# NEUE EUROPÄISCHE PATENTSCHRIFT

(11) **EP 2 473 486 B2**
(45) Veröffentlichungstag und Bekanntmachung des Hinweises auf die Entscheidung über den Einspruch: **22.09.2021**
(45) Hinweis auf die Patenterteilung: 28.10.2015
(21) Anmeldenummer: 10747859.6
(22) Anmeldetag: 31.08.2010
(51) Int. Cl.: C07D 239/47, C07D 251/18, C07D 251/66, C07D 401/04, C07D 401/14, C07D 403/04, A61K 31/53, A61K 31/506, A61K 31/5377, A61P 7/06

(54) **PYRIMIDINE ALS HEPCIDIN-ANTAGONISTEN**
PYRIMIDINES AS HEPCIDINE ANTAGONISTS
PYRIMIDINES EN TANT QU'ANTAGONISTES DE L'HEPCIDINE

(30) Priorität: 02.09.2009 EP 09169286
(43) Veröffentlichungstag der Anmeldung: 11.07.2012
(73) Patentinhaber: VIFOR (INTERNATIONAL) AG, 9001 St. Gallen (CH)
(72) Erfinder: DÜRRENBERGER, Franz, CH-4143 Dornach (CH); BURCKHARDT, Susanna, CH-8049 Zürich (CH); GEISSER, Peter, Otto, CH-9014 St. Gallen (CH); BUHR, Wilm, 78465 Konstanz (DE); FUNK, Felix, CH-8404 Winterthur (CH); BAINBRIDGE, Julia, Marie, Didcot Oxon Oxfordshire OX11 7FW (GB); CORDEN, Vincent, Anthony, Stanford in the Vale Oxon SN7 8PG (GB); COURTNEY, Stephen, Martin, Stanford in the Vale SN7 8FF (GB); DAVENPORT, Tara, Oxon OX13 5AA (GB); JAEGER, Stefan, 20251 Hamburg (DE); RIDGILL, Mark Peter, Horsham West Sussex RH12 4DB (GB); SLACK, Mark, 22527 Hamburg (DE); YARNOLD, Christopher, John, Didcot Oxon Oxfordshire OX11 8ST (GB); YAU, Wei, Tsung, Didcot Oxon Oxfordshire OX11 7FE (GB)
(74) Vertreter: Gille Hrabal Partnerschaftsgesellschaft mbB Patentanwälte
(86) Internationale Anmeldenummer: PCT/EP2010/062708
(87) Internationale Veröffentlichungsnummer: WO 2011/026835

(56) Entgegenhaltungen:
- EP-A1- 0 629 622
- WO-A1-00/39101
- WO-A1-03/091223
- WO-A1-2004/024159
- WO-A1-2004/046133
- WO-A1-2005/099711
- WO-A1-2006/029850
- WO-A1-2006/069258
- WO-A1-2006/116301
- WO-A1-2008/118455
- WO-A1-2008/146914
- WO-A1-2009/012421
- WO-A1-2009/029735
- WO-A1-2009/099193
- WO-A1-2009/117269
- WO-A1-2009/117269
- WO-A1-2011/026835
- WO-A2-02/102313
- WO-A2-2004/056786
- WO-A2-2008/003766
- WO-A2-2008/040778
- WO-A2-2009/064388
- WO-A2-2009/064388
- DE-A1-102007 010 801
- US-A1- 2006 199 804
- US-A1- 2006 293 343
- US-A1- 2008 194 577
- US-A1- 2008 260 736
- DATABASE CHEMCATS CHEMICAL ABSTRACT SERVICES, COLUMBUS, OHIO, US; 29. Juni 2009 (2009-06-29), XP002563315 gefunden im STN Database accession no. A3028/0127703, A2466/0104694 (ON) & "Chemical Block Stock Library" 29. Juni 2009 (2009-06-29), Chemical Block Ltd , Moscow, Russia
- DATABASE CHEMCATS CHEMICAL ABSTRACTS SERVICE, COLUMBUS, OHIO, US; 23. Januar 2009 (2009-01-23), XP002563316 gefunden im STN Database accession no. BAS00175970,BAS00125675, BAS00125664 (ON) & "ASINEX Express Gold Collection" 23. Januar 2009 (2009-01-23), ASINEX Ltd , Moscow, Russia
- DATABASE CHEMCATS CHEMICAL ABSTRACTS SERVICE, COLUMBUS, OHIO, US; 20. August 2009 (2009-08-20), XP002563317 gefunden im STN Database accession no. kasi-174891 (ON) & "Aurora Screening Library" 20. August 2009 (2009-08-20), Aurora Fine Chemicals LLC , san Diego, US
- DATABASE CHEMCAT CHEMICAL ABSTRACTS SERVICE, COLUMBUS, OHIO, US; 9. Februar 2009 (2009-02-09), XP002564573 Database accession no. KCD-024022 (on) & "Aurora Screening Library" 9. Februar 2009 (2009-02-09), Aurora Fine Chemicals LLC , San Diego, US
- MATSUNO ET AL: "Synthesis and aromatase-inhibitory activity of imidazolyl-1,3,5-triazine derivatives" CHEMICAL AND PHARMACEUTICAL BULLETIN, PHARMACEUTICAL SOCIETY OF JAPAN, TOKYO, JP, Bd. 45, Nr. 1, 1. Februar 1997 (1997-02-01), Seiten 291-296, XP002100044 ISSN: 0009-2363
- MUSONDA C C ET AL: "Synthesis and evaluation of 2-pyridyl pyrimidines with in vitro antiplasmodial and antileishmanial activity" BIOORGANIC & MEDICINAL CHEMISTRY LETTERS, PERGAMON, ELSEVIER SCIENCE, GB, Bd. 19, Nr. 2, 15. Januar 2009 (2009-01-15), Seiten 401-405, XP025816953 ISSN: 0960-894X [gefunden am 2008-12-03]
- ATANASIU VALERIU ET AL: "Hepcidin - central regulator of iron metabolism" EUROPEAN JOURNAL OF HAEMATOLOGY, Bd. 78, Nr. 1, Januar 2007 (2007-01), Seiten 1-10, XP002563305 ISSN: 0902-4441
- Derwent Abstract of D22

## Beschreibung

### EINLEITUNG:

Die Erfindung betrifft die neue Verwendung von Hepcidin-Antagonisten der allgemeinen Formel (I'), sowie diese umfassende pharmazeutischen Zusammensetzungen, zur Behandlung von Eisenmangelerkrankungen und/oder Eisenmangelanämien, insbesondere von Anämien im Zusammenhang mit chronischen Entzündungserkrankungen (anemia of chronic disease (ACD) und anemia of inflammation (Al)) oder von Eisenmangelerscheinungen.

### HINTERGRUND:

Eisen ist ein essentielles Spurenelement für fast alle Lebewesen und ist dabei insbesondere für das Wachstum und die Blutbildung relevant. Die Balance des Eisenhaushaltes wird dabei primär auf dem Level der Eisenwiedergewinnung aus Hämoglobin von alternden Erythrozyten und der duodenalen Absorption von nahrungsgebundenem Eisen reguliert. Das freigesetzte Eisen wird über den Darm insbesondere durch spezifische Transportsysteme (DMT-1, Ferroportin, Transferrin, Transferrin Rezeptoren) aufgenommen, in den Blutkreislauf transportiert und hierüber in die entsprechenden Gewebe und Organe weiter geleitet.

Das Element Eisen ist im menschlichen Körper unter anderem für den Sauerstofftransport, die Sauerstoffaufnahme, Zellfunktionen wie den mitochondrialen Elektronentransport und letztlich für den gesamten Energiestoffwechsel von großer Bedeutung.

Der Körper eines Menschen enthält im Durchschnitt 4 bis 5 g Eisen, wobei dies in Enzymen, in Hämoglobin und Myoglobin, sowie als Depot- oder Reserve-Eisen in Form von Ferritin und Hämosiderin vorliegt.
Etwa die Hälfte dieses Eisens ca. 2 g liegt als Häm Eisen gebunden im Hämoglobin der roten Blutkörperchen vor. Da diese Erythrozyten nur eine begrenzte Lebensdauer aufweisen (75-150 Tage), müssen ständig neue gebildet und alte eliminiert werden (über 2 Mio. Erythrozyten werden pro Sekunde neu gebildet). Diese hohe Neubildungskapazität wird durch Makrophagen erreicht, indem diese die alternden Erythrozyten phagozytotisch aufnehmen, lysieren und so das enthaltene Eisen für den Eisenhaushalt rezyklieren können. Somit wird die täglich für die Erythropoese benötige Eisenmenge von ca. 25 mg grösstenteils bereit gestellt.

Der tägliche Eisenbedarf eines erwachsenen Menschen beträgt zwischen 0,5 und 1,5 mg pro Tag, bei Kleinkindern sowie bei Frauen in der Schwangerschaft liegt der Eisenbedarf bei 2 bis 5 mg pro Tag. Die täglichen Eisenverluste, z.B. durch Abschilferung von Haut- und Epithelzellen ist vergleichsweise gering, erhöhte Eisenverluste treten beispielsweise bei der Menstruationsblutung bei Frauen auf. Generell können Blutverluste den Eisenhaushalt beträchtlich verringern, da pro 2 ml Blut etwa 1 mg Eisen verloren gehen. Der normale tägliche Eisenverlust von ca. 1 mg wird üblicherweise bei einem erwachsenen, gesunden Menschen über die tägliche Nahrungsaufnahme wieder ersetzt. Der Eisenhaushalt wird über Resorption reguliert, wobei die Resorptionsquote des in der Nahrung vorhandenen Eisens zwischen 6 und 12 % beträgt, bei Eisenmangel beträgt die Resorptionsquote bis zu 25 %. Die Resorptionsquote wird vom Organismus in Abhängigkeit vom Eisenbedarf und der Eisenspeichergröße reguliert. Dabei nutzt der menschliche Organismus sowohl zweiwertige als auch dreiwertige Eisenionen. Üblicherweise werden Eisen(III)-Verbindungen bei ausreichend saurem pH-Wert im Magen gelöst und damit für die Resorption verfügbar gemacht. Die Resorption des Eisens findet im oberen Dünndarm durch Mukosazellen statt. Dabei wird dreiwertiges nicht Häm Eisen für die Resorption z.B. durch Ferrireduktase (membranständiges, duodenales Cytochrom b) in der Darmzellmembran zunächst zu Fe²⁺ reduziert, um dann durch das Transportprotein DMT1 (divalent metal transporter 1) in die Darmzellen transportiert werden zu können. Dagegen gelangt Häm Eisen unverändert über die Zellmembran in die Enterozyten. In den Enterozyten wird Eisen entweder als Depot-Eisen in Ferritin gespeichert oder durch das Transportprotein Ferroportin, an Transferrin gebunden, ins Blut abgegeben. In diesem Vorgang spielt Hepcidin eine zentrale Rolle, da es den wesentlichen Regulationsfaktor der Eisenaufnahme darstellt. Das durch das Ferroportin ins Blut transportierte zweiwertige Eisen wird durch Oxidasen (Ceruloplasmin, Hephaestin) in dreiwertiges Eisen überführt, welches dann mittels Transferrin an die relevanten Stellen im Organismus transportiert wird (s. zum Beispiel: "Balancing acts: molecular control of mammalian iron metabolism". M.W. Hentze, Cell 117,2004,285-297.)

Die Regulation des Eisenspiegels wird dabei durch Hepcidin gesteuert bzw. reguliert.

Hepcidin ist ein Peptidhormon, welches in der Leber produziert wird. Die vorherrschende aktive Form besitzt 25 Aminosäuren (s. zum Beispiel: "Hepcidin, a key regulator of iron metabolism and mediator of anemia of inflammation".T. Ganz Blood 102,2003,783-8), obwohl auch zwei am Aminoende verkürzte Formen, Hepcidin-22 und Hepcidin-20, gefunden wurden. Hepcidin wirkt auf die Eisenaufnahme über den Darm, über die Plazenta sowie auf die Freisetzung von Eisen aus dem retikuloendotelialen System. Im Körper wird Hepcidin aus dem sogenannten Pro-Hepcidin in der Leber synthetisiert, wobei Pro-Hepcidin durch das sogenannte HAMP-Gen kodiert wird. Ist der Organismus ausreichend mit Eisen und Sauerstoff versorgt, so wird Hepcidin vermehrt gebildet. Hepcidin bindet in den Dünndarm-Mukosazellen und in den Makrophagen an Ferroportin, durch das üblicherweise Eisen aus dem Zellinneren ins Blut transportiert wird.

Bei dem Transportprotein Ferroportin handelt es sich um ein aus 571 Aminosäuren bestehendes Membrantransport-Protein, das in Leber, Milz, Nieren, Herz, Darm und Plazenta gebildet wird und lokalisiert ist. Insbesondere ist Ferroportin dabei in der basolateralen Membran von Darmepithelzellen lokalisiert. Das so gebundene Ferroportin bewirkt hierbei den Eisenexport in das Blut. Dabei transportiert Ferroportin Eisen höchstwahrscheinlich als Fe²⁺. Bindet Hepcidin an Ferroportin, wird Ferroportin in das Zellinnere transportiert und abgebaut, wodurch die Eisenabgabe aus den Zellen dann fast vollständig blockiert ist. Ist das Ferroportin über Hepcidin inaktiviert und kann somit das in den Mukosazellen gespeicherte Eisen nicht abtransportieren, geht das Eisen mit der natürlichen Zellabschilferung über den Stuhl verloren. Dadurch wird die Aufnahme von Eisen im Darm durch Hepcidin reduziert. Ist der Eisengehalt im Serum hingegen erniedrigt, so wird in den Hepatocyten der Leber die Hepcidin-Produktion reduziert, so dass weniger Hepcidin freigesetzt und somit weniger Ferroportin inaktiviert wird, wodurch eine erhöhte Eisenmenge ins Serum transportiert werden kann.

Außerdem ist Ferroportin stark im Retikuloendothelialen System (RES), zu dem auch die Makrophagen gehören, lokalisiert.
Hepcidin spielt hier eine wichtige Rolle bei gestörtem Eisenstoffwechsel im Rahmen chronischer Entzündungen, da bei solchen Entzündungen insbesondere Interleukin-6 erhöht ist, was zu einer Erhöhung des Hepcidin-Spiegels führt. Hierdurch wird vermehrt Hepcidn an das Ferroportin der Makrophagen gebunden, wodurch es hier zu einer Blockierung der Eisenfreisetzung kommt, die letztlich dann zu einer entzündungsbedingten Anämie (ACD oder Al) führt.

Da der Organismus von Säugetieren Eisen nicht aktiv ausscheiden kann, wird der Eisenmetabolismus im Wesentlichen über die zelluläre Freisetzung von Eisen aus Makrophagen, Hepatocyten und Enterozyten durch das Hepcidin gesteuert.

Hepcidin spielt somit eine wichtige Rolle bei der funktionellen Anämie. In diesem Fall wird trotz gefüllter Eisenspeicher der Eisenbedarf des Knochenmarks für die Erythropoese nicht ausreichend erfüllt. Als Grund hierfür wird eine erhöhte Hepcidinkonzentration angenommen, die insbesondere durch Blockierung des Ferroportins den Eisen Transport aus den Makrophagen einschränkt und somit die Freisetzung von phagozytotisch rezyklisiertem Eisen stark vermindert.

Bei einer Störung des Hepcidin-Regulationsmechanismus zeigt sich somit eine direkte Auswirkung auf den Eisenmetabolismus im Organismus. Wird beispielsweise die Hepcidin-Expression verhindert, beispielsweise durch einen genetischen Defekt, so führt dies unmittelbar zu einer Überladung an Eisen, was unter der Eisenspeicherkrankheit Hämochromatose bekannt ist.

Dahingegen resultiert eine Hepcidin-Überexpression, beispielsweise aufgrund von Entzündungsprozessen, beispielsweise bei chronischen Entzündungen, unmittelbar in verringerten Serum-Eisenspiegeln. Diese können in krankhaften Fällen zu verringertem Gehalt an Hämoglobin, verringerter Erythrozytenproduktion und damit zu einer Anämie führen.

Die Einsatzdauer von Chemotherapeutika bei Karzinombehandlungen kann durch eine bestehende Anämie deutlich verringert werden, da der Zustand der reduzierten Bildung von roten Blutkörperchen, hervorgerufen durch die eingesetzten Chemotherapeutika, durch eine bestehende Anämie noch weiter verstärkt wird.

Weitere Symptome von Anämien beinhalten Müdigkeit, Blässe sowie verringerte Aufmerksamkeitskapazitäten. Die klinischen Symptome einer Anämie beinhalten niedrige Serum-Eisengehalte (Hypoferrämie), geringe Hämoglobingehalte, geringe Hämatokritlevel sowie eine reduzierte Anzahl an roten Blutkörperchen, reduzierte Retikulozyten, erhöhte Werte an löslichen Transferrinrezeptoren.

Klassischerweise werden Eisenmangelerscheinungen oder Eisenanämien durch Eisenzufuhr behandelt. Dabei erfolgt die Substitution mit Eisen entweder auf dem oralen Weg oder durch intravenöse Eisengabe. Außerdem können zur Ankurbelung der Bildung von roten Blutkörperchen auch Erythropoetin und andere Erythropoese-stimulierende Substanzen bei der Behandlung von Anämien eingesetzt werden.

Anämien, die durch chronische Erkrankungen, z.B. chronische Entzündungserkrankungen verursacht werden, können nur unzureichend mit solchen klassischen Behandlungsmethoden behandelt werden. Bei Anämien, die auf chronischen Entzündungsprozessen basieren, spielen insbesondere Cytokine, wie insbesondere inflammatorische Cytokine, eine besondere Rolle. Eine Hepcidin-Überexpression tritt insbesondere bei solchen chronischen Entzündungserkrankungen auf und führt bekannterweise zu verringerter Eisenverfügbarkeit für die Bildung der roten Blutkörperchen.

Daraus ergibt sich die Notwendigkeit für eine wirksame Behandlungsmethode von Hepcidin-mediierten bzw. -vermittelten Anämien, insbesondere solcher, die nicht mit klassischen Eisensubstitutionen behandelt werden können wie solche Anämien, die durch chronische Entzündungserkrankungen (ACD und Al) hervorgerufen werden.

Anämie ist unter anderem zurückzuführen auf solche genannten chronischen Entzündungserkrankungen, sowie auf Mangelernährung bzw. eisenarme Diäten oder unausgewogene, eisenarme Ernährungsgewohnheiten, Außerdem treten Anämien durch verringerte bzw. schlechte Eisenabsorption beispielsweise aufgrund von Gastrektomien oder von Erkrankungen wie Crohn's-Disease auf. Auch kann ein Eisenmangel infolge eines erhöhten Blutverlustes z.B. durch eine Verletzung, starke Menstruationsblutung oder Blutspende auftreten. Auch ist ein erhöhter Eisenbedarf in der Wachstumsphase Heranwachsender und Kinder sowie von Schwangeren bekannt. Da ein Eisenmangel nicht nur zu einer verringerten Bildung von roten Blutkörperchen, sondern damit auch zu einer schlechten Versorgung des Organismus mit Sauerstoff führt, was zu den oben genannten Symptomen wie Müdigkeit, Blässe und Konzentrationsschwäche auch gerade bei Heranwachsenden zu langfristigen negativen Auswirkungen auf die kognitive Entwicklung führen kann, ist auch für diesen Bereich eine besonders wirksame Therapie neben den bekannten klassischen Substitutionstherapien von besonderem Interesse.

Verbindungen, die an Hepcidin oder an Ferroportin binden, und damit die Bindung von Hepcidin an Ferroportin inhibieren, und damit wiederum die Inaktivierung des Ferroportins durch das Hepcidin verhindern, oder Verbindungen, die obwohl Hepcidin an Ferroportin gebunden ist die Internalisierung des Hepcidin-Ferroportin Komplexes verhindern, und auf diese Weise die Inaktivierung des Ferroportins durch das Hepcidin verhindern, können allgemein als Hepcidin-Antagonisten bezeichnet werden.

Durch Verwendung solcher Hepcidin-Antagonisten besteht außerdem auch generell die Möglichkeit, beispielsweise durch Inhibierung der Hepcidin-Expression oder durch Blockierung der Hepcidin-Ferroportin-Interaktion auf den Regulationsmechanismus des Hepcidins direkt einzuwirken, und damit über diesen Weg eine Blockierung des Eisentransportweges aus Gewebemakrophagen, Leberzellen und Mukosazellen in das Serum über das Transportprotein Ferroportin zu verhindern. Damit stehen mit solchen Hepcidin-Antagonisten bzw. Hepcidin-Expressionsinhibitoren Substanzen zur Verfügung, die geeignet zur Herstellung von pharmazeutischen Zusammensetzungen oder Medikamenten in der Behandlung von Anämien, insbesondere Anämien bei chronischen Entzündungskrankeiten, sind. Diese Substanzen können zur Behandlung solcher Störungen und der daraus resultierenden Erkrankungen eingesetzt werden, da diese direkt Einfluss auf die Erhöhung der Freisetzung von rezykliertem Häm-Eisen durch Makrophagen haben, sowie eine Erhöhung der Eisenabsorption des aus der Nahrung freigesetzten Eisens im Intestinaltrakt bewirken. Damit werden solche Substanzen, Hepcidin-Expressionsinhibitoren bzw. Hepcidin-Antagonisten, für die Behandlung von Eisenmetabolismus-Störungen wie Eisenmangel-Erkrankungen, Anämien und Anämie-verwandten Erkrankungen verwendbar. Insbesondere umfasst dies auch solche Anämien, die durch akute oder chronische Entzündungserkrankungen hervorgerufen sind wie beispielsweise osteoartikuläre Erkrankungen wie rheumatoide Polyarthritis oder Erkrankungen, die mit inflammatorischen Syndromen assoziiert sind. Somit können solche Substanzen insbesondere in den Indikationen Krebs insbesondere kolorektal Krebs, multiples Myelom, ovarial und endometrial Krebs und Prostata-Krebs, CKD 3-5(chronic kidney disease stage 3-5), CHF(Chronic heart failure), RA (Rheumatoid arthritis), SLE (systemic lupus erythomatosus) und IBD (inflammatory bowel diseases) von besonderem Nutzen sein.

### STAND DER TECHNIK:

Aus dem Stand der Technik sind Hepcidin-Antagonisten oder Verbindungen, die auf die biochemischen Regulationswege im Eisenmetabolismus inhibierend oder unterstützend einwirken, grundsätzlich bekannt.

So beschreibt beispielsweise die WO 2008/036933 doppelsträngige dsRNA, die inhibierend auf die Expression von humanen HAMP-Genen in Zellen wirkt, und damit im Eisenmetabolismus-Signalweg bereits auf einer sehr frühen Stufe die Bildung von Hepcidin, das durch das HAMP-Gen kodiert wird, unterdrückt. Dadurch wird weniger Hepcidin gebildet, so dass Hepcidin zur Inhibierung von Ferroportin nicht zur Verfügung steht, so dass der Transport von Eisen aus der Zelle in das Blut durch Ferroportin ungehindert erfolgen kann.

Weitere Verbindungen, die direkt auf die Verringerung der Hepcidin-Expression abzielen, sind bekannt aus der US 2005/020487, worin Verbindungen beschrieben sind, die HIF-α stabilisierend wirken und damit zu einer Verringerung der Hepcidin-Expression führen.

Die US 2007/004618 hat siRNA zum Gegenstand, welche unmittelbar inhibierend auf die Hepcidin-mRNA-Expression einwirkt.

Somit handelt es sich bei allen diesen Verbindungen bzw. Verfahren um solche, die im Eisenmetabolismusweg vor Bildung des Hepcidin ansetzen und dessen generelle Bildung bereits herunterregulieren. Daneben sind jedoch auch solche Substanzen und Verbindungen bekannt und im Stand der Technik beschrieben, die an bereits gebildetes Hepcidin im Körper binden und damit dessen Bindungswirkung an das Membrantransportprotein Ferroportin inhibieren, so dass eine Inaktivierung des Ferroportins durch das Hepcidin nicht mehr möglich ist. Somit handelt es sich bei solchen Verbindungen um sogenannte Hepcidin-Antagonisten, wobei aus dieser Gruppe insbesondere solche auf Basis von Hepcidin-Antikörpern bekannt sind. Weiter sind auch solche Dokumente im Stand der Technik bekannt, die verschiedene Mechanismen zur Wirkung auf die Hepcidin-Expression beschreiben, beispielsweise durch Antisense-RNA oder DNA-Moleküle, Ribozyme sowie Anti-Hepcidin-Antikörper. Solche sind beispielsweise beschrieben in der EP 1 392 345.

Aus der WO09/058797 sind des weiteren Anti-Hepcidin-Antikörper und deren Verwendung zur spezifischen Bindung an humanes Hepcidin-25 bekannt und damit deren Verwendung zur therapeutischen Behandlung niedriger Eisengehalte, insbesondere von Anämien.

Weitere Verbindungen, die als Hepcidin-Antagonisten wirken und aus der Gruppe der Hepcidin-Antikörper ausgebildet sind, sind bekannt aus der EP 1 578 254, der WO08/097461, der US2006/019339, der WO09/044284 oder der WO09/027752.

Daneben sind auch Antikörper bekannt, die an Ferroportin-1 binden und damit Ferroportin aktivieren, um darüber den Eisentransport aus der Zelle in das Serum zu unterstützen. Solche Ferroportin-1-Antikörper sind beispielsweise bekannt aus der US2007/218055.

Bei allen diesen beschriebenen Verbindungen, die als Hepcidin-Antagonisten wirken oder in der Hepcidin-Expression eine inhibierende Wirkung entfalten können, handelt es sich um höhermolekulare Verbindungen, insbesondere um solche, die hauptsächlich über gentechnologische Verfahren erhältlich sind.

Daneben sind auch niedermolekulare Verbindungen, die eine Rolle im Eisenmetabolismus spielen, und die sowohl inhibierend oder auch unterstützend einwirken können, bekannt.

So beschreibt die WO08/109840 bestimmte tricyclische Verbindungen, die insbesondere zur Behandlung von Störungen des Eisenmetabolismus wie beispielsweise Ferroportinstörungen eingesetzt werden können, wobei diese Verbindungen durch Regulation von DMT-1 in Form von Inhibition oder Aktivierung wirken können. Dabei werden die Verbindungen dieser WO08/109840 insbesondere als DMT-1-Inhibitoren beschrieben, womit sie vorzugsweise bei Erkrankungen mit erhöhter Eisenakkumulation bzw. Eisenspeichererkrankungen wie Hämochromatose einsetzbar sind.

Auch aus der WO08/121861 sind niedermolekulare Verbindungen bekannt, die regulierend auf den DMT-1-Mechanismus wirken. Hierbei werden insbesondere bestimmte Pyrazol- und Pyrrol-Verbindungen behandelt, wobei auch hier insbesondere die Behandlung von Eisenüberladungsstörungen beispielsweise auf Grund von Ferroportinstörungen beschrieben wird.

Des weiteren sind Gegenstand der US2008/234384 bestimmte Diaryl- und Diheteroaryl-Verbindungen zur Behandlung von Störungen des Eisenmetabolismus wie beispielsweise Ferroportinstörungen, die ebenfalls durch Wirkung als DMT-1-Inhibitoren insbesondere zur Behandlung von Störungen aufgrund erhöhter Eisenakkumulation einsetzbar sind. In diesem Dokument werden jedoch ganz generell auch mögliche DMT-1-regulatorische Mechanismen erwähnt, die zum Einsatz bei Eisenmangelerscheinungen Verwendung finden können.

Das gleiche gilt für die WO08/151288, worin bestimmte aromatische und heteroaromatische Verbindungen mit Wirkung auf die DMT-1-Regulation und damit zur Behandlung von Störungen des Eisenmetabolismus beschrieben werden.

Somit sind die im Stand der Technik beschriebenen niedermolekularen Verbindungen, die auf den Eisenmetabolismus wirken, auf DMT-1-regulatorische Mechanismen bezogen und insbesondere zur Verwendung als Mittel zur Behandlung von Eisen-Akkumulationsstörungen bzw. Eisenüberladungs-Syndromen wie Hämochromatose offenbart.

"Hepcidin - Central-regulator of iron-metabolism" (Atanasiu Valeriu et al., European Journal of Haematolgy, 78 (1), 2007) gibt einen Überblick über Hepcidin und seine Funktion.

Aus DATABASE CHEMCATS CHEMICAL ABSTRACT SERVICES, COLUMBUS, OHIO, US; 29 June 2009 (2009-06-29), XPO02563315, SIN Database accession no. A3028/0127703, A2466/0104694 (ON); & "Chemical Block Stock Library" 29 June 2009 (2009-06-29), Chemical Block Ltd, Moscow, Russia ist eine Verbindung der Formel 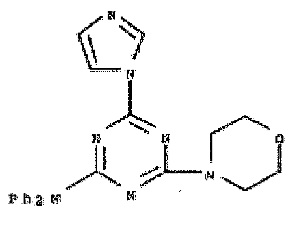 bekannt.

Aus DATABASE CHEMCATS CHEMICAL ABSTRACTS SERVICE, COLUMBUS, OHIO, US; 23 January 2009 (2009-01-23), XP002563316, SIN Database accession no. BAS00175970, BAS00125675, BAS00125664 (ON); s "ASINEX Express Gold Collection" 23 January 2009 (2009-01-23), ASINEX Ltd , Moscow, Russia ist eine Verbindung der Formel 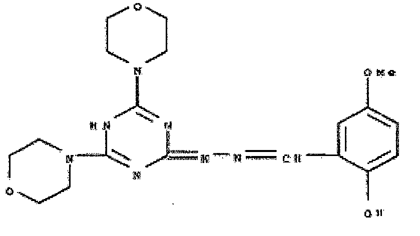 bekannt.

Aus DATABASE CHEMCATS CHEMICAL ABSTRACTS SERVICE, COLUMBUS, OHIO, US; 20 August 2009 (2009-08-20), XP002563317, SIN Database accession no. kas-174891 (ON); & "Aurora Screening Library" 20 August 2009 (2009-08-20), Aurora Fine Chemicals LLC , San Diego, US ist eine Verbindung der Formel 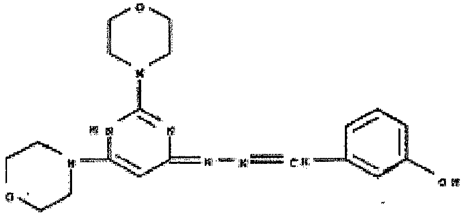 bekannt.

Aus DATABASE CHEMCAT CHEMICAL ABSTRACTS SERVICE, COLUMBUS, OHIO, US; 9 February 2009 (2009-02-09), XP002564573 Database accession no. KCD-024022 (on) ; & "Aurora Screening Library" 9 February 2009 (2009-02-09), Aurora Fine Chemicals LLC , San Diego, US ist eine Verbindung der Formel 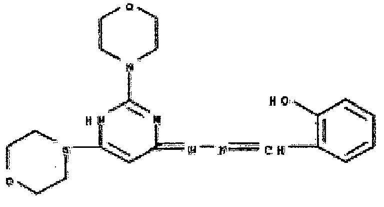 bekannt.

MATSUNO ET AL: "Synthesis and aromatase-inhibitory activity of imidazolyl-1,3,5-triazine derivatives" (CHEMICAL AND PHARMACEUTICAL BULLETIN, PHARMACEUTICAL SOCIETY OF JAPAN, TOKYO, JP, VOI. 45, Nr. 1, 1. Februar 1997 (1997-02-01), Seiten 291-296, XP002100044 ISSN: 0009-2363) beschreiben Untersuchungen zur Aromatase-inhibierenden Aktivität von Imidazolyl-1,3,5-triazin Derivaten.

MUSONDA C. C. ET AL: "Synthesis and evaluation of 2-pyridyl pyrimidines with in vitro antiplasmodial and antileishmanial activity" (BIOORGANIC & MEDICINAL CHEMISTRY LETTERS, PERGAMON, ELSEVIER SCIENCE, GB, Vol. 19, Nr. 2, 15. Januar 2009 (2009-01-15), Seiten 401-405, XP025816953 ISSN: 0960-894X) beschreiben 2-Pyridylpyrimidine mit in vitro Aktivität gegen Plasmodien und Parasiten der Gattung *Leishmania.*

Die EP 0 629 622 A1 offenbart S-Triazin Derivate und deren Verwendung zur Behandlung Östrogen-abhängiger Erkrankungen.

Die WO 2009/064388 A2 offenbart Pyrimidin-pyridin Verbindungen und deren Verwendung als Inhibitoren der humanen Methioninaminopeptidase.

Die WO 2006/116301 A1 offenbart Verbindungen der allgemeinen Formel (I) 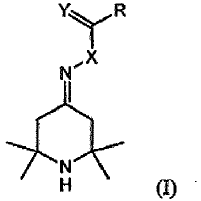 und deren Verwendung als neuroprotective Substanzen zur Behandlung optischer Neuropathien.

Die US 2006/199804 A1 offenbart Verbindungen mit Wirkung als mTOR Inhibitoren.

Die WO 2006/029850 A1 offenbart Hydrazonderivate und deren Verwendung als beta-Sekretasehemmer.

Die nachveröffentlichte internationale Anmeldung WO 2009/117269 A1 offenbart substituierte 4-Hydroxypyrimidin-5-carboxamide der allgemeinen Formel I 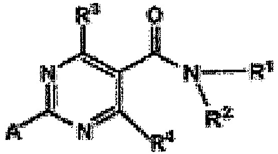 und deren Verwendung als HIF Prolylhydroxylase Inhibitoren zur Behandlung von Anämien.
Chemische Verbindungen auf struktureller Grundlage der Pyrimidine gemäß den Patentansprüchen wurden im Zusammenhang mit der Behandlung von Eisenmangelerkrankungen und/oder Eisenmangelanämien bislang noch nicht beschrieben. Außerdem wurden bisher noch keine niedermolekularen chemischen Strukturen, die ihre Wirkung als Hepcidin-Antagonisten entfalten und hierdurch zur Behandlung von Eisenmangelerkrankungen und/oder Eisenmangelanämien geeignet sind, beschrieben.

### AUFGABENSTELLUNG:

Die Aufgabe der vorliegenden Erfindung bestand darin, insbesondere solche Verbindungen bereitzustellen, die zur Verwendung von Eisenmangelstörungen oder Anämien insbesondere ACD und AI eingesetzt werden können und die im Eisenmetabolismus insbesondere als Hepcidin-Antagonisten wirken, und damit im Eisenmetabolismus in der Hepcidin-Ferroportin-Interaktion eine antagonistische und darüber regulierende Wirkung entfalten. Weiterhin war es insbesondere Aufgabe der vorliegenden Erfindung, dabei solche Verbindungen zur Verfügung zu stellen, die ausgewählt sind aus der Gruppe der niedermolekularen Verbindungen und die generell durch einfachere Synthesewege herstellbar sind als die durch gentechnologische Verfahren erhältlichen antagonistischen bzw. Hepcidininhibierenden Verbindungen wie RNA, DNA oder Antikörper.

### BESCHREIBUNG DER ERFINDUNG:

Die Erfinder fanden, dass bestimmte Verbindungen aus der Gruppe der Pyrimidine und Triazine eine Wirkung als Hepcidin-Antagonisten aufweisen.

Gegenstand der Erfindung sind die anspruchsgemäßen Verbindungen, die unter die allgemeine Strukturformel (I) fallen 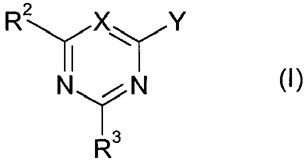 worin
X ausgewählt ist aus der Gruppe, die aus N oder C-R¹ besteht, worin
R¹ ausgewählt wird aus der Gruppe, die besteht aus:
   - Wasserstoff,
   - Hydroxyl,
   - Halogen,
   - Carboxyl,
   - Sulfonsäurerest (-SO₃H),
   - gegebenenfalls substituiertem Aminocarbonyl,
   - gegebenenfalls substituiertem Aminosulfonyl,
   - gegebenenfalls substituiertem Amino,
   - gegebenenfalls substituiertem Alkyl,
   - gegebenenfalls substituiertem Acyl,
   - gegebenenfalls substituiertem Alkoxycarbonyl,
   - gegebenenfalls substituiertem Acyloxy,
   - gegebenenfalls substituiertem Alkoxy
   - gegebenenfalls substituiertem Alkenyl,
   - gegebenenfalls substituiertem Alkinyl,
   - gegebenenfalls substituiertem Aryl,
   - gegebenenfalls substituiertem Heterocyclyl;
R² und R³ gleich oder verschieden sind, und jeweils ausgewählt werden aus der Gruppe, die besteht aus:
   - Wasserstoff,
   - Hydroxyl,
   - Halogen,
   - Carboxyl,
   - Sulfonsäurerest (-SO₃H),
   - gegebenenfalls substituiertem Aminocarbonyl,
   - gegebenenfalls substituiertem Aminosulfonyl,
   - gegebenenfalls substituiertem Amino,
   - gegebenenfalls substituiertem Alkyl,
   - gegebenenfalls substituiertem Acyl,
   - gegebenenfalls substituiertem Alkoxycarbonyl,
   - gegebenenfalls substituiertem Acyloxy,
   - gegebenenfalls substituiertem Alkoxy,
   - gegebenenfalls substituiertem Alkenyl,
   - gegebenenfalls substituiertem Alkinyl,
   - gegebenenfalls substituiertem Aryl,
   - gegebenenfalls substituiertem Heterocyclyl;
Y ausgewählt wird aus der Gruppe die besteht aus:
   - Wasserstoff
   - Hydroxyl,
   - Halogen, bevorzugt Chlor,
   - gegebenenfalls substituiertem Aryloxy, bevorzugt Phenoxy, und 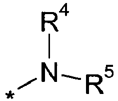
   - (* kennzeichnet hier und im Folgenden die Bindungsstelle des Restes)
      worin
      R⁴ und R⁵ gleich oder verschieden sind, und jeweils ausgewählt werden aus der Gruppe, die besteht aus:
      - Wasserstoff,
   - gegebenenfalls substituiertem Amino,
   - gegebenenfalls substituiertem Aminocarbonyl,
   - gegebenenfalls substituiertem Alkyl-, Aryl oder Heterocyclylsulfonyl,
   - gegebenenfalls substituiertem Alkyl,
   - gegebenenfalls substituiertem Alkenyl,
   - gegebenenfalls substituiertem Alkinyl,
   - gegebenenfalls substituiertem Acyl,
   - gegebenenfalls substituiertem Aryl,
   - gegebenenfalls substituiertem Heterocyclyl oder
   - worin R⁴ und R⁵ zusammen mit dem Stickstoffatom, an das sie gebunden sind, einen gesättigten oder ungesättigten, gegebenenfalls substituierten 3- bis 8-gliedrigen Ring ausbilden, der gegebenenfalls weitere Heteroatome enthalten kann;
   oder pharmazeutisch verträgliche Salze hiervon zur Verwendung in der Behandlung von Eisenmangelerkrankungen und/oder Eisenmangelanämien.

Gegenstand der Erfindung sind weiterhin und insbesondere die anspruchsgemäßen Verbindungen, die unter dieallgemeine Strukturformel (I') fallen 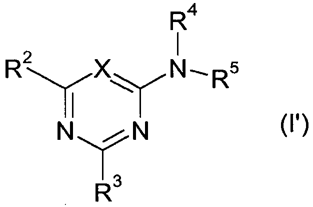 worin
X ausgewählt ist aus der Gruppe, die aus N oder C-R¹ besteht, worin
R¹ ausgewählt wird aus der Gruppe, die besteht aus:
   - Wasserstoff,
   - Hydroxyl,
   - Halogen,
   - Carboxyl,
   - Sulfonsäurerest (-SO₃H),
   - gegebenenfalls substituiertem Aminocarbonyl,
   - gegebenenfalls substituiertem Aminosulfonyl,
   - gegebenenfalls substituiertem Amino,
   - gegebenenfalls substituiertem Alkyl,
   - gegebenenfalls substituiertem Acyl,
   - gegebenenfalls substituiertem Alkoxycarbonyl,
   - gegebenenfalls substituiertem Acyloxy,
   - gegebenenfalls substituiertem Alkoxy
   - gegebenenfalls substituiertem Alkenyl,
   - gegebenenfalls substituiertem Alkinyl,
   - gegebenenfalls substituiertem Aryl,
   - gegebenenfalls substituiertem Heterocyclyl;
R² und R³ gleich oder verschieden sind, und jeweils ausgewählt werden aus der Gruppe, die besteht aus:
   - Wasserstoff,
   - Hydroxyl,
   - Halogen,
   - Carboxyl,
   - Sulfonsäurerest (-SO₃H),
   - gegebenenfalls substituiertem Aminocarbonyl,
   - gegebenenfalls substituiertem Aminosulfonyl,
   - gegebenenfalls substituiertem Amino,
   - gegebenenfalls substituiertem Alkyl,
   - gegebenenfalls substituiertem Acyl,
   - gegebenenfalls substituiertem Alkoxycarbonyl,
   - gegebenenfalls substituiertem Acyloxy,
   - gegebenenfalls substituiertem Alkoxy,
   - gegebenenfalls substituiertem Alkenyl,
   - gegebenenfalls substituiertem Alkinyl,
   - gegebenenfalls substituiertem Aryl,
   - gegebenenfalls substituiertem Heterocyclyl;
R⁴ und R⁵ gleich oder verschieden sind, und jeweils ausgewählt werden aus der Gruppe, die besteht aus:
   - Wasserstoff,
   - gegebenenfalls substituiertem Amino,
   - gegebenenfalls substituiertem Alkyl-, Aryl- oder Heterocyclylsulfonyl,
   - gegebenenfalls substituiertem Alkyl,
   - gegebenenfalls substituiertem Alkenyl,
   - gegebenenfalls substituiertem Alkinyl,
   - gegebenenfalls substituiertem Acyl,
   - gegebenenfalls substituiertem Aryl,
   - gegebenenfalls substituiertem Heterocyclyl oder
   - worin R⁴ und R⁵ zusammen mit dem Stickstoffatom, an das sie gebunden sind, einen gesättigten oder ungesättigten, gegebenenfalls substituierten 3- bis 8-gliedrigen Ring ausbilden, der gegebenenfalls weitere Heteroatome enthalten kann;
oder pharmazeutisch verträgliche Salze hiervon zur Verwendung in der Behandlung von Eisenmangelerkrankungen und/oder Eisenmangelanämien.

Im Rahmen der gesamten Erfindung werden die vorstehend genannten Substituentengruppen wie folgt definiert:
Gegebenenfalls substituiertes Alkyl schließt bevorzugt ein:
   Geradkettiges oder verzweigtes Alkyl mit bevorzugt 1 bis 8, bevorzugter 1 bis 6, besonders bevorzugt 1 bis 4 Kohlenstoffatomen. In einer Ausführungsform der Erfindung kann gegebenenfalls substituiertes geradkettiges oder verzweigtes Alkyl auch solche Alkylgruppen einschließen, in denen bevorzugt 1 bis 3 Kohlenstoffatom(e) durch entsprechende heteroanaloge Gruppen, die Stickstoff, Sauerstoff oder Schwefel enthalten, ersetzt sind. Dies bedeutet insbesondere, dass beispielsweise eine oder mehrere Methylengruppen in den genannten Alkylresten durch NH, O oder S ersetzt sein können.

Gegebenenfalls substituiertes Alkyl schließt weiterhin Cycloalkyl mit bevorzugt 3 bis 8, bevorzugter 5 oder 6, besonders bevorzugt 6 Kohlenstoffatomen ein.

Substituenten des zuvor definierten gegebenenfalls substituierten Alkyls schließen bevorzugt 1 bis 3 gleiche oder verschiedene Substituenten ein, die beispielsweise aus der Gruppe ausgewählt werden, die besteht aus: gegebenenfalls substituiertem Cycloalkyl, wie unten definiert, Hydroxy, Halogen, Cyano, Alkoxy, wie unten definiert, gegebenenfalls substituiertes Aryloxy, wie unten definiert, gegebenenfalls substituiertes Heterocyclyloxy, wie unten definiert, Carboxy, gegebenenfalls substituiertes Acyl, wie unten definiert, gegebenenfalls substituiertes Aryl, wie unten definiert, gegebenenfalls substituiertes Heterocyclyl, wie unten definiert, gegebenenfalls substituiertes Amino, wie unten definiert, Mercapto, gegebenenfalls substituiertes Alkyl-, Aryl- oder Heterocyclylsulfonyl (R-SO₂-), wie unten definiert.

Beispiele von Alkylresten mit 1 bis 8 Kohlenstoffatomen schließen ein: eine Methylgruppe, eine Ethylgruppe, eine n-Propylgruppe, eine i-Propylgruppe, eine n-Butylgruppe, eine i-Butylgruppe, eine sec-Butylgruppe, eine t-Butylgruppe, eine n-Pentylgruppe, eine i-Pentylgruppe, eine sec-Pentylgruppe, eine t-Pentylgruppe, eine 2-Methylbutylgruppe, eine n-Hexylgruppe, eine 1-Methylpentylgruppe, eine 2-Methylpentylgruppe, eine 3-Methylpentylgruppe, eine 4-Methylpentylgruppe, eine 1-Ethylbutylgruppe, eine 2-Ethylbutylgruppe, eine 3-Ethylbutylgruppe, eine 1,1-Dimethylbutylgruppe, eine 2,2-Dimethylbutylgruppe, eine 3,3-Dimethylbutylgruppe, eine 1-Ethyl-1-methylpropylgruppe, eine n-Heptylgruppe, eine 1-Methylhexylgruppe, eine 2-Methylhexylgruppe, eine 3-Methylhexylgruppe, eine 4-Methylhexylgruppe, eine 5-Methylhexylgruppe, eine 1-Ethylpentylgruppe, eine 2-Ethylpentylgruppe, eine 3-Ethylpentylgruppe, eine 4-Ethylpentylgruppe, eine 1,1-Dimethylpentylgruppe, eine 2,2-Dimethylpentylgruppe, eine 3,3-Dimethylpentylgruppe, eine 4,4-Dimethylpentylgruppe, eine 1-Propylbutylgruppe, eine n-Octylgruppe, eine 1-Methylheptylgruppe, eine 2-Methylheptylgruppe, eine 3-Methylheptylgruppe, eine 4-Methylheptylgruppe, eine 5-Methylheptylgruppe, eine 6-Methylheptylgruppe, eine 1-Ethylhexylgruppe, eine 2-Ethylhexylgruppe, eine 3-Ethylhexylgruppe, eine 4-Ethylhexylgruppe, eine 5-Ethylhexylgruppe, eine 1,1 -Dimethylhexylgruppe, eine 2,2-Dimethylhexylgruppe, eine 3,3-Dimethylhexylgruppe, eine 4,4-Dimethylhexylgruppe, eine 5,5-Dimethylhexylgruppe, eine 1-Propylpentylgruppe, eine 2-Propylpentylgruppe, usw. Bevorzugt sind solche mit 1 bis 6 Kohlenstoffatomen, insbesondere Methyl, Ethyl, n-Propyl und i-Propyl. Am meisten bevorzugt sind C₁-C₄ Alkyl, wie insbesondere Methyl, Ethyl und i-Propyl.

Beispiele von Alkylgruppen, die durch Austausch mit einer oder mehreren heteroanalogen Gruppe, wie -O-, -S- oder -NH- hervorgehen, sind bevorzugt solche, in denen eine oder mehrere Methylengruppen durch -O-unter Bildung einer oder mehrerer Ethergruppen ersetzt sind, wie Methoxymethyl, Ethoxymethyl, 2-Methoxyethyl usw. Erfindungsgemäß sind insbesondere auch Polyethergruppen, wie Poly(ethylenoxy)gruppen von der Definition von Alkyl umfasst.

Cycloalkylreste mit 3 bis 8 Kohlenstoffatomen schließen bevorzugt ein: eine Cyclopropylgruppe, eine Cyclobutylgruppe, eine Cyclopentylgruppe, eine Cyclohexylgruppe, eine Cycloheptylgruppe und eine Cyclooctylgruppe. Bevorzugt sind eine Cyclopropylgruppe, eine Cyclobutylgruppe, eine Cyclopentylgruppe und eine Cyclohexylgruppe. Besonders bevorzugt sind eine Cyclopentylgruppe und eine Cyclohexylgruppe.

Halogen schließt im Rahmen der vorliegenden Erfindung Fluor, Chlor, Brom und Iod, bevorzugt Fluor oder Chlor ein.

Beispiele eines mit Halogen substituierten linearen oder verzweigten Alkylrestes mit 1 bis 8 Kohlenstoffatomen ein:
eine Fluormethylgruppe, eine Difluormethylgruppe, eine Trifluormethylgruppe, eine Chlormethylgruppe, eine Dichlormethylgruppe,
eine Trichlormethylgruppe, eine Brommethylgruppe, eine Dibrommethylgruppe, eine Tribrommethylgruppe, eine 1-Fluorethylgruppe,
eine 1-Chlorethylgruppe, eine 1-Bromethylgruppe, eine 2-Fluorethylgruppe,
eine 2-Chlorethylgruppe, eine 2-Bromethylgruppe, eine 1,2-Difluorethylgruppe, eine 1,2-Dichlorethylgruppe, eine 1,2-Dibromethylgruppe, eine 2,2,2-Trifluorethylgruppe, eine Heptafluorethylgruppe, eine 1-Fluorpropylgruppe, eine 1-Chlorpropylgruppe,
eine 1-Brompropylgruppe, eine 2-Fluorpropylgruppe, eine 2-Chlorpropylgruppe, eine 2-Brompropylgruppe, eine 3-Fluorpropylgruppe,
eine 3-Chlorpropylgruppe, eine 3-Brompropylgruppe, eine 1,2-Difluorpropylgruppe, eine 1,2-Dichlorpropylgruppe, eine 1,2-Dibrompropylgruppe, eine 2,3-Difluorpropylgruppe, eine 2,3-Dichlorpropylgruppe, eine 2,3-Dibrompropylgruppe, eine 3,3,3-Trifluorpropylgruppe, eine 2,2,3,3,3-Pentafluorpropylgruppe, eine 2-Fluorbutylgruppe, eine 2-Chlorbutylgruppe, eine 2-Brombutylgruppe, eine 4-Fluorbutylgruppe, eine 4-Chlorbutylgruppe, eine 4-Brombutylgruppe, eine 4,4,4-Trifluorbutylgruppe, eine 2,2,3,3,4,4,4-Heptafluorbutylgruppe, eine Perfluorbutylgruppe, eine 2-Fluorpentylgruppe, eine 2-Chlorpentylgruppe,
eine 2-Brompentylgruppe, eine 5-Fluorpentylgruppe, eine 5-Chlorpentylgruppe, eine 5-Brompentylgruppe, eine Perfluorpentylgruppe,
eine 2-Fluorhexylgruppe, eine 2-Chlorhexylgruppe, eine 2-Bromhexylgruppe,
eine 6-Fluorhexylgruppe, eine 6-Chlorhexylgruppe, eine 6-Bromhexylgruppe,
eine Perfluorhexylgruppe, eine 2-Fluorheptylgruppe, eine 2-Chlorheptylgruppe, eine 2-Bromheptylgruppe, eine 7-Fluorheptylgruppe,
eine 7-Chlorheptylgruppe, eine 7-Bromheptylgruppe, eine Perfluorheptylgruppe, usw. Insbesondere sind Fluoroalkyl, Difluoroalkyl und Trifluoroalkyl, bevorzugt ist Trifluoromethyl zu nennen.

Beispiele eines mit Halogen substituierten Cycloalkylrestes mit 3 bis 8 Kohlenstoffatomen schließt ein: eine 2-Fluorcyclopentylgruppe, eine 2-Chlorcyclopentylgruppe, eine 2-Bromcyclopentylgruppe, eine 3-Fluorcyclopentylgruppe, eine 3-Chlorcyclopentylgruppe, eine 3-Bromcyclopentylgruppe, eine 2-Fluorcyclohexylgruppe, eine 2-Chlorcyclohexylgruppe, eine 2-Bromcyclohexylgruppe, eine 3-Fluorcyclohexylgruppe, eine 3-Chlorcyclohexylgruppe, eine 3-Bromcyclohexylgruppe, eine 4-Fluorcyclohexylgruppe, eine 4-Chlorcyclohexylgruppe, eine 4-Bromcyclohexylgruppe, eine Di-Fluorcyclopentylgruppe, eine Di-Chlorcyclopentylgruppe, eine Di-Bromcyclopentylgruppe, eine Di-Fluorcyclohexylgruppe, eine Di-Chlorcyclohexylgruppe, eine Di-Bromcyclohexylgruppe, eine Tri-Fluorcyclohexylgruppe, eine Tri-Chlorcyclohexylgruppe, eine Tri-Bromcyclohexylgruppe usw. Insbesondere sind Chlorocycloalkyl, Dichlorocycloalkyl und Trichlorocycloalkyl sowie Fluorocycloalkyl, Difluorocycloalkyl und Trifluorocycloalkyl zu nennen.

Beispiele eines mit Hydroxy substituierten Alkylrestes schließen die oben genanten Alkylreste ein, die 1 bis 3 Hydroxylreste aufweisen, wie zum Beispiel Hydroxymethyl, 2-Hydroxyethyl, 3-Hydroxypropyl etc.

Beispiele eines mit Alkoxy substituierten Alkylrestes schließen die oben genannten Alkylreste ein, die 1 bis 3 Alkoxyreste, wie unten definiert, aufweisen, wie zum Beispiel Methoxymethyl, Ethoxymethyl, 2-Methoxyethylen usw.

Beispiele eines mit Aryloxy substituierten Alkylrestes schließen die oben genannten Alkylreste ein, die 1 bis 3 Aryloxyreste, wie unten definiert, aufweisen, wie zum Beispiel Phenoxymethyl, 2-Phenoxyethyl und 2- oder 3-Phenoxypropyl usw. Besonders bevorzugt ist 2-Phenoxyethyl.

Beispiele eines mit Heterocyclyloxy substituierten Alkylrestes schließen die oben genannten Alkylreste ein, die 1 bis 3 Heterocyclyloxyreste, wie unten definiert, aufweisen, wie zum Beispiel Pyridin-2-yloxymethyl, -ethyl oder - propyl, Pyridin-3-yloxymethyl, -ethyl oder -propyl, Thiophen-2-yloxymethyl, -ethyl oder -propyl, Thiophen-3-yloxymethyl, -ethyl oder -propyl, Furan-2-yloxymethyl, -ethyl oder -propyl, Furan-3-yloxymethyl, -ethyl oder -propyl.

Beispiele eines mit Acyl substituierten Alkylrestes schließen die oben genannten Alkylreste ein, die 1 bis 3 Acylreste, wie unten definiert, aufweisen.

Beispiele einer mit Cycloalkyl substituierten Alkylgruppe schließen die oben genannten Alkylreste ein, die 1 bis 3, bevorzugt eine (gegebenenfalls substituierte) Cycloalkylgruppe aufweisen, wie zum Beispiel: Cyclohexylmethyl, 2-Cyclohexylethyl, 2- oder 3-Cyclohexylpropyl etc.

Beispiele einer mit Aryl substituierten Alkylgruppe schließen die oben genannten Alkylreste ein, die 1 bis 3, bevorzugt eine (gegebenenfalls substituierte) Arylgruppe, wie nachstehend definiert, aufweist wie zum Beispiel Phenylmethyl, 2-Phenylethyl, 2- oder 3-Phenylpropyl etc., wobei Phenylmethyl bevorzugt ist. Besonders bevorzugt sind weiterhin Alkylgruppen, wie vorstehend definiert, die mit substituiertem Aryl, wie unten definiert, insbesondere mit Halogen-substituiertem Aryl substituiert sind, wie besonders bevorzugt 2-Fluorphenylmethyl.

Beispiele einer mit Heterocyclyl substituierten Alkylgruppe schließen die oben genannten Alkylreste ein, die 1 bis 3, bevorzugt eine (gegebenenfalls substituierte) Heterocyclylgruppe, wie nachstehend definiert, aufweisen wie zum Beispiel 2-Pyridin-2-yl-ethyl, 2-Pyridin-3-yl-ethyl, Pyridin-2-yl-methyl, Pyridin-3-yl-methyl, 2-Furan-2-yl-ethyl, 2-Furan-3-yl-ethyl, Furan-2-yl-methyl, Furan-3-yl-methyl, 2-Thiophen-2-yl-ethyl, 2-Thiophen-3-yl-ethyl, Thiophen-2-yl-methyl, Thiophen-3-yl-methyl, 2-Morpholinylethyl, Morpholinylmethyl.

Beispiele eines mit Amino substituierten Alkylrestes schließen die oben genannten Alkylreste ein, die 1 bis 3, bevorzugt eine (gegebenenfalls substituierte) Aminogruppe, wie nachstehend definiert, aufweisen, wie zum Beispiel Methylaminomethyl, Methylaminoethyl, Methylaminopropyl, 2-Ethylaminomethyl, 3-Ethylaminomethyl, 2-Ethylaminoethyl, 3-Ethylaminoethyl etc.

Besonders bevorzugt sind Alkylgruppen, wie vorstehend definiert, die mit substituiertem Amino, wie nachstehend definiert, substituiert sind, insbesondere mit Aminogruppen, die mit gegebenenfalls substituiertem Aryl- oder Heterocyclyl substituiert sind, wie besonders bevorzugt 6-Trifluoromethyl-pyridin-2-yl-aminomethyl, 5-Trifluoromethyl-pyridin-2-yl-aminomethyl, 4-Trifluoromethyl-pyridin-2-yl-aminomethyl, 3-Trifluoromethyl-pyridin-2-yl-aminomethyl, 6-Trifluoromethyl-pyridin-3-yl-aminomethyl, 5-Trifluoromethyl-pyridin-3-yl-aminomethyl, 4-Trifluoromethyl-pyridin-3-yl-aminomethyl, 2-Trifluoromethyl-pyridin-3-yl-aminomethyl, 2-[6-Trifluoromethyl-pyridin-2-yl-amino]ethyl, 2-[5-Trifluoromethyl-pyridin-2-yl-amino]ethyl, 2-[4-Trifluoromethyl-pyridin-2-yl-amino]ethyl, 2-[3-Trifluoromethyl-pyridin-2-yl-amino]ethyl, 2-[6-Trifluoromethyl-pyridin-3-yl-amino]ethyl, 2-[5-Trifluoromethyl-pyridin-3-yl-amino]ethyl, 2-[4-Trifluoromethyl-pyridin-3-yl-amino]ethyl, 2-[2-Trifluoromethyl-pyridin-3-yl-amino]ethyl.
Besonders bevorzugt sind 2-[5-Trifluoromethyl-pyridin-2-yl-amino]ethyl: 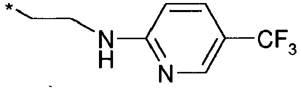 und 2-[4-Trifluoromethyl-pyridin-2-yl-amino]ethyl:

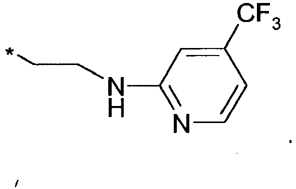

Gegebenenfalls substituiertes Alkoxy schließt eine gegebenenfalls substituierte Alkyl-O-Gruppe ein, worin hinsichtlich der Definition der Alkylgruppe auf vorstehende Definition verwiesen werden kann. Bevorzugte Alkoxygruppen sind lineare oder verzweigte Alkoxygruppen mit bis zu 6 Kohlenstoffatomen, wie eine Methoxygruppe, eine Ethoxygruppe, eine n-Propyloxygruppe, eine i-Propyloxygruppe, eine n-Butyloxygruppe, eine i-Butyloxygruppe, eine sec-Butyloxygruppe, eine t-Butyloxygruppe, eine n-Pentyloxygruppe, eine i-Pentyloxygruppe, eine sec-Pentyloxygruppe, eine t-Pentyloxygruppe, eine 2-Methylbutoxygrupe, eine n-Hexyloxygruppe, eine i-Hexyloxygruppe, eine t-Hexyloxygruppe, eine sec-Hexyloxygruppe, eine 2-Methylpentyloxygruppe, eine 3-Methylpentyloxygruppe, eine 1-Ethylbutyloxygruppe, eine 2-Ethylbutyloxygruppe, eine 1,1-Dimethylbutyloxygruppe, eine 2,2-Dimethylbutyloxygruppe, eine 3,3-Dimethylbutyloxygruppe, eine 1-Ethyl-1-methylpropyloxygruppe, sowie Cycloalkyloxy-Gruppen wie eine Cyclopentyloxygruppe oder eine Cyclohexyloxygruppe. Bevorzugt sind eine Methoxygruppe, eine Ethoxygruppe, eine n-Propyloxygruppe, eine i-Propyloxygruppe, eine n-Butyloxygruppe, eine i-Butyloxygruppe, eine sec-Butyloxygruppe, eine t-Butyloxygruppe. Besonders bevorzugt ist die Methoxygruppe.

Gegebenenfalls substituiertes Aryloxy schließt eine gegebenenfalls substituierte Aryl-O-Gruppe ein, worin hinsichtlich der Definition der Arylgruppe auf nachstehende Definition von gegebenenfalls substituiertem Aryl verwiesen werden kann. Bevorzugte Aryloxy-Gruppen umfassen 5- und 6-gliedrige Aryl-Gruppen, worunter Phenoxy, das gegebenenfalls substituiert sein kann, bevorzugt ist.

Gegebenenfalls substituiertes Heterocyclyloxy schließt eine gegebenenfalls substituierte Heterocyclyl-O-Gruppe ein, worin hinsichtlich der Definition der Heterocyclylgruppe auf nachstehende Definition von Heterocyclyl verwiesen werden kann. Bevorzugte Heterocyclyloxy-Gruppen umfassen gesättigte oder ungesättigte, wie aromatische 5- und 6-gliedrige Heterocyclyloxy-Gruppen, worunter Pyridin-2-yloxy, Pyridin-3-yloxy, Thiophen-2-yloxy, Thiophen-3-yloxy, Furan-2-yloxy, Furan-3-yloxy bevorzugt sind.

Gegebenenfalls substituiertes Alkenyl schließt im gesamten Rahmen der Erfindung bevorzugt ein:
Geradkettiges oder verzweigtkettiges Alkenyl mit 2 bis 8 Kohlenstoffatomen und Cycloalkenyl mit 3 bis 8 Kohlenstoffatomen, die gegebenenfalls durch bevorzugt 1 bis 3 gleichen oder verschiedenen Substituenten substituiert sein können, wie Hydroxy, Halogen oder Alkoxy. Beispiele schließen ein: Vinyl, 1-Methylvinyl, Allyl, 1-Butenyl, Isopropenyl, Cyclopropenyl, Cyclobutenyl, Cyclopentenyl, Cyclohexenyl. Bevorzugt sind Vinyl oder Allyl.

Gegebenenfalls substituiertes Alkinyl schließt im gesamten Rahmen der Erfindung bevorzugt ein:
Geradkettiges oder verzweigtkettiges Alkinyl mit 2 bis 8 Kohlenstoffatomen und Cycloalkinyl mit 5 bis 8 Kohlenstoffatomen, die gegebenenfalls durch bevorzugt 1 bis 3 gleichen oder verschiedenen Substituenten substituiert sein können. Bezüglich der Definition des gegebenenfalls substituierten Alkinyls wird auf die vorstehende Definition des gegebenenfalls substituierten Alkyls mit mehr als einem Kohlenstoffatom verwiesen, wobei die gegebenenfalls substituierten Alkine mindestens eine C≡C-Dreifachbindung umfassen. Beispiele schließen ein: Ethinyl, Propinyl, Butinyl, Pentinyl sowie gegebenenfalls wie vorstehend definierte substituierte Varianten davon. Bevorzugt ist Ethinyl sowie gegebenenfalls substituiertes Ethinyl.

Gegebenenfalls substituiertes Aryl schließt im gesamten Rahmen der Erfindung bevorzugt ein:
Aromatische Kohlenwasserstoffreste mit 6 bis 14 Kohlenstoffatomen (wobei die Kohlenstoffatome der möglichen Substituenten nicht mitgezählt sind), welche mono- oder bicyclisch sein können und die durch bevorzugt 1 bis 3 gleiche oder verschiedene Substituenten ausgewählt aus Hydroxy, Halogen, wie vorstehend definiert, Cyano, gegebenenfalls substituiertem Amino, wie nachstehend definiert, Mercapto, gegebenenfalls substituiertem Alkyl, wie vorstehend definiert, gegebenenfalls substituiertem Acyl, wie nachstehend definiert, und gegebenenfalls substituiertem Alkoxy, wie vorstehend definiert, gegebenenfalls substituiertem Aryloxy, wie vorstehend definiert, gegebenenfalls substituiertem Heterocyclyloxy, wie vorstehend definiert, gegebenenfalls substituiertem Aryl, wie hier definiert, gegebenenfalls substituiertem Heterocyclyl, wie nachstehend definiert, substituiert sein können. Aromatische Kohlenwasserstoffreste mit 6 bis 14 Kohlenstoffatomen schließen beispielweise ein: Phenyl, Naphthyl, Phenanthrenyl und Anthracenyl, die gegebenenfalls einfach oder mehrfach mit gleichen oder verschiedenen Resten substituiert sein können. Bevorzugt ist gegebenenfalls substituiertes Phenyl, wie halogensubstituiertes Phenyl.

Beispiele einer mit Alkyl substituierten Arylgruppe schließen bevorzugt ein: Aryl, wie oben beschrieben, welches mit geradkettigem oder verzweigtem Alkyl mit 1 bis 8, bevorzugt 1 bis 4 Kohlenstoffatomen, wie oben beschrieben, substituiert ist. Bevorzugtes Alkylaryl ist Toluyl.

Beispiele einer mit Hydroxy substituierten Arylgruppe schließen bevorzugt ein: Aryl, wie oben beschrieben, welches mit 1 bis 3 Hydroxylresten substituiert ist, wie zum Beispiel 2-Hydroxyphenyl, 3-Hydroxyphenyl, 4-Hydroxyphenyl, 2,4-Di-Hydroxyphenyl, 2,5-Di-Hydroxyphenyl, 2,6-Di-Hydroxyphenyl, 3,5-Di-Hydroxyphenyl, 3,6-Di-Hydroxyphenyl, 2,4,6-Tri-Hydroxyphenyl etc. Bevorzugt ist 2-Hydroxyphenyl, 3-Hydroxyphenyl und 2,4-Di-Hydroxyphenyl.

Beispiele einer mit Halogen substituierten Arylgruppe schließen bevorzugt ein: Aryl, wie oben beschrieben, welches mit 1 bis 3 Halogenatomen substituiert ist, wie zum Beispiel 2-Chlor- oder Fluorphenyl, 3-Chlor- oder Fluorphenyl, 4-Chlor- oder Fluorphenyl, 2,4-Di-(Chlor- und/oder Fluor)phenyl, 2,5-Di-(Chlor- und/oder Fluor)phenyl, 2,6-Di-(Chlor- und/oder Fluor)phenyl, 3,5-Di-(Chlor- und/oder Fluor)phenyl, 3,6-Di-(Chlor- und/oder Fluor)phenyl, 2,4,6-Tri-(Chlor- und/oder Fluor)phenyl etc. Bevorzugt ist 2-Fluorphenyl, 3-Fluorphenyl und 2,4-Di-fluorphenyl.

Beispiele einer mit Alkoxy substituierten Arylgruppe schließen bevorzugt ein: Aryl, wie oben beschrieben, welches mit 1 bis 3 Alkoxyresten, wie oben beschrieben, substituiert ist, wie bevorzugt 2-Methoxyphenyl, 3-Methoxyphenyl, 4-Methoxyphenyl, 2-Ethoxyphenyl, 3-Ethoxyphenyl, 4-Ethoxyphenyl, 2,4-Di-Methoxyphenyl etc.

Beispiele einer mit Hydroxy- und Alkoxy-substituierten Arylgruppe schließen ein: Aryl, wie oben beschrieben, welches mit 1 bis 2 Alkoxyresten, wie oben beschrieben, und mit 1 bis 2 Methoxyresten, wie oben beschrieben, substituiert ist. Bevorzugt ist 2-Hydroxy-5-Methoxyphenyl.

Gegebenenfalls substituiertes Heterocyclyl schließt im gesamten Rahmen der Erfindung bevorzugt ein:
Aliphatische, gesättigte oder ungesättigte heterocyclische 5 bis 8 gliedrige cyclische Reste, die 1 bis 3, bevorzugt 1 bis 2 Heteroatome, ausgewählt aus N, O oder S aufweisen, und die gegebenenfalls bevorzugt durch 1 bis 3 Substituenten substituiert sein können, wobei bezüglich möglicher Substituenten auf die Definition der möglichen Substituenten von Alkyl verwiesen werden kann. Bevorzugt sind 5- oder 6-gliedrige gesättigte oder ungesättigte, gegebenenfalls substituierte heterocyclische Reste, wie Tetrahydrofuran-2-yl, Tetrahydrofuran-3-yl, Tetrahydro-thiophen-2-yl, Tetrahydro-thiophen-3-yl, Pyrrolidin-1-yl, Pyrrolidin-2-yl, Pyrrolidin-3-yl, Morpholin-1-yl, Morpholin-2-yl, Morpholin-3-yl, Piperidin-1-yl, Piperidin-2-yl, Piperidin-3-yl, Piperidin-4-yl, Piperazin-1-yl, Piperazin-2-yl, Tetrahydropyran-2-yl, Tetrahydropyran-3-yl, Tetrahydropyran-4-yl, etc., welche gegebenenfalls mit aromatischen Ringen kondensiert sein können, etc.

Außerdem schließt gegebenenfalls substituiertes Heterocyclyl im gesamten Rahmen der Erfindung heteroaromatische Kohlenwasserstoffreste mit 4 bis 9 Ring-Kohlenstoffatomen, die zusätzlich bevorzugt 1 bis 3 gleiche oder verschiedene Heteroatome aus der Reihe S, O, N im Ring aufweisen, und die somit bevorzugt 5- bis 12-gliedrige heteroaromatische Reste bilden, welche bevorzugt monocyclisch aber auch bicyclisch sein können. Bevorzugte aromatische heterocyclische Reste schließen ein: Pyridinyl, wie Pyridin-2-yl, Pyridin-3-yl und Pyridin-4-yl, Pyridyl-N-oxid, Pyrimidyl, Pyridazinyl, Pyrazinyl, Thienyl, Furyl, Pyrrolyl, Pyrazolyl, Imidazolyl, Thiazolyl, Oxazolyl oder Isoxazolyl, Indolizinyl, Indolyl, Benzo[b]thienyl, Benzo[b]furyl, indazolyl, Chinolyl, Isochinolyl, Naphthyridinyl, Chinazolinyl. 5- oder 6-gliedrige aromatische Heterocyclen wie z.B. Pyridinyl, inbesondere Pyridin-2-yl, Pyridyl-N-oxid, Pyrimidyl, Pyridazinyl, Furyl und Thienyl sind bevorzugt.

Die erfindungsgemäßen Heterocyclyl-Reste können durch bevorzugt 1 bis 3 gleiche oder verschiedene Substituenten ausgewählt beispielsweise aus Hydroxy, Halogen, wie vorstehend definiert, Cyano, Amino, wie nachstehend definiert, Mercapto, Alkyl, wie vorstehend definiert, Acyl, wie nachstehend definiert, und Alkoxy, wie vorstehend definiert, Aryloxy, wie vorstehend definiert, Heterocyclyloxy, wie vorstehend definiert, Aryl, wie vorstehend definiert, Heterocyclyl, wie hier definiert, substituiert sein.

Heterocyclyl schließt bevorzugt ein: Tetrahydrofuranyl, Pyrrolidinyl, Morpholinyl, Piperidinyl oder Tetrahydropyranyl, Pyridinyl, Pyridyl-N-oxid, Pyrimidyl, Pyridazinyl, Pyrazinyl, Thienyl, Furanyl, Pyrrolyl, Pyrazolyl, Imidazolyl, Thiazolyl, Oxazolyl oder Isoxazolyl, Indolizinyl, Indolyl, Benzo[b]thienyl, Benzo[b]furyl, Indazolyl, Chinolyl, Isochinolyl, Naphthyridinyl, Chinazolinyl, Chinoxazolinyl. 5- oder 6-gliedrige Heterocyclen wie z. B. Morpholinyl sowie aromatische Heterocyclen wie z.B. Pyridyl, Pyridyl-N-oxid, Pyrimidyl, Pyridazinyl, Furanyl, Thienyl sowie Chinolyl und Isochinolyl sind bevorzugt, Bevorzugt sind Morpholinyl, Pyridyl, Pyrimidyl und Furanyl. Besonders bevorzugtes Heterocyclyl schließt ein: Morpholinyl, Pyridyl, wie Pyridin-2-yl, Pyridin-3-yl, Pyridin-4-yl, Pyrimidinyl, wie Pyrimidin-2-yl und Pyrimidin-5-yl, Pyrazin-2-yl, Thienyl, wie Thien-2-yl und Thien-3-yl sowie Furanyl, wie Furan-2-yl und Furan-3-yl.

Beispiele einer mit Alkyl substituierten Heterocyclylgruppe schließen bevorzugt ein: Heterocyclyl, wie oben beschrieben, welches mit geradkettigem oder verzweigtem gegebenenfalls substituiertem Alkyl mit 1 bis 8, bevorzugt 1 bis 4 Kohlenstoffatomen, wie oben beschrieben, substituiert ist. Bevorzugtes Alkylheterocyclyl sind Methylpyridinyl, Trifluormethylpyridinyl, wie insbesondere 3- oder 4- Trifluormethylpyridin-2-yl, Methylfuryl, Methylpyrimidyl, Methylpyrrolyl und Methylchinolinyl wie insbesondere 2-Methylchinolin-6-yl:

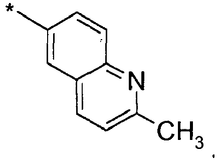

Beispiele einer mit Hydroxy substituierten Heterocyclylgruppe schließen bevorzugt ein: Heterocyclyl, wie oben beschrieben, welches mit 1 bis 3 Hydroxylresten substituiert ist, wie zum Beispiel 3-Hydroxypyridyl, 4-Hydroxypyridyl 3-Hydroxyfuryl, 2-Hydroxypyrimidyl 5-Hydroxypyrimidyl, 3-Hydroxypyrrolyl, 3,5-Di-Hydroxypyridyl, 2,5-Di-Hydroxypyrimidyl etc.

Beispiele einer mit Alkoxy substituierten Heterocyclylgruppe schließen bevorzugt ein:
Heterocyclyl, wie oben beschrieben, welches mit 1 bis 3 Alkoxyresten, wie oben beschrieben, substituiert ist, wie bevorzugt 3-Alkoxypyridyl, 4-Alkoxypyridyl 3-Alkoxyfuryl, 2-Alkoxypyrimidyl 5-Alkoxypyrimidyl, 3-Alkoxypyrrolyl, 3,5-Di-Alkoxypyridin-2-yl, 2,5-Di-Alkoxypyrimidyl etc.

Gegebenenfalls substituiertes Acyl schließt hier und im folgenden ein: Formyl (-CH(=O)), gegebenenfalls substituiertes aliphatisches Acyl (Alkanoyl = Alkyl-CO-, worin bezüglich der Alkylgruppe auf vorstehende Definition von gegebenenfalls substituiertem Alkyl verwiesen werden kann), gegebenenfalls substituiertes aromatisches Acyl (Aroyl = Aryl-CO-, worin bezüglich der Arylgruppe auf vorstehende Definition von gegebenenfalls substituiertem Aryl verwiesen werden kann) oder heterocyclisches Acyl (Heterocycloyl = Heterocyclyl-CO-, worin bezüglich der heterocyclischen Gruppe auf vorstehende Definition von gegebenenfalls substituiertem Heterocyclyl verwiesen werden kann. Bevorzugt ist heteroaromatisches Acyl = Heteroaryl-CO-).

Dabei schließt gegebenenfalls substituiertes aliphatisches Acyl (Alkanoyl) bevorzugt ein: C₁ bis C₆ Alkanoyl, wie Formyl, Acetyl, Propionyl, Butyryl, Isobutyryl, Valeryl, Isovaleryl, Pivaloyl, Hexanoyl, usw.

Beispiele von substituiertem aliphatischen Acyl schließen beispielsweise ein: gegebenenfalls Aryl- oder Heterocycyl-substituiertes C₂ bis C₆ Alkanoyl, worin bezüglich der Definitionen von Aryl, Heterocycyl und C₂ bis C₆ Alkanoyl auf die vorstehenden Definitionen verwiesen werden kann, wie Phenylacetyl, Thiophen-2-yl-acetyl, Thiophen-3-yl-acetyl, Furan-2-yl-acetyl, Furan-3-yl-acetyl, 2- oder 3- Phenylpropionyl, 2- oder 3-Thiophen-2-yl-propionyl, 2- oder 3-Thiophen-3-yl-propionyl, 2- oder 3-Furan-2-yl-propionyl, 2- oder 3-Furan-3-yl-propionyl, bevorzugt Thiophen-2-yl-acetyl.

Gegebenenfalls substituiertes aromatisches Acyl (Aroyl) schließt ein: C₆ bis C₁₀ Aroyl, wie Benzoyl, Toluoyl, Xyloyl, usw.

Gegebenenfalls substituiertes heteroaromatisches Acyl (Hetaroyl) schließt insbesondere ein: C₆ bis C₁₀ Hetaroyl, wie Furanoyl, Pyridinoyl etc.

Gegebenenfalls substituiertes Amino schließt im gesamten Rahmen der Erfindung bevorzugt ein: Amino, Mono- oder Dialkylamino, Mono- oder Diarylamino, (N-Alkyl)(N-aryl)amino, Mono- oder Diheterocyclylamino, (N-Alkyl)(N-heterocyclyl)amino, (N-Aryl)(N-heterocyclyl)amino, Mono- oder Diacylamino etc., wobei bezüglich Alkyl, Aryl, Heterocyclyl und Acyl auf die entsprechenden vorstehenden Definition für gegebenenfalls substituiertes Alkyl, gegebenenfalls substituiertes Aryl, gegebenenfalls substituiertes Heterocyclyl und gegebenenfalls substituiertes Acyl verwiesen werden kann, und substituiertes Alkyl hier bevorzugt Aryl- oder Heterocyclyl-substituiertes Alkyl einschließt.

Mono- oder Dialkylamino schließt dabei insbesondere ein: geradkettiges oder verzweigtes Mono- oder Dialkylamino mit 1 bis 8, bevorzugt 1 bis 4 gesättigten oder ungesättigten, gegebenenfalls wie vorstehend beschrieben substituierten Kohlenstoffatomen in jeder Alkylgruppe, insbesondere Methylamino, Dimethylamino, Ethylamino, wobei die Alkylgruppen mit bevorzugt einem Substituenten substituiert sein können.

Mono- oder Diarylamino schließt dabei insbesondere ein: Mono- oder Diarylamino mit 3- bis 8-, bevorzugt 5- bis 6-gliedrigen, gegebenenfalls wie vorstehend beschrieben substituierten Arylresten, insbesondere Phenylamino oder Diphenylamino, wobei die Arylgruppen gegebenenfalls mit einem oder zwei Substituenten substituiert sein können.

(N-Alkyl)(N-aryl)amino beschreibt insbesondere ein substituiertes Amino, das jeweils am Stickstoffatom mit einem Alkylrest und mit einem Arylrest substituiert ist, wie insbesondere (N-Methyl)(N-Phenyl)amino.

Mono- oder Diheterocyclylamino schließt insbesondere ein: Mono- oder Diheterocyclylamino mit 3- bis 8-, bevorzugt 5- bis 6-gliedrigen, gegebenenfalls wie vorstehend beschrieben substituierten Heterocyclylresten, insbesondere Pyridylamino oder Dipyridylamino.

(N-Alkyl)(N-heterocyclyl)amino beschreibt insbesondere ein substituiertes Amino, das jeweils am Stickstoffatom mit einem Alkylrest und mit einem Heterocyclylrest substituiert ist.

(N-Aryl)(N-heterocyclyl)amino beschreibt insbesondere ein substituiertes Amino, das jeweils am Stickstoffatom mit einem Arylrest und mit einem Heterocyclylrest substituiert ist.

Mono- oder Diacylamino schließt insbesondere ein substituiertes Amino ein, das mit einem oder zwei Acylresten substituiert ist etc.

Bezüglich der Definition von Alkyl, Aryl, Heterocyclyl und Acyl kann auf die entsprechenden vorstehenden Definitionen für gegebenenfalls substituiertes Alkyl, gegebenenfalls substituiertes Aryl und gegebenenfalls substituiertes Heterocyclyl und gegebenenfalls substituiertes Acyl verwiesen werden.

Gegebenenfalls substituiertes Amino schließt weiterhin eine bevorzugt substituierte Methylenaminogruppe ein: 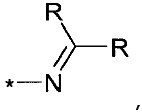 worin hier R jeweils eine organische Gruppe und/oder Wasserstoff ist, insbesondere R⁶ und R⁷, wie unten definiert. Bevorzugt ist R hier Wasserstoff und/oder eine gegebenenfalls substituierte Alkyl-, Aryl- oder Heterocyclylgruppe, die jeweils wie vorstehend definiert sind. Besonders bevorzugt ist R hier Wasserstoff und eine gegebenenfalls substituierte Arylgruppe oder R ist eine gegebenenfalls substituierte Alkylgruppe und eine gegebenenfalls substituierte Arylgruppe, wie zum Beispiel:

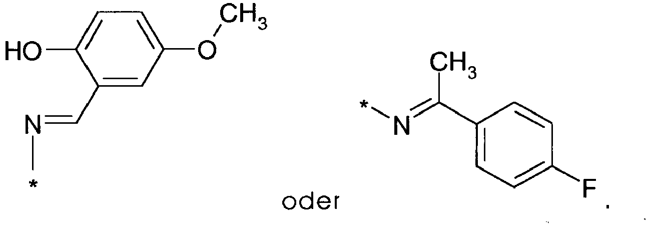

In der Bedeutung von R⁵ bildet die gegebenenfalls substituierte Aminogruppe, wie vorstehend beschrieben zusammen mit dem Stickstoffatom, an das sie gebunden ist, bevorzugt eine gegebenenfalls substituierte Hydrazingruppe (-NH-NH₂), wie Hydrazinyl, eine gegebenenfalls substituierte Mono- oder Dialkylhydrazinylgruppe (-NH-NHR oder -NH-NR₂) wie gegebenenfalls substituiertes Methylhydrazin, Methylenhydrazin (-NH-N=CR₂), Ethylhydrazin, Propylhydrazin etc. oder (gegebenenfalls substituierte) Aryl- und/oder Heterocyclylhydrazinyl wie beispielsweise (gegebenenfalls substituiertey) Phenylhydrazin (-NH-NH-Phenyl).
Besonders bevorzugte gegebenenfalls substituierte Aminogruppen sind: Amino, Diphenylamino, (N-Methyl)(N-Phenyl)amino sowie Aminogruppen der Formel 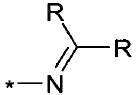 wie oben definiert, bevorzugt solche in denen R hier Wasserstoff, eine gegebenenfalls substituierte Alkylgruppe oder eine gegebenenfalls substituierte Arylgruppe darstellt, wie insbesondere:
2-Hydroxy-phenyl-meth-(E oder Z)-yliden]-amino: 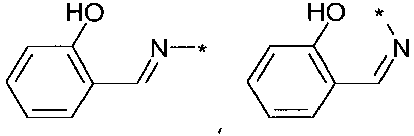
(3-Hydroxy-phenyl)-meth-(E oder Z)-yliden]-amino: 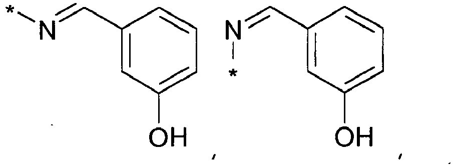
1-(2,4-Dihydroxy-phenyl)-meth-(E oder Z)-yliden]-amino 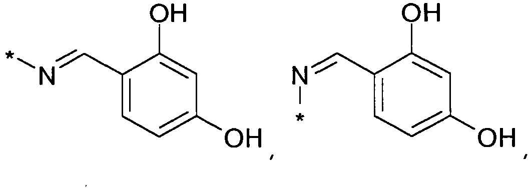
1-(2-Hydroxy-5-methoxy-phenyl)-meth-(E oder Z)-yliden]-amino: 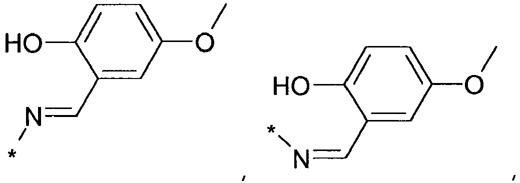
1-(4-Fluorphenyl)-eth-(E oder Z)-ylidenamino:

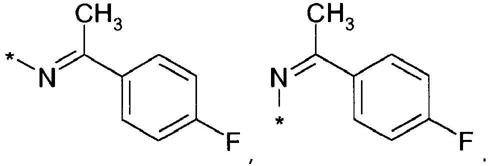

Gegebenenfalls substituiertes Aminocarbonyl stellt im Rahmen der gesamten Erfindung gegebenenfalls substituiertes Amino-CO- dar, worin hinsichtlich der Definition von gegebenenfalls substituiertem Amino auf vorstehende Definition verwiesen werden kann. Bevorzugt stellt gegebenenfalls substituiertes Aminocarbonyl gegebenenfalls substituiertes Carbamoyl (H₂NCO-), wie H₂NCO-, Mono- oder Dialkylaminocarbonyl (H(Alkyl)N-CO- oder (Alkyl)₂N-CO-), Mono- oder Diarylaminocarbonyl (H(Aryl)N-CO- oder (Aryl)₂N-CO-) oder Mono- oder Diheterocyclylaminocarbonyl (H(Heterocyclyl)N-CO- oder (Heterocyclyl)₂N-CO-) dar, worin hinsichtlich der Definition von Alkyl, Aryl oder Hyeterocyclyl auf die oben stehenden Erläuterungen für gegebenenfalls substituiertes Alkyl, Aryl oder Heterocyclyl verwiesen werden kann.

Des weiteren stellt gegebenenfalls substituiertes Aminosulfonyl im Rahmen der gesamten Erfindung gegebenenfalls substituiertes Amino-SO₂- dar, worin hinsichtlich der Definition von gegebenenfalls substituiertem Amino auf vorstehende Definition verwiesen werden kann. Bevorzugt sind gegebenenfalls substituiertes Sulfamoyl (H₂N-SO₂-), wie Sulfamoyl (H₂N-SO₂-) oder Mono- oder Dialkylaminosulfonyl (Alkyl)₂N-SO₂-, worin hinsichtlich der Definition von Alkyl auf die oben stehenden Erläuterungen für gegebenenfalls substituiertes Alkyl verwiesen werden kann.

Weiterhin stellt gegebenenfalls substituiertes Alkyl-, Aryl- oder Heterocyclylsulfonyl (R-SO₂-, worin R gegebenenfalls substituiertes Alkyl, gegebenenfalls substituiertes Aryl oder gegebenenfalls substituiertes Heterocyclyl, jeweils wie vorstehend definiert, sind) vorzugsweise Methylsulfonyl, Ethylsulfonyl, Phenylsulfonyl, Tolylsulfonyl oder Benzylsulfonyl dar.

Gegebenenfalls substituiertes Alkoxycarbonyl (RO(O=)C-) schließt hinsichtlich der Definition von Alkoxy oben erwähntes gegebenenfalls substituiertes Alkoxy ein.

Gegebenenfalls substituiertes Acyloxy (R-C(=O)-O-) schließt hinsichtlich der Definition von Acyl oben erwähntes gegebenenfalls substituiertes Acyl ein.

### BEVORZUGTE AUSFÜHRUNGSFORMEN:

Verbindungen der Formel (I) können folgende Substituentendefinitionen aufweisen:
X hat die Bedeutung N oder C-R¹, worin
R¹ ausgewählt wird aus der Gruppe, die besteht aus:
   - Wasserstoff,
   - Halogen,
   - gegebenenfalls substituiertem Amino,
   - gegebenenfalls substituiertem Alkyl,
   - gegebenenfalls substituiertem Alkoxy
   - gegebenenfalls substituiertem Aryl,
   - gegebenenfalls substituiertem Heterocyclyl;
R² und R³ sind gleich oder verschieden, und werden jeweils ausgewählt aus der Gruppe, die besteht aus:
   - Wasserstoff,
   - Halogen,
   - gegebenenfalls substituiertem Amino,
   - gegebenenfalls substituiertem Alkyl,
   - gegebenenfalls substituiertem Alkoxy,
   - gegebenenfalls substituiertem Aryl,
   - gegebenenfalls substituiertem Heterocyclyl;
R⁴ und R⁵ sind gleich oder verschieden, und werden jeweils ausgewählt aus der Gruppe, die besteht aus:
   - Wasserstoff,
   - gegebenenfalls substituiertem Amino,
   - gegebenenfalls substituiertem Alkyl,
   - gegebenenfalls substituiertem Aryl,
   - gegebenenfalls substituiertem Heterocyclyl oder
   - R⁴ und R⁵ bilden zusammen mit dem Stickstoffatom, an das sie gebunden sind, einen gesättigten oder ungesättigten, gegebenenfalls substituierten 5- bis 6-gliedrigen Ring aus, der gegebenenfalls weitere Heteroatome enthalten kann.

Weiterhin kann die Verbindung der Formel (I) folgende Substituentendefinitionen aufweisen:
X hat die Bedeutung N oder C-R¹, worin
R¹ ausgewählt wird aus der Gruppe, die besteht aus:
   - Wasserstoff,
   - Halogen,
   - gegebenenfalls substituiertem Alkyl,
   - gegebenenfalls substituiertem Alkoxy
   - gegebenenfalls substituiertem Aryl,
   - gegebenenfalls substituiertem Heterocyclyl;
R² und R³ sind gleich oder verschieden, und werden jeweils ausgewählt aus der Gruppe, die besteht aus:
   - Wasserstoff,
   - Halogen,
   - gegebenenfalls substituiertem Amino,
   - gegebenenfalls substituiertem Alkyl,
   - gegebenenfalls substituiertem Aryl,
   - gegebenenfalls substituiertem Heterocyclyl;
R⁴ und R⁵ sind gleich oder verschieden, und werden jeweils ausgewählt aus der Gruppe, die besteht aus:
   - Wasserstoff,
   - gegebenenfalls substituiertem Amino,
   - gegebenenfalls substituiertem Alkyl,
   - gegebenenfalls substituiertem Aryl,
   - gegebenenfalls substituiertem Heterocyclyl oder
   - R⁴ und R⁵ bilden zusammen mit dem Stickstoffatom, an dass sie gebunden sind, einen gesättigten oder ungesättigten, gegebenenfalls substituierten 5- bis 6-gliedrigen Ring aus, der gegebenenfalls ein bis zwei weitere Heteroatome enthalten kann.

Weiterhin kann die Verbindung der Formel (I) folgende Substituentendefinitionen aufweisen:
X hat die Bedeutung N oder C-R¹, worin
R¹ ausgewählt wird aus der Gruppe, die besteht aus:
   - Wasserstoff,
   - Halogen,
   - gegebenenfalls substituiertem Alkyl,
   - gegebenenfalls substituiertem Alkoxy,
R² und R³ sind gleich oder verschieden und werden ausgewählt aus der Gruppe, die besteht aus:
   - Wasserstoff,
   - gegebenenfalls substituiertem Amino,
   - gegebenenfalls substituiertem Alkyl,
   - gegebenenfalls substituiertem Heterocyclyl,
R⁴ und R⁵ sind gleich oder verschieden, und werden jeweils ausgewählt aus der Gruppe, die besteht aus:
   - Wasserstoff,
   - gegebenenfalls substituiertem Amino,
   - gegebenenfalls substituiertem Alkyl;
   - gegebenenfalls substituiertem Heterocyclyl; oder
   R⁴ und R⁵ bilden zusammen mit dem Stickstoffatom, an das sie gebunden sind, einen gesättigten oder ungesättigten, gegebenenfalls substituierten 5- bis 6-gliedrigen Ring aus, der gegebenenfalls ein bis zwei weitere Heteroatome enthalten kann.

Weiterhin können in der allgemeinen Formel (I) bzw. (I') die einzelnen Substituenten jeweils folgende Definitionen aufweisen:
1. Y hat die Bedeutung von -NR⁴R⁵.
2. X hat die Bedeutung von N und R², R³, R⁴ und R⁵ weisen die Bedeutung einer der vorstehend beschriebenen Ausführungsformen auf.
3. X hat die Bedeutung C-R¹ und R¹ wird ausgewählt aus der Gruppe, die besteht aus:
   ∘ Wasserstoff,
   ∘ Halogen,
   ∘ gegebenenfalls substituiertem Alkyl,
   ∘ gegebenenfalls substituiertem Alkoxy,
   und R², R³, R⁴ und R⁵ weisen die Bedeutung einer der vorstehend beschriebenen Ausführungsformen auf.
4. R² und R³ sind gleich oder verschieden und werden ausgewählt aus der Gruppe, die besteht aus:
   ∘ Wasserstoff,
   ∘ gegebenenfalls substituiertem Amino,
   ∘ gegebenenfalls substituiertem Alkyl,
   ∘ gegebenenfalls substituiertem Heterocyclyl,
   und X, R¹, R⁴ und R⁵ weisen die Bedeutung einer der vorstehend beschriebenen Ausführungsformen auf.
5. R⁴ und R⁵ sind gleich oder verschieden, und werden jeweils ausgewählt aus der Gruppe, die besteht aus:
   ∘ Wasserstoff,
   ∘ gegebenenfalls substituiertem Amino;
   ∘ gegebenenfalls substituiertem Alkyl;
   ∘ gegebenenfalls substituiertem Heterocyclyl; oder
   R⁴ und R⁵ bilden zusammen mit dem Stickstoffatom, an das sie gebunden sind, einen gesättigten oder ungesättigten, gegebenenfalls substituierten 5- bis 6-gliedrigen Ring aus, der gegebenenfalls ein bis zwei weitere Heteroatome enthalten kann
   und X, R¹ R² und R³ weisen die Bedeutung einer der vorstehend beschriebenen Ausführungsformen auf.

In der allgemeinen Formel (I) können die einzelnen Substituenten jeweils folgende Definitionen aufweisen:
X bedeutet N oder C- R¹ worin R¹ ausgewählt wird aus der Gruppe, die besteht aus:
   - Wasserstoff,
   - Halogen, wie insbesondere Chlor,
      - gegebenenfalls substituiertem Alkyl, wie insbesondere geradkettigem oder verzweigtem Alkyl, wie vorstehend definiert, insbesondere bevorzugt Methyl, und das gegebenenfalls durch (gegebenenfalls substituiertes, wie Alkyl-, Halogen-, und/oder Alkoxy-, substituiertes) Aryl, wie vorstehend definiert, substituiert sein kann, insbesondere mit gegebenenfalls Alkyl-, Halogen-, und/oder Alkoxy -substituiertem Aryl substituiertes Alkyl, wie Benzyl, Halogen-, Alkyl- und oder Alkoxysubstituiertes Benzyl, wie beispielsweise 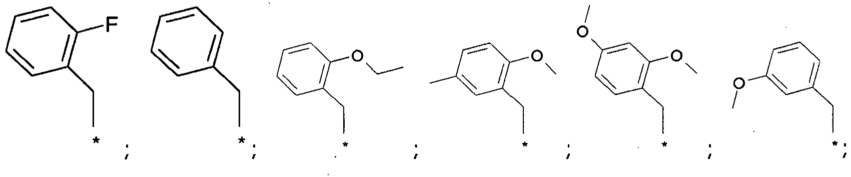 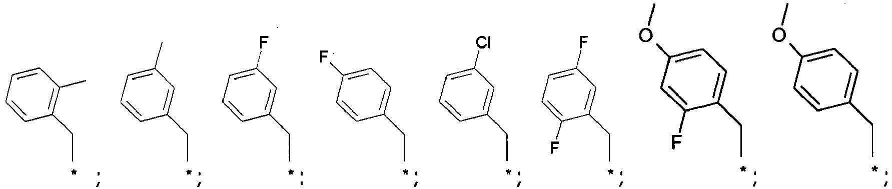 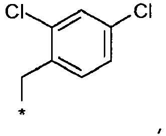 bevorzugt 2-Fluorphenylmethyl: 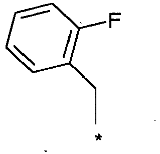 (* bezeichnet hier und im Folgenden jeweils die Bindungsposition des Restes hier R¹);
         oder
   - gegebenenfalls substituiertem Alkoxy, wie Isopropoxy, Methoxy, insbesondere Methoxy,
R² wird ausgewählt aus der Gruppe, die besteht aus:
   - Wasserstoff,
   - Hydroxy,
   - Halogen, wie Chlor,
   - gegebenenfalls substituiertem Alkyl, wie insbesondere geradkettigem oder verzweigtem Alkyl, wie vorstehend definiert, das gegebenenfalls, substituiert sein kann, wie vorstehend beschrieben, wobei insbesondere Methyl bevorzugt ist;
   - gegebenenfalls substituiertem Alkoxy, wie insbesondere Alkoxy substituiert mit gegebenenfalls substituiertem Aryl, wie 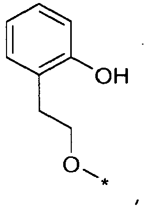
   - gegebenenfalls substituiertem Amino, wie Amino, Mono- oder Dialkylamino, wie Isopropylamino, insbesondere Amino (-NH₂);
   - gegebenenfalls substituiertem Heterocyclyl, wie insbesondere aliphatisches Heterocyclyl, wie vorstehend beschrieben, wobei Morpholinyl, insbesondere Morpholin-4-yl: 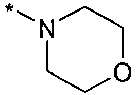 bevorzugt ist;
R³ wird ausgewählt aus der Gruppe, die besteht aus:
   - Wasserstoff,
   - gegebenenfalls substituiertem Alkyl, wie insbesondere geradkettigem oder verzweigtem Alkyl, wie vorstehend definiert, das gegebenenfalls, substituiert sein kann, wie vorstehend beschrieben, wie Aminomethyl und Methyl, wobei insbesondere Methyl bevorzugt ist;
   - gegebenenfalls substituiertem Amino, wie insbesondere Diarylamino, worin Aryl gegebenenfalls substituiert sein kann, wie vorstehend beschrieben, wobei Diphenylamino bevorzugt ist, oder (N-Alkyl)(N-aryl)amino, worin Alkyl und Aryl gegebenenfalls substituiert sein können, wie vorstehend beschrieben, wobei (N-Methyl)(N-phenyl)amino bevorzugt ist; oder
   - gegebenenfalls substituiertem Aryl, wie Phenyl,
   - gegebenenfalls substituiertem Heterocyclyl, wie insbesondere aliphatischem Heterocyclyl, wie vorstehend beschrieben, wobei Morpholinyl, insbesondere Morpholin-4-yl: 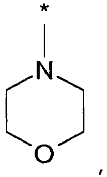 bevorzugt ist, oder gegebenenfalls substituiertem ungesättigten und/oder aromatischen Heterocyclyl, wie vorstehend beschrieben, wie gegebenenfalls substituiertem insbesondere stickstoff-haltiges Heterocyclyl, wie 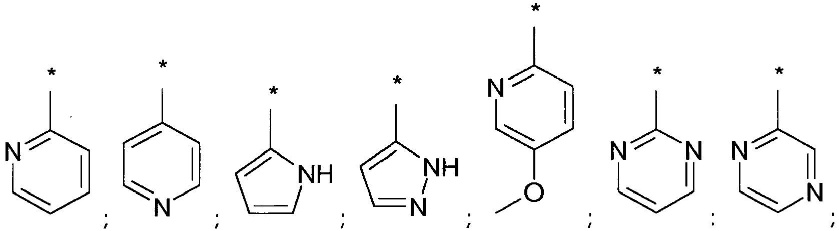 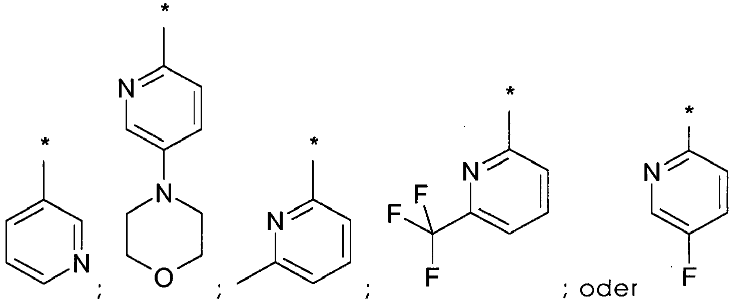 wobei Pyridinyl, wie insbesondere 2-Pyridinyl 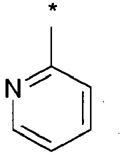 besonders bevorzugt ist;
R⁴ und R⁵ sind gleich oder verschieden und bedeuten:
   - Wasserstoff (bevorzugt ist einer von R⁴ oder R⁵ Wasserstoff, oder beide sind Wasserstoff),
   - gegebenenfalls substituiertes Alkyl, wie insbesondere geradkettiges, verzweigtes und/oder cyclisches Alkyl, wie vorstehend definiert, wie insbesondere bevorzugt Methyl, Ethyl, n-Propyl, besonders bevorzugt ist Isopropyl 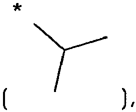 n-Butyl, Isobutyl 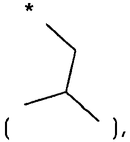 Cyclopropylmethyl 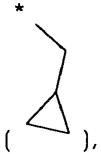 Cyclohexylmethyl 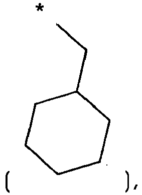 und das gegebenenfalls durch (gegebenenfalls substituiertes) Amino, wie vorstehend definiert, substituiert sein kann, wobei insbesondere mit (gegebenenfalls substituiertem) Aryl- oder Heterocyclyl substituiertem Amino substituiertes Alkyl bevorzugt ist, wie insbesondere Benzyl, Phenethyl, Phenylpropyl 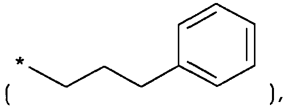 Hydroxyphenethyl (wie 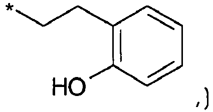 2-(5-Trifluormethyl-pyridin-2-ylamino)-ethyl: 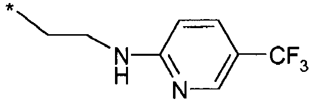 und
      2-(4-Trifluoromethyl-pyridin-2-ylamino)-ethyl: 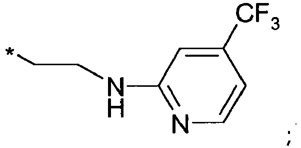
   - gegebenenfalls substituiertes Amino, wie eine gegebenenfalls substituierte Acylaminogruppe, wie: 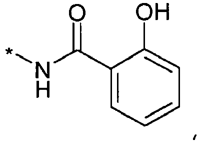 bevorzugt eine einfach oder zweifach substituierte Methylenaminogruppe: 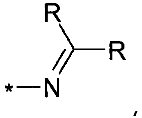 worin hier R jeweils eine organische Gruppe und/oder Wasserstoff ist, wie R⁶ und R⁷ wie unten definiert. Bevorzugt ist R Wasserstoff und/oder eine gegebenenfalls substituierte Alkyl-, Aryl- oder Heterocyclylgruppe, die jeweils wie vorstehend definiert sind. Besonders bevorzugt ist R hier Wasserstoff und eine gegebenenfalls substituierte Arylgruppe oder R ist eine gegebenenfalls substituierte Alkylgruppe und eine gegebenenfalls substituierte Arylgruppe, wie zum Beispiel:

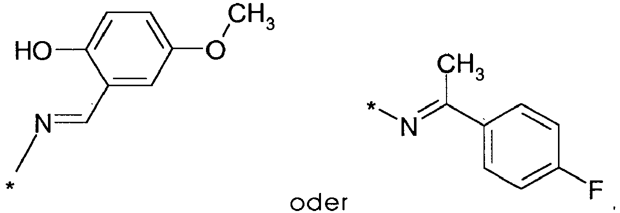

Besonders bevorzugte gegebenenfalls substituierte Aminogruppen für R⁵ sind:
2-Hydroxy-phenyl-meth-(E oder Z)-yliden]-amino: 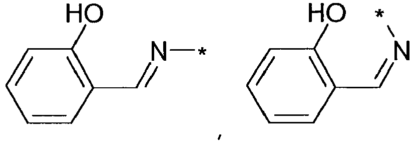 (3-Hydroxy-phenyl)-meth-(E oder Z)-yliden]-amino: 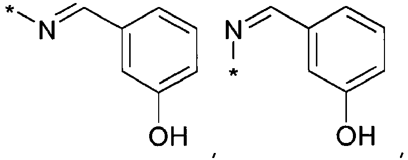 1-(2,4-Dihydroxy-phenyl)-meth-(E oder Z)-yliden]-amino 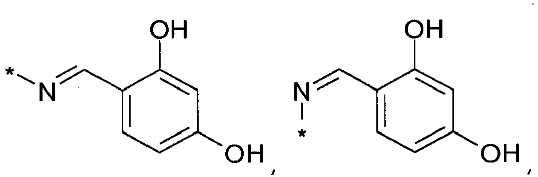 1-(2-Hydroxy-5-methoxy-phenyl)-meth-(E oder Z)-yliden]-amino: 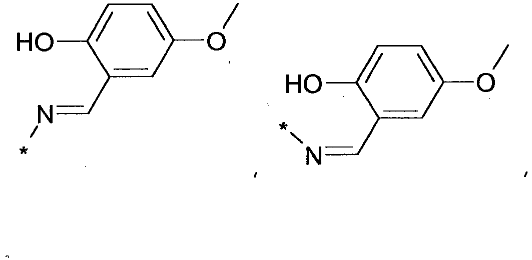 oder
1-(4-Fluorphenyl)-eth-(E oder Z)-ylidenamino: 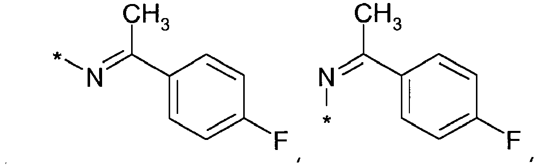 oder 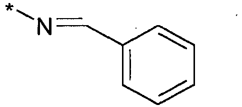
   - gegebenenfalls substituiertes Heterocyclyl, wie insbesondere aromatisches Heterocyclyl, wie vorstehend beschrieben, wobei insbesondere Chinolyl oder Alkyl-substituiertes Chinolyl wie 5-Methylchinolyl bevorzugt ist;
   - gegebenenfalls substituiertes Acyl, wie insbesondere aliphatische oder aromatisches Acyl, wie Acetyl, Benzoyl,
   - gegebenenfalls substituiertes Alkyl- oder Arylsulfonyl, Methylsulfonyl, Phenylsulfonyl,
   - gegebenenfalls substituiertem Aminocarbonyl, wie Mono- oder DiAlkyl- und/oder Arylaminocarbonyl, wie 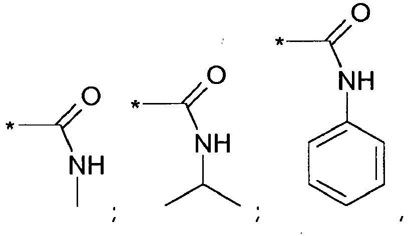 oder
   - R⁴ und R⁵ bilden zusammen mit dem Stickstoffatom, an das sie gebunden sind, einen gesättigten oder ungesättigten, gegebenenfalls substituierten 5- bis 6-gliedrigen Ring aus, der gegebenenfalls ein bis zwei weitere Heteroatome enthalten kann, insbesondere bilden R⁴ und R⁵ bevorzugt zusammen mit dem Stickstoffatom, an das sie gebunden sind, einen gesättiigten oder ungesättigten, wie aromatischen 5- oder 6-gliedrigen gliedrigen Heterocylylring, wie insbesondere gegebenenfalls substituiertes Pyrazolyl, Imidazolyl, Triazolyl; Piperidinyl, Morpholinyl, Piperazinyl, wie 4-Methylpiperazinyl, Pyrrolidinyl, aus.Besonders bevorzugt ist, daß R⁴ und R⁵ zusammen Reste der Formeln: 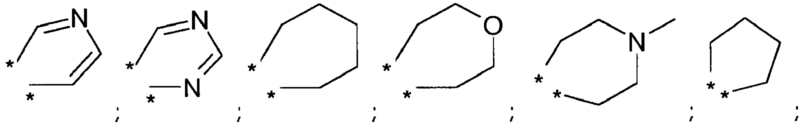 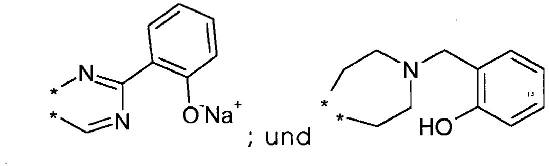 bilden.

In einer besonders bevorzugten Variante ist R⁴ Wasserstoff und R⁵ ist Isopropyl.

Mögliche Verbindungen der allgemeinen Formel (I) sind in folgender Tabelle gezeigt:

| Beispiel | Verbindung | 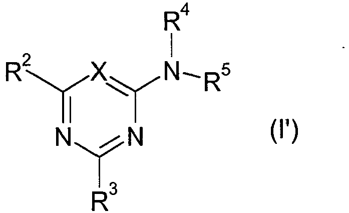 | | | | | |
|---|---|---|---|---|---|---|---|
| | | X | R¹ | R² | R³ | R⁴ | R⁵ |
| 1# | 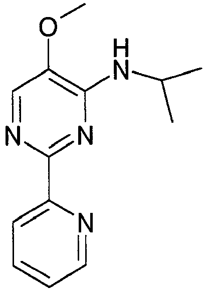 | C-R¹ | -OCH₃ | H | 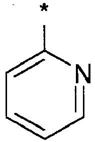 | H | 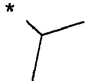 |
| 2# | 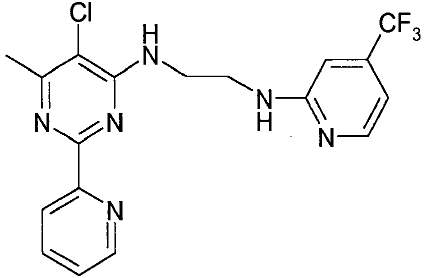 | C-R¹ | -Cl | -CH₃ | 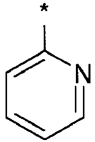 | H | 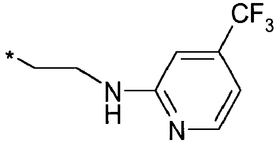 |

| Beispiel | Verbindung | 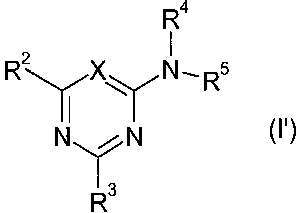 | | | | | |
|---|---|---|---|---|---|---|---|
| | | X | R¹ | R² | R³ | R⁴ | R⁵ |
| 3# | 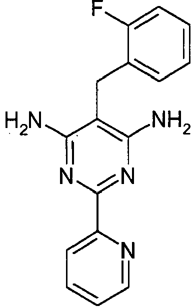 | C-R¹ | 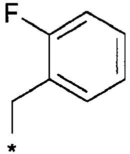 | -NH₂ | 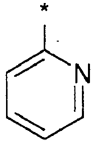 | H | H |
| 4# | 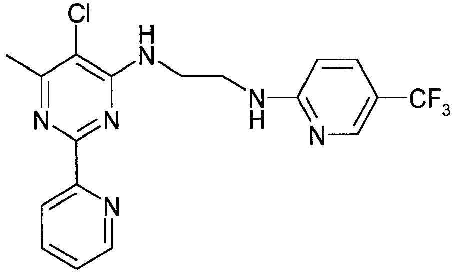 | C-R¹ | Cl | -CH₃ | 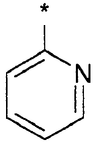 | H | 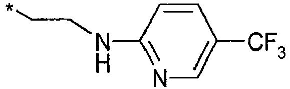 |
| 5# | 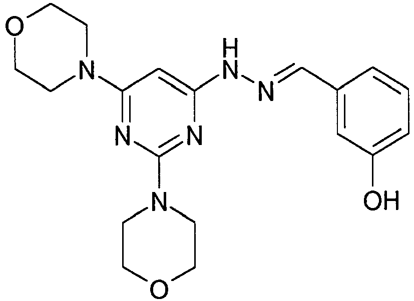 | C-R¹ | H | 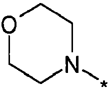 | 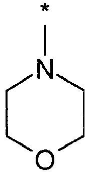 | H | 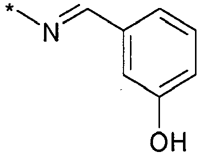 |
| 6# | 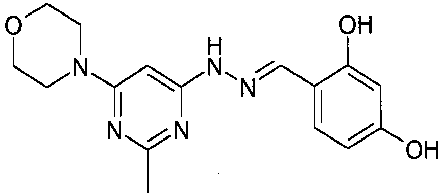 | C-R¹ | H | 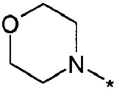 | -CH₃ | H | 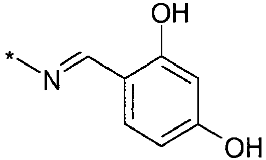 |
| 7# | 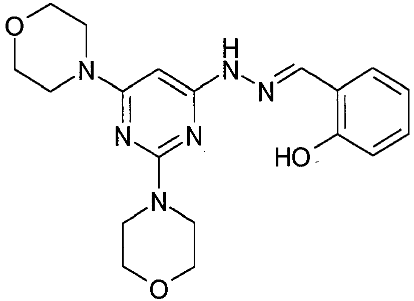 | C-R¹ | H | 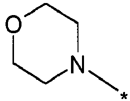 | 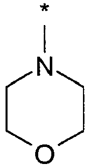 | H | 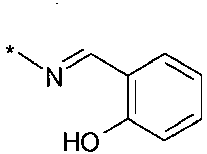 |
| 8# | 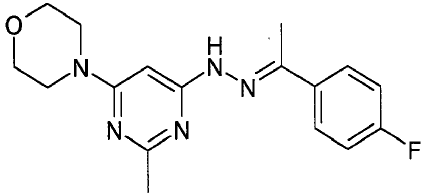 | C-R¹ | H | 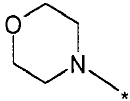 | -CH₃ | H | 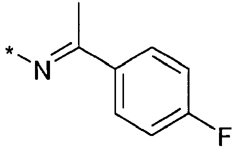 |
| 9 | 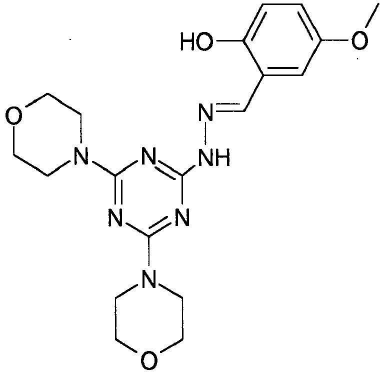 | N | - | 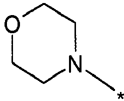 | 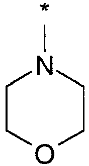 | H | 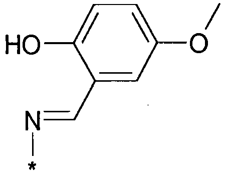 |
| 10 | 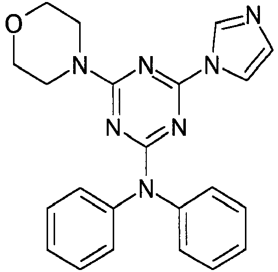 | N | - | 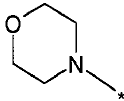 | 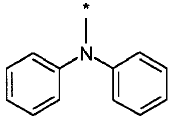 | 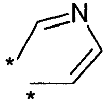 | |
| 11 | 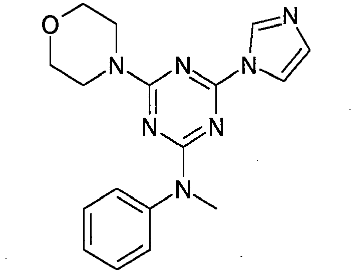 | N | - | 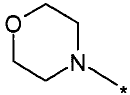 | 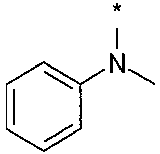 | 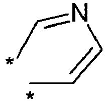 | |
| 12 | 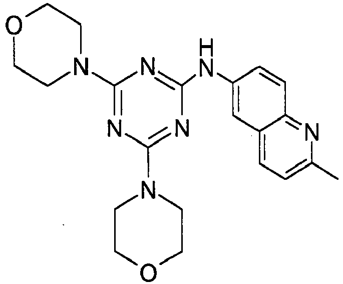 | N | - | 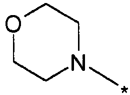 | 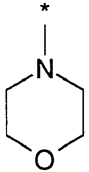 | H | 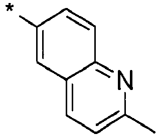 |
| 13 | 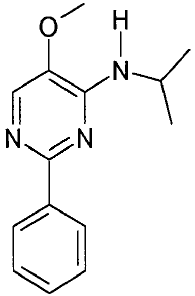 | CR¹ | -OCH₃ | H | Phenyl | H | 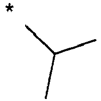 |
| 14 | 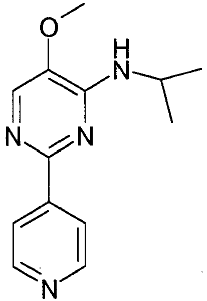 | CR¹ | -OCH₃ | H | 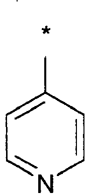 | H | 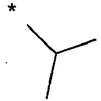 |
| 15 | 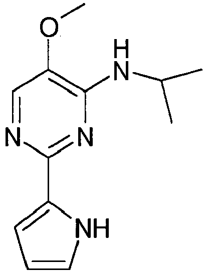 | CR¹ | -OCH₃ | H | 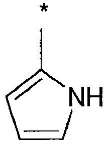 | H | 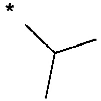 |
| 16 | 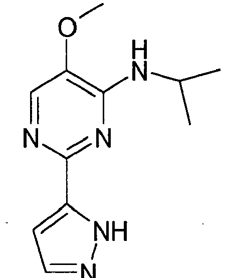 | CR¹ | -OCH₃ | H | 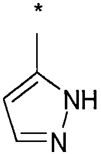 | H | 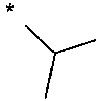 |
| 17 | 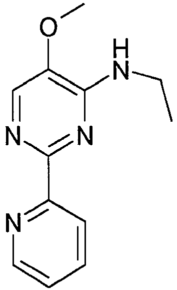 | CR¹ | -OCH₃ | H | 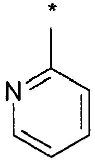 | H | 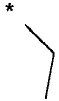 |
| 18 | 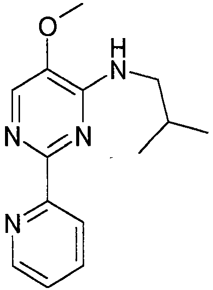 | CR¹ | -OCH₃ | H | 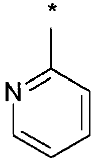 | H | 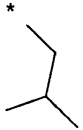 |
| 19 | 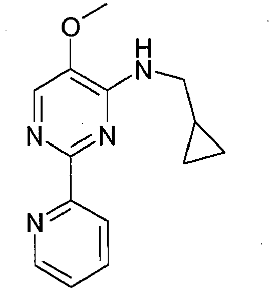 | CR¹ | -OCH₃ | H | 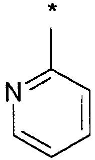 | H | 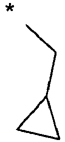 |
| 20 | 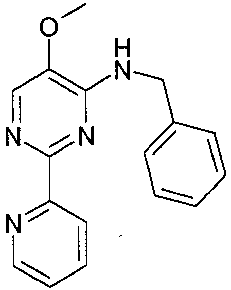 | CR¹ | -OCH₃ | H | 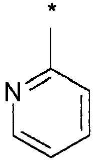 | H | 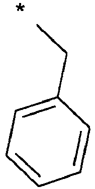 |
| 21 | 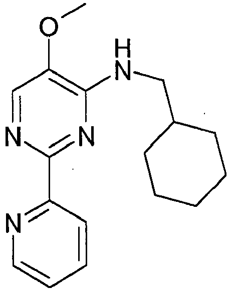 | CR¹ | -OCH₃ | H | 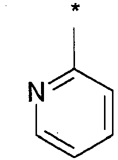 | H | 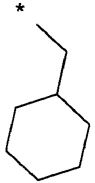 |
| 22 | 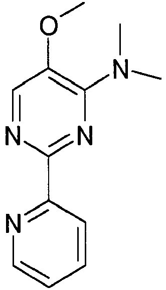 | CR¹ | -OCH₃ | H | 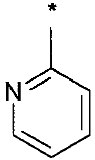 | -CH₃ | -CH₃ |
| 23 | 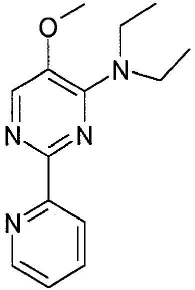 | CR¹ | -OCH₃ | H | 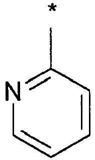 | Ethyl | Ethyl |
| 24 | 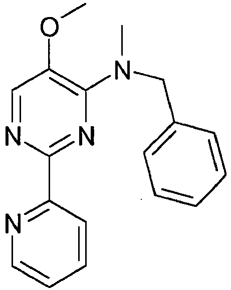 | CR¹ | -OCH₃ | H | 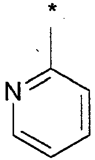 | Ethyl | Benzyl |
| 25 | 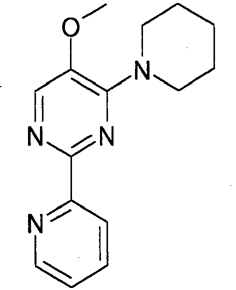 | CR¹ | -OCH₃ | H | 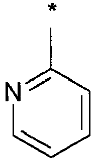 | 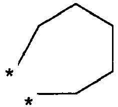 | |
| 26 | 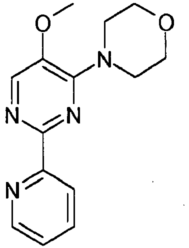 | CR¹ | -OCH₃ | H | 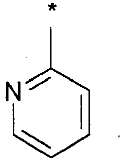 | 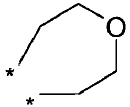 | |
| 27 | 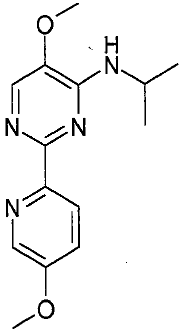 | CR¹ | -OCH₃ | H | 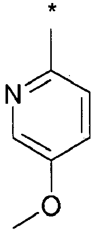 | H | 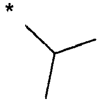 |
| 30 | 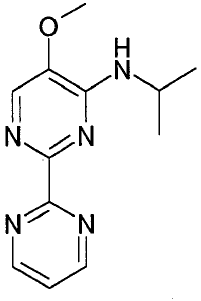 | CR¹ | -OCH₃ | H | 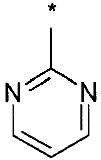 | H | 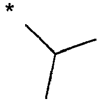 |
| 31 | 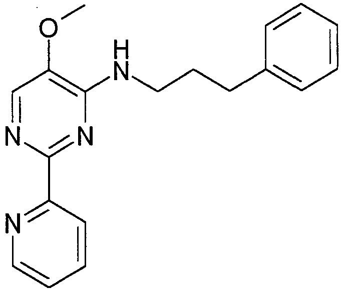 | CR¹ | -OCH₃ | H | 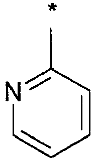 | H | 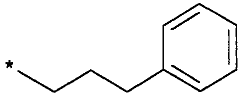 |
| 32 | 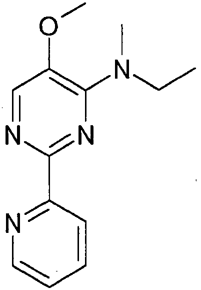 | CR¹ | -OCH₃ | H | 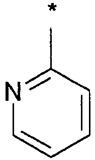 | -CH₃ | Ethyl |
| 33 | 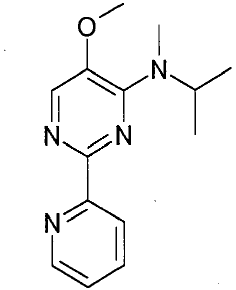 | CR¹ | -OCH₃ | H | 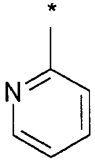 | -CH₃ | 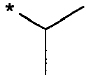 |
| 34 | 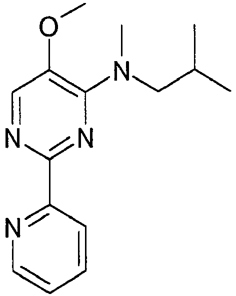 | CR¹ | -OCH₃ | H | 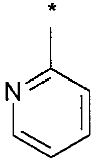 | -CH₃ | 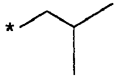 |
| 35 | 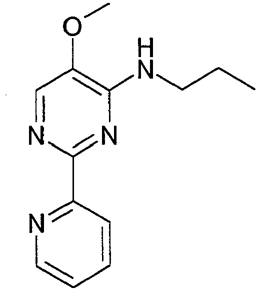 | CR¹ | -OCH₃ | H | 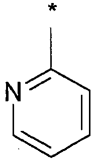 | H | 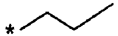 |
| 36 | 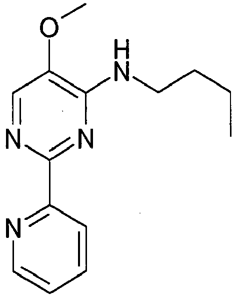 | CR¹ | -OCH₃ | H | 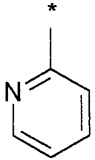 | H | 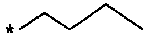 |
| 37 | 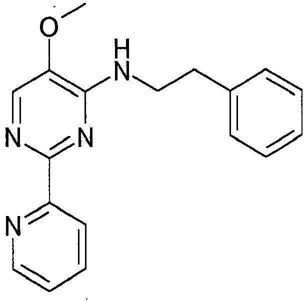 | CR¹ | -OCH₃ | H | 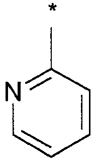 | H | 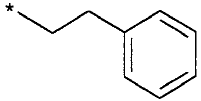 |
| 38 | 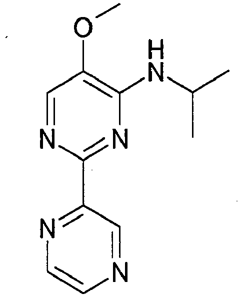 | CR¹ | -OCH₃ | H | 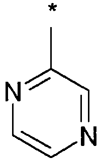 | H | 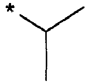 |
| 39 | 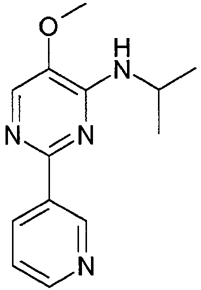 | CR¹ | -OCH₃ | H | 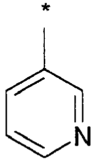 | H | 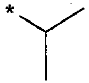 |
| 41 | 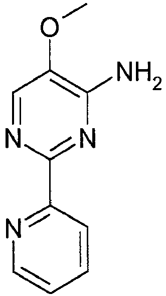 | CR¹ | -OCH₃ | H | 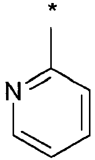 | H | H |
| 42 | 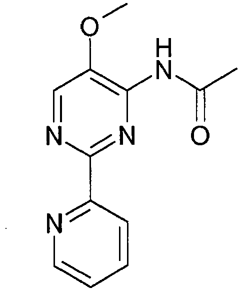 | CR¹ | -OCH₃ | H | 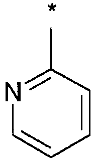 | H | 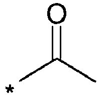 |
| 43 | 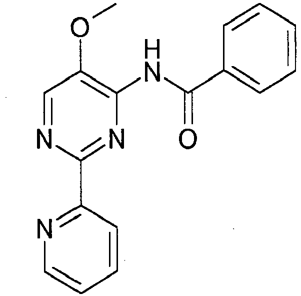 | CR¹ | -OCH₃ | H | 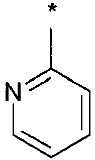 | H | 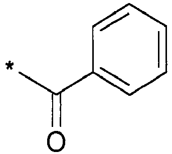 |
| 44 | 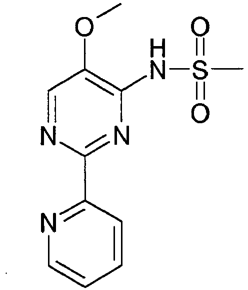 | CR¹ | -OCH₃ | H | 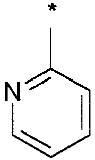 | H | 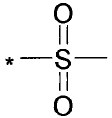 |
| 45 | 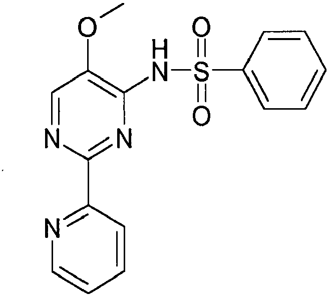 | CR¹ | -OCH₃ | H | 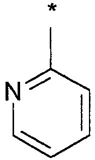 | H | 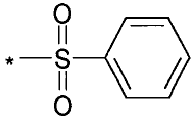 |
| 46 | 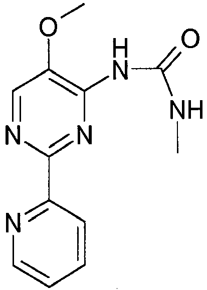 | CR¹ | -OCH₃ | H | 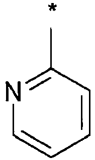 | H | 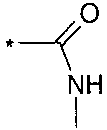 |
| 47 | 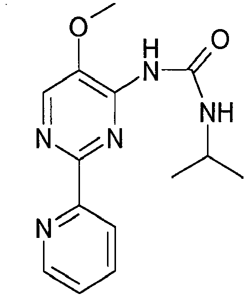 | CR¹ | -OCH₃ | H | 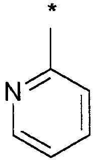 | H | 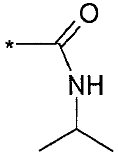 |
| 48 | 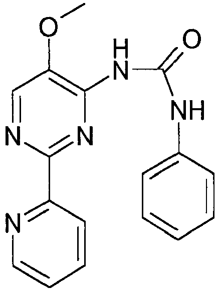 | CR¹ | -OCH₃ | H | 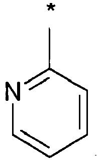 | H | 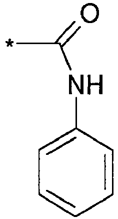 |
| 49 | 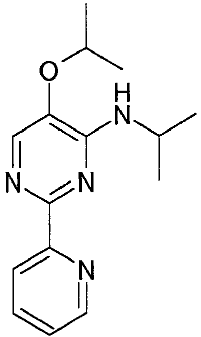 | CR¹ | 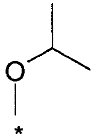 | H | 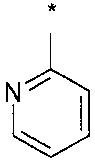 | H | 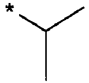 |
| 50 | 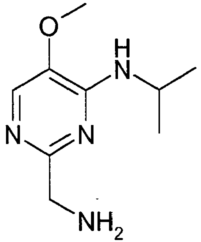 | CR¹ | -OCH₃ | H | 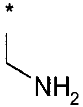 | H | 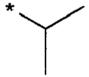 |
| 55 | 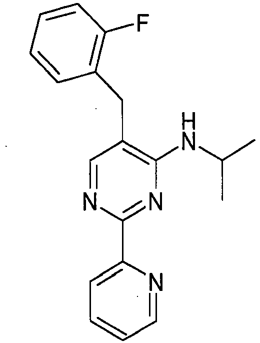 | CR¹ | 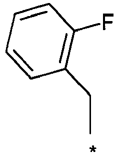 | H | 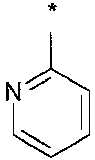 | H | 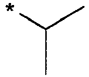 |
| 56 | 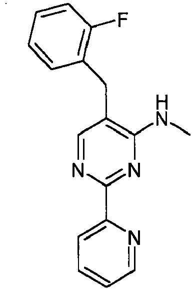 | CR¹ | 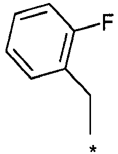 | H | 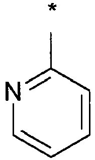 | H | -CH₃ |
| 57 | 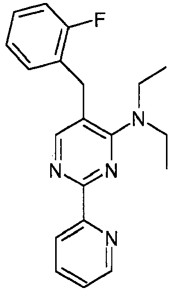 | CR¹ | 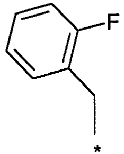 | H | 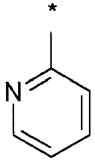 | Ethyl | Ethyl |
| 58 | 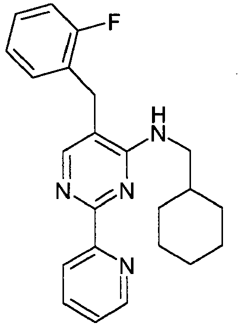 | CR¹ | 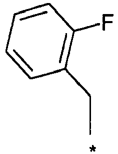 | H | 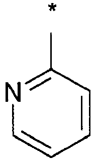 | H | 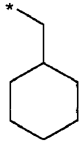 |
| 59 | 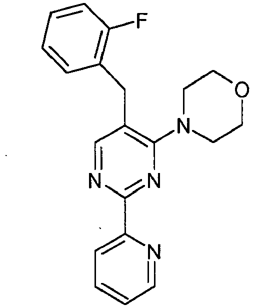 | CR¹ | 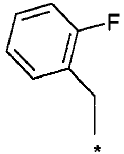 | H | 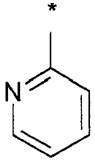 | 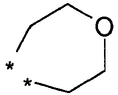 | |
| 60 | 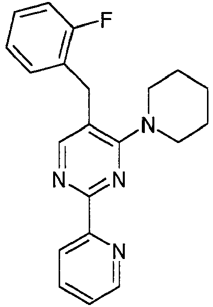 | CR¹ | 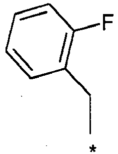 | H | 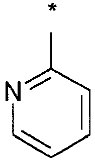 | 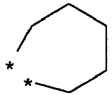 | |
| 61 | 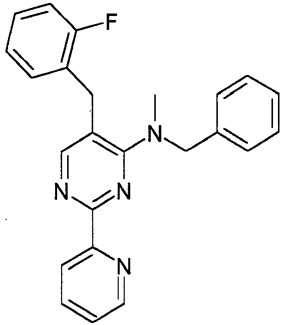 | CR¹ | 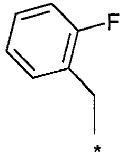 | H | 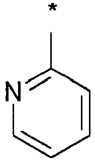 | -CH₃ | Benzyl |
| 62 | 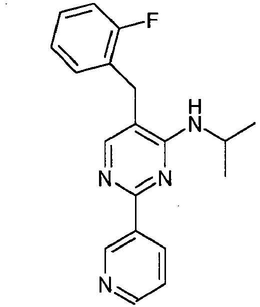 | CR¹ | 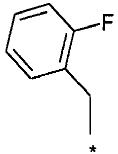 | H | 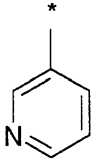 | H | 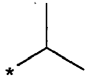 |
| 63 | 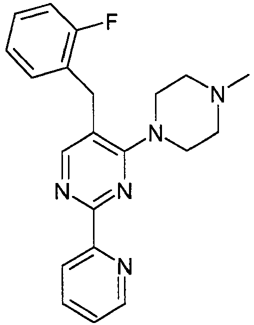 | CR¹ | 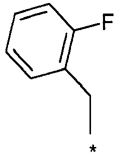 | H | 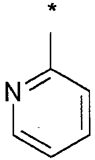 | 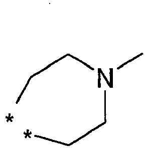 | |
| 69 | 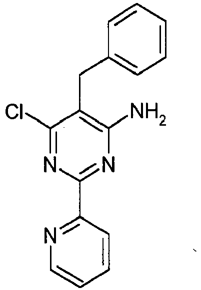 | CR¹ | 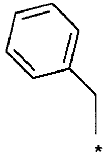 | Cl | 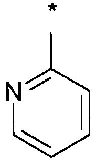 | H | H |
| 70 | 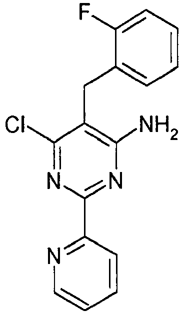 | CR¹ | 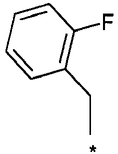 | Cl | 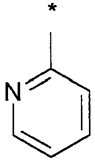 | H | H |
| 71 | 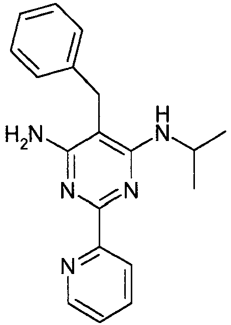 | CR¹ | 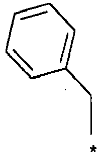 | -NH₂ | 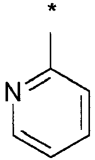 | H | 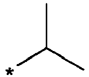 |
| 72 | 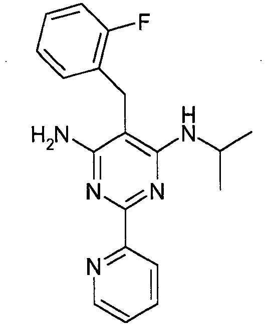 | CR¹ | 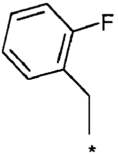 | -NH₂ | 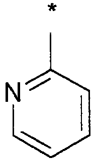 | H | 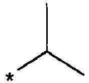 |
| 73 | 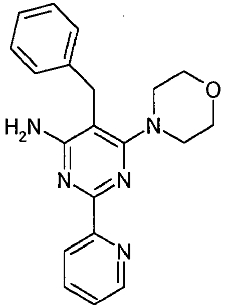 | CR¹ | 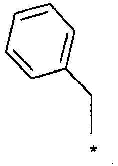 | -NH₂ | 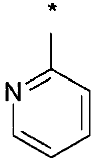 | 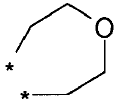 | |
| 74 | 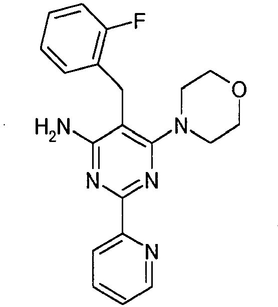 | CR¹ | 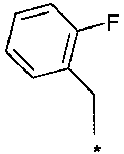 | -NH₂ | 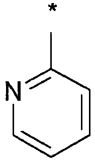 | 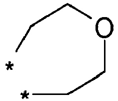 | |
| 75 | 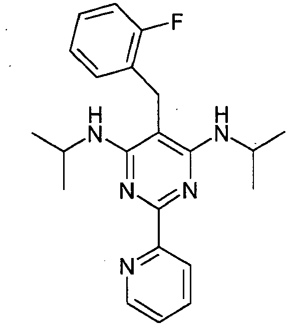 | CR¹ | 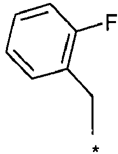 | 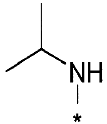 | 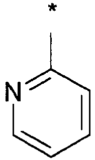 | H | 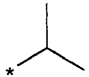 |
| 76 | 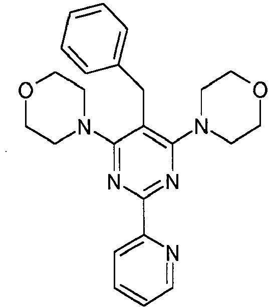 | CR¹ | 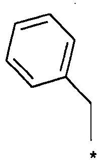 | 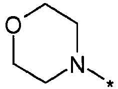 | 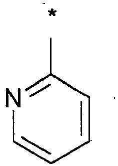 | 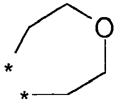 | |
| 77 | 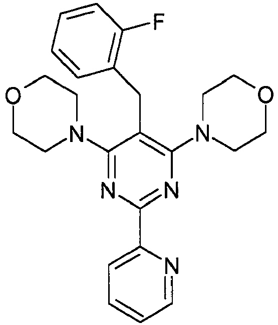 | CR¹ | 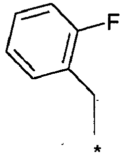 | 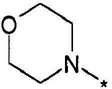 | 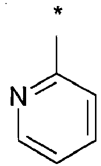 | 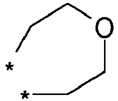 | |
| 78 | 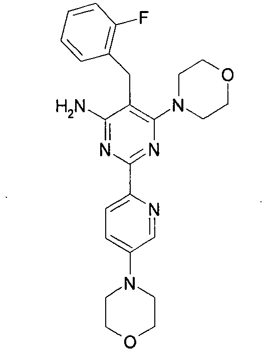 | CR¹ | 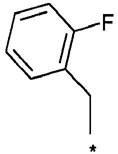 | -NH₂ | 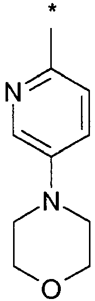 | 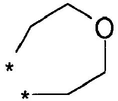 | |
| 79 | 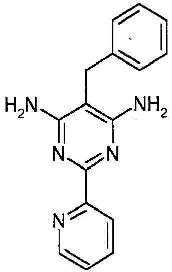 | CR¹ | 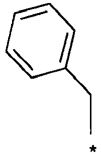 | -NH₂ | 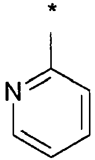 | H | H |
| 80 | 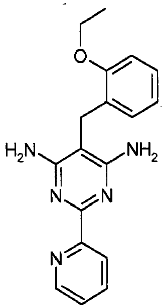 | CR¹ | 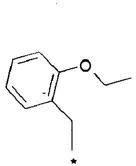 | -NH₂ | 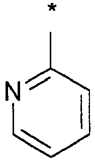 | H | H |
| 81 | 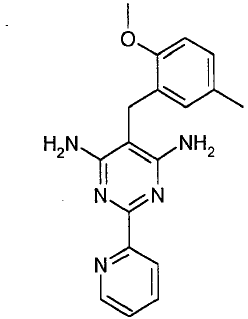 | CR¹ | 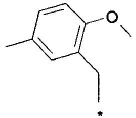 | -NH₂ | 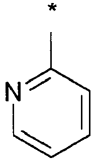 | H | H |
| 82 | 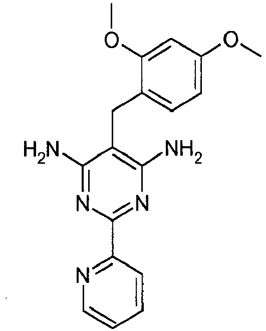 | CR¹ | 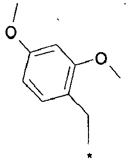 | -NH₂ | 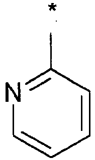 | H | H |
| 83 | 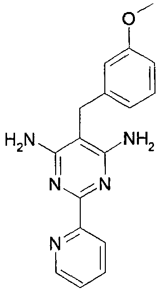 | CR¹ | 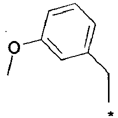 | -NH₂ | 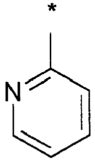 | H | H |
| 84 | 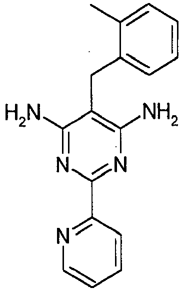 | CR¹ | 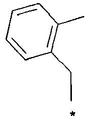 | -NH₂ | 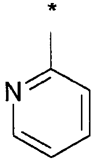 | H | H |
| 85 | 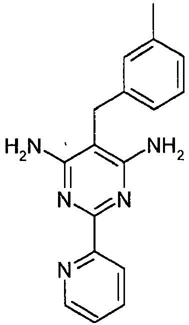 | CR¹ | 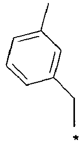 | -NH₂ | 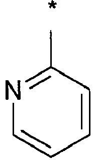 | H | H |
| 86 | 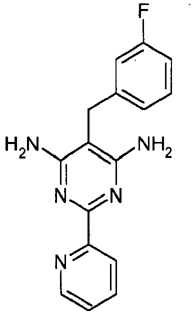 | CR¹ | 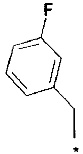 | -NH₂ | 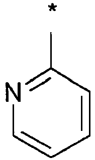 | H | H |
| 87 | 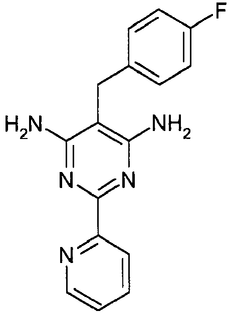 | CR¹ | 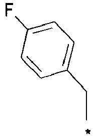 | -NH₂ | 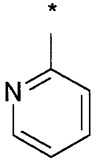 | H | H |
| 88 | 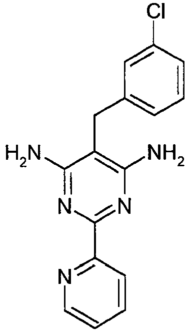 | CR¹ | 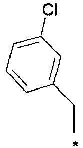 | -NH₂ | 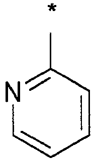 | H | H |
| 89 | 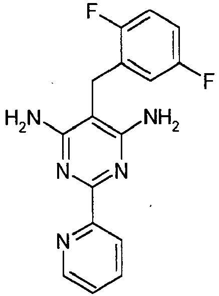 | CR¹ | 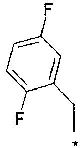 | -NH₂ | 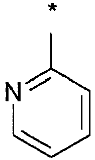 | H | H |
| 90 | 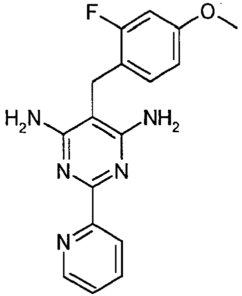 | CR¹ | 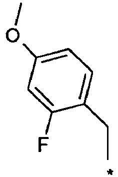 | -NH₂ | 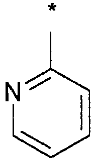 | H | H |
| 91 | 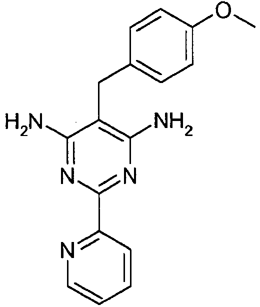 | CR¹ | 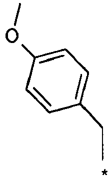 | -NH₂ | 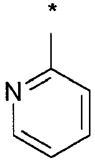 | H | H |
| 92 | 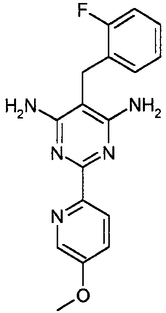 | CR¹ | 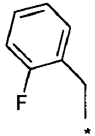 | -NH₂ | 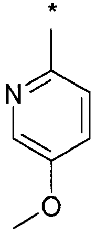 | H | H |
| 93 | 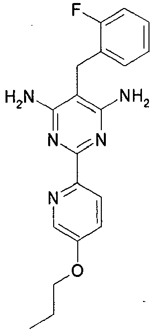 | CR¹ | 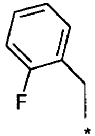 | -NH₂ | 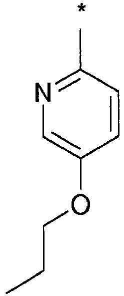 | H | H |
| 94 | 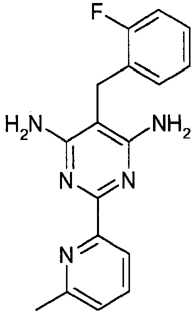 | CR¹ | 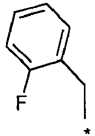 | -NH₂ | 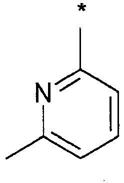 | H | H |
| 95 | 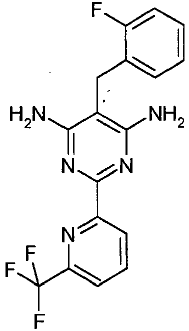 | CR¹ | 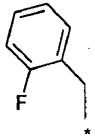 | -NH₂ | 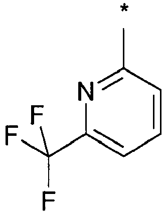 | H | H |
| 97 | 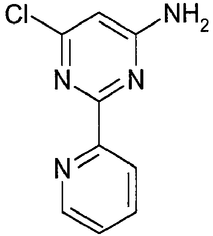 | CR¹ | H | -Cl | 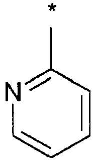 | H | H |
| 98 | 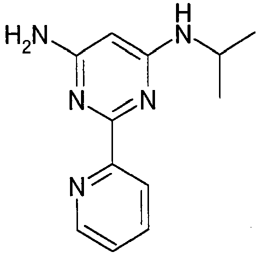 | CR¹ | H | -NH₂ | 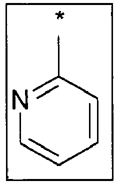 | H | 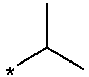 |
| 99 | 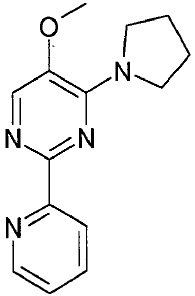 | CR¹ | -OCH₃ | H | 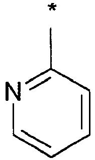 | 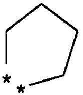 | |
| 100 | 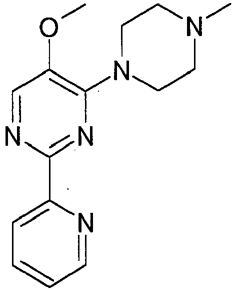 | CR¹ | -OCH₃ | H | 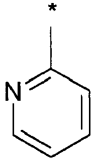 | 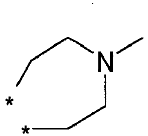 | |
| 101 | 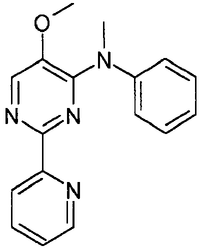 | CR¹ | -OCH₃ | H | 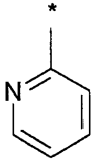 | -CH₃ | Phenyl |
| 103 | 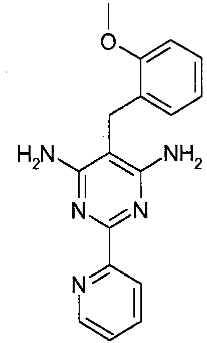 | C-R¹ | 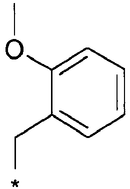 | -NH₂ | 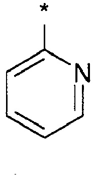 | H | H |
| 104 | 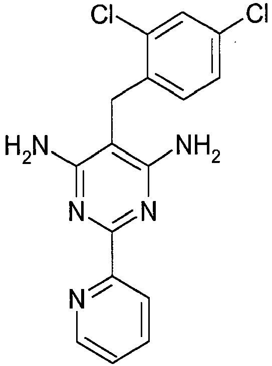 | C-R¹ | 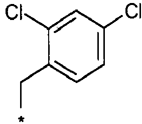 | -NH₂ | 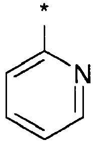 | H | H |
| 105 | 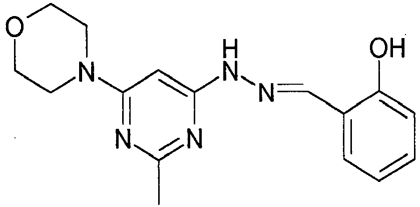 | C-R¹ | H | 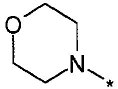 | -CH₃ | H | 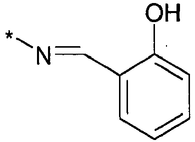 |
| 106 | 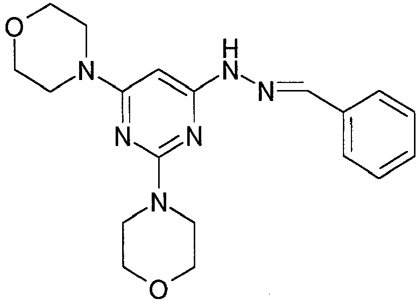 | C-R¹ | H | 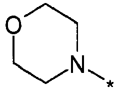 | 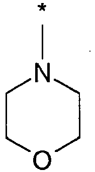 | H | 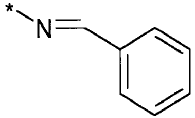 |
| 107 | 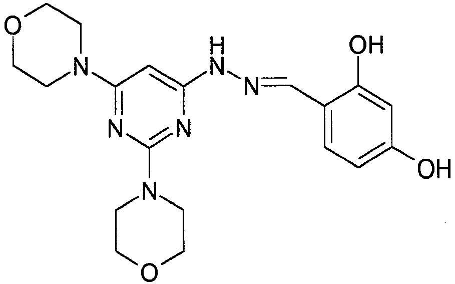 | C-R¹ | H | 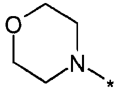 | 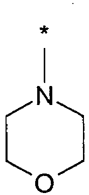 | H | 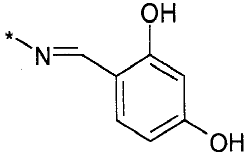 |
| 108 | 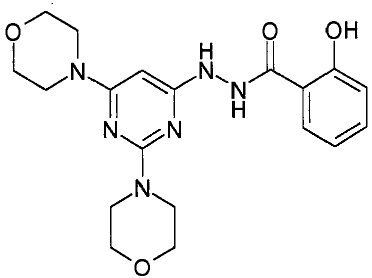 | C-R¹ | H | 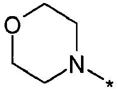 | 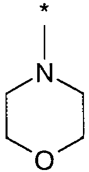 | H | 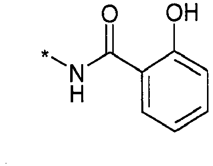 |
| 109 | 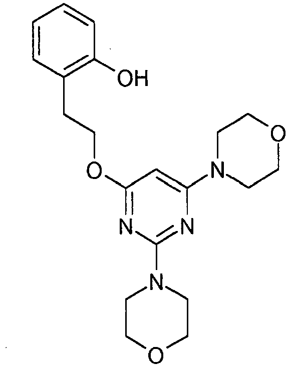 | C-R¹ | H | 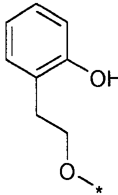 | 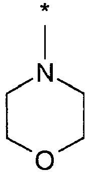 | 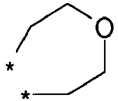 | |
| 110 | 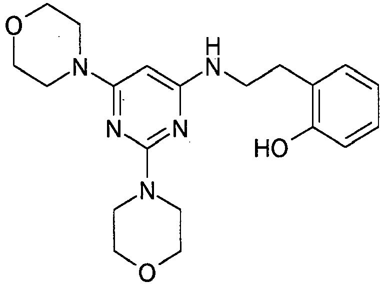 | C-R¹ | H | 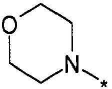 | 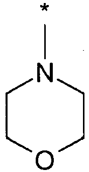 | H | 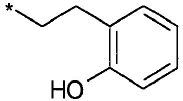 |
| 111 | 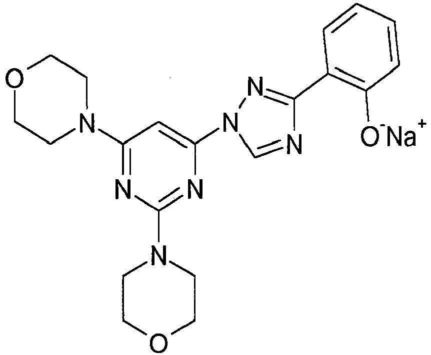 | C-R¹ | H | 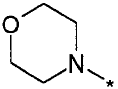 | 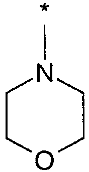 | 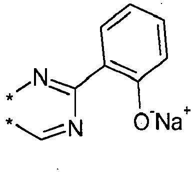 | |
| 112 | 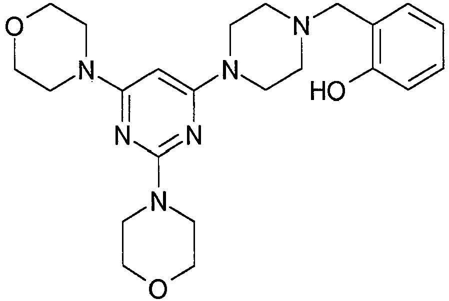 | C-R¹ | H | 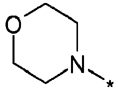 | 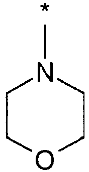 | 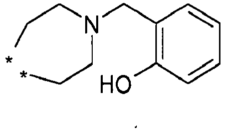 | |
| 113 | 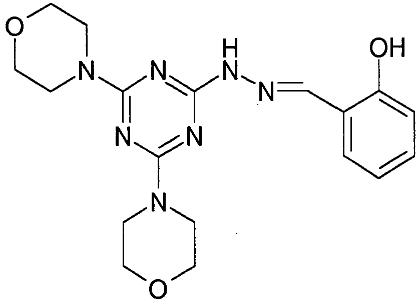 | N | - | 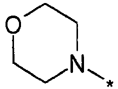 | 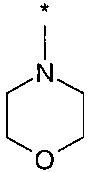 | H | 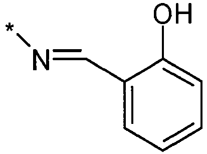 |
| 114 | 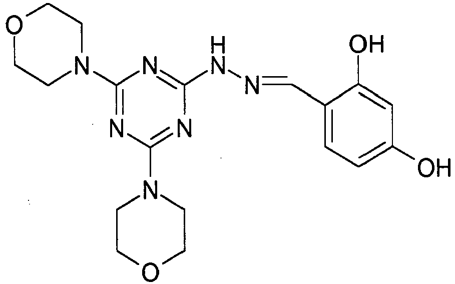 | N | - | 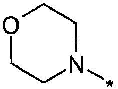 | 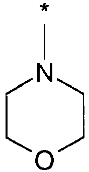 | H | 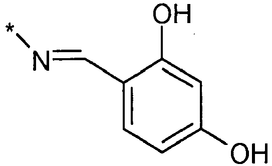 |
| 115 | 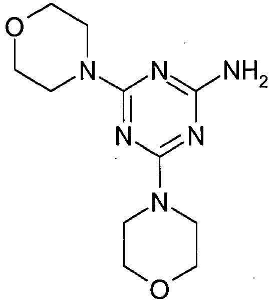 | N | - | 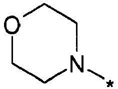 | 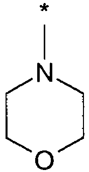 | H | H |
| 116 | 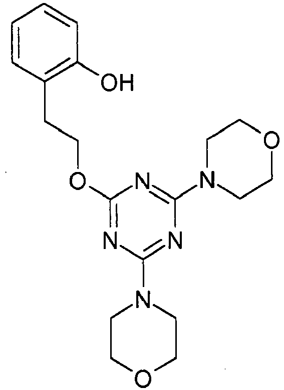 | N | - | 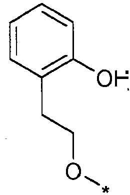 | 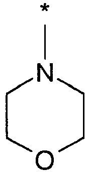 | 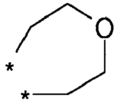 | |
| 117 | 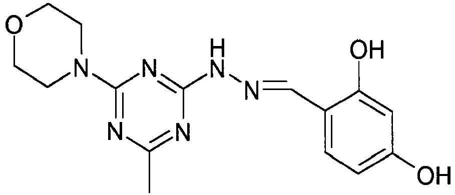 | N | - | 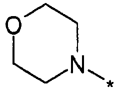 | -CH₃ | H | 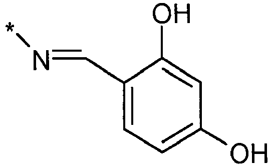 |

| X | R¹ | R² | R³ | | | Y | |
|---|---|---|---|---|---|---|---|
| 28 | 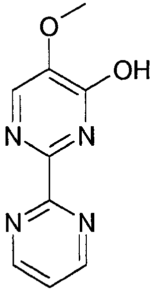 | C-R¹ | -OCH₃ | H | 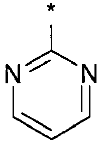 | -OH | |
| 29 | 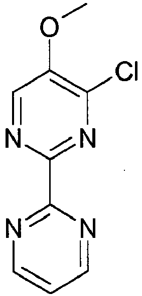 | C-R¹ | -OCH₃ | H | 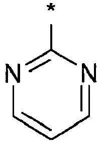 | -Cl | |
| 40 | 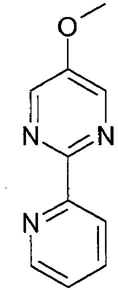 | C-R¹ | -OCH₃ | H | 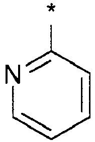 | -H | |
| 51 | 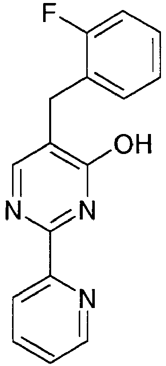 | C-R¹ | 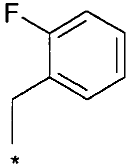 | H | 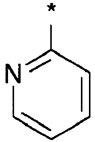 | -OH | |
| 52 | 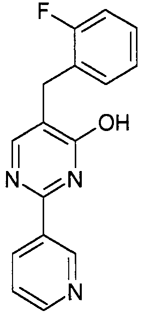 | C-R¹ | 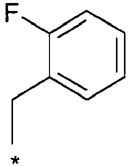 | H | 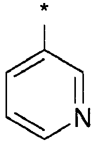 | -OH | |
| 53 | 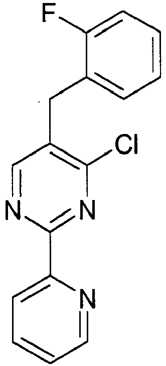 | C-R¹ | 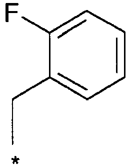 | H | 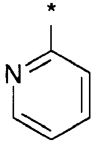 | -Cl | |
| 54 | 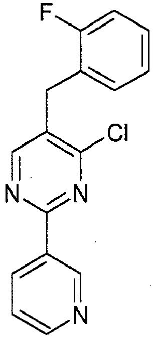 | C-R¹ | 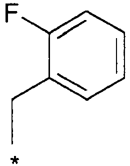 | H | 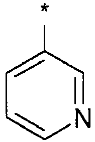 | -Cl | |
| 64 | 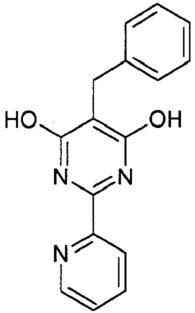 | C-R¹ | 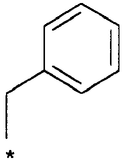 | -OH | 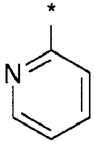 | -OH | |
| 65 | 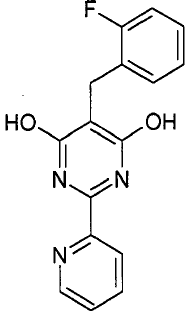 | C-R¹ | 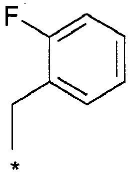 | -OH | 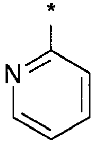 | -OH | |
| 66 | 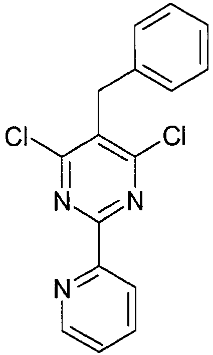 | C-R¹ | 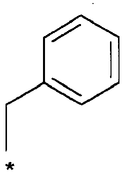 | -Cl | 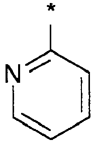 | -Cl | |
| 67 | 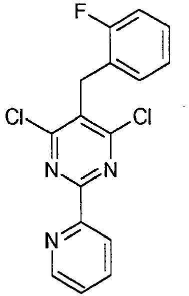 | C-R¹ | 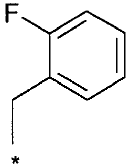 | -Cl | 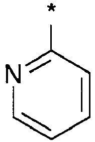 | -Cl | |
| 68 | 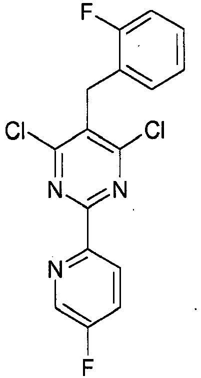 | C-R¹ | 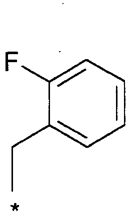 | -Cl | 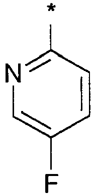 | -Cl | |
| 96 | 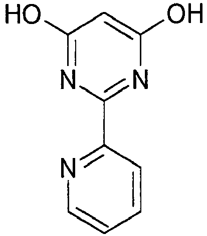 | C-R¹ | -H | -OH | 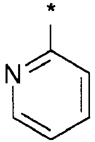 | -OH | |
| 102 | 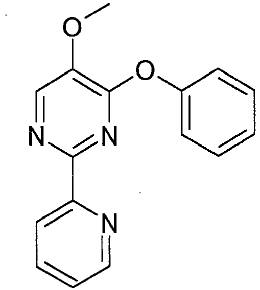 | C-R¹ | -OCH₃ | H | 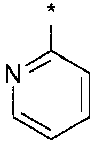 | -O-Phenyl | |

| | | | | | | | |
|---|---|---|---|---|---|---|---|
| (* = Bindungsposition) | | | | | | | |

sowie pharmazeutisch verträgliche Salze hiervon.

Darin bezeichnen die mit "#" markierten Verbindungen erfindungsgemäße Beispielverbindungen.

Die erfindungsgemäßen Verbindungen können in Abhängigkeit von ihrer Struktur bei Vorliegen asymmetrischer Kohlenstoffatome in stereoisomeren Formen (Enantiomere, Diastereomere) existieren. Die Erfindung umfasst deshalb die Verwendung der Enantiomeren oder Diastereomeren und ihrer jeweiligen Mischungen. Die enantiomerenreinen Formen können gegebenenfalls durch übliche Verfahren der optischen Auflösung, wie durch fraktionierte Kristallisation von Diastereomeren daraus durch Umsetzung mit optisch aktiven Verbindungen erhalten werden. Sofern die erfindungsgemäßen Verbindungen in tautomeren Formen vorkommen können, umfasst die vorliegende Erfindung die Verwendung sämtlicher tautomerer Formen.

Die erfindungsgemäß vorgesehenen Verbindungen können als Mischungen verschiedener möglicher isomerer Formen, insbesondere von Stereoisomeren, wie z.B. E- und Z-, syn und anti, sowie optischen Isomeren vorliegen. Es werden sowohl die E- als auch die Z-Isomeren, sowie die optischen Isomeren, sowie beliebige Mischungen dieser Isomeren beansprucht.

Die erfindungsgemäßen Verbindungen der allgemeinen Strukturformel (I) können grundsätzlich durch die nachfolgend erläuterten Verfahren sowie den im experimentellen Teil gezeigten allgemeinen Reaktionsschemata (siehe beispielsweise entsprechende Stufen bei den Routen 1 bis 20 der Herstellungsbeispiele 13 bis 104, die entsprechenden Stufen bei den Routen 1 bis 7 der Herstellungsbeispiele 105 bis 112, sowie die entsprechenden Stufen bei den Routen 1 bis 5 der Herstellungsbeispiele 113 bis 117), gewonnen werden:
Verfahren, worin
   (a1) Verbindungen der allgemeinen Formel 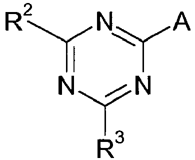 worin R² und R³ wie oben definiert sind, A eine Abgangsgruppe, wie insbesondere Halogen, bevorzugt Chlor ist, mit einer Verbindung der allgemeinen Formel 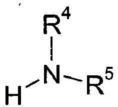 worin R⁴ und R⁵ wie oben definiert sind,
      zu Verbindungen der allgemeinen Formel (Ia) umgesetzt werden: 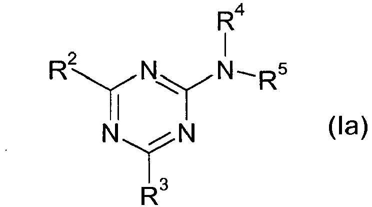 worin R², R³, R⁴ und R⁵ wie oben definiert sind, (siehe beispielsweise entsprechende Stufen bei den Routen 1, 2, 3, 4, 6, 7, 10, 12, 13, 14, 15, 16, 19, 20 der Herstellungsbeispiele 13 bis 104 sowie entsprechende Stufen bei den Routen 1, 2, 3 der Herstellungsbeispiele 105 bis 112 sowie die entsprechenden Stufen bei den Routen 1, 2, 3, 4, 5 der Herstellungsbeispiele 113 bis 117), oder
   (a2) Verbindungen der allgemeinen Formel 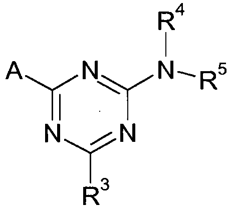 worin R³, R⁴ und R⁵ wie oben definiert sind, A eine Abgangsgruppe, wie insbesondere Halogen, bevorzugt Chlor ist, mit einer Verbindung der allgemeinen Formel

      R²-E

      worin R² wie oben definiert ist, und E hier und im Folgenden im Rahmen der gesamten Erfindung, eine geeignete Gruppe oder ein geeignetes Element ist, welches R² zu einem Nucleophil macht, wie zum Beispiel H (insbesondere wenn R eine Aminogruppe ist), Metalle (insbesondere wenn R ein Kohlenwasserstoffrest ist), wie insbesondere Alkalimetalle, wie Lithium, Natrium und Kalium, Erdalkalimetalle, wie Calcium oder Magnesium, -MgBr (Grignard-Verbindungen), welche die nukleophile Substitution von A durch R² ermöglichen,
      zu Verbindungen der allgemeinen Formel (Ia), wie oben definiert, umgesetzt werden (siehe beispielsweise entsprechende Stufen bei den Routen 1, 2, 3, 4, 6, 7, 10, 12, 13, 14, 15, 16, 19, 20 der Herstellungsbeispiele 13 bis 104 sowie entsprechende Stufen bei den Routen 1, 2, 3 der Herstellungsbeispiele 105 bis 112 sowie die entsprechenden Stufen bei den Routen 1, 2, 3, 4, 5 der Herstellungsbeispiele 113 bis 117), oder
   (a3) Verbindungen der allgemeinen Formel 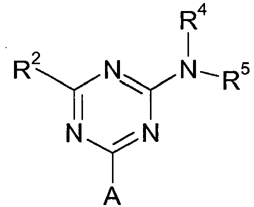 worin R², R⁴ und R⁵ wie oben definiert sind, A eine Abgangsgruppe, wie insbesondere Halogen, bevorzugt Chlor ist, mit einer Verbindung der allgemeinen Formel

      R³-E

      worin R³ wie oben definiert ist, und E eine geeignete Abgangsgruppe, wie zuvor definiert, ist, welche die Substitution von A durch R³ ermöglicht,
      zu Verbindungen der allgemeinen Formel (Ia), wie oben definiert, umgesetzt werden (siehe beispielsweise entsprechende Stufen bei den Routen 1, 2, 3, 4, 6, 7, 10, 12, 13, 14, 15, 16, 19, 20 der Herstellungsbeispiele 13 bis 104 sowie entsprechende Stufen bei den Routen 1, 2, 3 der Herstellungsbeispiele 105 bis 112 sowie die entsprechenden Stufen bei den Routen 1, 2, 3, 4, 5 der Herstellungsbeispiele 113 bis 117), oder
   (a4) Verbindungen der allgemeinen Formel 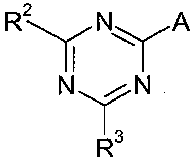 worin R² und R³ wie oben definiert sind, A eine Abgangsgruppe, wie insbesondere Halogen, bevorzugt Chlor ist, mit

      H₂N-NH₂

      zu einer Verbindung der allgemeinen Formel 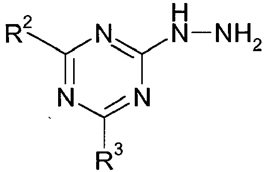 worin R² und R³ wie oben definiert sind, umgesetzt werden, die nachfolgend mit einer Verbindung der Formel 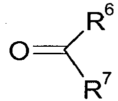 worin R⁶ und R⁷ gleich oder verschieden sind und ausgewählt werden aus:
      - Wasserstoff,
      - gegebenenfalls substituiertem Alkyl,
      - gegebenenfalls substituiertem Alkenyl,
      - gegebenenfalls substituiertem Alkinyl,
      - gegebenenfalls substituiertem Aryl, oder
      - gegebenenfalls substituiertem Heterocyclyl,
      zu Verbindungen der Formel 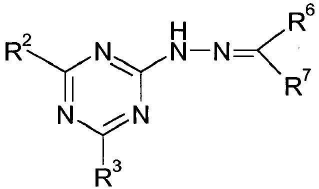 worin R², R³, R⁶ und R⁷ wie oben definiert sind, umgesetzt werden (siehe beispielsweise entsprechende Stufen bei den Routen 1, 2, 3 der Herstellungsbeispiele 105 bis 112), oder
   (a5) Verbindungen der Formel 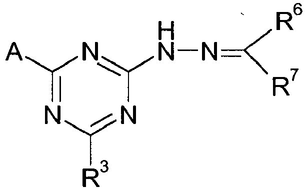 worin A, R³, R⁶ und R⁷ wie oben definiert sind, mit Verbindungen der Formel
      R²-E, worin R² wie oben definiert ist und E eine geeignete Abgangsgruppe, wie zuvor definiert, ist, welche die Substitution von A durch R² ermöglicht, zu Verbindungen der Formel 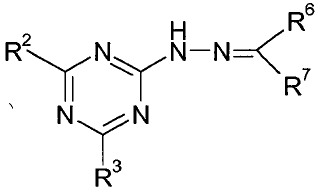 worin R², R³, R⁶ und R⁷ wie oben definiert sind, umgesetzt werden, oder
   (a6) Verbindungen der Formel 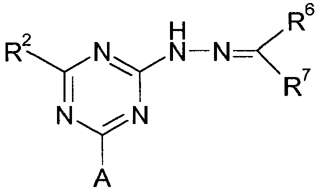
      worin A, R², R⁶ und R⁷ wie oben definiert sind, mit Verbindungen der Formel
      R³-E, worin R³ wie oben definiert ist und E, wie zuvor definiert, eine geeignete Abgangsgruppe ist, welche die Substitution von A durch R³ ermöglicht, zu Verbindungen der Formel 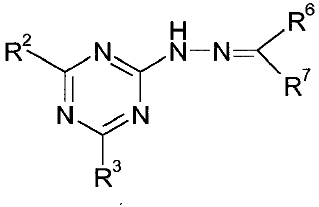
      worin R², R³, R⁶ und R⁷ wie oben definiert sind, umgesetzt werden, oder
   (b1) Verbindungen der allgemeinen Formel 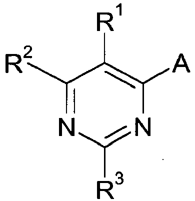 worin R¹, R² und R³ wie oben definiert sind, A eine Abgangsgruppe, wie insbesondere Halogen, bevorzugt Chlor ist, mit einer Verbindung der allgemeinen Formel 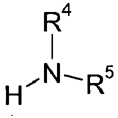 worin R⁴ und R⁵ wie oben definiert sind,
      zu Verbindungen der allgemeinen Formel (Ib) umgesetzt werden: 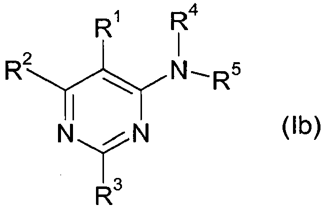 worin R¹, R², R³, R⁴ und R⁵ wie oben definiert sind, (siehe beispielsweise entsprechende Stufen bei den Routen 1, 2, 3, 4, 6, 7, 10, 12, 13, 14, 15, 16, 19, 20 der Herstellungsbeispiele 13 bis 104 sowie entsprechende Stufen bei den Routen 1, 2, 3 der Herstellungsbeispiele 105 bis 112 sowie die entsprechenden Stufen bei den Routen 1, 2, 3, 4, 5 der Herstellungsbeispiele 113 bis 117), oder
   (b2) Verbindungen der allgemeinen Formel 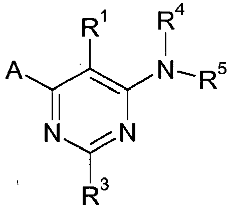 worin R¹, R³, R⁴ und R⁵ wie oben definiert sind, A eine Abgangsgruppe, wie insbesondere Halogen, bevorzugt Chlor ist, mit einer Verbindung der allgemeinen Formel

      R²-E

      worin R² wie oben definiert ist und E eine geeignete Abgangsgruppe, wie zuvor definiert, ist, welche die Substitution von A durch R² ermöglicht,
      zu Verbindungen der allgemeinen Formel (Ib), wie oben definiert, umgesetzt werden (siehe beispielsweise entsprechende Stufen bei den Routen 1, 2, 3, 4, 6, 7, 10, 12, 13, 14, 15, 16, 19, 20 der Herstellungsbeispiele 13 bis 104 sowie entsprechende Stufen bei den Routen 1, 2, 3 der Herstellungsbeispiele 105 bis 112 sowie die entsprechenden Stufen bei den Routen 1, 2, 3, 4, 5 der Herstellungsbeispiele 113 bis 117), oder
   (b3) Verbindungen der allgemeinen Formel 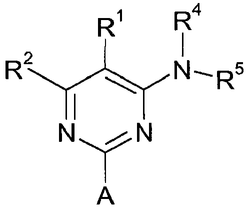 worin R¹, R², R⁴ und R⁵ wie oben definiert sind, A eine Abgangsgruppe, wie insbesondere Halogen, bevorzugt Chlor ist, mit einer Verbindung der allgemeinen Formel

      R³-E

      worin R³ wie oben definiert ist und E eine geeignete Abgangsgruppe ist, wie zuvor definiert, welche die Substitution von A durch R³ ermöglicht,
      zu Verbindungen der allgemeinen Formel (Ib), wie oben definiert, umgesetzt werden (siehe beispielsweise entsprechende Stufen bei den Routen 1, 2, 3, 4, 6, 7, 10, 12, 13, 14, 15, 16, 19, 20 der Herstellungsbeispiele 13 bis 104 sowie entsprechende Stufen bei den Routen 1, 2, 3 der Herstellungsbeispiele 105 bis 112 sowie die entsprechenden Stufen bei den Routen 1, 2, 3, 4, 5 der Herstellungsbeispiele 113 bis 117), oder
   (b4) Verbindungen der allgemeinen Formel 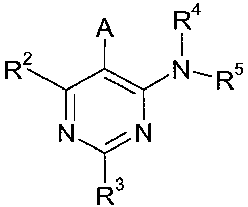 worin R², R³, R⁴ und R⁵ wie oben definiert sind, A eine Abgangsgruppe, wie insbesondere Halogen, bevorzugt Chlor ist, mit einer Verbindung der allgemeinen Formel

      R¹-E

      worin R¹ wie oben definiert ist und E eine geeignete Abgangsgruppe, wie zuvor definiert, ist, welche die Substitution von A durch R¹ ermöglicht,
      zu Verbindungen der allgemeinen Formel (Ib), wie oben definiert, umgesetzt werden (siehe beispielsweise entsprechende Stufen bei den Routen 1, 2, 3, 4, 6, 7, 10, 12, 13, 14, 15, 16, 19, 20 der Herstellungsbeispiele 13 bis 104 sowie entsprechende Stufen bei den Routen 1, 2, 3 der Herstellungsbeispiele 105 bis 112 sowie die entsprechenden Stufen bei den Routen 1, 2, 3, 4, 5 der Herstellungsbeispiele 113 bis 117), oder
   (b5) Verbindungen der allgemeinen Formel 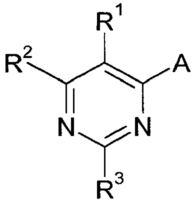 worin R¹, R² und R³ wie oben definiert sind, A eine Abgangsgruppe, wie insbesondere Halogen, bevorzugt Chlor ist, mit

      H₂N-NH₂

      zu Verbindungen der allgemeinen Formel 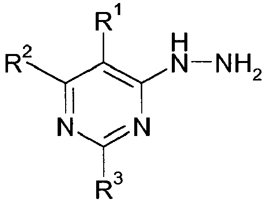 worin R¹, R² und R³ wie oben definiert sind, umgesetzt werden, die nachfolgend mit einer Verbindung der Formel 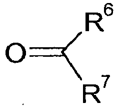 worin R⁶ und R⁷ gleich oder verschieden sind und wie vorstehend definiert sind, zu Verbindungen der Formel 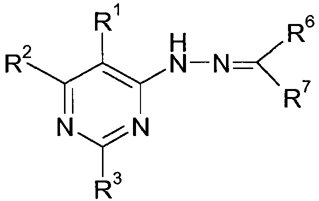 worin R¹, R², R³, R⁶ und R⁷ wie oben definiert sind, umgesetzt werden (siehe beispielsweise entsprechende Stufen bei den Routen 1, 2, 3 der Herstellungsbeispiele 105 bis 112), oder
   (b6) Verbindungen der Formel 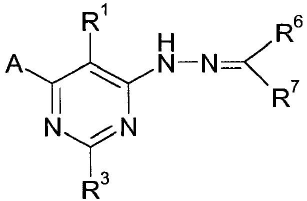 worin A, R¹, R³, R⁶ und R⁷ wie oben definiert sind, mit Verbindungen der Formel
      R²-E, worin R² wie oben definiert ist und E eine geeignete Abgangsgruppe, wie zuvor definiert, ist, welche die Substitution von A durch R² ermöglicht, zu Verbindungen der Formel 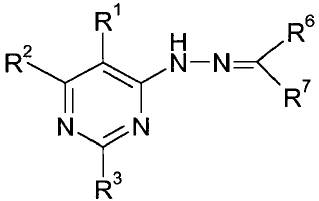 worin R¹, R², R³, R⁶ und R⁷ wie oben definiert sind, umgesetzt werden, oder
   (b7) Verbindungen der Formel 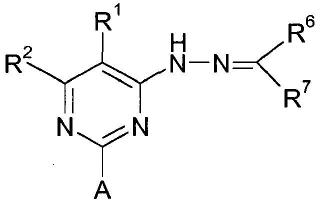 worin A, R¹, R², R⁶ und R⁷ wie oben definiert sind, mit Verbindungen der Formel
      R³-E, worin R³ wie oben definiert ist und E eine geeignete Abgangsgruppe, wie zuvor definiert, ist, welche die Substitution von A durch R³ ermöglicht, zu Verbindungen der Formel 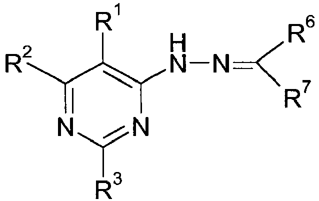 worin R¹, R², R³, R⁶ und R⁷ wie oben definiert sind, umgesetzt werden, oder
   (b8) Verbindungen der Formel 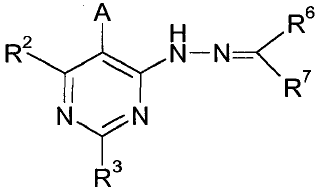 worin A, R², R⁶ und R⁷ wie oben definiert sind, mit Verbindungen der Formel
      R¹-E, worin R¹ wie oben definiert ist und E eine geeignete Abgangsgruppe, wie zuvor definiert, ist, welche die Substitution von A durch R¹ ermöglicht, zu Verbindungen der Formel 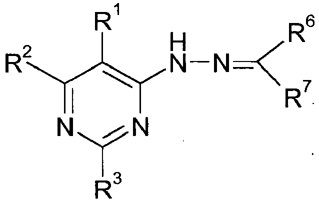 worin R¹, R², R³, R⁶ und R⁷ wie oben definiert sind, umgesetzt werden.

Insbesondere können erfindungsgemäße Verbindungen der allgemeinen Formel (I), durch die nachfolgend erläuterten Verfahren gewonnen werden.

Ausgangspunkt für die Synthese von Verbindungen der allgemeinen Formel (I), worin X für C-R¹ steht und worin R¹ ausgewählt ist aus der Gruppe die Alkoxy, Halogen, gegebenenfalls substituiertes Alkyl, gegebenenfalls substituiertes Aryl oder gegebenenfalls substituiertes Heterocyclyl umfasst, und worin R², R³, R⁴ und R⁵ eine der vorstehenden Bedeutungen aufweisen, ist das käufliche Alkylimidamid, der allgemeinen Formel (II), welches unter Standardbedingungen [s. z.B.: Henze et al, JOC, 17, 1952, 1320-1322; R. Ferris, JACS, 62, 1940, 606; S. Biggs, Journal of the Chemistry Society, 1959, 1849-1854] mit 1,3-Diketoverbindungen, der allgemeinen Formel (III) zum Pyrimidinon, der allgemeinen Formel (IV) cyclisiert werden kann.

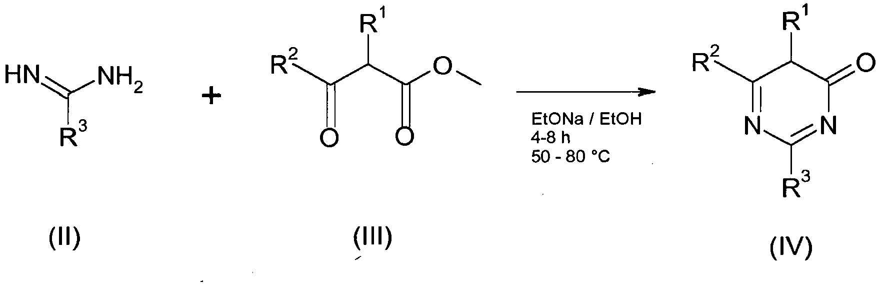

Durch anschließende Behandlung der Pyrimidinone der allgemeinen Formel (IV) mit Phosphorylchlorid gemäß bekannten Methoden [s. z. B.: B. Singh, Heterocycles, 31, 1990, 2163-2172] erhält man die entsprechenden Chlorsubstituierten Pyrimidine der allgemeinen Formel (V).

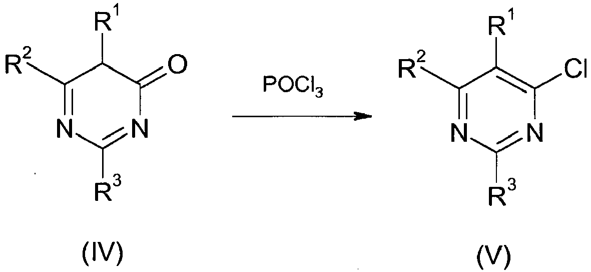

Diese können dann unter dem Fachmann vertrauten Standardbedingungen [s. z. B.: K.A. Kolmakov, Journal of Heterocyclic Chemistry, 45, 2008, 533-539] unter basischen Reaktionsbedingungen mit Amin der allgemeinen Formel (VI) weiter zu den Endverbindungen der allgemeinen Formel (I) derivatisiert werden.

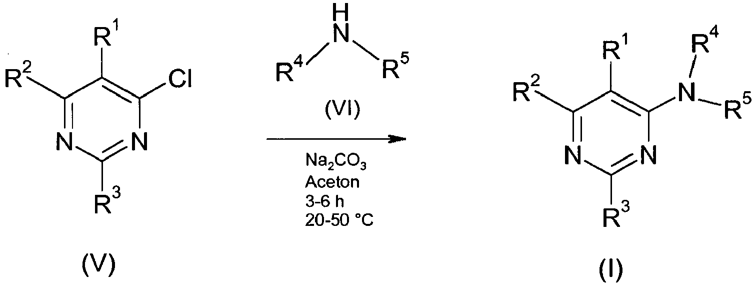

Weitere ähnliche universell anwendbare Verfahren zum Aufbau der Pyrimidine sind beispielsweise in den Routen 3, 4, 10, 13, 14, 17, 18, 19 und 20 der Herstellungsbeispiele 13 bis 104 beschrieben.

Im Allgemeinen gibt es in der Literatur eine Vielzahl von weiteren Synthesen für substituierte Pyrimidine. Eine dieser Synthesen um hochsubstituierte Pyrimidine, der allgemeinen Formeln (I) aufzubauen sieht folgendermaßen aus [s. z. B.: A.G. Martinez, JOC, 57, 1992, 1627]:

Ketone, der allgemeinen Formel (III') werden unter Trifluoressigsäureanhydrid-Katalyse mit Nitrilen, insbesondere Chlorcyan zu den Pyrimidinen, der allgemeinen Formel (V') kondensiert.

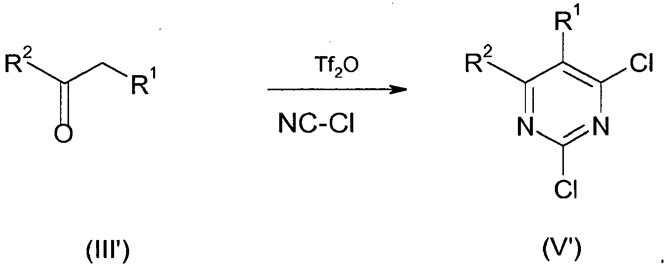

Die Verbindungen der allgemeinen Formel (V') können dann mittels geeigneter, dem Fachmann bekannter Methoden [s. z. B.: B. Singh, Heterocycles, 31,1990, 2163-2172] zu Verbindungen der allgemeinen Formel (V) und weiter nach bekannten Methoden [s. z. B.: K.A. Kolmakov, Journal of Heterocyclic Chemistry, 45, 2008, 533-539], wie vorstehend beschrieben, zu Verbindungen der allgemeinen Formel (I) umgesetzt werden.

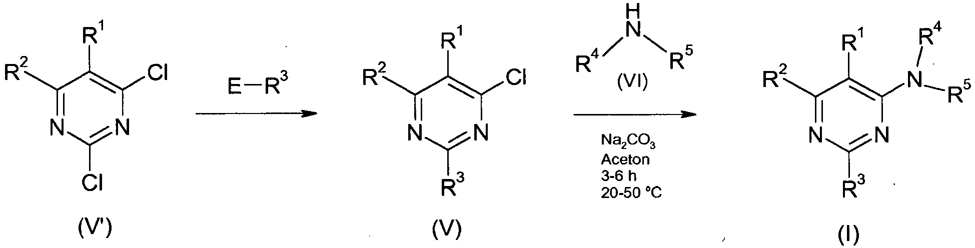

Dabei steht E, wie oben dargelegt, für eine geeignete Abgangsgruppe, welche die Substitution von Cl durch R³ ermöglicht.

Nach diesen beschriebenen Synthesewegen sind insbesondere auch die erfindungsgemäßen Verbindungen gemäß den Beispielen 1, 2, 3 und 4 erhältlich.

Darüber hinaus steht ein weiterer erfindungsgemäßer Verfahrensweg zur Verfügung, der geeignet ist zur Herstellung der erfindungsgemäßen Verbindungen der allgemeinen Formel (I), worin X für C-R¹ mit der Bedeutung für R¹ von Wasserstoff steht, und worin weiter R² die Bedeutung von gegebenenfalls substituiertem Amino wie vorstehend definiert aufweist und worin weiter R³ eine der vorstehenden Bedeutungen aufweist und worin R⁴ und R⁵ ebenfalls eine der vorstehenden Bedeutungen aufweisen, wobei einer der Substituenten R⁴ oder R⁵ die Bedeutung von gegebenenfalls substituiertem Amino aufweist und darunter ausgewählt ist aus der Gruppe derer, die zusammen mit dem Stickstoffatom, an das sie gebunden sind, unter Bildung einer gegebenenfalls substituierten Hydrazongruppe, hervorgehen.

Ausgangspunkt für die Synthese derartiger erfindungsgemäßer Verbindungen ist das käufliche 2,4,6-Trichlorpyrimidin (VII), welches nach dem Fachmann bekannten Standardmethoden [s. z. B.: B. Singh, Heterocycles, 31,1990, 2163-2172] zu Verbindungen der Formel (VIII') umgesetzt werden kann. Diese werden dann weiter unter dem Fachmann bekannten Bedingungen [s. z. B.: T.J. Delia, Journal of Heterocyclic Chemistry,36, 1999, 1259-1262] mit Verbindungen der Formel R²-H, worin R² eine gegebenenfalls substituierte Aminoverbindung darstellt, zu Verbindungen der allgemeinen Formel (VIII) derivatisiert. Diese werden dann in einem weiteren Schritt mit Hydrazin Hydrat unter Standardbedingungen in das Hydrazin der allgemeinen Formel (IX) überführt [s. z. B.: Chesterfield et al, Journal of the Chemical Society, 1955, 3478-3481], welches anschließend durch Umsetzung mit Aldehyden der allgemeinen Formel R⁶-(C=O)-R⁷, gemäß untenstehendem Reaktionsschema, zu den entsprechenden Hydrazonen der allgemeinen Formel (X) umgesetzt wird [s. z. B.: Claesen, Bulletin des Societes Chimiques Belges, 68, 1959, 47-57; L.F. Kuyper, Bioorganic & Medicinal Chemistry, 4, 1996, 593-602]. Dabei ist es prinzipiell auch möglich, Verbindungen der Formel (VIII') erst mit Hydrazin-Hydrat und Aldehyden zu den entsprechenden Hydrazonen umzusetzen und anschließend die Derivatisierung mit der Verbindung R²-E durchzuführen. In dem nachfolgenden Reaktionsschema steht E für eine geeignete Abgangsgruppe, wie zuvor definiert, welche die Substitution von Cl durch R² oder R³ ermöglicht, und R⁶ und R⁷ sind gleich oder verschieden und werden ausgewählt aus:
- Wasserstoff,
- gegebenenfalls substituiertem Alkyl,
- gegebenenfalls substituiertem Alkenyl,
- gegebenenfalls substituiertem Alkinyl,
- gegebenenfalls substituiertem Aryl, oder
- gegebenenfalls substituiertem Heterocyclyl.

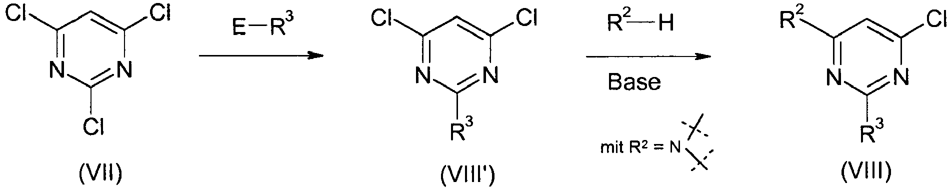

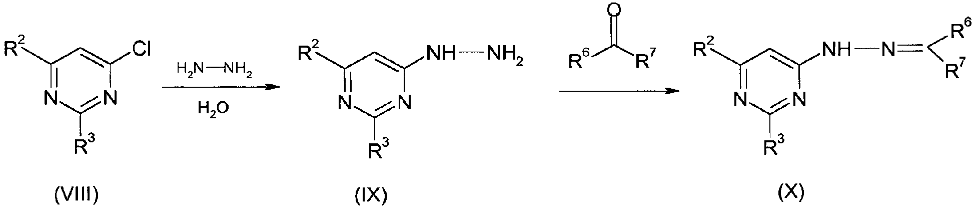

(Die Schreibweise 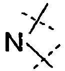 bedeutet hier und im folgenden, dass das Stickstoffatom Substituenten aufweist welche die Bedeutungen aufweisen, wie für die Erfindung definiert. Grundsätzlich kann im gesamten Rahmen der Erfindung im Falle, dass R² = R³ ist, die Umsetzung zur entsprechenden Zielverbindung mit R² und R³ auch in einer Stufe erfolgen. (Siehe beispielsweise entsprechende Stufen bei den Routen 1, 2, 3 der Herstellungsbeispiele 105 bis 112)).

Die so erhältlichen Verbindungen (X) entsprechen erfindungsgemäßen Verbindungen der Formel (I), worin X die Bedeutung C mit R¹=H hat, R² insbesondere eine gegebenenfalls substituierte Aminogruppe bedeutet, R³ eine der vorstehend genannten erfindungsgemäßen Bedeutungen aufweist und worin einer der Substituenten R⁴ oder R⁵ Wasserstoff ist und der jeweils andere ein gegebenenfalls substituiertes Amino, ausgewählt aus der Gruppe derer, die zusammen mit dem Stickstoffatom an das sie gebunden sind eine gegebenenfalls substituierte Hydrazongruppe bilden:

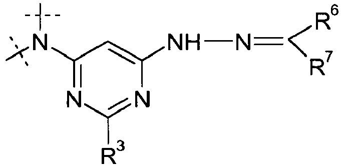

Nach diesem beschriebenen Syntheseweg sind insbesondere auch die erfindungsgemäßen Verbindungen gemäß den Beispielen 6 und 8 erhältlich.

Um solche erfindungsgemäßen Verbindungen zu erhalten, in denen außerdem auch R³ eine gegebenenfalls substituierte Aminogruppe bedeutet, erfolgt die Umsetzung der Verbindung der Formel (VII) gemäß vorstehendem Syntheseschema unter dem Fachmann bekannten Bedingungen [s. z. B.: T.J. Delia, Journal of Heterocyclic Chemistry, 36, 1999, 1259-1262] mittels Verbindungen der Formel R³-H, worin R³ eine gegebenenfalls substituierte Aminoverbindung darstellt, zu Verbindungen der allgemeinen Formel (VIII"), sowie anschließender Derivatisierung mit R²-E, wie oben definiert und Umsetzung zu den entsprechenden Hydrazonverbindungen wie oben dargestellt.

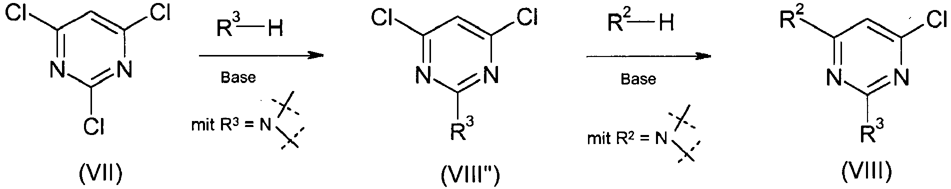

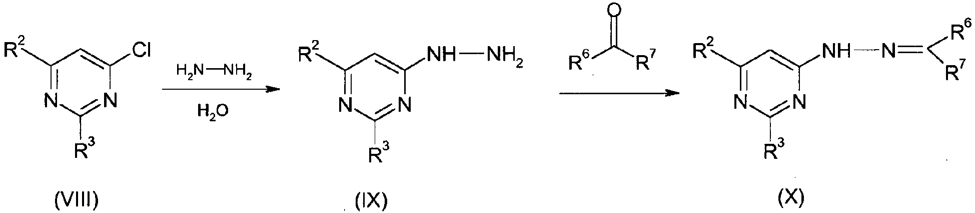

Darin sind in Verbindung (X) sowohl der Substituent R² als auch der Substituent R³ jeweils über ein Stickstoffatom an den Pyrimidin-Ring gebunden:

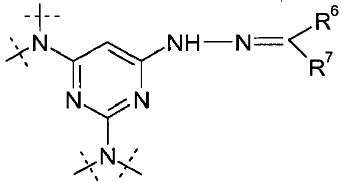

Nach diesem Syntheseweg sind insbesondere auch die erfindungsgemäßen Verbindungen gemäß den Beispielen 5 und 7 erhältlich.

Weiterhin steht mit nachfolgendem Syntheseweg ein Verfahren zur Herstellung erfindungsgemäßer Verbindungen der allgemeinen Formel (I), worin X für N steht und worin die Substituenten R² und R³ gegebenenfalls substituierte Aminoverbindungen oder gegebenenfalls substituierte Heterocyclylverbindungen, die über ein Hetero-Stickstoffatom gebunden sind, bedeuten, zur Verfügung.

Ausgangspunkt für die Synthese derartiger Verbindungen der Formel (I) bildet das käufliche 2,4,6-Trichloro-1,3,5-triazin der Formel (XI), welches über die beschriebenen, dem Fachmann bekannten Verfahren umgesetzt werden kann.

Dabei wird zu Beginn das käufliche Triazin (XI) unter basischen Reaktionsbedingungen mit Amin der allgemeinen Formel R⁴-NH-R⁵, nach dem Fachmann bekannten Standardmethoden [s. z. B.: K. A. Kolmakov, Journal of Heterocyclic Chemistry, 45, 2008, 533-539] zu Verbindung der allgemeinen Formel (XI') umgesetzt. Das so erhaltene Aminotriazin (XI') kann dann analog mit weiteren Aminen R³-H und R²-H unter basischen Reaktionsbedingungen über Diaminotriazin (XI") zur Zielverbindung der allgemeinen Formel (I) umgesetzt werden [s. z. B.: H. E. Birkett, Magnetic Resonance in Chemistry, 41, 2003,324-336; J. P. Mathias, JACS, 116, 1994, 4326-4340].

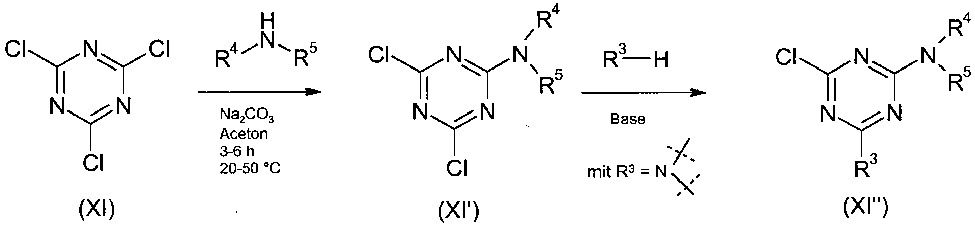

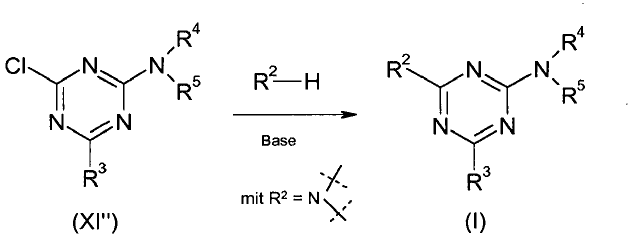

Darin sind in Verbindung (I) sowohl der Substituent R² als auch der Substituent R³ jeweils über ein Stickstoffatom an den Triazin-Ring gebunden im Sinne der allgemeinen Formel:

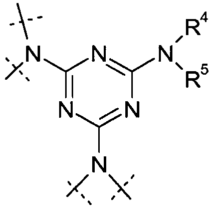

Nach diesem Syntheseweg sind insbesondere auch die erfindungsgemäßen Verbindungen gemäß den Beispielen 9, 10, 11 und 12 erhältlich. (Siehe beispielsweise auch entsprechende Stufen bei den Routen 1 bis 5 der Herstellungsbeispiele 113 bis 117).

Um entsprechende Triazin-Verbindungen zu erhalten, in denen entweder R² oder R³ eine andere der vorstehend genannten Bedeutungen für R² bzw. R³ aufweist als die einer gegebenenfalls substituierten Aminoverbindung, können die entsprechenden Diaminotriazine (XI") bzw. (XI''') auch mit anderen Nukleophilen zur Verbindung (I) [s. z. B.: P.A. Belyakoy, Russian Chemical Bulletin, 54, 2005, 2441-2451] umgesetzt werden: 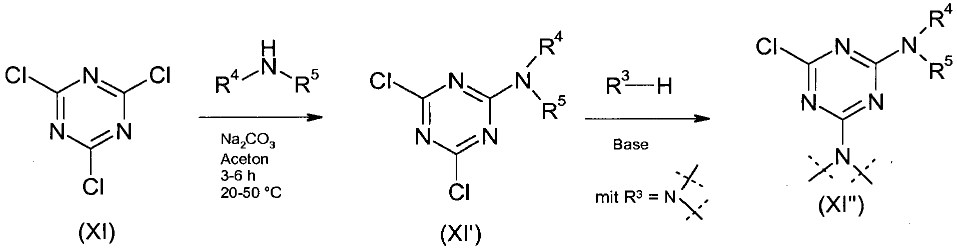 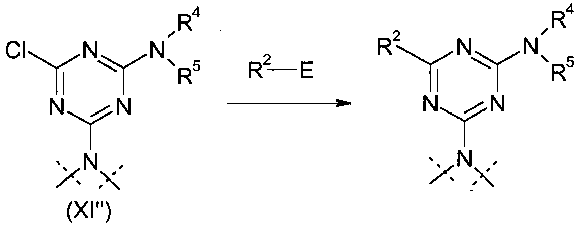 worin R² eine der vorstehenden erfindungsgemäßen Bedeutungen hat, und worin E eine geeignete Abgangsgruppe, wie zuvor definiert, ist, bzw.: 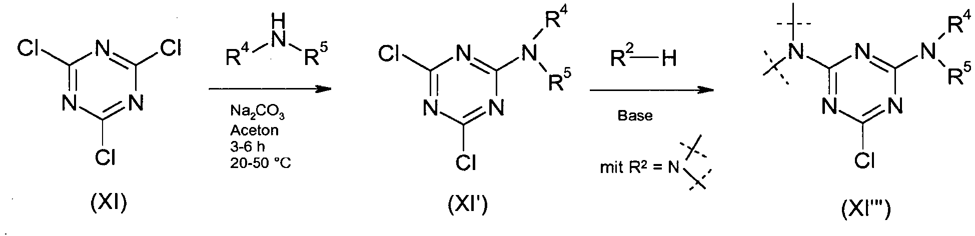 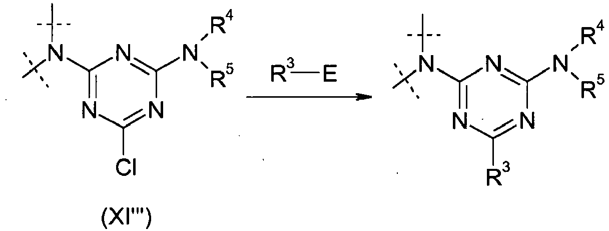 worin R³ eine der vorstehenden erfindungsgemäßen Bedeutungen hat, und worin E eine geeignete Abgangsgruppe, wie zuvor definiert, ist.

Im Rahmen der Erfindung handelt es sich bei Verbindungen R-E, wie insbesondere R³-E und R²-E um solche, worin R² und R³ die Bedeutungen, wie oben definiert, aufweisen und worin E eine geeignete Abgangsgruppe ist, die insbesondere geeignet ist, das Chloratom im entsprechenden Triazinyl- oder Pyrimidin-Grundkörper mittels der Gruppe R zu substituieren, wie zuvor definiert.

Die hier dargestellten Reaktionswege stellen an sich bekannte Reaktionstypen dar, die in an sich bekannter Weise durchgeführt werden können. Durch Umsetzen mit einer pharmazeutische verträglichen Base oder Säure erhält man entsprechende Salze.

Die Umsetzung der verschiedenen Reaktionspartner kann in verschiedenen Lösemitteln durchgeführt werden und unterliegt in dieser Hinsicht keiner besonderen Beschränkung. Entsprechende Beispiele für geeignete Lösemittel sind somit Wasser, Ethanol, Aceton, Dichlorethan, Dichlormethan, Dimethoxyethan, Diglym, Acetontril, Butyronitril, THF, Dioxan, Ethylacetat, Butylacetat, Dimethylacetamid, Toluol und Chlorbenzol. Darüber hinaus ist es möglich, die Reaktion in einem im Wesentlichen homogenen Gemisch aus Wasser und Lösemitteln durchzuführen, falls das organische Lösemittel mit Wasser mischbar ist.

Die erfindungsgemäße Umsetzung der Reaktionspartner wird beispielsweise weise bei Raumtemperatur durchgeführt. Es können jedoch auch Temperaturen oberhalb von Raumtemperatur, beispielsweise bis 70 °C, und Temperaturen unterhalb Raumtemperatur, beispielsweise bis -20°C oder weniger angewendet werden.

Der pH-Wert, bei welchen die erfindungsgemäße Umsetzung der Reaktionspartner, insbesondere die R²- und R³-Substituierung, durchgeführt wird, wird geeignet eingestellt.

Die Einstellung des pH-Wertes insbesondere bei der R²- und R³-Substituierung sowie bei der Aminierung mit R⁴-NH-R⁵, erfolgt vorzugsweise durch Zugabe einer Base. Als Basen können sowohl organische wie auch anorganische Basen verwendet werden. Bevorzugt werden anorganische Basen wie beispielsweise LiOH, NaOH, KOH, Ca(OH)₂, Ba(OH)₂, Li₂CO₃, K₂CO₃, Na₂CO₃, NaHCO₃, oder organische Basen wie Amine (wie beispielsweise bevorzugt Triethylamin, Diethylisopropylamin), Bu₄NOH, Piperidin, Morpholin, Alkylpyridine, verwendet. Besonders bevorzugt werden anorganische Basen, ganz besonders bevorzugt, Na₂CO₃, LiOH, NaOH und KOH verwendet.

Die Einstellung des pH-Wertes kann ggf. auch mittels Säuren erfolgen, wie insbesondere bei der Cyclisierung zu Pyrimidineonen. Als Säuren können sowohl organische wie auch anorganische Säuren verwendet werden. Bevorzugt werden anorganische Säuren wie beispielsweise HCl, HBr, HF, H₂SO₄, H₃PO₄ oder organische Säuren wie CF₃COOH, CH₃COOH, p-Toluolsulfonsäure, sowie deren Salze verwendet. Besonders bevorzugt werden anorganische Säuren, wie HCl und H₂SO₄ sowie organische Säuren wie Triflouressigsäure (CF₃COOH), Triflouressigsäureanhydrid (Tf₂O)) und Essigsäure (CH₃COOH) bzw. deren Natriumsalz (EtONa) verwendet.

Ein Fachmann ist hier in der Lage für den entsprechenden Syntheseweg das am besten geeignete Lösemittel und die optimalen Reaktionsbedingungen, insbesondere hinsichtlich Temperatur, pH-Wert, Katalysator und Lösungsmittel auszuwählen.

Die Erfinder fanden überraschend, dass die Verbindungen, die Gegenstand der vorliegenden Erfindung sind und die durch die allgemeine Strukturformel (I') dargestellt werden, eine Wirkung als Hepcidin-Antagonist zeigen und somit zur Verwendung als Arzneimittel zur Behandlung Hepcidin-vermittelter Erkrankungen und der damit einhergehenden oder damit assozierten Symptome geeignet sind. Insbesondere sind die erfindungsgemäßen Verbindungen geeignet in der Anwendung zur Behandlung von Eisenmangelerkrankungen und/oder Eisenmangelanämien insbesondere bei ACD und AI.

Die die Verbindungen der allgemeine Strukturformel (I') enthaltenden Arzneimittel sind dabei zum Einsatz in der Human- und der Veterinärmedizin geeignet.

Die erfindungsgemäßen Verbindungen eignen sich somit auch zur Herstellung eines Medikamentes zur Behandlung von Patienten, die unter Symptomen einer Eisenmangelanämie wie zum Beispiel: Ermüdung, Antriebslosigkeit, Konzentrationschwäche, geringe kognitive Effizienz, Schwierigkeiten beim Finden der richtige Worte, Vergesslichkeit, unnatürliche Blässe, Reizbarkeit, Beschleunigung der Herzfrequenz (Tachykardie), wunde oder geschwollene Zunge, vergrößerte Milz, Schwangerengelüste (Pica), Kopfschmerzen, Appetitlosigkeit, erhöhte Infektionsanfälligkeit, depressive Verstimmungen oder unter einer ACD oder einer AI leiden.

Die erfindungsgemäßen Verbindungen eignen sich somit auch zur Herstellung eines Medikamentes zur Behandlung von Patienten, die unter Symptomen einer Eisenmangelanämie leiden.

Die Verabreichung kann über einen Zeitraum von mehreren Monaten bis zur Verbesserung des Eisenstatus, reflektiert beispielsweise durch den Hämoglobin-Wert, die Transferrin-Sättigung und den Ferritin-Wert der Patienten, oder zur gewünschten Verbesserung einer durch Eisenmangelanämie oder durch ACD oder AI hervorgerufenen Beeinträchtigung des Gesundheitszustandes erfolgen.

Das erfindungsgemäße Präparat kann von Kindern, Jugendlichen und Erwachsenen eingenommen werden.

Die Verbindungen der vorliegenden Erfindung können außerdem auch in Kombination mit weiteren in der Behandlung von Eisenmetabolismus-Störungen bekannten Wirkstoffen oder Arzneimitteln und/oder mit Wirkstoffen oder Arzneimitteln, die begleitend mit Mitteln zur Behandlung von Erkrankungen, die mit Eisenmangel und/oder Anämien assoziiert sind, verabreicht werden, verwendet werden. Beispiele solcher in Kombination anwendbarer Mittel zur Behandlung von Eisenmetabolismus-Störungen und weiterer mit Eisenmangel und/oder Anämien assoziierter Erkrankungen können beispielsweise umfassen Eisen-haltige Verbindungen wie z. B. Eisensalze, Eisenkohlenhydrat-Komplexverbindungen, wie Eisen-Maltose- oder Eisen-Dextrin-Komplexverbindungen, Vitamin D und/oder Derivate davon.

Die in Kombination mit den erfindungsgemäßen Verbindungen angewendeten Verbindungen können dabei sowohl oral als auch parenteral verabreicht werden, oder die Verabreichung der erfindungsgemäßen Verbindungen und der in Kombination angewendeten Verbindungen kann durch Kombination der genannten Verabreichungsmöglichkeiten erfolgen.

Die erfindungsgemäßen Verbindungen sowie die vorgenannten Kombinationen der erfindungsgemäßen Verbindungen mit weiteren Wirkstoffen oder Arzneimitteln können in der Behandlung von Eisenmangelerkrankungen und/oder Eisenmangelanämien, insbesondere Anämien bei Krebs, Anämie ausgelöst durch Chemotherapie, Anämie ausgelöst durch Inflammation (AI), Anämien bei kongestiver Herzinsuffizienz (CHF;congestive heart failure), Anämie bei chronischer Niereninsuffizienz Stadium 3 -5 (CKD 3-5; chronic kidney diseases stage 3-5), Anämie ausgelöst durch chronische Inflammation (ACD), Anämie bei rheumatischer Arthritis (RA; rheumatoid arthritis), Anämie bei systemischem Lupus erythemotodes (SLE; systemic lupus erythematosus) und Anämie bei inflammatorischen Darmerkrankungen (IBD; inflammatory bowel diseases) eingesetzt werden oder zur Herstellung von Medikamenten zur Behandlung der dieser Erkrankungen verwendet werden.

Die erfindungsgemäßen Verbindungen sowie die vorgenannten Kombinationen der erfindungsgemäßen Verbindungen mit weiteren Wirkstoffen oder Arzneimitteln können insbesondere zur Herstellung von Medikamenten zur Behandlung der Eisenmangelanämie verwendet werden, wie Eisenmangelanämien bei Schwangeren, der latenten Eisenmangelanämie bei Kindern und Heranwachsenden, der Eisenmangelanämie infolge gastrointestinaler Abnormalitäten, der Eisenmangelanämie infolge von Blutverlusten, wie durch gastrointestinale Blutungen (z.B. infolge von Geschwüren, Karzinomen, Hämorrhoiden, entzündlichen Störungen, Einnahme von Acetylsalicylsäure), Menstruation, Verletzungen, Eisenmangelanämie infolge von Psilosis (Sprue), Eisenmangelanämie infolge verminderter Eisenaufnahme bei der Ernährung, insbesondere bei selektiv essenden Kindern und Heranwachsenden, Immunschwäche hervorgerufen durch Eisenmangelanämie, Beeinträchtigung der Hirnleistung hervorgerufen durch Eisenmangelanämie, Restless Leg Syndrom.

Die erfindungsgemäße Anwendung führt zu einer Verbesserung der Eisen-, Hämoglobin-, Ferritin- und Transferrinwerte, welche insbesondere bei Heranwachsenden und Kindern aber auch bei Erwachsenen mit einer Verbesserung im Kurzzeitgedächtnistests (STM), im Langzeitgedächtnistest (LTM), im Test der progressiven Matrices nach Raven, in der Welschers Erwachsenenintelligenzskala (WAIS) und/oder im emotionalen Koeffizienten (Baron EQ-i, YV-Test; Jugendversion), oder zu einer Verbesserung der Neutrophilen-Niveaus, der Antikörperniveaus und/oder der Lymphozytenfunktion einhergeht.

Die vorliegende Erfindung betrifft weiterhin pharmazeutische Zusammensetzungen, enthaltend eine oder mehrere der erfindungsgemäßen Verbindungen nach der Formel (I), sowie gegebenenfalls eine oder mehrere weitere pharmazeutisch wirksame Verbindung sowie gegebenenfalls einen oder mehrere pharmakologisch verträgliche Träger und/oder Hilfsstoffe und/oder Lösungsmittel.

Dabei handelt es sich um übliche pharmazeutische Träger, Hilfsstoffe oder Lösungsmittel. Genannte pharmazeutische Zusammensetzungen sind beispielsweise geeignet zur intravenösen, intraperitonealen, intramuskulären, intravaginalen, intrabuccalen, perkutanen, subkutanen, mucokutanen, oralen, rektalen, transdermalen, topikalen, intradermalen, intragastralen oder intrakutanen Applikation und liegen beispielsweise in der Form von Pillen, Tabletten, magensaftresistenten Tabletten, Filmtabletten, Schichttabletten, Retardformulierungen zur oralen, subkutanen oder kutanen Verabreichung (insbesondere als Pflaster), Depotformulierung, Dragees, Zäpfchen, Gelen, Salben, Sirup, Granulaten, Suppositorien, Emulsionen, Dispersionen, Mikrokapseln, Mikroformulierungen, Nanoformulierungen, liposomalen Formulierungen, Kapseln, magensaftresistente Kapseln, Pulvern, Inhalationspulvern, mikrokristallinen Formulierungen, Inhalationssprays, Puder, Tropfen, Nasentropfen, Nasensprays, Aerosolen, Ampullen, Lösungen, Säften, Suspensionen, Infusionslösungen oder Injektionslösungen etc. vor. Bevorzugt werden die erfindungsgemäßen Verbindungen sowie pharmazeutische Zusammensetzungen, enthaltend solche Verbindungen oral und/oder parenteral, insbesondere intravenös appliziert.

Dazu liegen die erfindungsgemäßen Verbindungen bevorzugt in pharmazeutischen Zusammensetzungen in Form von Pillen, Tabletten, magensaftresistenten Tabletten, Filmtabletten, Schichttabletten, Retardformulierungen zur oralen Verabreichung, Depotformulierungen, Dragees, Granulaten, Emulsionen, Dispersionen, Mikrokapseln, Mikroformulierungen, Nanoformulierungen, liposomalen Formulierungen, Kapseln, magensaftresistente Kapseln, Pulvern, mikrokristallinen Formulierungen, Puder, Tropfen, Ampullen, Lösungen, Suspensionen, Infusionslösungen oder Injektionslösungen vor.

Die erfindungsgemäßen Verbindungen können in pharmazeutischen Zusammensetzung verabreicht werden, die verschiedene organische oder anorganische Träger- und/oder Hilfsmaterialien enthalten können, wie sie üblicherweise für pharmazeutische Zwecke insbesondere für feste Arzneimittelformulierungen verwendet werden, wie beispielsweise Exzipienten (wie Saccharose, Stärke, Mannit, Sorbit, Lactose, Glucose, Cellulose, Talk, Calciumphosphat, Calciumcarbonat), Bindemittel (wie Cellulose, Methylcellulose, Hydroxypropylcellulose, Polypropylpyrrolidon, Gelatine, Gummiarabicum, Polyethylenglykol, Saccharose, Stärke), Desintegrationsmittel (wie Stärke, hydrolysierte Stärke, Carboxymethylcellulose, Calciumsalz von Carboxymethylcellulose, Hydroxypropylstärke, Natriumglycolstärke, Natriumbicarbonat, Calciumphosphat, Calciumcitrat), Gleit- bzw. Schmiermittel (wie Magnesiumstearat, Talk, Natriumlaurylsulfat), ein Geschmacksbildner (wie Citronensäure, Menthol, Glycin, Orangenpulver), Konservierungsmittel (wie Natriumbenzoat, Natriumbisulfit, Methylparaben, Propylparaben), Stabilisatoren (wie Citronensäure, Natriumcitrat, Essigsäure, und Multicarbonsäuren aus der Titriplex Reihe wie z.B. Diethylentriaminpentaessigsäure (DTPA), Suspendiermittel (wie Methylcellulose, Polyvinylpyrrolidon, Aluminiumstearat), Dispergiermittel, Verdünnungsmittel (wie Wasser, organische Lösungsmittel), Bienenwachs, Kakaobutter, Polyethylenglykol, weißes Petrolatum etc..

Flüssige Arzneimittelformulierungen, wie Lösungen, Suspensionen und Gele enthalten üblicherweise einen flüssigen Träger, wie Wasser und/oder pharmazeutisch verträgliche organische Lösungsmittel. Weiterhin können derartige flüssige Formulierungen auch pH-einstellende Mittel, Emulgatoren oder dispergierende Agenzien, puffernde Agenzien, Konservierungsmittel, Netzmittel, Geliermittel (beispielsweise Methylcellulose), Färbemittel und/oder Aromastoffe enthalten. Die Zusammensetzungen können isotonisch sein, das heisst diese können den gleichen osmotischen Druck wie Blut haben. Die Isotonie der Zusammensetzung kann durch die Verwendung von Natriumchlorid oder anderer pharmazeutisch annehmbare Agenzien wie beispielsweise Dextrose, Maltose, Borsäure, Natriumtartrat, Propylenglykol oder andere anorganische oder organisch lösliche Substanzen eingestellt werden. Die Viskosität der flüssigen Zusammensetzungen kann unter Verwendung eines pharmazeutisch annehmbaren Verdickungsmittels, wie Methylcellulose eingestellt werden. Andere geeignete Verdickungsmittel umfassen beispielsweise Xanthan, Carboxymethylcellulose, Hydroxypropylcellulose, Carbomer und dergleichen. Die bevorzugte Konzentration des Verdickungsmittels wird von dem ausgewählten Agens abhängen. Pharmazeutisch annehmbare Konservierungsmittel können verwendet werden, um die Haltbarkeit der flüssigen Zusammensetzung zu erhöhen. Benzylalkohol kann geeignet sein, obwohl eine Vielzahl von Konservierungsmitteln einschließlich beispielsweise Paraben, Thimerosal, Chlorbutanol oder Benzalkoniumchlorid ebenfalls verwendet werden können.

Der Wirkstoff kann beispielsweise mit einer Einheitsdosis von 0,001 mg/kg bis 500 mg/kg Körpergewicht beispielweise bis zu 1 bis 4 mal am Tag verabreicht werden. Die Dosierung kann jedoch je nach Alter, Gewicht, Zustand des Patienten, Schwere der Erkrankung oder Art der Verabreichung erhöht oder herabgesetzt werden.

Eine bevorzugte Ausführungsform betrifft die Verwendung der erfindungsgemäßen Verbindungen, sowie der die erfindungsgemäßen Verbindungen enthaltenden erfindungsgemäßen Zusammensetzungen sowie der die erfindungsgemäßen Verbindungen und Zusammensetzungen enthaltenden erfindungsgemäßen Kombinationspräparate zur Herstellung eines Arzneimittels zur oralen oder parenteralen Verabreichung.

Die Erfindung wird durch die folgenden Beispiele näher veranschaulicht. Die Beispiele stellen lediglich Exemplifizierungen dar, und der Fachmann ist in der Lage die spezifischen Beispiele auf weitere beanspruchte Verbindungen auszudehnen.

### BEISPIELE

### PHARMAKOLOGISCHE WIRKVERSUCHE:

Folgende Materialien wurden verwendet:

| Reagenzien | Batch-No. | Kommentar |
|---|---|---|
| MDCK-FPN-HaloTag Klon 7 | | |
| Hepcidin 100 µM Stammlösung in Wasser | Lot# 571007 | Peptides International |
| HaloTag®TMR Ligand | Lot# 257780 | Promega, Kat#G8251 |
| Opera confocal plate imager | | PerkinElmer |
| Perkin Elmer 384 Cell carrier plates | | Kat#6007430 |
| Paraformaldehyd | Lot# 080416 | Electron Microscopy Sciences Kat#15710-S |
| Draq5 | | Biostatus, Kat Nr: DR51000 |

Die Hepcidin-antagonistische Wirkung der Pyrimidin- und Triazin-Verbindungen der vorliegenden Erfindung wurde mittels des nachfolgend beschriebenen "Ferroportin Internalisation Assays" bestimmt.

### Prinzip des "Ferroportin Internalisation Assays"

Organische Verbindungen mit geringem Molekulargewicht, die den biologischen Wirkungen von Hepcidin auf seinen Rezeptor, den Eisenexporter Ferroportin (Fpn), entgegenwirken, wurden auf der Basis ihrer Fähigkeit, Hepcidin-induzierte Internalisierung von Fpn in lebenden Zellen zu hemmen, identifiziert. Zu diesem Zweck wurde eine stabile Zell-Linie (Madin-Darby Canine Kidney, MDCK) erzeugt, die konstitutiv menschliches Ferroportin, das an seinem C-Terminus mit einem fluoreszierenden Reporterprotein (HaloTag®, Promega Corp.) rekombinant fusioniert ist, exprimiert. Die Internalisierung von Fpn wurde verfolgt, indem diese Zellen mit fluoreszierenden Liganden (HaloTag®-TMR, Tetramethylrhodamin) markiert wurden, die sich kovalent an das mit dem Fpn fusionierten HaloTag-Reportergen anfügen. Die Bildgebung mit konfokaler Fluoreszenzmikroskopie zeigte eine Zelloberflächenlokalisation von Fpn bei Abwesenheit von Hepcidin und das Fehlen von Fpn-Oberflächenfärbung bei Anwesenheit von Hepcidin. Optimierte Bildanalysealgorithmen wurden zur Erfassung der Zelloberfläche und zur Quantifizierung der entsprechenden, mit dem Fpn-HaloTag-Fusionsprotein assoziierten Membranfluoreszenz verwendet. Dieser Assay erlaubt eine quantitative bildbasierte Analyse, um schnell Verbindungen zu bewerten, die Hepcidin-induzierte Internalisierung von Fpn blockieren können. Dieser Assay ist ein direktes In-Vitro-Pendant des für Arzneimittelkandidaten vorgeschlagenen in vivo Wirkmechanismus, und ist somit geeignet als ein initialer Assay mit hohem Durchsatz für die Identifizierung von Verbindungen, die der Wirkung von Hepcidin auf seinen Rezeptor Ferroportin entgegenwirken.

### Detaillierter Ablauf des Assays

- 7500 Zellen pro Vertiefung (MDCK-FPN-HaloTag) wurden pro Vertiefung in 50 *µ*L DMEM-Medium (Dulbeccos Modified Eagle Medium mit 10% fötalem Rinderserum (fetal bovine serum, FBS), das 1% Penicillin, 1% Streptomycin und 450 *µ*g/ml G-418 enthielt) in Mikrotiterplatten mit 384 Vertiefungen überimpft (384 Cell carrier plates, Perkin Elmer, Kat. Nr. 6007430), gefolgt von einer Inkubation über Nacht bei 37°C/5 % CO₂.
- Das Volumen des Mediums wurde auf 10 *µ*L reduziert, und 10 *µ*L von 5 *µ*M HaloTag-TMR-Liganden (Promega, Kat. Nr. G 8251) wurden in DMEM-Medium hinzugegeben, um das Fpn-HaloTag Fusionsprotein anzufärben.
- 15 min Inkubation bei 37°C/5% CO₂
- HaloTag-TMR-Ligand wurde entfernt, und die Zellen wurden mit frischem DMEM-Medium gewaschen, und das Volumen wurde auf 20 *µ*L DMEM-Medium reduziert.
- Pro Vertiefung wurden 3 *µ*L einer Lösung der Testverbindung (gelöstes DMSO) hinzugegeben (10 *µ*L Endvolumen).
- 7 *µ*L 43 *µ*M Hepcidin (Peptides International, Kat. Nr. PLP-4392-s, 100 *µ*M Stammlösung in Wasser verdünnt in DMEM-Medium) wurde pro Vertiefung bis zu einer endgültigen Hepcidinkonzentration von 100 nM hinzugefügt.
- Die Zellen wurden über Nacht bei 37°C/5% CO₂ inkubiert.
- Die Zellen wurden fixiert, indem Paraformaldehyd (PFA, Electron Microscopy Sciences, Kat. Nr. 15710-S) direkt zu den Zellen bis zu einer endgültigen Konzentration von 4% hinzugegeben wurde, gefolgt von einer 15-20 minütigen Inkubation bei Zimmertemperatur.
- Die PFA-Lösung wurde entfernt und die Zellen mit PBS (Phophatgepufferte Salzlösung) gewaschen, wobei jeweils 30 *µ*l in der Platte verblieben.
- 20 *µ*L Draq5 (Biostatus, Kat. Nr. DR 51000) wurden bis zu einer endgültigen Konzentration von 2,5 *µ*M hinzugefügt, um die Zellkerne zu färben, und die Platten wurden mit Plattenversiegelung aus Folie versiegelt.
- Die Platten wurden mit dem Opera Plate Imager (Opera Confocal Plate Imager, Perkin Elmer) mit 7 Bildern pro Vertiefung; 440 ms Belichtungszeit pro Bild, 1 *µ*M Brennpunkthöhe analysiert.

Datenanalyse
- Optimierte Algorithmen wurden für die Bildanalyse zur Erfassung und Quantifizierung der mit der Zelloberfläche assoziierten Fluoreszenz als Maß für die Zelloberflächenlokalisierung von Fpn-HaloTag verwendet.
- Die endgültige Anzeige entsprach dem prozentualen Anteil an Zellen, die Membranfluoreszenz zeigten: mit 100 nM Hepcidin behandelte Vertiefungen ergaben die geringsten Werte (negative Kontrollanzeige = 0% Hemmung der Fpn Internalisierung) und Vertiefungen, die nicht mit Hepcidin behandelte wurden, ergaben den maximalen prozentualen Anteil an Zellen mit Membranfluoreszenz (positive Kontrollanzeige 100% Hemmung der Fpn Internalisierung)
- Auf jeder Platte wurde der Medianwert der 6 positiven und 6 negativen Kontrollwerte verwendet, um die prozentuale Hemmung der Verbindungen, die getestet wurden, gemäß folgender Formel zu berechnen: $I = 100 \times \frac{{R}_{neg} - {R}_{compound}}{{R}_{neg} - {R}_{pos}}$ mit:
   - Rₚₒₛ: positiver Kontrollanzeigewert (Median)
   - R_{neg}: negativer Kontrollanzeigewert (Median)
   - R_{compound}: Anzeigewert der untersuchten Verbindung
   - I: prozentuale Hemmung der jeweiligen Verbindung
- In Dosiswirkungsversuchen wurden Verdünnungsreihen (11 Konzentrationen, 1:2 Verdünnungsschritte) der Verbindungen getestet (Konzentrationsbereich von 0,04 bis 40 *µ*M), und normierte Signalwerte replizierter Versuche (durchschnittlich 6 Titrationen auf unabhängigen Platten) wurden zur Kurvenanpassung nach einem robusten Standard-Dosiswirkungsmodell mit vier Parametern (untere Asymptote, obere Asymptote, IC50, Steigung) verwendet.

Folgende Ergebnisse wurden für die Beispiele erhalten:

| Beispiel | Verbindung | IC50 [*µ*M] | I [%] (Median Inhibition [%] bei 10*µ*M Substanz Konz.) |
|---|---|---|---|
| 1# | 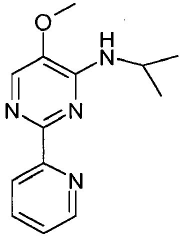 | < 50 | > 50 |
| 2# | 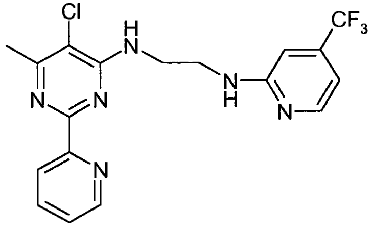 | > 40 | < 50 |
| 3# | 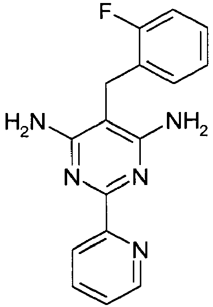 | > 40 | < 50 |
| 4# | 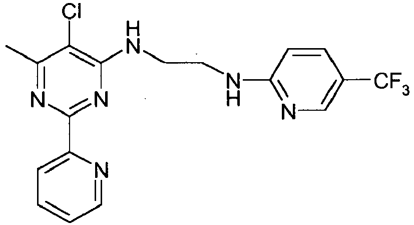 | > 40 | < 50 |
| 5# | 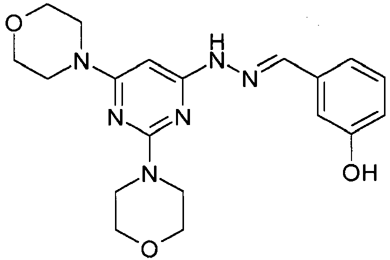 | > 40 | > 50 |
| 6# | 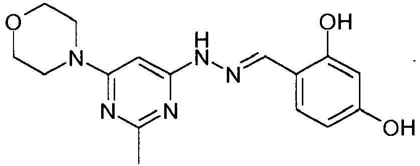 | > 40 | > 50 |
| 7# | 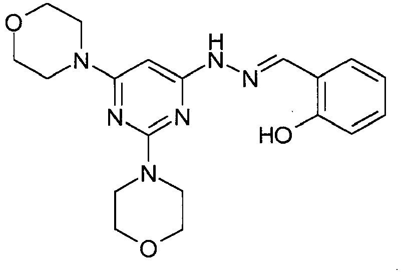 | < 50 | > 50 |
| 8# | 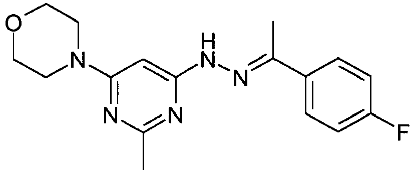 | > 40 | < 50 |
| 9 | 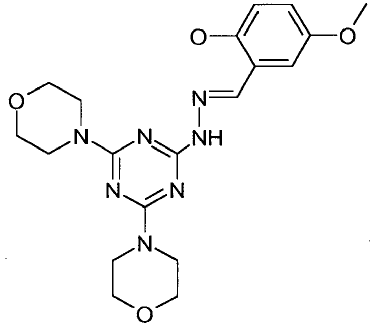 | < 50 | > 50 |
| 10 | 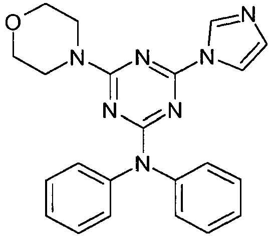 | < 50 | > 50 |
| 11 | 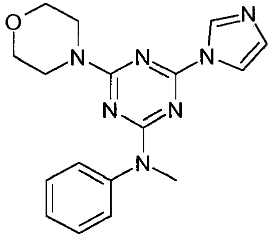 | < 50 | < 50 |
| 12 | 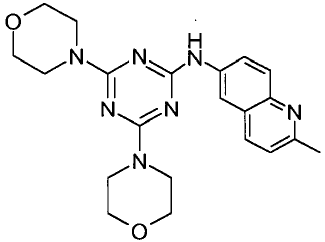 | < 50 | < 50 |

| Beispiel | Verbindung | IC50 [*µ*M] | |
|---|---|---|---|
| 13 | 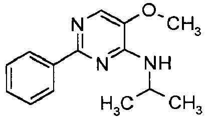 | >50 | |
| 14 | 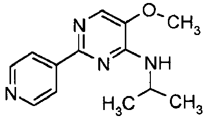 | >50 | |
| 15 | 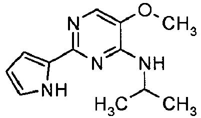 | >50 | |
| 16 | 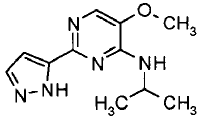 | >50 | |
| 17 | 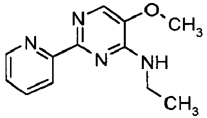 | <50 | |
| 18 | 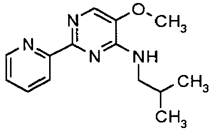 | <50 | |
| 19 | 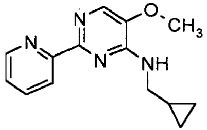 | <50 | |
| 20 | 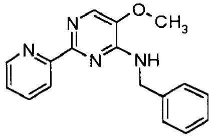 | <50 | |
| 21 | 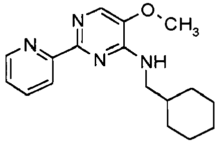 | <50 | |
| 22 | 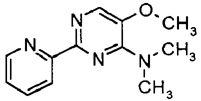 | <50 | |
| 23 | 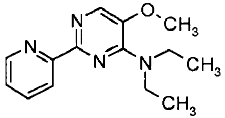 | >50 | |
| 24 | 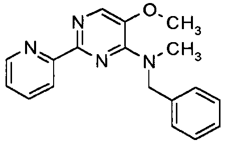 | | |
| 25 | 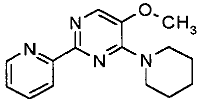 | >50 | |
| 26 | 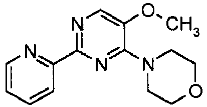 | <50 | |
| 27 | 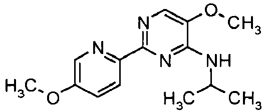 | <50 | |
| 28 | 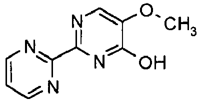 | >50 | |
| 29 | 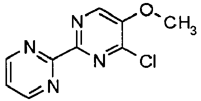 | >50 | |
| 30 | 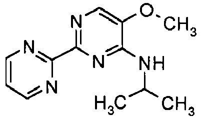 | >50 | |
| 31 | 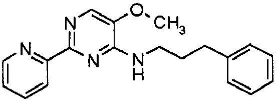 | >50 | |
| 32 | 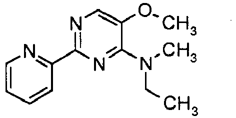 | <50 | |
| 33 | 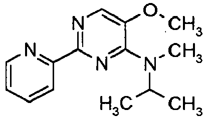 | >50 | |
| 34 | 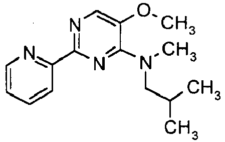 | >50 | |
| 35 | 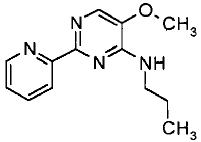 | <50 | |
| 36 | 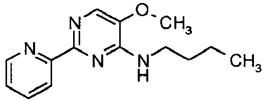 | <50 | |
| 37 | 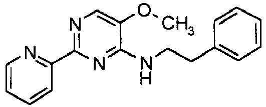 | <50 | |
| 38 | 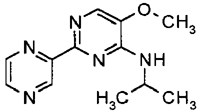 | >50 | |
| 39 | 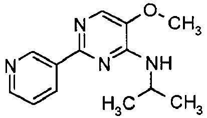 | >50 | |
| 40 | 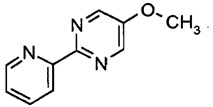 | >50 | |
| 41 | 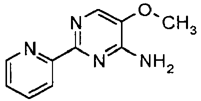 | >50 | |
| 42 | 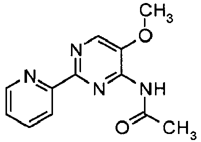 | >50 | |
| 43 | 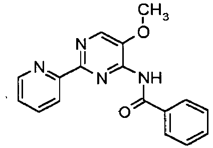 | <50 | |
| 44 | 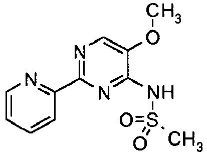 | <50 | |
| 45 | 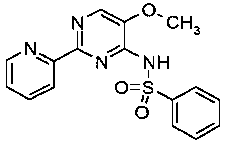 | <50 | |
| 46 | 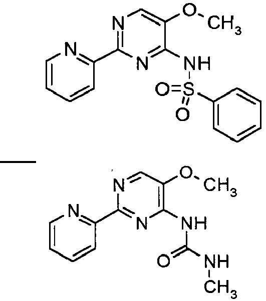 | >50 | |
| 47 | 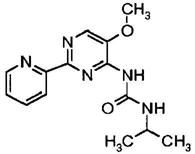 | >50 | |
| 48 | 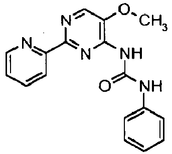 | >50 | |
| 49 | 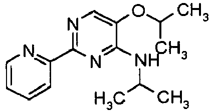 | >50 | |
| 50 | 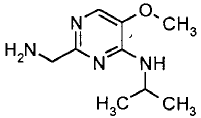 | <50 | |
| 51 | 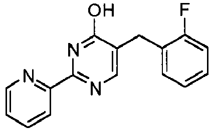 | <50 | |
| 52 | 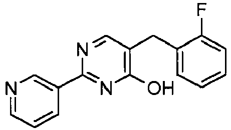 | >50 | |
| 53 | 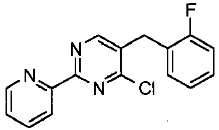 | <50 | |
| 54 | 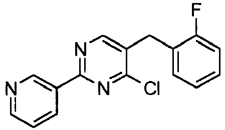 | >50 | |
| 55 | 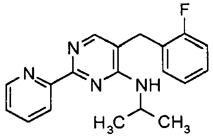 | <50 | |
| 56 | 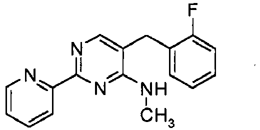 | >50 | |
| 57 | 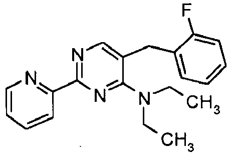 | <50 | |
| 58 | 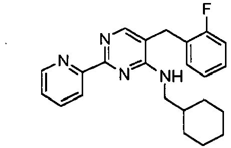 | >50 | |
| 59 | 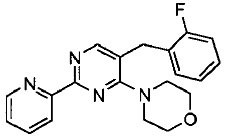 | <50 | |
| 60 | 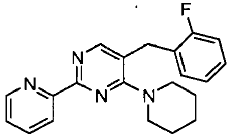 | <50 | |
| 61 | 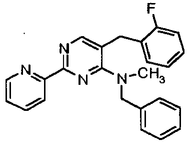 | >50 | |
| 62 | 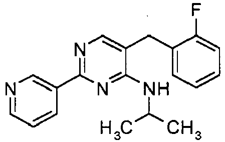 | >50 | |
| 63 | 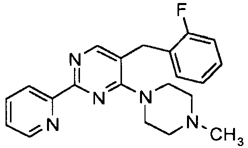 | <50 | |
| 64 | 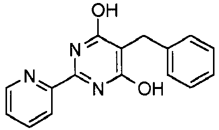 | >50 | |
| 65 | 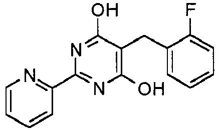 | >50 | |
| 66 | 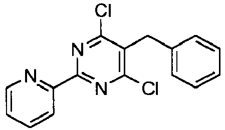 | >50 | |
| 67 | 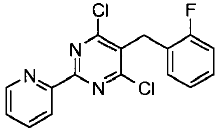 | >50 | |
| 68 | 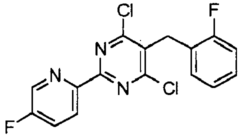 | >50 | |
| 69 | 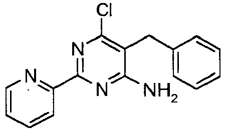 | >50 | |
| 70 | 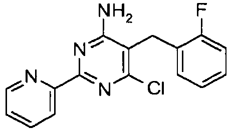 | >50 | |
| 71 | 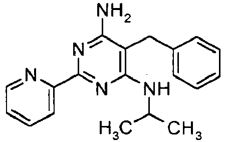 | <50 | |
| 72 | 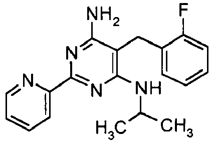 | <50 | |
| 73 | 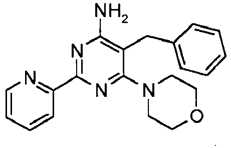 | <50 | |
| 74 | 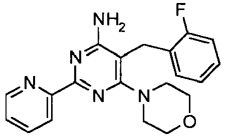 | <50 | |
| 75 | 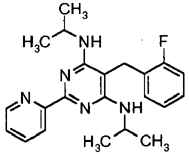 | >50 | |
| 76 | 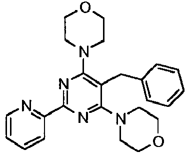 | >50 | |
| 77 | 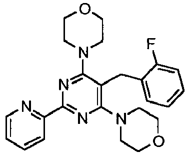 | >50 | |
| 78 | 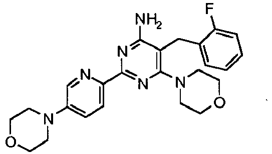 | >50 | |
| 79 | 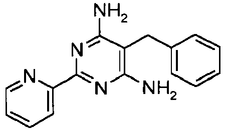 | <50 | |
| 80 | 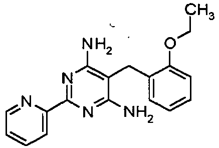 | >50 | |
| 81 | 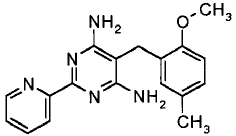 | >50 | |
| 82 | 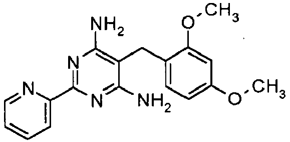 | <50 | |
| 83 | 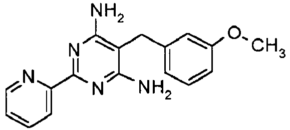 | <50 | |
| 84 | 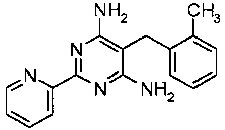 | <50 | |
| 85 | 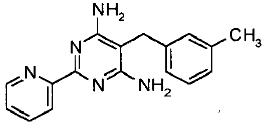 | <50 | |
| 86 | 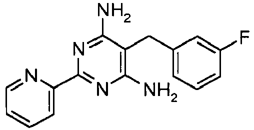 | <50 | |
| 87 | 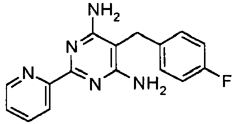 | <50 | |
| 88 | 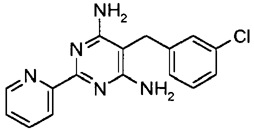 | <50 | |
| 89 | 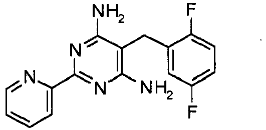 | <50 | |
| 90 | 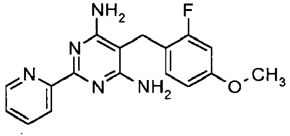 | <50 | |
| 91 | 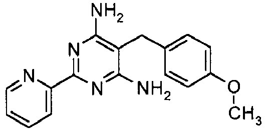 | >50 | |
| 92 | 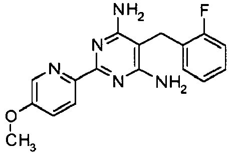 | <50 | |
| 93 | 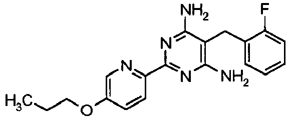 | <50 | |
| 94 | 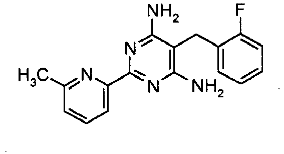 | >50 | |
| 95 | 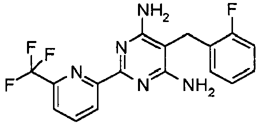 | >50 | |
| 96 | 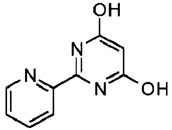 | >50 | |
| 97 | 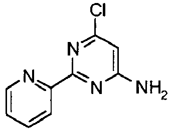 | >50 | |
| 98 | 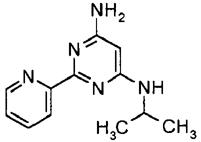 | <50 | |
| 99 | 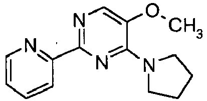 | <50 | |
| 100 | 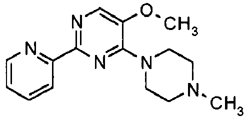 | >50 | |
| 101 | 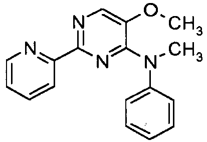 | <50 | |
| 102 | 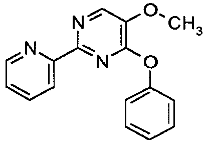 | >50 | |
| 103 | 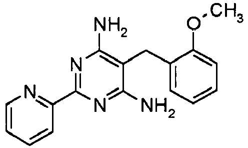 | <50 | |
| 104 | 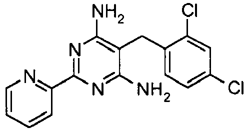 | <50 | |
| 105 | 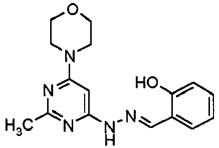 | >50 | |
| 106 | 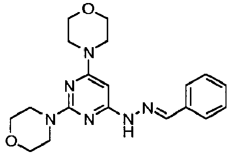 | >50 | |
| 107 | 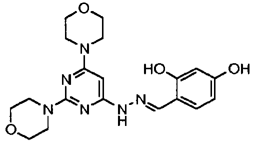 | >50 | |
| 108 | 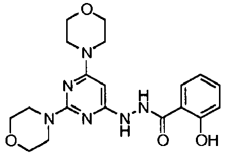 | >50 | |
| 109 | 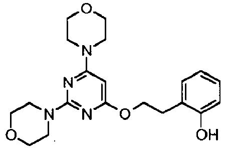 | >50 | |
| 110 | 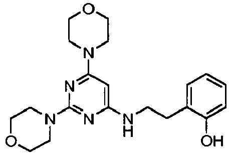 | >50 | |
| 111 | 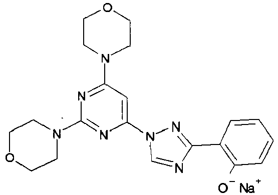 | >50 | |
| 112 | 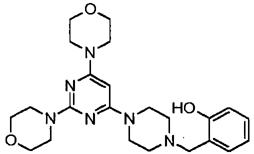 | >50 | |
| 113 | 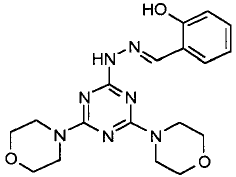 | >50 | |
| 114 | 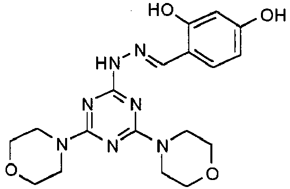 | >50 | |
| 115 | 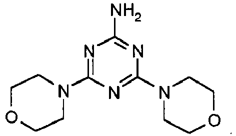 | >50 | |
| 116 | 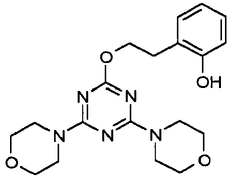 | >50 | |
| 117 | 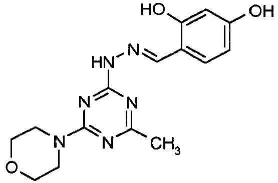 | >50 | |

Darin bezeichnen die mit "#" markierten Verbindungen erfindungsgemäße Beispielverbindungen.

### HERSTELLUNGSBEISPIELE 1 BIS 12:

Der Nachweis und die Reinheit der Verbindungen 1 bis 12 erfolgte jeweils mittels HPLC MS (Hochleistungsflüssigkeitschromatographie (High Performance Liquid Chromatography) mit Massenspektrometrie (MS)) bzw. mittels HPLC mit UV-Detektion (PDA; Photo Diode Array).

Darin handelt es sich bei den Verbindungen 1 bis 8 um erfindungsgemäße Beispielverbindungen.

Hierbei wurde folgende Methode verwendet:

| | |
|---|---|
| Methode: | MS19_7MIN_HIRES_POS / High resolution method |
| Stationäre Phase / Säule: | Waters Atlantis dC18 100 x 2.1 mm, 3µm Säule, 40 °C |
| Mobile Phase: | A - 0.1% Ameisensäure (Wasser) |
| | B - 0.1% Ameisensäure (Acetonitril) |
| Fliessrate: | 0.6 ml/min |
| Injektionsvolumen: | 3µl |
| UV-Detektor: bzw. | 215nm (nominal) |
| MS-Detektion: | TIC (Total Ion Count) |

| Gradient | Zeit (min) | organischer Anteil (%) |
|---|---|---|
| | 0.00 | 5 |
| | 5.00 | 100 |
| | 5.40 | 100 |
| | 5.42 | 5 |

HPLC-MS System: Shimadzu LCMS 2010EV system
Massenbereich: 100-1000 m/z
Scangeschwindigkeit: 2000 amu/sec

**Erfindungsgemäße Verbindung nach Beispiel 1:**

Isopropyl-(5-methoxy-2-pyridin-2-yl-pyrimidin-4-yl)-amin: 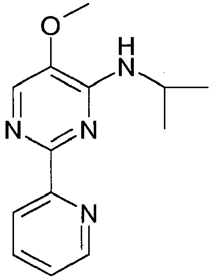
HP-B002012-C01
MW:244.29
Hersteller: BIONET
UV Spektrum: λ max [nm]: 214, 235, 321, 345.
HPLCMS: [m/z] : 245

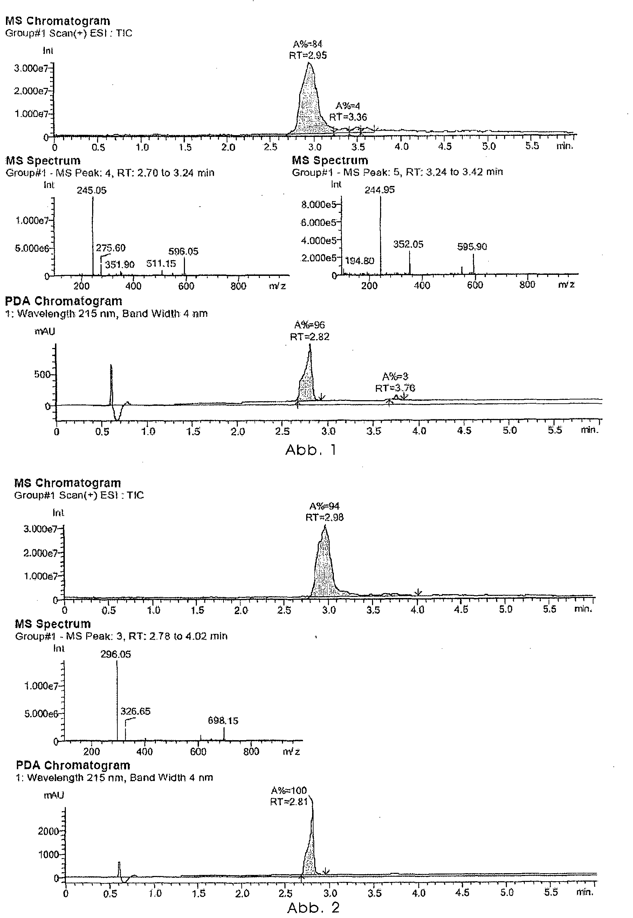

Das Ergebnis zeigt Abbildung 1.

### Erfindungsgemäße Verbindung nach Beispiel 2:

N-(5-Chlor-6-methyl-2-pyridin-2-yl-pyrimidin-4-yl)-N'-(4-trifluornnethyl-pyridin-2-yl)-ethan-1,2-diamin

### ROUTE 21

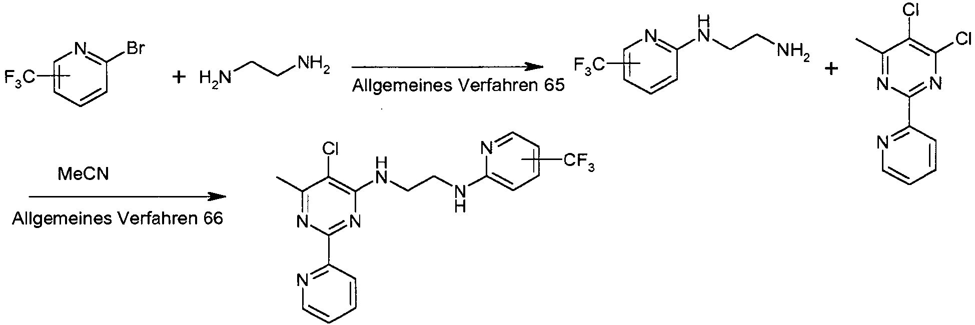

### Allgemeines Verfahren 65:

### N*1*-(4-Trifluormethyl-pyridin-2-yl)-ethan-1,2-diamin

2-Brom-4-(trifluormethyl)pyridin (500 mg, 2.2 mmol) und Ethan-1,2-diamin (12.5 ml, 187.5 mmol) wurden unter Rückfluss für 2 h erhitzt. Nach Abkühlen wurde die Mischung im Vakuum eingeengt, und der Rückstand wurde zwischen DCM und Wasser verteilt. Die wässrige Phase wurde mit DCM extrahiert, und die vereinigten organischen Phasen wurden mit Wasser gewaschen, getrocknet (MgSO₄) und im Vakuum eingeengt, um die Titelverbindung zu ergeben (330 mg, 72 %), die ohne weitere Reinigung eingesetzt wurde. Die Verbindung konnte nicht durch HPLCMS nachgewiesen werden, die Struktur wurde daher durch NMR bestätigt.

### Allgemeines Verfahren 66:

### N-(5-Chlor-6-methyl-2-pyridin-2-yl-pyrimidin-4-yl)-N'-(4-trifluormethyl-pyridin-2-yl)-ethan-1,2-diamin (Beispiel 2)

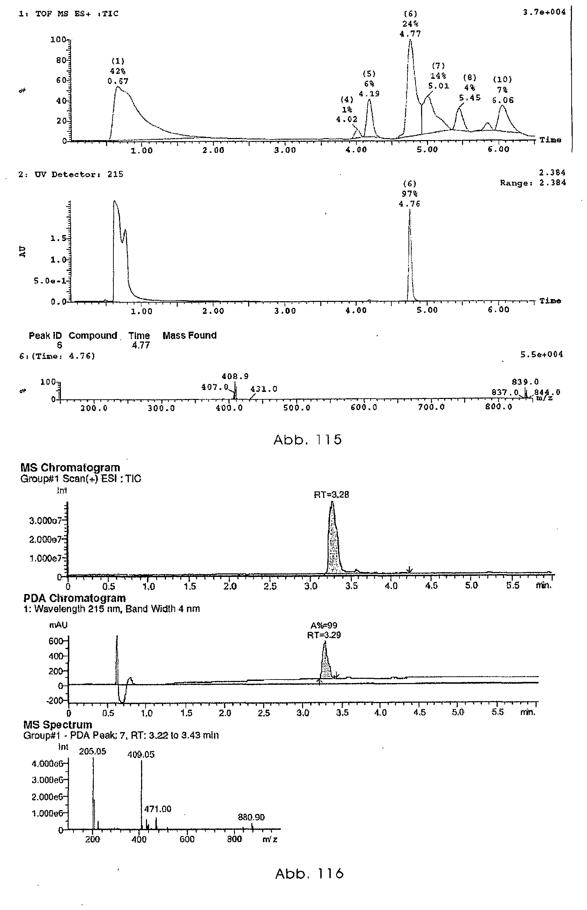

4,5-Dichlor-6-methyl-2-pyridin-2-yl-pyrimidin (144 mg, 0.63 mmol) wurde zu einer Lösung von *N**1*-(4-Trifluormethyl-pyridin-2-yl)-ethan-1,2-diamin (120 mg, 0.63 mmol) in MeCN (5 ml) gegeben, und die Mischung wurde bei Raumtemperatur für 18 h gerührt, gefolgt von Erwärmen unter Rückfluss für 4 h. Nach Abkühlung wurde die Mischung im Vakuum eingeengt. Der Rohrückstand wurde durch Säulenchromatographie mit EtOAc/Heptan (0:100 - 100:0) als Eluent gereinigt, um die Titelverbindung zu ergeben (35 mg, 13 %).
MW: 408.8
HPLCMS (Methode A wie für die Verbindungen der Beispiele 13-104 beschrieben):
[m/z]: 408.9
Abb. 115 zeigt die Chromatogramme/Spektren der Verbindung von Beispiel 2.
IC50 [*µ*M]: >40

### Erfindungsgemäße Verbindung nach Beispiel 3:

### 5-(2-Fluor-benzyl)-2-pyridin-2-yl-pyrimidin-4,6-diamin

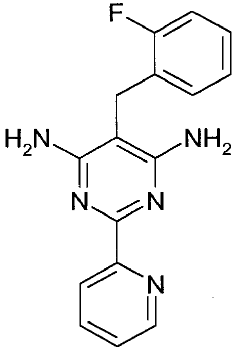

HP-AB002020-B09
MW: 295.31
Hersteller: BIONET
UV Spektrum: λ max [nm]: 195, 225, 293
HPLCMS: [m/z] : 296

Das Ergebnis zeigt Abbildung 2.

### Erfindungsgemäße Verbindung nach Beispiel 4:

### N*1*-(5-Trifluormethyl-pyridin-2-yl)-ethan-1,2-diamin

In ähnlicher Weise, unter Verwendung von Route 21, allgemeines Verfahren 65 (siehe Beispiel 2), ergaben 2-Brom-5-(trifluormethyl)pyridin (100 mg, 0.44 mmol) und Ethane-1,2-diamin (2.5 ml, 37.5 mmol) die Titelverbindung (60 mg, 65 %), die ohne weitere Reinigung verwendet wurde. Die Verbindung konnte nicht durch HPLCMS nachgewiesen werden, die Struktur wurde daher durch NMR bestätigt.

### N-(5-Chlor-6-methyl-2-pyridin-2-yl-pyrimidin-4-yl)-N'-(5-trifluormethyl-pyridin-2-yl)-ethan-1,2-diamin (Beispiel 4)

In ähnlicher Weise ergaben, unter Verwendung von Route 21, allgemeines Verfahren 66 (siehe Beispiel 2) *N**1*-(5-Trifluormethyl-pyridin-2-yl)-ethan-1,2-diamin (60 mg, 0.32 mmol) und 4,5-Dichlor-6-methyl-2-pyridin-2-yl-pyrimidin (77 mg, 0.32 mmol) in Dioxan (5 ml) die Titelverbindung.
MW: 408.8
HPLCMS (Methode A wie für die Verbindungen der Beispiele 13-104 beschrieben):
[m/z]: 409
Abb. 116 zeigt das MS-Chromatogramm, das MS-Spektrum und das PDA-Chromatogramm der Verbindung von Beispiel 4.
IC50 [*µ*M]: >40

### Erfindungsgemäße Verbindung nach Beispiel 5:

### 3-[(2,6-Di-morpholin-4-yl-pyrimidin-4-yl)-hydrazonomethyl]-phenol

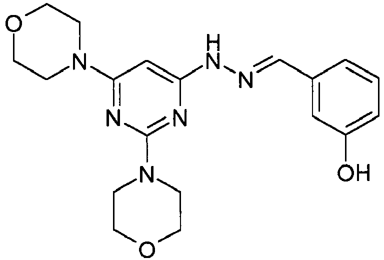

HP-AN003030-E11
MW:384.43
Hersteller: VITAS M LABS
UV Spektrum: λ max [nm]: 214, 235, 321, 345.
HPLCMS: [m/z] : 385

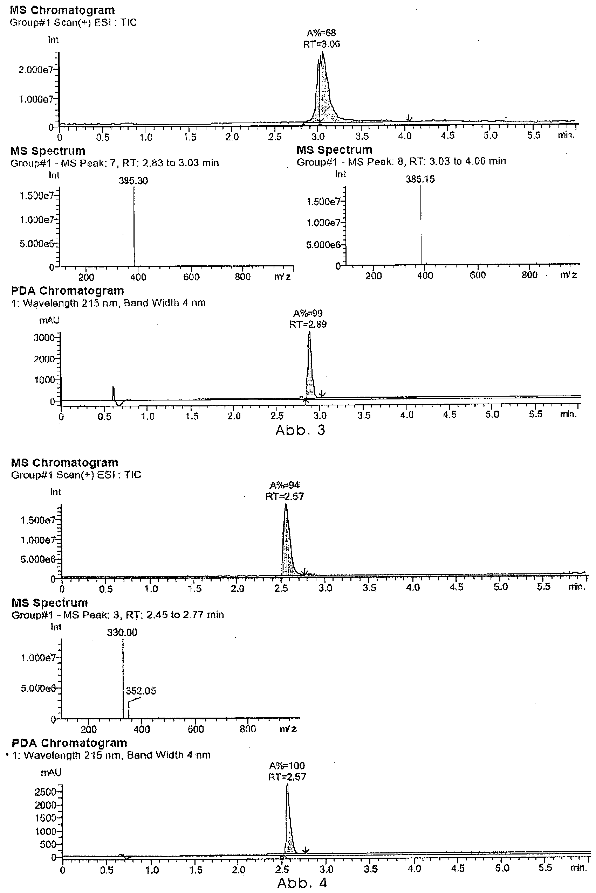

Das Ergebnis zeigt Abbildung 3.

### Erfindungsgemäße Verbindung nach Beispiel 6:

### 4-[(2-Methyl-6-morpholin-4-yl-pyrimidin-4-yl)-hydrazonomethyl]-benzen-1,3-diol

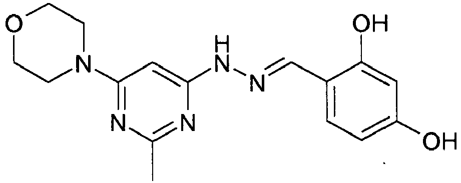

HP-AA004168-B11
MW:329.35
Hersteller: ASINEX
UV Spektrum: λ max [nm]: 212, 241, 346
HPLCMS: [m/z] : 330

Das Ergebnis zeigt Abbildung 4.

### Erfindungsgemäße Verbindung nach Beispiel 7:

### 2-[(2,6-Di-morpholin-4-yl-pyrimidin-4-yl)-hydrazonomethyl]-phenol

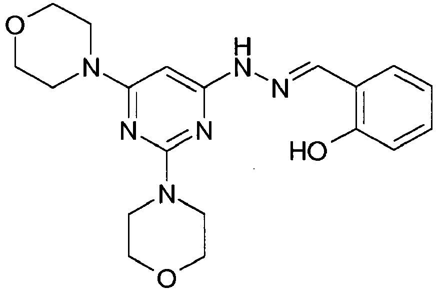

HP-AN003030-F11
MW:384.43
Hersteller: VITAS M LABS
UV Spektrum: λ max [nm]: 222, 284,332
HPLCMS: [m/z] : 385

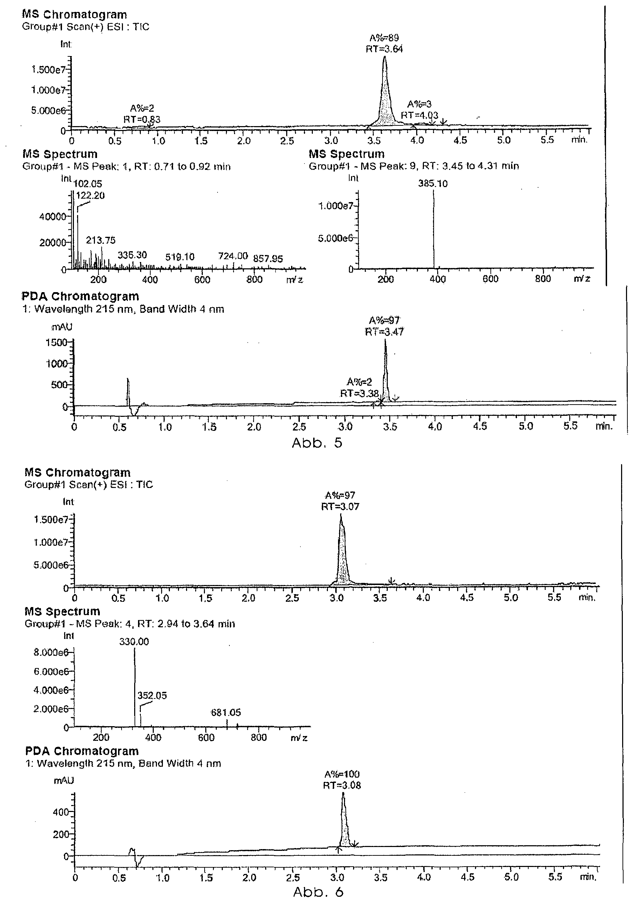

Das Ergebnis zeigt Abbildung 5.

### Erfindungsgemäße Verbindung nach Beispiel 8:

### N-[1-(4-Fluorphenyl)-ethyliden]-N'-(2-methyl-6-morpholin-4-yl-pyrimidin-4-yl)-hydrazin

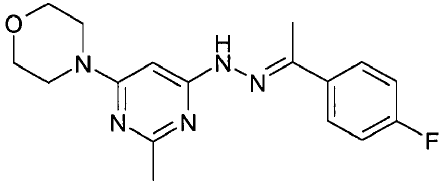

HP-AA004168-D11
MW:329.37
Hersteller: ASINEX
UV Spektrum: λ max [nm]: 198, 230, 322
HPLCMS: [m/z] : 330

Das Ergebnis zeigt Abbildung 6.

### Verbindung nach Beispiel 9:

### 2-[(4,6-Di-morpholin-4-yl-[1,3,5]triazin-2-yl)-hydrazonomethyl]-4-methoxyphenol

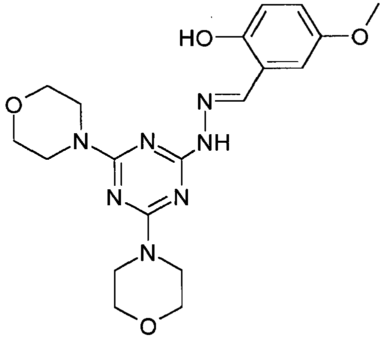

HP-AA004154-A01
MW:415.45
Hersteller: ASINEX
UV Spektrum: λ max [nm]: 232, 290, 343
HPLCMS: [m/z] : 416

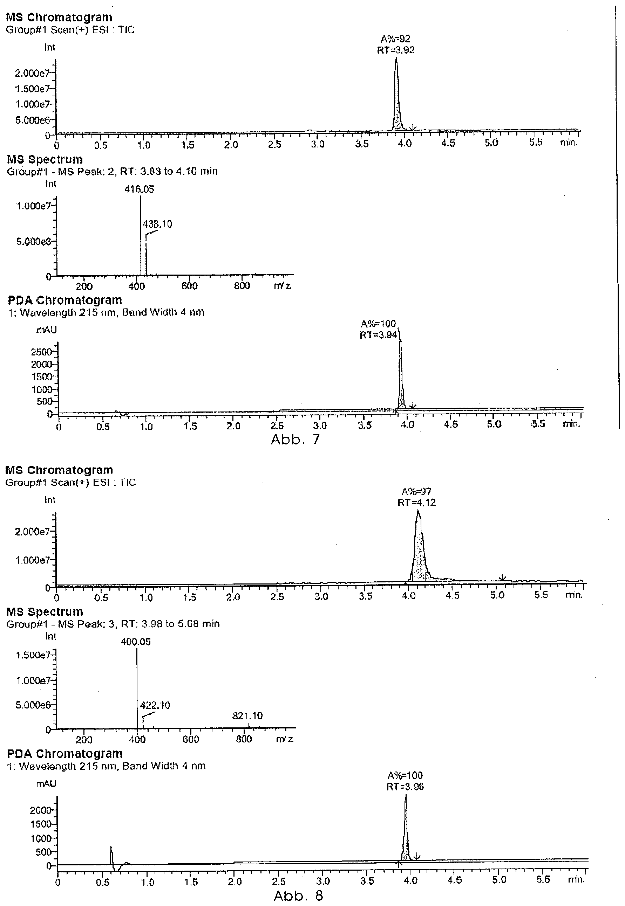

Das Ergebnis zeigt Abbildung 7.

### Verbindung nach Beispiel 10:

### (4-Imidazol-1-yl-6-morpholin-4-yl-[1,3,5]triazin-2-yl)-diphenyl-amin

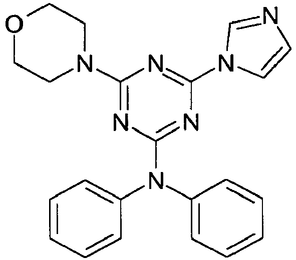

HP-AN004039-H04
MW:399.48
Hersteller: VITASMLAB
UV Spektrum: λ max [nm]: 195, 239
HPLCMS: [m/z] : 400

Das Ergebnis zeigt Abbildung 8.

### Verbindung nach Beispiel 11:

### (4-Imidazol-1-yl-6-morpholin-4-yl-[1,3,5]triazin-2-yl)-methyl-phenyl-amin

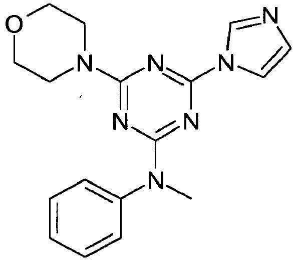

HP-AN004039-F04
MW:337.38
Hersteller: VITASMLAB
UV Spektrum: λ max [nm]: 190, 202, 235
HPLCMS: [m/z] : 338

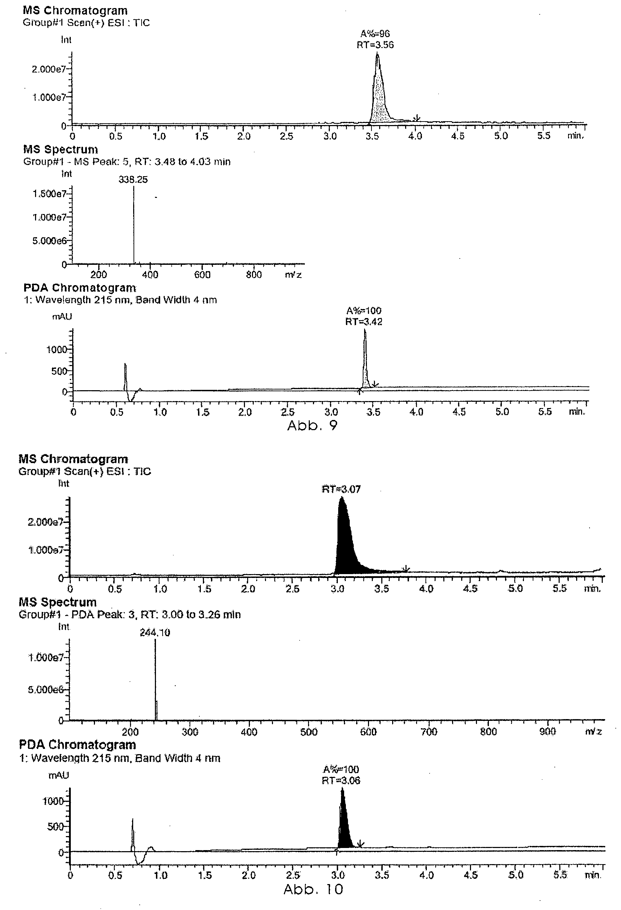

Das Ergebnis zeigt Abbildung 9.

### Beispiel 12

### (4,6-Di-morpholin-4-yl-[1,3,5]triazin-2-yl)-(2-methyl-chinolin-6-yl)-amin

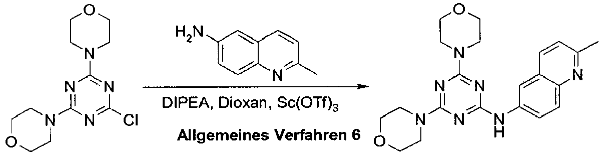

6-Amino-2-methylchinolin (30 mg, 0.19 mmol) wurde zu einer Lösung von 2-Chlor-4,6-di-morpholin-4-yl-[1,3,5]triazin (50 mg, 0.18 mmol) in Dioxan (0.5 ml), gefolgt von DIPEA (92 *µ*l, 0.53 mmol) gegeben, und die Mischung wurde bei 50°C für 1 Stunde erhitzt. Die Temperatur wurde auf 90°C für 1 h und auf 100°C für 18 h erhöht. Lediglich 4 % Umsatz zum gewünschten Produkt traten auf, daher wurde die Mischung in eine Mikrowellenröhre zusammen mit einem Überschuss von 6-Amino-2-methylchinolin und katalytischen Mengen Scandiumtriflat überführt. Die Mischung wurde auf 150°C in der Mikrowelle für insgesamt 3,5 h erhitzt. Nach Abkühlen wurde die Mischung im Vakuum eingeengt. Der Rohrückstand wurde mit MeOH verfestigt, um die Titelverbindung (17 mg, 24 %) zu ergeben.
MW: 407.48
HPLCMS (Methode A wie für die Verbindungen der Beispiele 113-117) beschrieben: [m/z]: 408

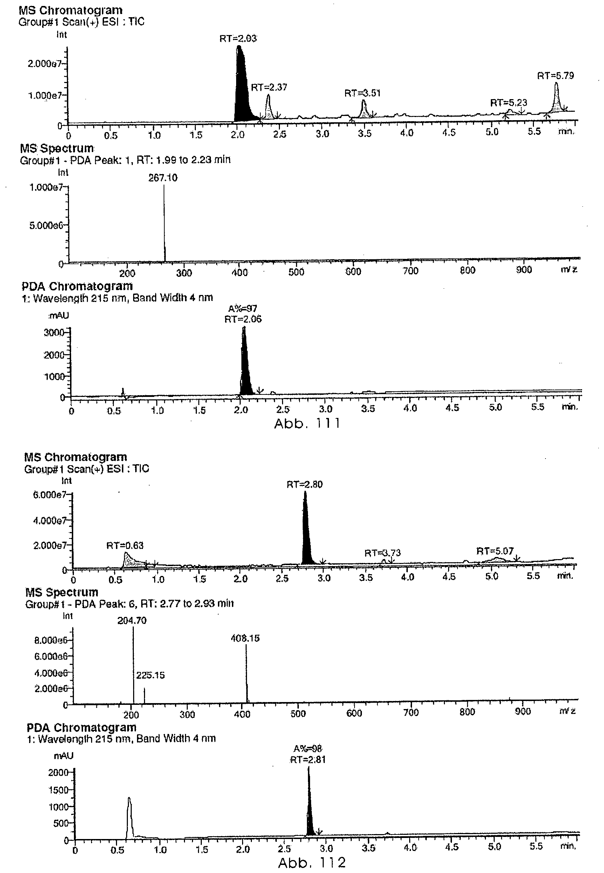

Abb. 112 zeigt das MS-Chromatogramm, das MS-Spektrum und das PDA-Chromatogramm der Verbindung von Beispiel 12.
IC50 [*µ*M]: <50

### HERSTELLUNGSBEISPIELE 13 BIS 104:

Bei den nachfolgenden Beispielen 13 bis 104 wurden folgende analytische Methoden angewendet:

### Analytische HPLC-MS

### Methode A

Säule: Waters Atlantis dC18 (2.1 x100mm, 3*µ*m Säule)
Fließgeschwindigkeit bzw. Fließrate: 0.6 ml/min
Lösungsmittel A: 0.1% Ameisensäure / Wasser
Lösungsmittel B: 0.1% Ameisensäure / Acetonitril
Injektions-Volumen: 3 *µ*l
Säulentemperatur: 40°C
UV Detektions-Wellenlänge: 215nm
Eluent: 0 min (= Minuten) bis 5 min, konstanter Gradient von 95% Lösungsmittel A + 5% Lösungsmittel B bis 100% Lösungsmittel B; 5 min bis 5.4 min, 100% Lösungsmittel B; 5.4 min bis 5.42 min, konstanter Gradient von 100% Lösungsmittel B bis 95% Lösungsmittel A + 5% Lösungsmittel B; 5.42 min bis 7.00 min, 95% Lösungsmittel A + 5% Lösungsmittel B

### Methode B

Säule: Waters Atlantis dC18 (2.1 x 50mm, 3*µ*m)
Lösungsmittel A: 0.1% Ameisensäure / Wasser
Lösungsmittel B: 0.1% Ameisensäure / Acetonitril
Fließgeschwindigkeit 1 ml/min
Injektions-Volumen 3*µ*l
UV Detektions-Wellenlänge: 215nm
Eluent: 0 to 2.5 min, konstanter Gradient von 95% Lösungsmittel A + 5% Lösungsmittel B bis 100% Lösungsmittel B; 2.5 min bis 2.7 min, 100% Lösungsmittel B; 2.71 bis 3.0 min, 95% Lösungsmittel A + 5% Lösungsmittel B.

### Methode C

Säule: Waters Atlantis dC18 (2.1 x 30mm, 3*µ*m Säule)
Fließgeschwindigkeit: 1 ml/min
Lösungsmittel A: 0.1% Ameisensäure / Wasser
Lösungsmittel B: 0.1% Ameisensäure / Acetonitril
Injektions-Volumen: 3*µ*l
UV Detektions-Wellenlänge: 215nm
Eluent: 0 min bis 1.5 min, konstanter Gradient von 95% Lösungsmittel A + 5% Lösungsmittel B bis 100% Lösungsmittel B; 1.5 min bis 1.6 min, 100% Lösungsmittel B; 1.60 min bis 1.61 min, konstanter Gradient von 100% Lösungsmittel B bis 95% Lösungsmittel A + 5% Lösungsmittel B; 1.61 min bis 2.00 min, 95% Lösungsmittel A + 5% Lösungsmittel B.
MS Detektion unter Verwendung von Waters LCT oder LCT Premier, oder ZQ oder ZMD
UV Detektion unter Verwendung von Waters 2996 Photodioden-Array oder Waters 2787 UV oder Waters 2788 UV

### Methode D

Säule: Atlantis dC18 50mm x 3 mm; 3 *µ*m
Mobile Phase A: 0.1% Ameisensäure / Wasser
Mobile Phase B: 0.1% Ameisensäure / Acetonitril
Fließgeschwindigkeit: 0.8 ml/min.
Detektions-Wellenlänge: Dioden-Array-Spektrum I max (mit Scan im Bereich von 210-350nm)
Sampling-Rate: 5
Säulentemperatur: 35°C
Injektions-Volumen: 5 *µ*l
Eluent: 0 min 95% Lösungsmittel A + 5% Lösungsmittel B, 0.2 min 95% Lösungsmittel A + 5% Lösungsmittel B; 0.2 min bis 3.2 min konstanter Gradient von 95% Lösungsmittel A + 5% Lösungsmittel B bis 5% Lösungsmittel A und 95% Lösungsmittel B; 5 min 5% Lösungsmittel A und 95% Lösungsmittel B; 5 min bis 5.2 min konstanter Gradient von 5% Lösungsmittel A und 95% Lösungsmittel B bis 95% Lösungsmittel A + 5% Lösungsmittel B; 5.5 min 95% Lösungsmittel A und 5% Lösungsmittel B.
MS Detektion unter Verwendung von Waters LCT oder LCT Premier, oder ZQ oder ZMD
UV Detektion unter Verwendung von Waters 2996 Photodioden-Array oder Waters 2787 UV oder Waters 2788 UV

### Methode E

Säule: Phenomenex Gemini C18 2.0 x 100mm; 3 *µ*m
Mobile Phase A: 2 mM Ammoniumbicarbonat, auf pH=10 gepuffert
Mobile Phase B: Acetonitril
Fließgeschwindigkeit: 0.5 ml/min.
UV Detektions-Wellenlänge: 215 nm
Säulentemperatur: 60°C
Injektions-Volumen: 3 *µ*l
Eluent: 0 min 95% Lösungsmittel A + 5% Lösungsmittel B, 0.2 min bis 5.50 min konstanter Gradient von 95% Lösungsmittel A + 5% Lösungsmittel B bis 100% Lösungsmittel B; 5.50 - 5.90 min 100% Lösungsmittel B; 5.90 - 5.92 min Gradient von 100% Lösungsmittel B bis 95% Lösungsmittel A + 5% Lösungsmittel B.

### Präparative HPLC - Neutrale Bedingungen

Säule: Waters SunFire Prep C18 OBD (5*µ*m 19 x 100mm)
Fließgeschwindigkeit: 20ml/min
Lösungsmittel A: Wasser
Lösungsmittel B: Acetonitril
Injektions-Volumen: 1000□l
Säulentemperatur: Raumtemperatur
Detektion: UV gerichtet
Eluent: 0 min bis 2 min, 5% Lösungsmittel B + 95% Lösungsmittel A; 2 min bis 2.5 min konstanter Gradient bis 10% Lösungsmittel B + 90% Lösungsmittel A, 2.5 min bis 14.5 min konstanter Gradient bis 100% Lösungsmittel B; 14.5 min bis 16.5 min 100% Lösungsmittel B; 16.5 bis 16.7 min konstanter Gradient bis 5% B + 95% A; 16.7 min bis 17.2 min 5% Lösungsmittel B + 95% Lösungsmittel A.
Gilson semi-präparative HPLC-Module mit 119 UV Detektor und 5.11 Unipoint Steuerungssoftware

### Präparative HPLC - Saure Bedingungen

Säule: Waters SunFire Prep C18 OBD (5*µ*m 19 x 100mm)
Fließgeschwindigkeit: 26ml/min
Lösungsmittel A: 0.1% TFA / Wasser
Lösungsmittel B: 0.1% TFA / Acetonitril
Injektions-Volumen: 1000*µ*l
Säulentemperatur: Raumtemperatur
Detektion: Massengerichtet
Eluent: 0 min bis 1 min 90% Lösungsmittel A + 10% Lösungsmittel B; 1 min bis 7.5 min, konstanter Gradient von 90% Lösungsmittel A + 10% Lösungsmittel B bis 100% Lösungsmittel B; 7.5 min bis 9 min, 100% Lösungsmittel B; 9 min bis 9.1 min, konstanter Gradient von 100% Lösungsmittel B bis 90% Lösungsmittel A + 10% Lösungsmittel B; 9.1 min bis 10 min, 90% Lösungsmittel A + 10% Lösungsmittel B.
Waters Micromass Plattform LCZ "single quadrupole mass spectrometer".
Waters 600 Lösungsmittelsbereitstellungsmodul
Waters 515 Hilfspumpen
Waters 2487 UV Detektor
Gilson 215 Autosampler und Fraktionssammler

### Präparative HPLC - Basische Bedingungen

Säule: XBridge Prep C18 OBD (5*µ*m 19 x 100mm)
Fließgeschwindigkeit: 20ml/min
Lösungsmittel A: Wasser + 0.2% Ammoniumhydroxid
Lösungsmittel B: Acetonitril + 0.2% Ammoniumhydroxid
Injektions-Volumen: 1000*µ*l
Säulentemperatur: Raumtemperatur
Detektion: UV-gerichtet
Eluent: 0 min bis 2 min, 5% Lösungsmittel B + 95% Lösungsmittel A; 2 min bis 2.5 min konstanter Gradient bis 10% Lösungsmittel B + 90% Lösungsmittel A, 2.5 min bis 14.5 min konstanter Gradient bis 100% Lösungsmittel B; 14.5 min bis 16.5 min 100% Lösungsmittel B; 16.5 bis 16.7 min konstanter Gradient bis 5% B + 95% A; 16.7 min bis 17.2 min 5% Lösungsmittel B + 95% Lösungsmittel A.
Gilson semi-präparative HPLC-Module mit 119 UV Detektor und 5.11 Unipoint Steuerungssoftware

Flash-Silicagel-Chromatograpie wurde an Silicagel 230-400 mesh oder an vorgepackten Silica-Kartuschen durchgeführt.

Mikrowellen-Reaktionen wurden unter Verwendung eines CEM Discover oder Explorer fokussierten Mikrowellen-Apparates durchgeführt.

### Verbindungsbenennung

Einige Verbindungen wurden als TFA oder HCl-Salze isoliert, was sich nicht in deren chemischer Benennung niederschlägt. Soweit anwendbar bezeichnet Im Rahmen der vorliegenden Erfindung der chemische Name daher die Verbindung sowohl in der neutralen Form als auch als TFA-Salz oder als irgendein anderes Salz, insbesondere ein pharmazeutisch verträgliches Salz. Abkürzungen:
- nBuLi: n-Butyllithium
- nBuOH: n-Butanol
- cat: katalytisch
- mCPBA: m-Chlorperoxybenzoesäure
- DCM: Dichlormethan
- DIPEA: N,N-Diisopropylethylamin
- DMF: N,N-Dimethylformamid
- Et₂O: Diethylether
- EtOAc: Ethylacetat
- EtOH: Ethanol
- h: Stunde(n)
- HPLC: High Performance Liquid Chromatography
- LiHMDS: Lithiumhexamethyldisilazid
- MeCN: Acetonitril
- MeOH: Methanol
- min: Minute(n)
- MW: Molekulargewicht
- NaOMe: Natriummethoxid
- Pd₂(dba)₃: Tris(dibenzylidenaceton)dipalladium(0)
- nPrOH: n-Propanol
- Py: Pyridin
- TEA: Triethylamin
- THF: Tetrahydrofuran
- TMSOTf: Trimethylsilyltrifluormethansulfonat

IC50 [*µ*M]-Werte wurden wie oben beschrieben bestimmt.

Einige Ausgangsverbindungen sind kommerziell erhältlich, wie zum Beispiel einige Dichlorpyimidine und Trichlorpyrimidine. Diese wurden analog zu den allgemein beschriebenen Synthesemethoden (siehe Patenttext und nachfolgende allgemeinen Schemata), wie dem Fachmann vertraut, zu den Endprodukten umgesetzt. Als käufliche Ausgangsverbindungen seien hier beispielhaft das 4,6-Dichlorpyrimidin [1193-21-1] und das 2,4,6-Trichlorpyrimidin [3764-01-01] von Sigma Aldrich genannt.

### Beispiel 13:

Die Verbindung von Beispiel 13 wurde entsprechend nachfolgender Route 1 hergestellt:

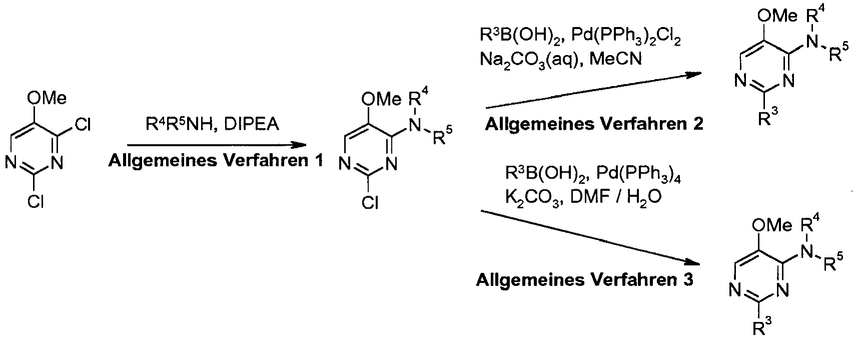

### Allgemeines Verfahren 1:

### 2-(Chlor-5-methoxy-pyrimidin-4-yl)-isopropyl-amin

Isopropylamin (0.86 ml, 10.02 mmol) wurde tropfenweise zu einer Lösung von 2,4-Dichlor-5-methoxy-pyrimidin (1.63 g, 9.11 mmol) und DIPEA (1.91 ml, 10.93 mmol) in EtOH (33 ml) gegeben. Die Reaktionsmischung wurde bei Raumtemperatur für 29 h gerührt und im Vakuum eingeengt. Der Rückstand wurde in EtOAc gelöst und mit gesättigter wässriger NaHCO₃-Lösung und Kochsalzlösung gewaschen. Die organische Phase wurde getrocknet (Na₂SO₄) und im Vakuum eingeengt. Das Rohprodukt wurde durch Säulenchromatographie mit EtOAc/Heptan (45:55) als Eluent gereingt, um die Titelverbindung zu ergeben (1.1 g, 60 %).
MW: 201.66
HPLCMS (Methode B):[m/z]: 202

### Allgemeines Verfahren 2:

### Isopropyl-(5-methoxy-2-phenyl-pyrimidin-4-yl)-amin (Beispiel 13)

Bis(triphenylphosphin)palladium(II)-dichlorid (27 mg, 36 *µ*mol) wurde zu einer Mischung von (2-Chlor-5-methoxy-pyrimidin-4-yl)-isopropyl-amin (150 mg, 0.75 mmol), Phenylborsäure (90 mg, 0.75 mmol), Na₂CO₃ (1 M Lösung in Wasser, 0.75 ml, 1.50 mmol) und MeCN (1.5 ml) in einer Mikrowellenröhre gegeben. Die Mischung wurde mit N₂ für 5 min. entgast. Die Reaktionsmischung wurde bei 150°C für 5 min in der Mikrowelle erhitzt. Die Reaktionsmischung wurde abfiltriert und die organische Phase des Filtrates wurde abgetrennt. Die wässrige Phase wurde mit EtOAc (x3) extrahiert. Die vereinigten organischen Phasen wurden getrocknet (Na₂SO₄) und im Vakuum eingeengt. Das Rohprodukt wurde durch präparative HPLC (neutrale Bedingungen) gereinigt, um die Titelverbindung (95 mg, 52 %) zu ergeben.
MW: 243.31
HPLCMS (Methode A):[m/z]: 244
Abb. 10 zeigt das MS-Chromatogramm, das MS-Spektrum und das PDA-Chromatogramm der Verbindung von Beispiel 13.
IC50 [*µ*M]: >50

### Beispiel 14

### Isopropyl-(5-methoxy-2-pyridin-4-yl-pyrimidin-4-yl)-amin

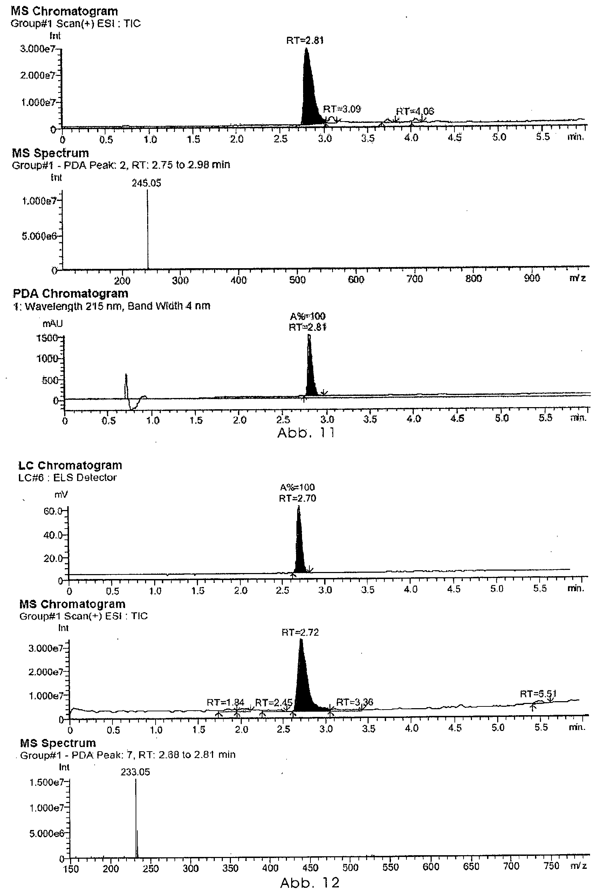

In ähnlicher Weise, unter Verwendung von Route 1, allgemeines Verfahren 2, ergab Bis(triphenylphosphin)palladium(II)-dichlorid (36 mg, 51 *µ*mol), (2-Chlor-5-methoxy-pyrimidin-4-yl)-isopropyl-amin (200 mg, 1.0 mmol), Pyridin-4-yl-borsäure (120 mg, 1.0 mmol), Na₂CO₃ (1 M Lösung in Wasser, 0.5 ml, 2.0 mmol) die Titelverbindung (20 mg, 7 %) nach Reinigung durch präparative HPLC (neutrale Bedingungen).
MW: 244.30
HPLCMS (Methode A):[m/z]: 245
Abb. 11 zeigt das MS-Chromatogramm, das MS-Spektrum und das PDA-Chromatogramm der Verbindung von Beispiel 14.
IC50 [*µ*M]: >50

### Beispiel 15

### Allgemeines Verfahren 3:

### Isopropyl-[5-methoxy-2-(1H-pyrrol-2-yl)pyrimidin-4-yl]-amin

(2-Chlor-5-methoxy-pyrimidin-4-yl)-isopropyl-amin (0.2 g, 0.99 mmol), Kaliumarbonat (0.27 g, 1.9 mmol), N-Boc-2-pyrrolborsäure (0.31 g, 1.4 mmol) in DMF (3 ml) und Wasser (1.5 ml) wurden entgast und Tetrakis(triphenylphosphin)palladium(0) (57 mg, 0.05 mmol) wurde unter Argon zugegeben. Die Reaktionsmischung wurde für 10 min. bei 150°C in der Mikrowelle erhitzt. Wasser (10 ml) wurde hinzugegeben und die wässrige Phase wurde mit DCM (x3) extrahiert. Die vereinigten organischen Phasen wurden getrocknet (Na₂SO₄) und im Vakuum eingeengt. Der Rohrückstand wurde durch Säulenchromatographie mit EtOAc/Hexan (1:9 - 3:7) als Eluent gereinigt, um die Titelverbindung zu ergeben (0.048 g, 21 %).
MW: 232.28
HPLCMS (Methode A): [m/z]: 233
Abb. 12 zeigt das LC-Chromatogramm, das MS-Spektrum und das MS-Chromatogramm der Verbindung von Beispiel 15.
IC50 [*µ*M] > 50

### Beispiel 16

### Isopropyl-[5-methoxy-2-(1H-pyrazol-5-yl)pyrimidin-4-yl]-amin

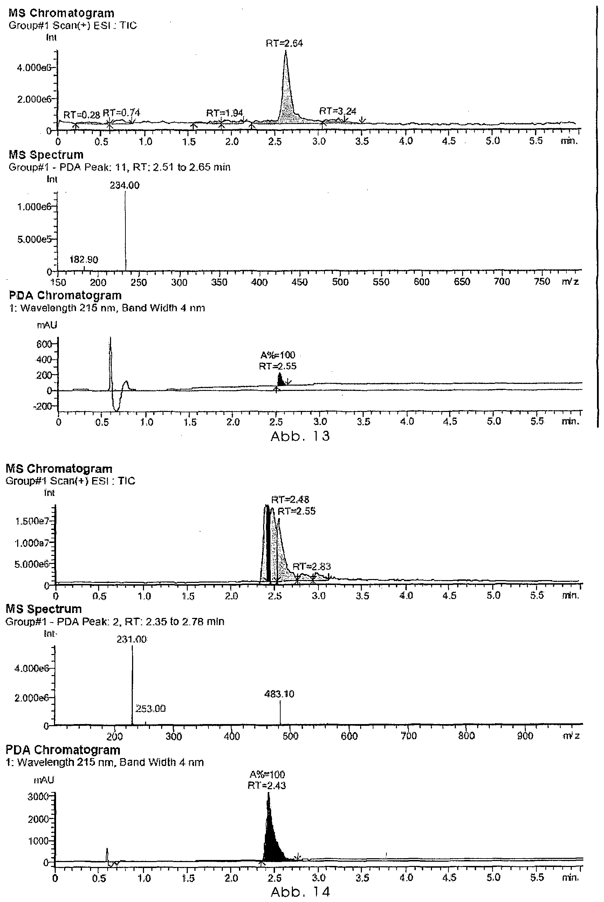

In ähnlicher Weise, unter Verwendung von Route 1, allgemeines Verfahren 3, ergaben (2-Chlor-5-methoxy-pyrimidin-4-yl)-isopropyl-amin (0.1 g, 0.4 mmol), Kaliumcarbonat (0.14 g, 0.98 mmol), 1H-Pyrrazol-5-borsäure (82 mg, 0.68 mmol) und Tetrakis(triphenylphosphin)palladium (0) (0.06 g, 0.034 mmol) die Titelverbindung (27 mg, 25 %) nach Reinigung durch Säulenchromatographie mit DCM/MeOH (98:2) als Eluent.
MW: 233.27
HPLCMS (Methode A): [m/z]: 234
Abb. 13 zeigt das MS-Chromatogramm, das MS-Spektrum und das PDA-Chromatogramm der Verbindung von Beispiel 16.
IC50 [*µ*M]: > 50

### ROUTE 2

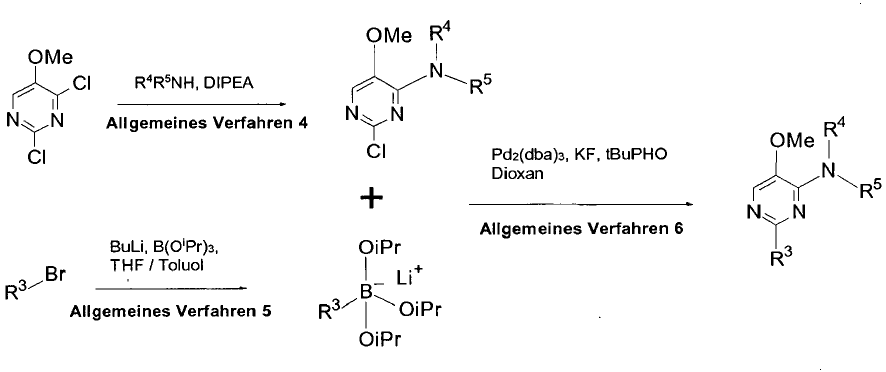

### Allgemeines Verfahren 4:

### (2-Chlor-5-methoxy-pyrimidin-4-yl)-ethyl-amin

2,4-Dichlor-5-methoxypyrimidin (0.1 g, 0.56 mmol), Ethylamin (27 mg, 0.64 mmol) und DIPEA (0.12 ml, 0.67 mmol) wurden in Ethanol (2 ml) gelöst, und die Mischung wurde bei Raumtemperatur für 15 h gerührt. Die Mischung wurde im Vakuum eingeengt. Der Rückstand wurde mit Wasser (15 ml) verdünnt, und die Reaktionsmischung wurde mit EtOAc (x 3) extrahiert. Die vereinigten organischen Phasen wurden getrocknet (Na₂SO₄) und im Vakuum eingeengt, um die Titelverbindung zu ergeben (104 mg, 100 %).
MW: 187.63
HPLCMS (Methode D): [m/z]: 188

### (2-Chlor-5-methoxy-pyrimidin-4-yl)-isobutyl-amin

In ähnlicher Weise, unter Verwendung von Route 2, allgemeines Verfahren 4, ergaben 2,4-Dichlor-5-methoxypyrimidin (0.3 g, 1.6 mmol), Isobutylamin (0.13 g, 1.84 mmol) und DIPEA (0.58 ml, 3.3 mmol) die Titelverbindung (0.36 g, 99 %).
MW: 215.68
HPLCMS (Methode D): [m/z]: 216

### (2-Chlor-5-methoxy-pyrimidin-4-yl)-cyclopropylmethyl-amin

In ähnlicher Weise, unter Verwendung von Route 2, allgemeines Verfahren 4, ergaben 2,4-Dichlor-5-methoxypyrimidin (0.3 g, 1.6 mmol), Cyclopropanmethylamin-hydrochlorid (0.20 g, 1.84 mmol) und DIPEA (0.58 ml, 3.3 mmol) die Titelverbindung (0.36 g, 99 %).
MW: 213.67
HPLCMS (Methode D): [m/z]: 214

### Benzyl-(2-chlor-5-methoxy-pyrimidin-4-yl)-amin

In ähnlicher Weise, unter Verwendung von Route 2, allgemeines Verfahren 4, ergaben 2,4-Dichlor-5-methoxypyrimidin (0.3 g, 1.6 mmol), Benzylamin (0.20 g, 1.84 mmol) und DIPEA (0.58 ml, 3.3 mmol) die Titelverbindung (0.42 g, 97 %).
MW: 249.70
HPLCMS (Methode D): [m/z]: 250

### (2-Chlor-5-methoxy-pyrimidin-4-yl)-cyclohexylmethyl-amin

In ähnlicher Weise, unter Verwendung von Route 2, allgemeines Verfahren 4, ergaben 2,4-Dichlor-5-methoxypyrimidin (0.3 g, 1.6 mmol), Cyclohexanmethylamin (0.21 g, 1.84 mmol) und DIPEA (0.58 ml, 3.3 mmol) die Titelverbindung (0.43 g, 100 %).
MW: 255.75
HPLCMS (Methode D): [m/z]: 258

### (2-Chlor-5-methoxy-pyrimidin-4-yl)-dimethyl-amin

In ähnlicher Weise, unter Verwendung von Route 2, allgemeines Verfahren 4, ergaben 2,4-Dichlor-5-methoxypyrimidin (0.3 g, 1.6 mmol), Dimethylamin (83 mg, 1.84 mmol) und DIPEA (0.58 ml, 3.3 mmol) die Titelverbindung (0.31g, 97 %).
MW: 187.63
HPLCMS (Methode D): [m/z]: 188

### (2-Chlor-5-methoxy-pyrimidin-4-yl)-diethyl-amin

In ähnlicher Weise, unter Verwendung von Route 2, allgemeines Verfahren 4, ergaben 2,4-Dichlor-5-methoxypyrimidin (0.3 g, 1.6 mmol), Diethylamin (0.13 g, 1.84 mmol) und DIPEA (0.58 ml, 3.3 mmol) die Titelverbindung (0.34 g, 94 %).
MW: 215.68
HPLCMS (Methode D): [m/z]: 216

### Benzyl-(2-chlor-5-methoxy-pyrimidin-4-yl)-methyl-amin

In ähnlicher Weise, unter Verwendung von Route 2, allgemeines Verfahren 4, ergaben 2,4-Dichlor-5-methoxypyrimidin (0.3 g, 1.6 mmol), N-Methylbenzylamin (0.22 g, 1.84 mmol) und DIPEA (0.58 ml, 3.3 mmol) die Titelverbindung (0.37 g, 83 %).
MW: 263.73
HPLCMS (Methode D): [m/z]: 264

### 2-Chlor-5-methoxy-4-piperidin-1-yl-pyrimidin

In ähnlicher Weise, unter Verwendung von Route 2, allgemeines Verfahren 4, ergaben 2,4-Dichlor-5-methoxypyrimidin (0.3 g, 1.6 mmol), Piperidin (0.16 g, 1.84 mmol) und DIPEA (0.58 ml, 3.3 mmol) die Titelverbindung (0.37 g, 96 %).
MW: 227.7
HPLCMS (Methode D): [m/z]: 228

### 4-(2-Chlor-5-methoxy-pyrimidin-4-yl)-morpholin

In ähnlicher Weise, unter Verwendung von Route 2, allgemeines Verfahren 4, ergaben 2,4-Dichlor-5-methoxypyrimidin (0.3 g, 1.6 mmol), Morpholin (0.16 g, 1.84 mmol) und DIPEA (0.58 ml, 3.3 mmol) die Titelverbindung (0.38 g, 98 %).
MW: 229.67
HPLCMS (Methode D): [m/z]: 230

### Allgemeines Verfghren 5:

### Lithium-tris(propan-2-yloxy)(pyridin-2-yl)borat

n-BuLi (791 *µ*l, 1.74 mmol) wurde tropfenweise zu einer Lösung von Triisopropoxyborat (400 *µ*l, 1.74 mmol) und 2-Brompyridin (250 mg, 1.58 mmol) in THF/Toluol (1:4, 7.5 ml) bei -78°C gegeben. Die Reaktionsmischung wurde bei -78°C für 1,5 h gerührt und dann über Nacht auf Raumtemperatur erwärmen gelassen. Der Ansatz wurde im Vakuum eingeengt, um die Titelverbindung (421 mg, 88 %) zu ergeben, die ohne weitere Reinigung eingesetzt wurde. Die Verbindung konnte durch HPLCMS nicht nachgewiesen werden, daher wurde ihre Struktur durch NMR bestätigt.

### Lithium-(5-methoxypyridin-2-yl)tris(propan-2-yloxy)borat

In ähnlicher Weise, unter Verwendung von Route 2, allgemeines Verfahren 5, ergaben n-BuLi (791 *µ*l, 1.74 mmol), Triisopropoxyborat (400 *µ*l, 1.74 mmol) und 2-Brom-5-methoxy-pyridin (198 mg, 1.58 mmol) die Titelverbindung (404 mg, 94 %), die ohne weitere Reinigung eingesetzt wurde. Die Verbindung konnte nicht durch HPLCMS nachgewiesen werden, daher wurde ihre Struktur durch 1H-NMR bestätigt.

### Allgemeines Verfahren 6:

### Beispiel 17

### Ethyl-(5-methoxy-2-pyridin-2-yl-pyrimidin-4-yl)-amin

Pd₂(dba)₃ (10 mg, 0.01 mmol) wurde zu einer Mischung von Lithium-tris(propan-2-yloxy)(pyridin-2-yl)borat (367 mg, 1.50 mmol), KF (87 mg, 1.50 mmol), t-Bu₂PHO (10 mg, 0.06 mmol) und (2-Chlor-5-methoxy-pyrimidin-4-yl)-ethyl-amin (94 mg, 0.50 mmol) in entgastem Dioxan (2 ml) gegeben. Der Ansatz wurde auf 110°C für 48 h erwärmt. Die Reaktionsmischung wurde abkühlen gelassen und filtriert. Der Filterkuchen wurde mit EtOAc gewaschen, und das Filtrat wurde mit Wasser gewaschen. Die wässrigen Waschlösungen wurden mit EtOAc (x 2) extrahiert. Die vereinigten organischen Phasen wurden getrocknet (Na₂SO₄) und im Vakuum eingeengt. Der Rohrückstand wurde durch präparative HPLC (neutrale Bedingungen) gereinigt, um die Titelverbindung zu ergeben (9 mg, 8 %).
MW: 230.26
HPLCMS (Methode A):[m/z]: 231
Abb. 14 zeigt das MS-Chromatogramm, das MS-Spektrum und das PDA-Chromatogramm der Verbindung von Beispiel 17.
IC50 [*µ*M]: <50.

### Beispiel 18

### Isobutyl-(5-methoxy-2-pyridin-2-yl-pyrimidin-4-yl)-amin

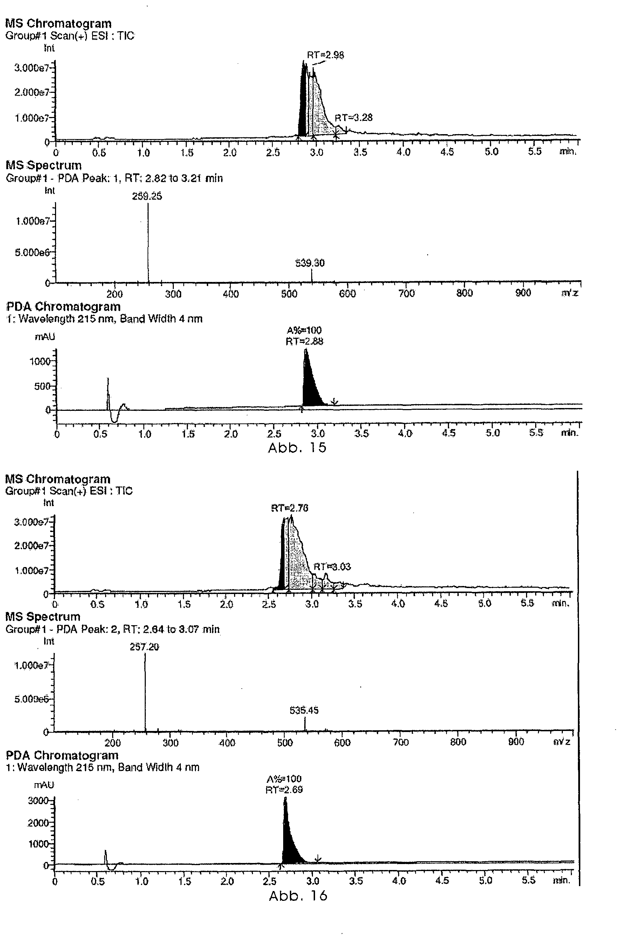

In ähnlicher Weise, unter Verwendung von Route 2, allgemeines Verfahren 6, ergaben Pd₂(dba)₃ (10 mg, 0.01 mmol), Lithium-tris(propan-2-yloxy)(pyridin-2-yl)borat (367 mg, 1.50 mmol), KF (87 mg, 1.50 mmol), †-Bu₂PHO (10 mg, 0.06 mmol) und (2-Chlor-5-methoxy-pyrimidin-4-yl)-isobutyl-amin (101 mg, 0.50 mmol) die Titelverbindung (5 mg, 4 %) nach Reinigung durch präparative HPLC (neutrale Bedingungen).
MW: 258.32
HPLCMS (Methode A): [m/z]: 259
Abb. 15 zeigt das MS-Chromatogramm, das MS-Spektrum und das PDA-Chromatogramm der Verbindung von Beispiel 18.
IC50 [*µ*M]: <50.

### Beispiel 19

### Cyclopropylmethyl-(5-methoxy-2-pyridin-2-yl-pyrimidin-4-yl)-amin

In ähnlicher Weise, unter Verwendung von Route 2, allgemeines Verfahren 6, ergaben Pd₂(dba)₃ (10 mg, 0.01 mmol), Lithium-tris(propan-2-yloxy)(pyridin-2-yl)borat (367 mg, 1.50 mmol), KF (87 mg, 1.50 mmol), t-Bu₂PHO (10 mg, 0.06 mmol) und (2-Chlor-5-methoxy-pyrimidin-4-yl)-cyclopropylmethyl-amin (107 mg, 0.50 mmol) die Titelverbindung (4 mg, 3 %) nach HPLC (neutrale Bedingungen).
MW: 256.30
HPLCMS (Methode A):[m/z]: 257
Abb. 16 zeigt das MS-Chromatogramm, das MS-Spektrum und das PDA-Chromatogramm der Verbindung von Beispiel 19.
IC50 [*µ*M]: <50.

### Beispiel 20

### Benzyl-(5-methoxy-2-pyridin-2-yl-pyrimidin-4-yl)-amin

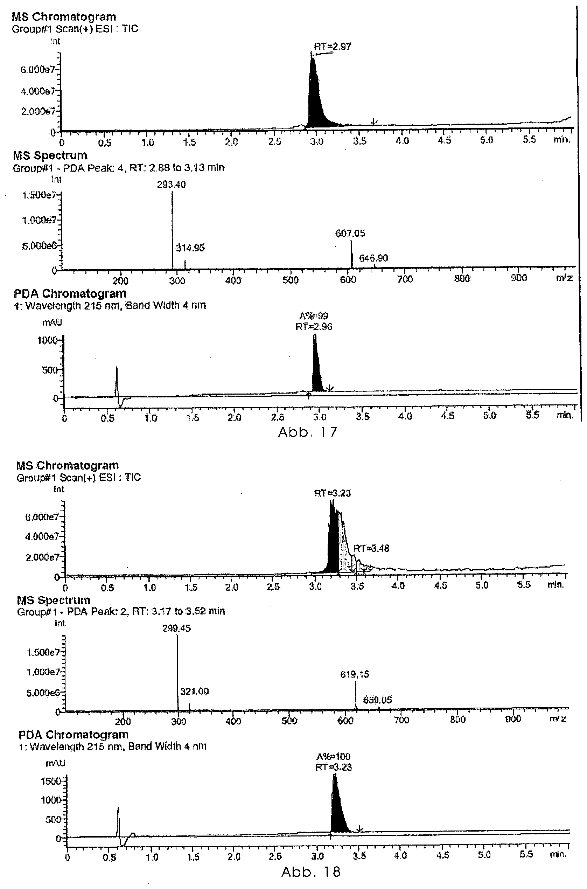

In ähnlicher Weise, unter Verwendung von Route 2, allgemeines Verfahren 6, ergaben Pd₂(dba)₃ (19 mg, 0.02 mmol), Lithium-tris(propan-2-yloxy)(pyridin-2-yl)borat (780 mg, 3.19 mmol), KF (185 mg, 3.19 mmol), t-Bu₂PHO (21 mg, 0.13 mmol) und Benzyl-(2-chlor-5-methoxy-pyrimidin-4-yl)-amin (265 mg, 1.06 mmol) die Titelverbindung (4 mg, 3 %) nach Reinigung durch präparative HPLC (saure Bedingungen).
MW: 292.34
HPLCMS (Methode A):[m/z]: 293
Abb. 17 zeigt das MS-Chromatogramm, das MS-Spektrum und das PDA-Chromatogramm der Verbindung von Beispiel 20.
IC50 [*µ*M]: <50.

### Beispiel 21

### Cyclohexylmethyl-(5-methoxy-2-pyridin-2-yl-pyrimidin-4-yl)-amin

In ähnlicher Weise, unter Verwendung von Route 2, allgemeines Verfahren 6, ergaben Pd₂(dba)₃ (10 mg, 0.01 mmol), Lithium-tris(propan-2-yloxy)(pyridin-2-yl)borat (367 mg, 1.50 mmol), KF (87 mg, 1.50 mmol), t-Bu₂PHO (10 mg, 0.06 mmol) und (2-Chlor-5-methoxy-pyrimidin-4-yl)-cyclohexylmethyl-amin (128 mg, 0.50 mmol) die Titelverbindung (9 mg, 6 %) nach Reinigung durch präparative HPLC (saure Bedingungen).
MW: 298.38
HPLCMS (Methode A):[m/z]: 299
Abb. 18 zeigt das MS-Chromatogramm, das MS-Spektrum und das PDA-Chromatogramm der Verbindung von Beispiel 21.
IC50 [*µ*M] <50.

### Beispiel 22

### (5-Methoxy-2-pyridin-2-yl-pyrimidin-4-yl)-dimethyl-amin

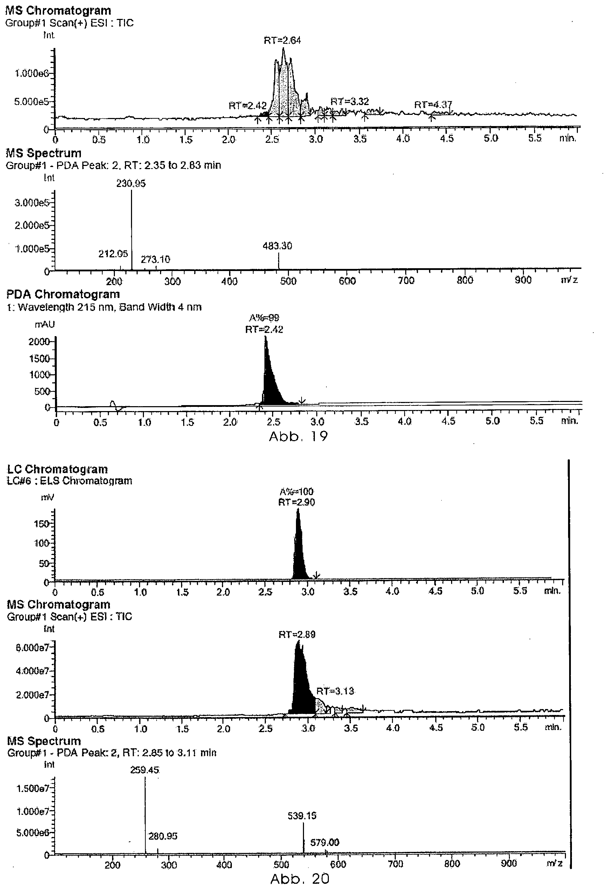

In ähnlicher Weise, unter Verwendung von Route 2, allgemeines Verfahren 6, ergaben Pd₂(dba)₃ (20 mg, 0.02 mmol), Lithium-tris(propan-2-yloxy)(pyridin-2-yl)borat (790 mg, 3.25 mmol), KF (189 mg, 3.25 mmol), †-Bu₂PHO (217 mg, 0.13 mmol) und (2-Chlor-5-methoxy-pyrimidin-4-yl)-dimethyl-amin (203 mg, 1.08 mmol) die Titelverbindung (27 mg, 23 %) nach Reinigung durch HPLC (saure Bedingungen).
MW: 230.27
HPLCMS (Methode A):[m/z]: 230.95
Abb. 19 zeigt das MS-Chromatogramm, das MS-Spektrum und das PDA-Chromatogramm der Verbindung von Beispiel 22.
IC50 [*µ*M] <50.

### Beispiel 23

### Diethyl-(5-methoxy-2-pyridin-2-yl-pyrimidin-4-yl)-amin

In ähnlicher Weise, unter Verwendung von Route 2, allgemeines Verfahren 6, ergaben Pd₂(dba)₃ (10 mg, 0.01 mmol), Lithium-tris(propan-2-yloxy)(pyridin-2-yl)borat 16 (367 mg, 1.50 mmol), KF (87 mg,1.50 mmol), †-Bu₂PHO (10 mg, 0.06 mmol) und (2-Chlor-5-methoxy-pyrimidin-4-yl)-diethyl-amin (108 mg, 0.50 mmol) die Titelverbindung (11 mg, 9 %) nach Reinigung durch präparative HPLC (saure Bedingungen).
MW: 258.32
HPLCMS (Methode A):[m/z]: 259
Abb. 20 zeigt das MS-Chromatogramm, das MS-Spektrum und das PDA-Chromatogramm der Verbindung von Beispiel 23.
IC50 [*µ*M]: >50.

### Beispiel 24

### Benzyl-(5-methoxy-2-pyridin-2-yl-pyrimidin-4-yl)-methyl-amin

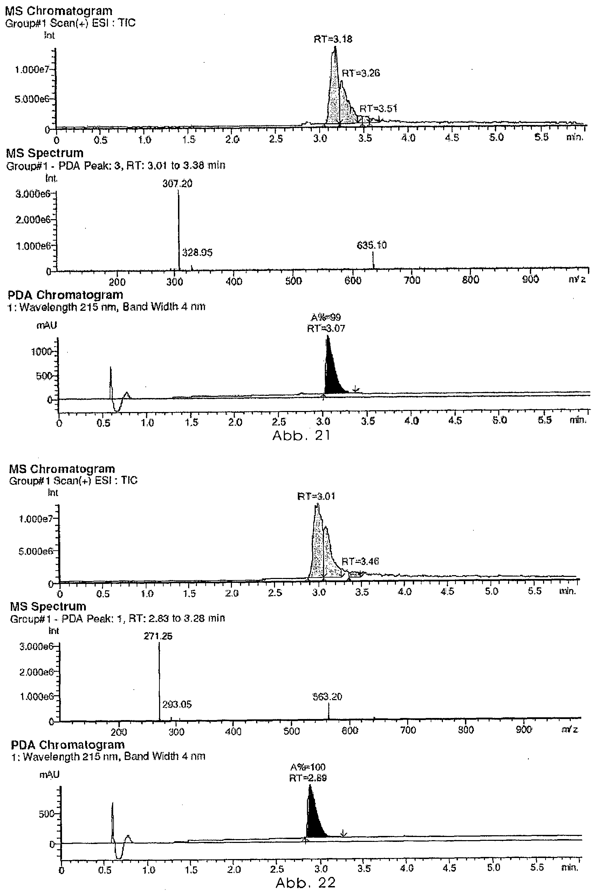

In ähnlicher Weise, unter Verwendung von Route 2, allgemeines Verfahren 6, ergaben Pd₂(dba)₃ (10 mg, 0.01 mmol), Lithium-tris(propan-2-yloxy)(pyridin-2-yl)borat (367 mg, 1.50 mmol), KF (87 mg, 1.50 mmol), †-Bu₂PHO (10 mg, 0.06 mmol) und Benzyl-(2-chlor-5-methoxy-pyrimidin-4-yl)-methyl-amin (132 mg, 0.50 mmol) die Titelverbindung (16 mg, 10 %) nach Reinigung durch präparative HPLC (saure Bedingungen).
MW: 306.36
HPLCMS (Methode A):[m/z]: 307
Abb. 21 zeigt das MS-Chromatogramm, das MS-Spektrum und das PDA-Chromatogramm der Verbindung von Beispiel 24.
IC50 [*µ*M]: >50.

### Beispiel 25

### 5-Methoxy-4-piperidin-1-yl-2-pyridin-2-yl-pyrimidin

In ähnlicher Weise, unter Verwendung von Route 2, allgemeines Verfahren 6, ergaben, Pd₂(dba)₃ (10 mg, 0.01 mmol), Lithium-tris(propan-2-yloxy)(pyridin-2-yl)borat (367 mg, 1.50 mmol), KF (87 mg, 1.50 mmol), †-Bu₂PHO (10 mg, 0.06 mmol) und 2-Chlor-5-methoxy-4-piperidin-1-yl-pyrimidin (114 mg, 0.50 mmol) die Titelverbindung (20 mg, 15 %) nach Reinigung durch präparative HPLC (saure Bedingungen).
MW: 270.33
HPLCMS (Methode A):[m/z]: 271
Abb. 22 zeigt das MS-Chromatogramm, das MS-Spektrum und das PDA-Chromatogramm der Verbindung von Beispiel 25.
IC50 [*µ*M]: >50.

### Beispiel 26

### 4-(5-Methoxy-2-pyridin-2-yl-pyrimidin-4-yl)-morpholin

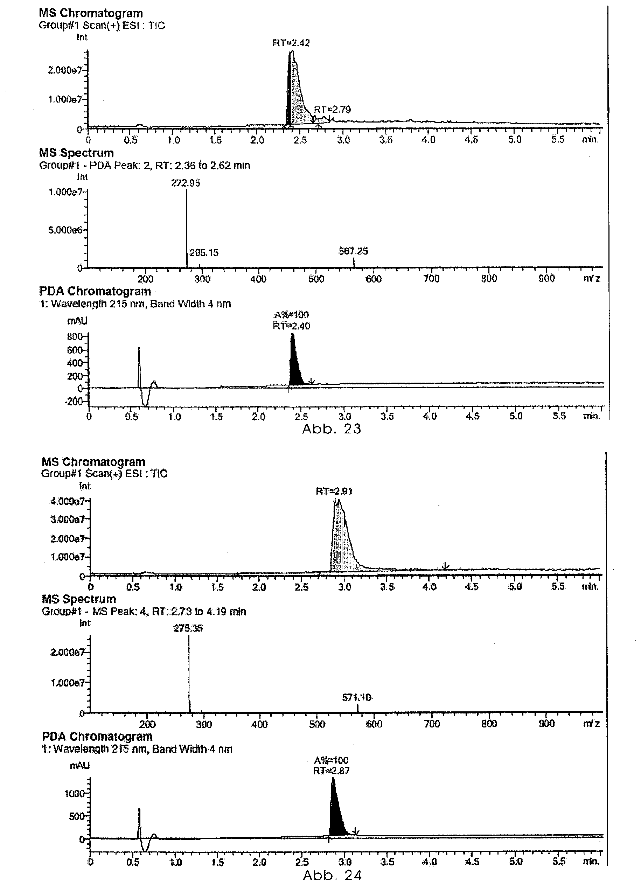

In ähnlicher Weise, unter Verwendung von Route 2, allgemeines Verfahren 6, ergaben Pd₂(dba)₃ (20 mg, 0.02 mmol), Lithium-tris(propan-2-yloxy)(pyridin-2-yl)borat (820 mg, 3.35 mmol), KF (194 mg, 3.35 mmol), †-Bu₂PHO (22 mg, 0.13 mmol) und 4-(2-Chlor-5-methoxy-pyrimidin-4-yl)-morpholin (256 mg, 1.12 mmol) die Titelverbindung (42 mg, 15 %) nach Reinigung durch präparative HPLC (saure Bedingungen).
MW: 272.30
HPLCMS (Methode A):[m/z]: 273
Abb. 23 zeigt das MS-Chromatogramm, das MS-Spektrum und das PDA-Chromatogramm der Verbindung von Beispiel 26.
IC50 [*µ*M]: <50.

### Beispiel 27

### Isopropyl-[5-methoxy-2-(5-methoxy-pyridin-2-yl)-pyrimidin-4-yl]-amin

In ähnlicher Weise, unter Verwendung von Route 2, allgemeines Verfahren 6, ergaben Pd₂(dba)₃ (18 mg, 0.02 mmol), Lithium-(5-methoxypyridin-2-yl)tris(propan-2-yloxy)borat (902 mg, 2.98 mmol), KF (173 mg, 2.98 mmol), t-Bu₂PHO (19 mg, 0.12 mmol) und (2-Chlor-5-methoxy-pyrimidin-4-yl)-isopropyl-amin (200 mg, 0.9 mmol) die Titelverbindung (55 mg, 20 %) nach Reinigung durch präparative HPLC (saure Bedingungen).
MW: 274.32
HPLCMS (Methode A):[m/z]: 275
Abb. 24 zeigt das MS-Chromatogramm, das MS-Spektrum und das PDA-Chromatogramm der Verbindung von Beispiel 27.
IC50 [*µ*M] <50.

### ROUTE 3

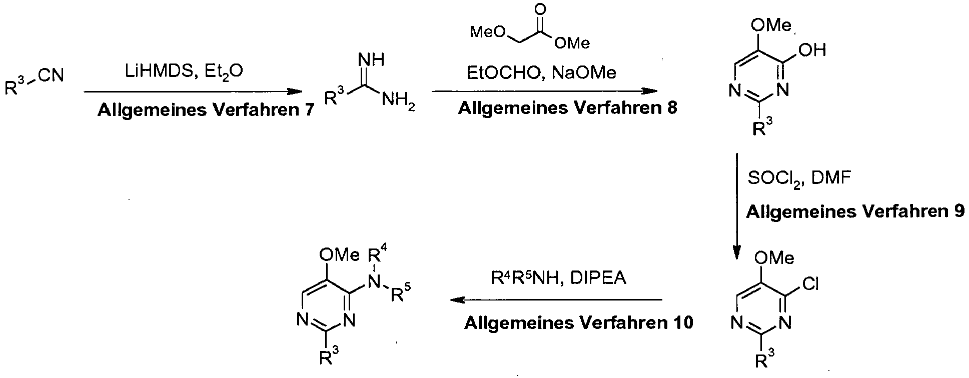

### Allgemeines Verfahren 7:

### Beispiel 28

### Pyrimidin-2-carboxamidin (Ausgangsmaterial)

Lithiumhexamethyldisilazid (1 M Lösung in THF, 20.0 ml, 20.0 mmol) wurde zu einer Lösung von Pyrimidin-2-carbonitril (1.0 g, 9.5 mmol) in Et₂O (30 ml) bei 0°C gegeben. Die Reaktion wurde auf Raumtemperatur über Nacht erwärmen gelassen. Der Ansatz wurde auf 0°C abgekühlt und 3 M HCl (54 ml) wurden hinzugegeben, und der Ansatz wurde für 30 min. gerührt. Wasser (135 ml) wurde hinzugegeben und die organische Phase abgetrennt und verworfen. Die wässrige Phase wurde mit gesättigter wässriger NaOH auf pH 14 basisch gemacht und mit DCM (x 3) extrahiert. Die vereinigten organischen Extrakte wurden getrocknet (Na₂SO₄) und im Vakuum eingeengt, um die Titelverbindung zu ergeben (0.46 g, 40 %).
MW: 122.13
HPLCMS (Methode B):[m/z]: 123

### Allgemeines Verfahren 8:

### 5-Methoxy-[2,2']bipyrimidinyl-4-ol (Beispiel 28)

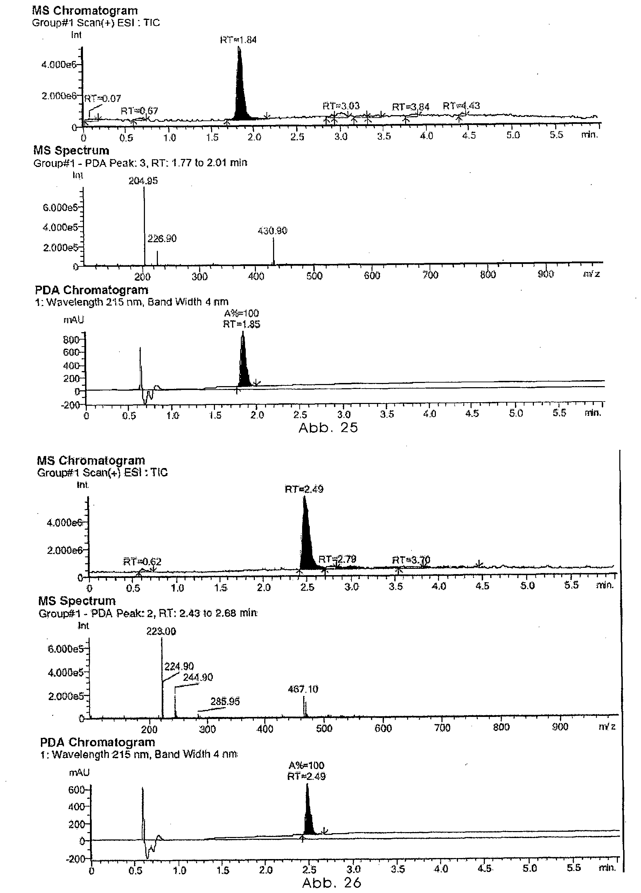

NaOMe (0.49 g, 9.00 mmol) wurde zu einer Lösung von Methylmethoxyacetat (0.81 ml, 8.19 mmol) und Ameisensäureethylester (0.99 ml, 12.28 mmol) in MeOH (10 ml) gegeben. Die Reaktionsmischung wurde bei Raumtemperatur für 5 h gerührt. Eine Lösung von Pyrimidin-2-carboxamidin (1.0 g, 8.19 mmol) in MeOH (5 ml) wurde hinzugegeben, gefolgt von NaOMe (0.44 g, 8.19 mmol). Die Mischung wurde unter Rückfluss für 18 h erhitzt und wurde im Vakuum eingeengt. Der Rohrückstand wurde durch Säulenchromatographie mit MeOH/DCM (5:95 - 50:50) als Eluent gereinigt, um die Titelverbindung zu ergeben (0.55 g, 22 %).
MW: 204.19
HPLCMS (Methode A):[m/z]: 205
Abb. 25 zeigt das MS-Chromatogramm, das MS-Spektrum und das PDA-Chromatogramm der Verbindung von Beispiel 28.
IC50 [*µ*M]: >50.

### Beispiel 29

### Allgemeines Verfahren 9:

### 4-Chlor-5-methoxy-[2,2']bipyrimidinyl

DMF (Kat.) wurde zu einer Lösung von 5-Methoxy-[2,2']bipyrimidinyl-4-ol (520 mg, 2.55 mmol) in Thionylchlorid (5 ml) gegeben, und die Mischung wurde bei 80°C für 15 min. erhitzt. Die Mischung wurde im Vakuum eingeengt. Der Rückstand wurde mit gesättigter wässriger NaHCO₃-Lösung (50 ml) basisch gemacht und mit DCM (x 3) extrahiert. Die vereinigten organischen Phasen wurden getrocknet (Na₂SO₄) und im Vakuum eingeengt, um die Titelverbindung zu ergeben (570 mg, 100 %).
MW: 222.64
HPLCMS (Methode A):[m/z]: 223
Abb. 26 zeigt das MS-Chromatogramm, das MS-Spektrum und das PDA-Chromatogramm der Verbindung von Beispiel 29.
IC50 [*µ*M]: >50.

### Beispiel 30

### Allgemeines Verfahren 10:

### Isopropyl-(5-methoxy-[2,2']bipyrimidinyl-4-yl)-amin

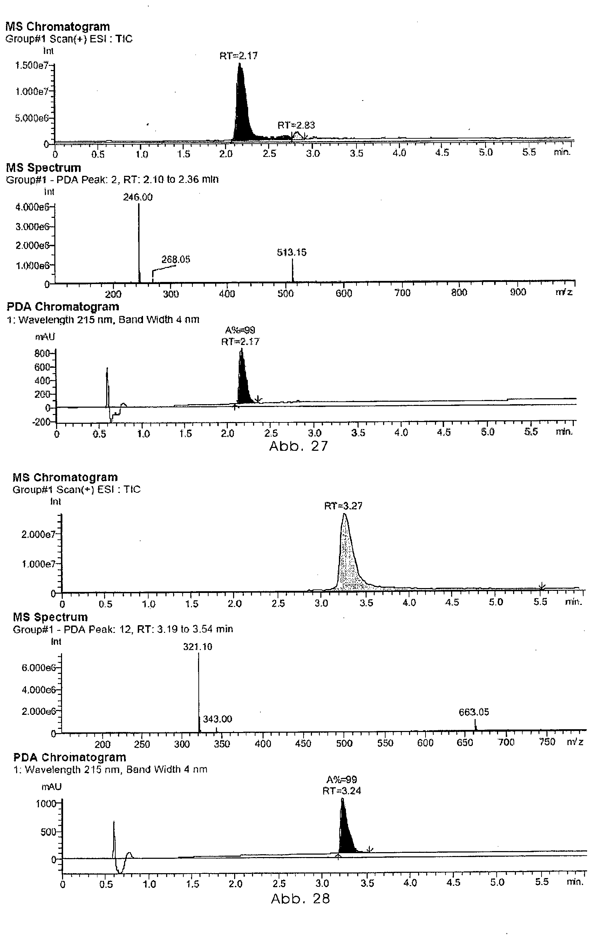

Diisopropylamin (173 *µ*l, 2.02 mmol) wurde zu einer Lösung von 4-Chlor-5-methoxy-[2,2']bipyrimidinyl (100 mg, 0.45 mmol) in EtOH (1.0 ml) und die Mischung wurde unter Rückfluss für 18 h erhitzt. Die Reaktionsmischung wurde im Vakuum eingeengt. Der Rückstand wurde mit gesättigter NaHCO₃-Lösung (1 ml) basisch gemacht und mit DCM (x 3) extrahiert. Die organische Phase wurde mit Wasser (x 2) gewaschen, getrocknet (Na₂SO₄) und im Vakuum eingeengt, um die Titelverbindung zu ergeben (89 mg, 81 %).
MW: 245.29
HPLCMS (Methode A):[m/z]: 246
Abb. 27 zeigt das MS-Chromatogramm, das MS-Spektrum und das PDA-Chromatogramm der Verbindung von Beispiel 30.
IC50 [*µ*M]: >50.

### ROUTE 4

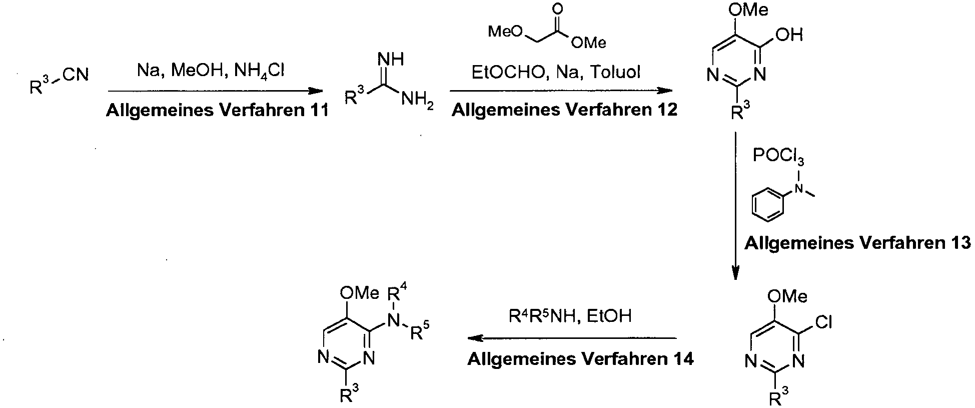

### Allgemeines Verfahren 11:

### Pyridin-2-carboxamidin

Eine Lösung von Natriummetall (74 mg, 3.2 mmol) in MeOH (5 ml) wurde zu einer Lösung von 2-Cyanopyridin (3 g, 28 mmol) in MeOH (25 ml) gegeben, und die Mischung wurde 16 h bei Raumtemperatur gerührt. Ammoniumchlorid (4.5 g, 84 mmol) wurde hinzugegeben und die Mischung wurde bei 70°C für 3 h gerührt. Nach Abkühlen wurde die Mischung im Vakuum eingeengt. Der Rückstand wurde mit EtOH (40 ml) verdünnt und die Mischung wurde erhitzt unter Rückfluss für 0,5 h. Nach Abkühlen wurde die Mischung filtriert und das Filtrat im Vakuum eingeengt. Der Rohrückstand wurde mit Et₂O/Isopropanol (4:1) getrocknet, um die Titelverbindung als HCl-Salz zu ergeben (4.5 g, 99 %).
MW: 121.4
HPLCMS (Methode D): [m/z]: 122

### Pyrazin-2-carboxamidin

In ähnlicher Weise, unter Verwendung von Route 4, allgemeines Verfahren 11, ergaben Pyrazin-2-carbonitril (2 g, 19 mmol), Natriummetall (49 mg, 2.15 mmol), MeOH (23 ml) und Ammoniumchlorid (3.05 g, 57.1 mmol) die Titelverbindung (2.7 g, 93 %) nach Verfestigung (Trituration) aus EtOH.
MW: 122.13
HPLCMS (Methode D): [m/z]:122

### Allgemeines Verfahren 12:

### 5-Methoxy-2-pyridin-2-yl-3H-pyrimidin-4-on

Methylmethoxyacetat (4.0 g, 38 mmol) und Ameisensäureethylester (2.81 g, 38 mmol) wurden gleichzeitig zu einer gerührten Suspension von Natrium (0.87 g, 38 mmol) in Toluol (20 ml) gegeben, und die Mischung wurde bei Raumtemperatur für 12 h gerührt. Toluol wurde dekantiert, der Rückstand wurde mit EtOH (20 ml) verdünnt und Pyridin-2-carboxamidin (4.7 g, 30 mmol) wurde hinzugegeben, gefolgt von einer Lösung von Natriumethoxid (hergestellt aus Na 1.39 g, 60 mmol und 5 ml Ethanol). Die Reaktionsmischung wurde für 15 h unter Rückfluss erhitzt. Nach Abkühlen wurde die Mischung filtriert und der Rückstand mit 1N HCl (10 ml) neutralisiert. Die Mischung wurde im Vakuum eingeengt. Der Rohrückstand wurde mit MeOH (20 ml) verdünnt, für 0,25 h gerührt und über Zelithe filtriert. Das Filtrat wurde im Vakuum eingeengt, um die Titelverbindung zu ergeben (3.7 g, 61 %).
MW: 203.19
HPLCMS (Methode D): [m/z]: 204

### 5-Methoxy-2-pyrazin-2-yl-3H-pyrimidin-4-on

In ähnlicher Weise, unter Verwendung von Route 4, allgemeines Verfahren 12, ergaben Methylmethoxyacetat (1.0 g, 9.6 mmol), Ameisensäureethylester (0.71 g, 9.6 mmol) und Natrium (0.22 g, 9.6 mmol), gefolgt von Pyrazin-2-carboxamidin (1.2 g, 7.6 mmol) und Natriumethoxid (hergestellt aus Na 0.17 g, 7.6 mmol und 5 ml Ethanol) die Titelverbindung (0.75 g, 38 %) nach Reinigung durch Verfestigung aus MeOH.
MW: 204.18
HPLCMS (Methode A): [m/z]: 205

### 5-Methoxy-2-pyridin-3-yl-3H-pyrimidin-4-on

In ähnlicher Weise, unter Verwendung von Route 4, allgemeines Verfahren 12, ergaben Methylmethoxyacetat (2.0 g, 19.2 mmol), Ameisensäureethylester (1.42 g, 19.2 mmol), Natrium (0.44 g, 19.2 mmol) in Toluol (20 ml), Nicotinamidinhydrochlorid (2.4 g, 15 mmol) die Titelverbindung (1.23 g, 39 %).
MW: 203.19
HPLCMS (Methode D): [m/z]: 204

### Allgemeines Verfahren 13:

### 4-Chlor-5-methoxy-2-pyridin-2-yl-pyrimidin

5-Methoxy-2-pyridin-2-yl-3H-pyrimidin-4-on (4.2 g, 20.68 mmol) und POCl₃ (31.58 g, 206 mmol) in N,N-Dimethylanilin (6 ml) wurden unter Rückfluss für 1 h erhitzt. Nach Abkühlen wurde die Mischung auf Eis (200 ml) gegossen, und die Mischung auf pH 8 - 9 mit gesättigter wässriger NaHCO₃. basisch gemacht. Die wässrige Phase wurde mit EtOAc (x 3) extrahiert. Die vereinigten organischen Phasen wurden getrocknet (Na₂SO₄) und im Vakuum eingeengt. Der Rohrückstand wurde durch Säulenchromatographie mit DCM/MeOH (97:3) als Eluent gereinigt, um die Titelverbindung zu ergeben (2.2 g, 48%).
MW: 221.64
HPLCMS (Methode D): [m/z]: 223

### 4-Chlor-5-methoxy-2-pyrazin-2-yl-pyrimidin

In ähnlicher Weise, unter Verwendung von Route 4, allgemeines Verfahren 13, ergaben 5-Methoxy-2-pyrazin-2-yl-3H-pyrimidin-4-on (0.6 g, 2.94 mmol), POCl₃ (4.5 g, 29.4 mmol) und N,N-Dimethylanilin (0.8 ml) die Titelverbindung (44 mg, 6 %) nach Reinigung durch Säulenchromatographie EtOAc/Hexan (3:7) als Eluent.
MW: 222.63
HPLCMS (Methode D): [m/z]: 223

### 4-Chlor-5-methoxy-2-pyridin-3-yl-pyrimidin

In ähnlicher Weise, unter Verwendung von Route 4, allgemeines Verfahren 13, ergaben 5-Methoxy-2-pyridin-3-yl-3H-pyrimidin-4-on (0.4 g, 19 mmol), POCl₃ (3 g, 19 mmol) und N,N-Dimethylanilin (0.3 ml) die Titelverbindung (0.16 g, 43 %) nach Reinigung durch Säulenchromatographie mit DCM/MeOH (95:5).
MW: 221.64
HPLCMS (Methode D): [m/z]: 222

### Beispiel 31

### Allgemeines Verfahren 14:

(5-Methoxy-2-pyridin-2-yl-pyrimidin-4-yl)-(3-phenyl-propyl)-amin 4-Chlor-5-methoxy-2-pyridin-2-yl-pyrimidin (0.1 g, 0.45 mmol), 3-Phenylpropan-1-amin (73 mg, 0.54 mmol) und DIPEA (0.12 g, 0.9 mmol) wurden in EtOH (2 ml) gelöst, und die Mischung wurde bei 80°C für 15 h gerührt. Nach Abkühlen wurden die Mischung im Vakuum eingeengt. Der Rückstand wurde mit Wasser (15 ml) verdünnt, und die wässrige Phase mit EtOAc (x 3) extrahiert. Die vereinigten organischen Phasen wurden getrocknet (Na₂SO₄) und im Vakuum eingeengt. Der Rohrückstand wurde durch Säulenchromatographie mit DCM/MeOH (95:5) als Eluent gereinigt, um die Titelverbindung zu ergeben (65 mg, 45 %).
MW: 320.38
HPLCMS (Methode A): [m/z]: 321
Abb. 28 zeigt das MS-Chromatogramm, das MS-Spektrum und das PDA-Chromatogramm der Verbindung von Beispiel 31.
IC50 [*µ*M]: >50.

### Beispiel 32

### Ethyl-(5-methoxy-2-pyridin-2-yl-pyrimidin-4-yl)-methyl-amin

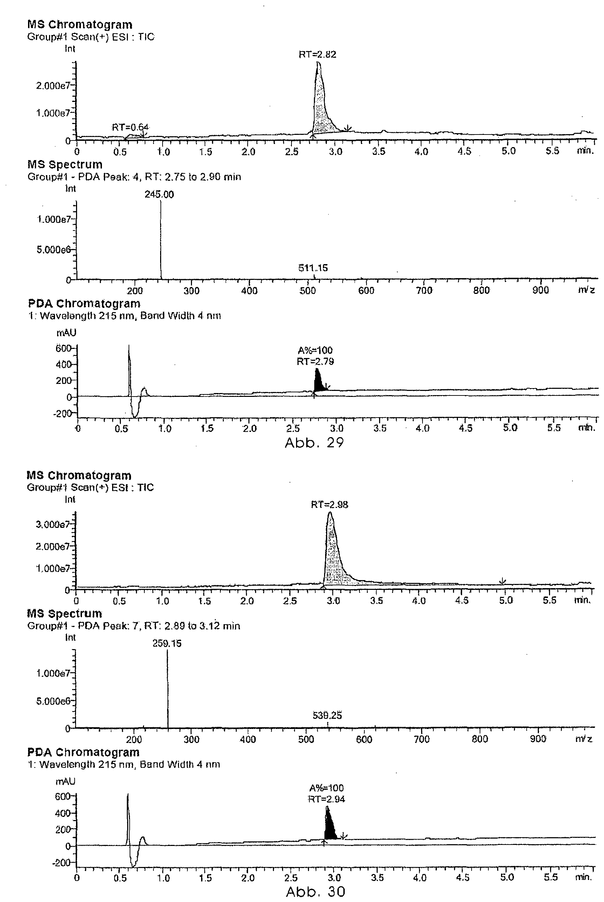

In ähnlicher Weise, unter Verwendung von Route 4, allgemeines Verfahren 14, ergaben 4-Chlor-5-methoxy-2-pyridin-2-yl-pyrimidin (50 mg, 0.22 mmol), N-Methylethylamin (15 *µ*l, 0.27 mmol) und DIPEA (50 *µ*l, 0.27 mmol) die Titelverbindung (29 mg, 53 %) nach Reinigung durch Säulenchromatographie mit DCM/1 % NH₃ in MeOH (95:5) als Eluent.
MW: 244.29
HPLCMS (Methode A): [m/z]: 245
Abb. 29 zeigt das MS-Chromatogramm, das MS-Spektrum und das PDA-Chromatogramm der Verbindung von Beispiel 32.
IC50 [*µ*M]: <50.

### Beispiel 33

### Isopropyl-(5-methoxy-2-pyridin-2-yl-pyrimidin-4-yl)-methyl-amin

In ähnlicher Weise, unter Verwendung von Route 4, allgemeines Verfahren 14, ergaben 4-Chloro-5-methoxy-2-pyridin-2-yl-pyrimidin (50 mg, 0.22 mmol), N-Methyl-iso-propylamin (19 mg, 0.27 mmol) und DIPEA (0.05 ml, 0.27 mmol) die Titelverbindung (23 mg, 39 %) nach Reinigung durch Säulenchromatographie mit DCM/1% NH₃ in MeOH (95:5) als Eluent.
MW: 258.31
HPLCMS (Methode A): [m/z]: 259
Abb. 30 zeigt das MS-Chromatogramm, das MS-Spektrum und das PDA-Chromatogramm der Verbindung von Beispiel 33.
IC50 [*µ*M]: >50.

### Beispiel 34

### Isobutyl-(5-methoxy-2-pyridin-2-yl-pyrimidin-4-yl)-methyl-amin

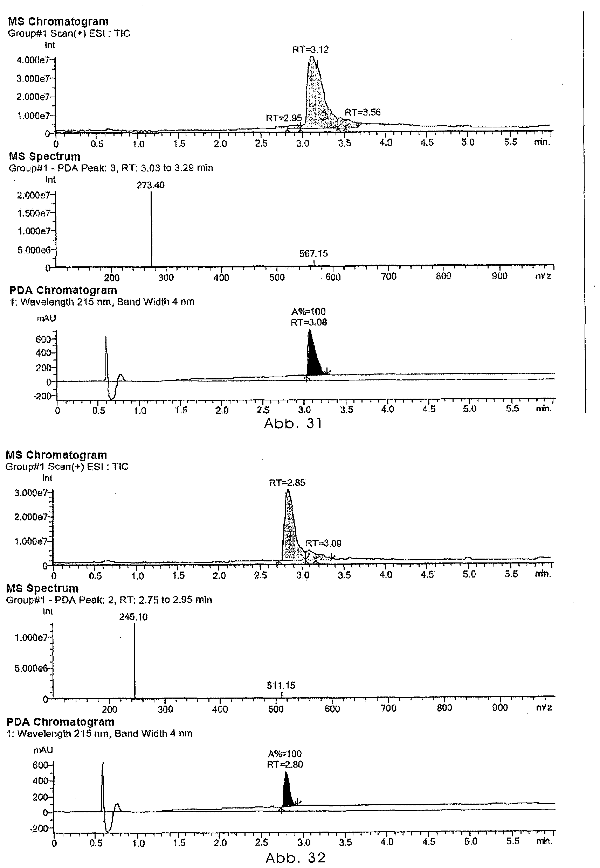

In ähnlicher Weise, unter Verwendung von Route 4, allgemeines Verfahren 14, ergaben 4-Chlor-5-methoxy-2-pyridin-2-yl-pyrimidin (50 mg, 0.22 mmol), N-Methyl-iso-butylamin (20 *µ*l, 0.27 mmol) und DIPEA (50 *µ*l, 0.27 mmol) die Titelverbindung (30 mg, 49 %) nach Reinigung durch Säulenchromatographie mit DCM/1% NH₃ in MeOH (95:5) als Eluent.
MW: 272.35
HPLCMS (Methode A): [m/z]: 273
Abb. 31 zeigt das MS-Chromatogramm, das MS-Spektrum und das PDA-Chromatogramm der Verbindung von Beispiel 34.
IC50 [*µ*M]: >50.

### Beispiel 35

### (5-Methoxy-2-pyridin-2-yl-pyrimidin-4-yl)-propyl-amin

In ähnlicher Weise, unter Verwendung von Route 4, allgemeines Verfahren 14, ergaben 4-Chlor-5-methoxy-2-pyridin-2-yl-pyrimidin (50 mg, 0.22 mmol), Propylamin (15 *µ*l, 0.27 mmol) und DIPEA (50 *µ*l, 0.27 mmol) die Titelverbindung (24 mg, 44 %) nach Reinigung durch Säulenchromatographie mit DCM/1% NH₃ in MeOH (95:5) als Eluent.
MW: 244.29
HPLCMS (Methode A): [m/z]: 245
Abb. 32 zeigt das MS-Chromatogramm, das MS-Spektrum und das PDA-Chromatogramm der Verbindung von Beispiel 35.
IC50 [*µ*M]: <50.

### Beispiel 36

### Butyl-(5-methoxy-2-pyridin-2-yl-pyrimidin-4-yl)-amin

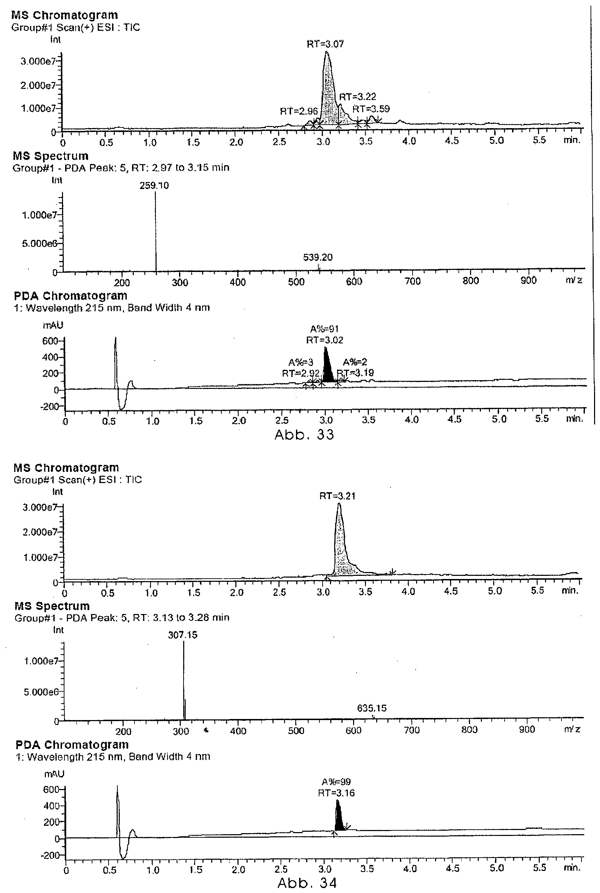

In ähnlicher Weise, unter Verwendung von Route 4, allgemeines Verfahren 14, ergaben 4-Chlor-5-methoxy-2-pyridin-2-yl-pyrimidin (50 mg, 0.22 mmol), Butylamin (20 *µ*l, 0.27 mmol) und DIPEA (50 *µ*l, 0.27 mmol) die Titelverbindung (26 mg, 45 %) nach Reinigung durch Säulenchromatographie mit DCM/1% NH₃ in MeOH (95:5) als Eluent.
MW: 258.31
HPLCMS (Methode A): [m/z]: 259
Abb. 33 zeigt das MS-Chromatogramm, das MS-Spektrum und das PDA-Chromatogramm der Verbindung von Beispiel 36.
IC50 [*µ*M]: <50.

### Beispiel 37

### (5-Methoxy-2-pyridin-2-yl-pyrimidin-4-yl)-phenethyl-amin

In ähnlicher Weise, unter Verwendung von Route 4, allgemeines Verfahren 14, ergaben 4-Chlor-5-methoxy-2-pyridin-2-yl-pyrimidin (50 mg, 0.22 mmol), Phenylethylamin (30 *µ*l, 0.27 mmol) und DIPEA (50 *µ*l, 0.27 mmol) die Titelverbindung (28 mg, 48 %) nach Reinigung durch Säulenchromatographie mit DCM/1 % NH₃ in MeOH (95:5) als Eluent.
MW: 306.32
HPLCMS (Methode A): [m/z]: 307
Abb. 34 zeigt das MS-Chromatogramm, das MS-Spektrum und das PDA-Chromatogramm der Verbindung von Beispiel 37.
IC50 [*µ*M]: <50.

### Beispiel 38

### Isopropyl-(5-methoxy-2-pyrazin-2-yl-pyrimidin-4-yl)-amin

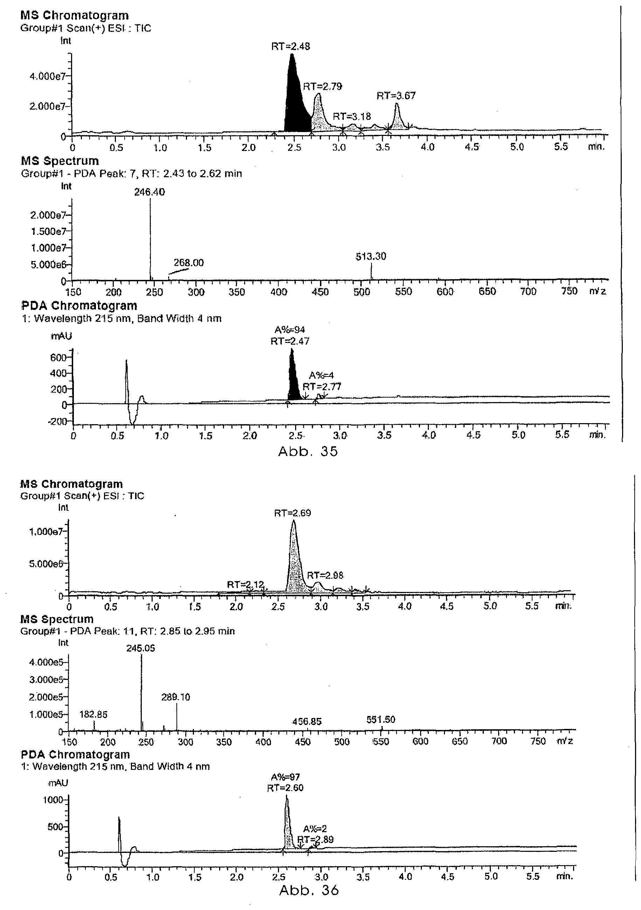

In ähnlicher Weise, unter Verwendung von Route 4, allgemeines Verfahren 14, ergaben 4-Chlor-5-methoxy-2-pyrazin-2-yl-pyrimidin (44 mg, 0.19 mmol), Isopropylamin (25 *µ*l, 0.29 mmol) und DIPEA (67 *µ*l, 0.39 mmol) die Titelverbindung (27 mg, 60 %) nach Reinigung durch Säulenchromatographie mit DCM/MeOH (95:5) als Eluent.
MW: 245.28
HPLCMS (Methode A) [m/z]: 246
Abb. 35 zeigt das MS-Chromatogramm, das MS-Spektrum und das PDA-Chromatogramm der Verbindung von Beispiel 38.
IC50 [*µ*M]: >50.

### Beispiel 39

### Isopropyl-(5-methoxy-2-pyridin-3-yl-pyrimidin-4-yl)-amin

In ähnlicher Weise, unter Verwendung des allgemeinen Verfahrens 14, ergaben 4-Chlor-5-methoxy-2-pyridin-3-yl-pyrimidin (0.15 g, 0.67 mmol), Isopropylamin (43 *µ*l, 0.74 mmol) und DIPEA (0.13 ml, 0.81 mmol) die Titelverbindung (39 mg, 24 %) nach Reinigung durch Säulenchromatographie mit DCM/MeOH (98:2) als Eluent.
MW: 244.29
HPLCMS (Methode A): [m/z]: 245
Abb. 36 zeigt das MS-Chromatogramm, das MS-Spektrum und das PDA-Chromatogramm der Verbindung von Beispiel 39.
IC50 [*µ*M]: >50.

### ROUTE 5

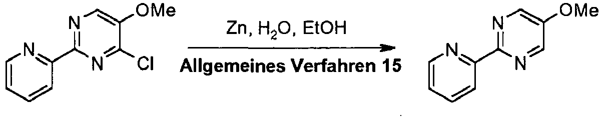

### Beispiel 40

### Allgemeines Verfahren 15:

### 5-Methoxy-2-pyridin-2-yl-pyrimidin

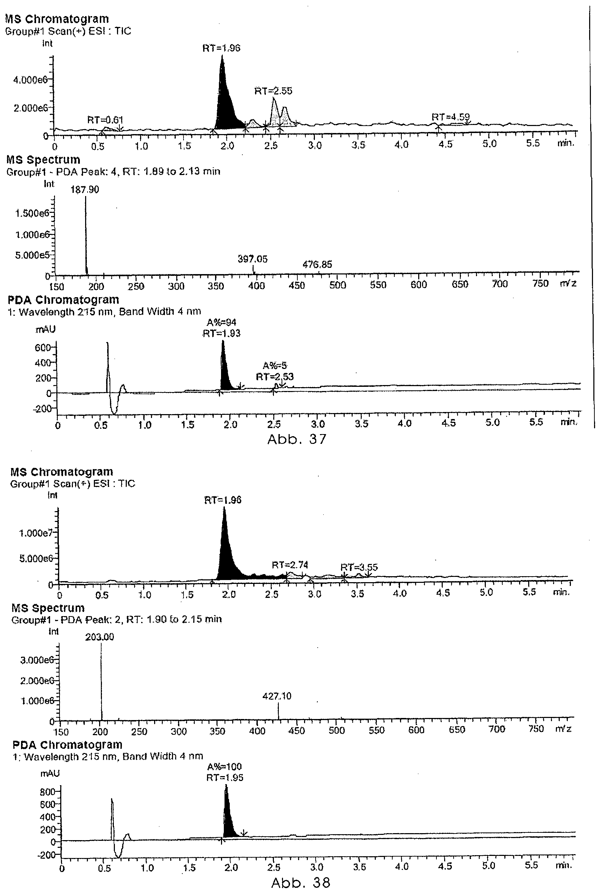

Zinkpulver (1.0 g, 15.8 mmol) und Wasser (2.4 ml) wurden zu einer Lösung von 4-Chlor-5-methoxy-2-pyridin-2-yl-pyrimidin (0.2 g, 0.9 mmol) in EtOH (5.4 ml) gegeben, und die Mischung wurde auf 60°C für 5 h erhitzt. Nach Abkühlen wurde die Reaktionsmischung filtriert und das Filtrat im Vakuum eingeengt. Der Rohrückstand wurde durch Säulenchromatographie mit DCM/1 % NH₃ in MeOH (95:5) als Eluent gereinigt, um die Titelverbindung zu ergeben (23 mg, 14 %).
MW: 187.20
HPLCMS (Methode A): [m/z]: 188
Abb. 37 zeigt das MS-Chromatogramm, das MS-Spektrum und das PDA-Chromatogramm der Verbindung von Beispiel 40.
IC50 [*µ*M]: >50.

### ROUTE 6

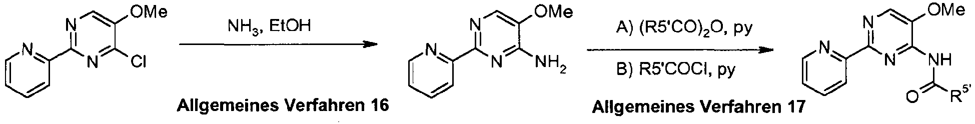

### Allgemeines Verfahren 16:

### Beispiel 41

### 5-Methoxy-2-pyridin-2-yl-pyrimidin-4-ylamin

4-Chlor-5-methoxy-2-pyridin-2-yl-pyrimidin (1.0 g, 4.52 mmol) in EtOH (5 ml) wurde mit Ammoniakgas bei 0°C für 0,3 h gespült. Die Reaktionsmischung wurde auf 140°C für 12 h erhitzt. Nach Abkühlen wurde die Mischung im Vakuum eingeengt. Der Rohrückstand wurde durch Säulenchromatographie mit DCM/1% NH₃ in MeOH (97:3) als Eluent gereinigt, um die Titelverbindung zu ergeben (0.7 g, 78 %).
MW: 202.21
HPLCMS (Methode A): [m/z]: 203
Abb. 38 zeigt das MS-Chromatogramm, das MS-Spektrum und das PDA-Chromatogramm der Verbindung von Beispiel 41.
IC50 [*µ*M]: >50.

### Beispiel 42

### Allgemeines Verfahren 17:

### N-(5-Methoxy-2-pyridin-2-yl-pyrimidin-4-yl)-acetamid

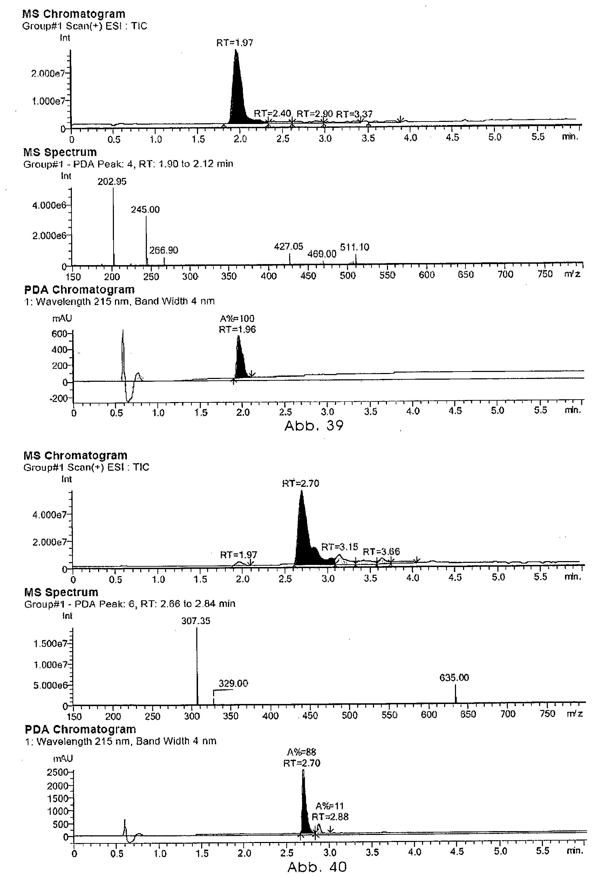

Essigsäureanhydrid (0.05 g, 0.49 mmol) wurde zu einer Lösung von 5-Methoxy-2-pyridin-2-yl-pyrimidin-4-ylamin (0.05 g, 0.25 mmol) in Pyridin (0.5 ml) bei 0°C gegeben, und die Mischung wurde bei Raumtemperatur für 12 h gerührt. Die Mischung wurde mit Wasser (7 ml) verdünnt und die wässrige Phase wurde extrahiert mit DCM (x 3). Die vereinigten organischen Phasen wurden getrocknet (Na₂SO₄) und eingeengt im Vakuum. Der Rohrückstand wurde durch Säulenchromatographie mit DCM/1%NH₃ in MeOH (95:5) und 1 % Ammoniak als Eluent gereinigt, um die Titelverbindung zu ergeben (25 mg, 41 %).
MW: 244.29
HPLCMS (Methode A): [m/z]: 245
Abb. 39 zeigt das MS-Chromatogramm, das MS-Spektrum und das PDA-Chromatogramm der Verbindung von Beispiel 42.
IC50 [*µ*M]: >50.

### Beispiel 43

### N-(5-Methoxy-2-pyridin-2-yl-pyrimidin-4-yl)-benzamid

In ähnlicher Weise, unter Verwendung von Route 6, allgemeines Verfahren 17, ergaben 5-Methoxy-2-pyridin-2-yl-pyrimidin-4-ylamin (45 mg, 0.22 mmol), Benzoylchlorid (59 mg, 0.42 mmol) und Pyridin (0.5 ml) die Titelverbindung (20 mg, 29 %) nach Reinigung durch Säulenchromatographie mit DCM/MeOH (95:5) als Eluent.
MW: 306.31
HPLCMS (Methode A): [m/z]: 307
Abb. 40 zeigt das MS-Chromatogramm, das MS-Spektrum und das PDA-Chromatogramm der Verbindung von Beispiel 43.
IC50 [*µ*M]: <50.

### ROUTE 7

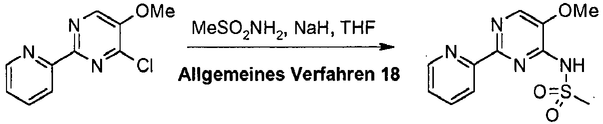

### Allgemeines Verfahren 18:

### Beispiel 44

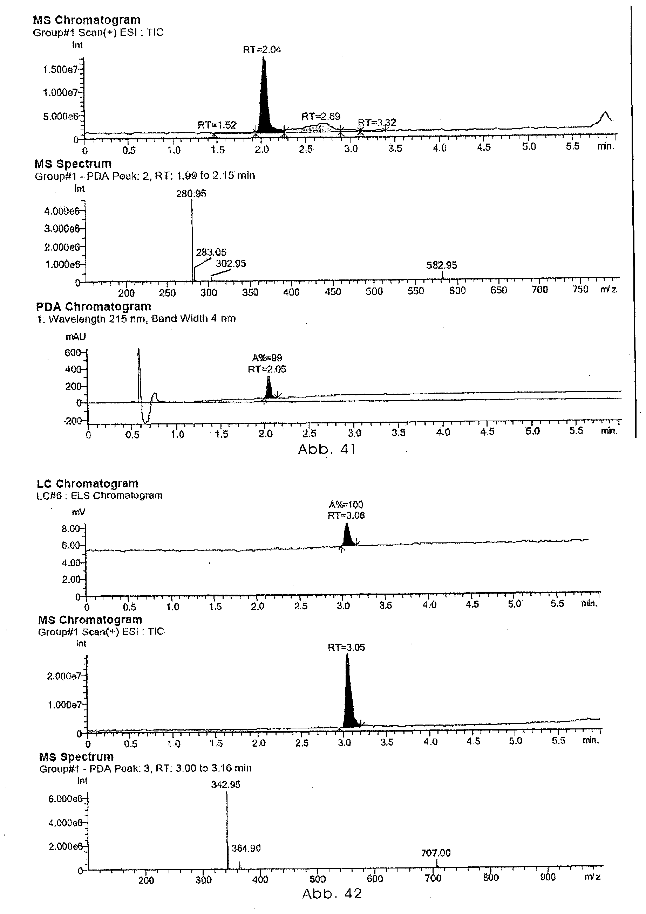

Synthese von N-(5-Methoxy-2-pyridin-2-yl-pyrimidin-4-yl)-methansulfonamid Methansulfonamid (47 mg, 0.49 mmol) wurde in eine Lösung von Natriumhydrid (60 %ig in Mineralöl, 20 mg, 0.5 mmol) in THF (0.5 ml) gegeben, und die Mischung wurde bei Raumtemperatur für 0,5 h gerührt. 4-Chlor-5-methoxy-2-pyridin-2-yl-pyrimidin (0.10 g, 0.45 mmol) in DMSO (0.5 ml) wurde hinzugegeben, und die Mischung wurde auf 120°C für 1 h erhitzt. Nach Abkühlen wurde die Mischung im Vakuum eingeengt. Der Rohrückstand wurde durch Säulenchromatographie mit DCM/1% NH₃ in MeOH (97:3) als Eluent gereinigt, um die Titelverbindung zu ergeben (27 mg, 27 %).
MW: 280.30
HPLCMS (Methode A): [m/z]: 281
Abb. 41 zeigt das MS-Chromatogramm, das MS-Spektrum und das PDA-Chromatogramm der Verbindung von Beispiel 44.
IC50 [*µ*M]: <50.

### ROUTE 8

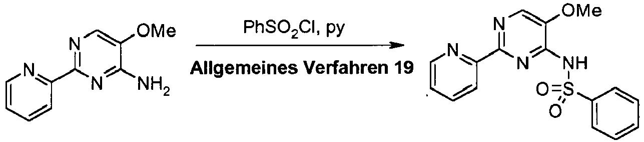

### Allgemeines Verfahren 19:

### Beispiel 45

### N-(5-Methoxy-2-pyridin-2-yl-pyrimidin-4-yl)-benzolsulfonamid

Benzolsulfonylchlorid (43 mg, 0.24 mmol) wurde zu einer Lösung von 5-Methoxy-2-pyridin-2-yl-pyrimidin-4-ylamin (50 mg, 0.24 mmol) in Pyridin (0.3 ml) gegeben, und die Mischung wurde bei 80°C für 16 h erwärmt. Nach Abkühlen wurde die Reaktionsmischung mit Wasser (10 ml) verdünnt, und die wässrige Phase mit DCM (x 3) extrahiert. Die vereinigten organischen Phasen wurden getrocknet (Na₂SO₄) und im Vakuum eingeengt. Der Rohrückstand wurde durch Säulenchromatographie mit DCM/1% NH₃ in MeOH (95:5) als Eluent gereinigt, um die Titelverbindung zu ergeben (15 mg, 18 %).
MW: 342.37
HPLCMS (Methode A): [m/z]: 343
Abb. 42 zeigt das MS-Chromatogramm, das MS-Spektrum und das PDA-Chromatogramm der Verbindung von Beispiel 45.
IC50 [*µ*M] <50.

### ROUTE 9

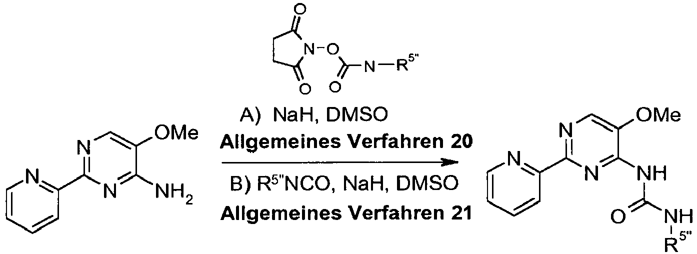

### Allgemeines Verfahren 20:

### Beispiel 46

### 1-(5-Methoxy-2-pyridin-2-yl-pyrimidin-4-yl)-3-methyl-harnstoff

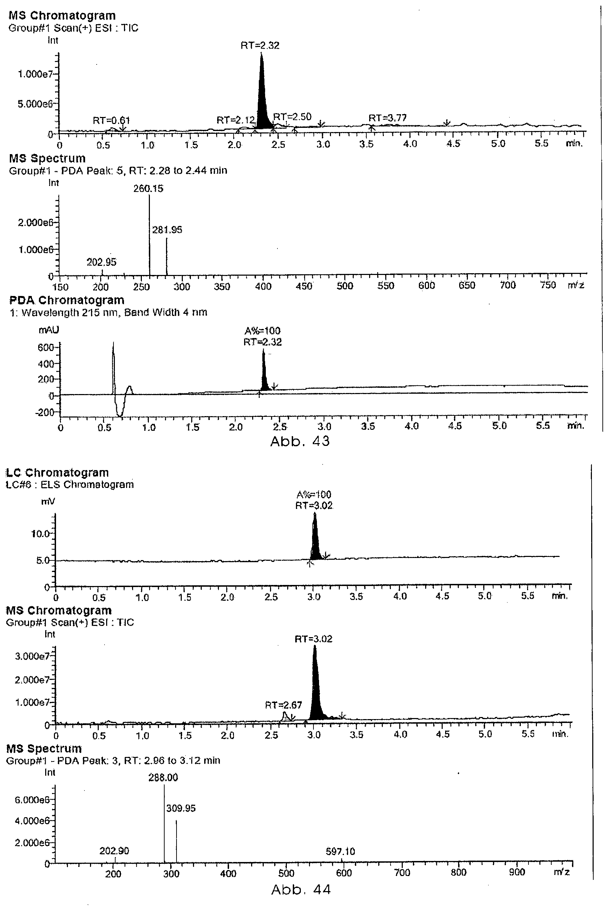

Natriumhydrid (60 %ig in Mineralöl, 12 mg, 0.29 mmol) wurde bei 0°C zu einer Lösung von 5-Methoxy-2-pyridin-2-yl-pyrimidin-4-ylamin (50 mg, 0.24 mmol) in DMSO (1 ml) gegeben, und die Mischung wurde für 0,25 h gerührt. N-Succinimidyl-N-methylcarbamat (51 mg, 0.29 mmol) wurde tropfenweise hinzugegeben und die Mischung wurde bei Raumtemperatur für 4 h gerührt. Die Reaktionsmischung wurde mit Eiswasser (10 ml) verdünnt, und die wässrige Phase wurde mit EtOAc (x 2) extrahiert. Die vereinigten organischen Phasen wurden mit Kochsalzlösung gewaschen, getrocknet (Na₂SO₄) und im Vakuum eingeengt. Der Rohrückstand wurde durch Säulenchromatographie mit DCM/0.1% NH₃ in MeOH (97:3) als Eluent gereinigt, um die Titelverbindung zu ergeben (21 mg, 32 %).
MW: 259.26
HPLCMS (Methode A): [m/z]: 260
Abb. 43 zeigt das MS-Chromatogramm, das MS-Spektrum und das PDA-Chromatogramm der Verbindung von Beispiel 46.
IC50 [*µ*M]: >50.

### Beispiel 47

### Allgemeines Verfahren 21:

### 1-Isopropyl-3-(5-methoxy-2-pyridin-2-yl-pyrimidin-4-yl)-harnstoff

Natriumhydrid (60 %ig in Mineralöl, 13 mg, 0.3 mmol) wurde zu einer Lösung von 5-Methoxy-2-pyridin-2-yl-pyrimidin-4-ylamin (50 mg, 0.24 mmol) in DMSO (1 ml) gegeben, und die Mischung wurde bei Raumtemperatur für 0,25 h gerührt. Isopropylisocyanat (42 mg, 0.49 mmol) wurde bei Raumtemperatur hinzugegeben, und die Mischung wurde bei 80°C für 14 h gerührt. Die Mischung wurde mit Wasser (10 ml) verdünnt, und die wässrige Phase mit EtOAc (x 2) extrahiert. Die vereinigten organischen Phasen wurden mit Kochsalzlösung gewaschen, getrocknet (Na₂SO₄) und im Vakuum eingeengt. Der Rohrückstand wurde durch Säulenchromatographie mit DCM/3% NH₃ in MeOH (95:5) als Eluent gereinigt, um die Titelverbindung zu ergeben (23 mg, 32 %).
MW: 287.31
HPLCMS (Methode A): [m/z]: 288
Abb. 44 zeigt das MS-Chromatogramm, das MS-Spektrum und das PDA-Chromatogramm der Verbindung von Beispiel 47.
IC50 [*µ*M]: >50.

### Beispiel 48

### Synthese von 1-(5-Methoxy-2-pyridin-2-yl-pyrimidin-4-yl)-3-phenyl-harnstoff

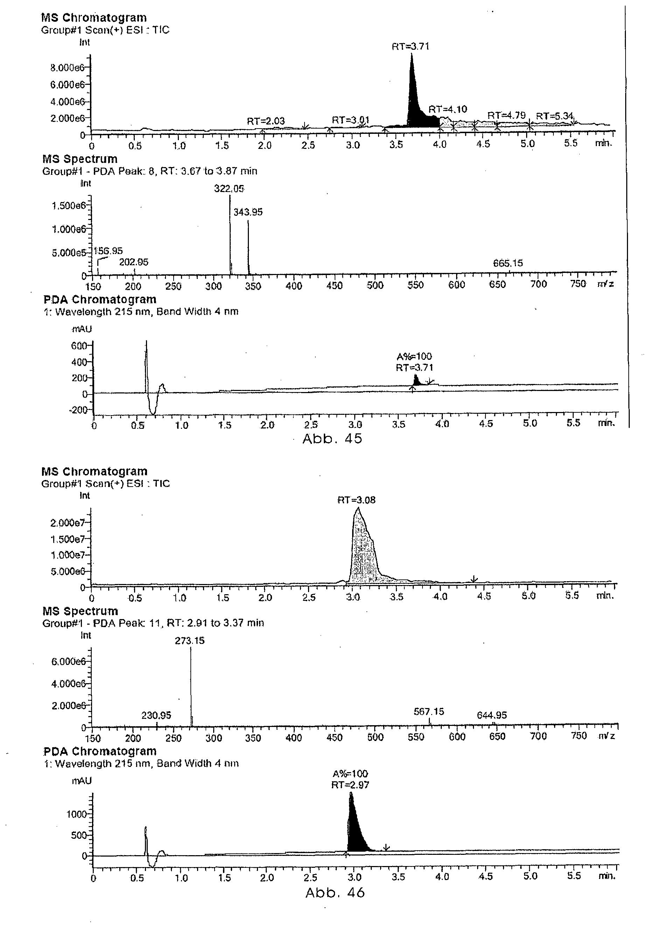

In ähnlicher Weise, unter Verwendung von Route 9, allgemeines Verfahren 21, ergaben 5-Methoxy-2-pyridin-2-yl-pyrimidin-4-ylamin (50 mg, 0.24 mmol), Natriumhydrid (60% ig in Mineralöl, 12 mg, 0.29 mmol) und Phenylisocyanat (35 mg, 0.29 mmol) die Titelverbindung (16 mg, 0 %) nach Reinigung durch Säulenchromatographie mit DCM/0.1 % NH₃ in MeOH (95:5) als Eluent.
MW: 321.33
HPLCMS (Methode A): [m/z]: 322
Abb. 45 zeigt das MS-Chromatogramm, das MS-Spektrum und das PDA-Chromatogramm der Verbindung von Beispiel 48.
IC50 [*µ*M]: >50.

### ROUTE 10

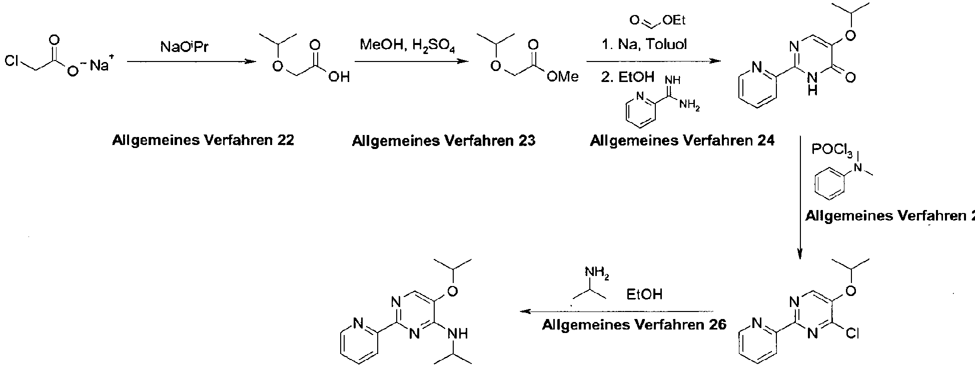

### Allgemeines Verfahren 22:

### Isopropoxyessigsäure

Das Natriumsalz von Chloressigsäure (20 g, 171 mmol) wurde portionsweise bei 80°C zu Natriumisopropoxid-Lösung gegeben (hergestellt aus 5.92 g Natrium und 60 ml Isopropanol). Die Reaktionsmischung wurde unter Rückfluss für 4 h erhitzt. Nach Abkühlen wurde die Mischung im Vakuum eingeengt. Der Rückstand wurde mit Wasser (80 ml) verdünnt und auf pH 2-3 mit 1N HCl angesäuert. Die wässrige Phase wurde mit EtOAc (x 6) extrahiert. Die vereinigten organischen Phasen wurde getrocknet (Na₂SO₄) und im Vakuum eingeengt, um die Titelverbindung zu ergeben (18 g, 89 %), die ohne weitere Reinigung eingesetzt wurde.

### Allgemeines Verfahren 23:

### Isopropoxyessigsäuremethytester

Thionylchlorid (22.2 ml, 303 mmol) wurde tropfenweise zu einer Lösung von Isopropoxyessigsäure (17.9 g, 179 mmol) in MeOH (70 ml) bei -5 °C gegeben. Die Reaktionsmischung wurde unter Rückfluss für 9 h erhitzt. Nach Abkühlen wurde die Mischung im Vakuum eingeengt. Der Rückstand wurde mit gesättigter wässriger NaHCO₃-Lösung (100 ml) verdünnt und mit Et₂O (x 2) extrahiert. Die vereinigten organischen Phasen wurden mit Kochsalzlösung gewaschen, getrocknet (Na₂SO₄) und im Vakuum eingeengt, um die Titelverbindung als gelbes Öl zu ergeben (15.5 g, 78 %), welches ohne weitere Reinigung verwendet wurde.

### Allgemeines Verfahren 24:

### 5-Isopropoxy-2-pyridin-2-yl-3H-pyrimidin-4-on

Isopropoxyessigsäuremethylester (1.0 g, 7.5 mmol) und Ameisensäureethylester (0.56 g, 7.5 mmol) wurden gleichzeitig zu einer gerührten Suspension von Natrium (0.18 g, 7.5 mmol) in Toluol (20 ml) gegeben, und die Mischung wurde bei Raumtemperatur für 12 h gerührt. Das Toluol wurde dekantiert, der Rückstand mit EtOH (20 ml) verdünnt und Pyridin-2-carboxamidin (0.83 g, 5.3 mmol) wurde hinzugegeben, gefolgt von einer Lösung von Natriumethoxid (hergestellt aus Na 0.35 g, 15 mmol in 5 ml von EtOH). Die Reaktionsmischung wurde unter Rückfluss für 20 h erhitzt. Die Mischung wurde filtriert und der Rückstand mit 1N HCl (10 ml) neutralisiert. Die Mischung wurde im Vakuum eingeengt, und der Rohrückstand durch Säulenchromatographie mit DCM/1% NH₃ in MeOH (98:2) als Eluent gereinigt, um die Titelverbindung zu ergeben (0.18 g, 11 %).
MW: 231.25
HPLCMS (Methode D): [m/z]: 232

### Allgemeines Verfahren 25:

### 4-Chlor-5-isopropoxy-2-pyridin-2-yl-pyrimidin

Eine Lösung von 5-Isopropoxy-2-pyridin-2-yl-3H-pyrimidin-4-on (0.18 g, 0.78 mmol) und POCl₃ (0.76 ml, 7.8 mmol) in N,N-Dimethylanilin (0.22 ml) wurde für 1 h unter Rückfluss erhitzt. Die Reaktionsmischung wurde auf Eis (50 ml) gegossen und mit gesättigter wässriger NaHCO₃-Lösung auf pH 8 - 9 basisch gemacht. Die wässrige Phase wurde mit EtOAc (x 3) extrahiert. Die vereinigten organischen Phasen wurden getrocknet (Na₂SO₄) und im Vakuum eingeengt. Der Rohrückstand wurde durch Säulenchromatographie mit DCM/1% NH₃ in MeOH (98:2) als Eluent gereinigt, um die Titelverbindung zu ergeben (0.14 g, 72 %).
MW: 249.69
HPLCMS (Methode D): [m/z]: 250

### Allgemeines Verfahren 26:

### Beispiel 49

### (5-Isopropoxy-2-pyridin-2-yl-pyrimidin-4-yl)-isopropyl-amin

4-Chlor-5-isopropoxy-2-pyridin-2-yl-pyrimidin (0.13 g, 0.52 mmol), Isopropylamin (45 *µ*l, 0.52 mmol) und DIPEA (0.18 ml, 1.04 mmol) wurden in EtOH (2 ml) gelöst und die Mischung wurde für 15 h bei 80°C gerührt. Nach Abkühlen wurde die Mischung im Vakuum eingeengt. Der Rückstand wurde mit Wasser (15 ml) verdünnt, und die wässrige Phase mit EtOAc (x 3) extrahiert. Die vereinigten organischen Phasen wurden getrocknet (Na₂SO₄) und im Vakuum eingeengt. Der Rohrückstand wurde durch Säulenchromatographie mit DCM/1% NH₃ in MeOH (95:5) als Eluent gereinigt, um die Titelverbindung zu ergeben (55 mg, 38 %).
MW: 272.34
HPLCMS (Methode A): [m/z]: 273
Abb. 46 zeigt das MS-Chromatogramm, das MS-Spektrum und das PDA-Chromatogramm der Verbindung von Beispiel 49.
IC50 [*µ*M]: >50.

### ROUTE 12

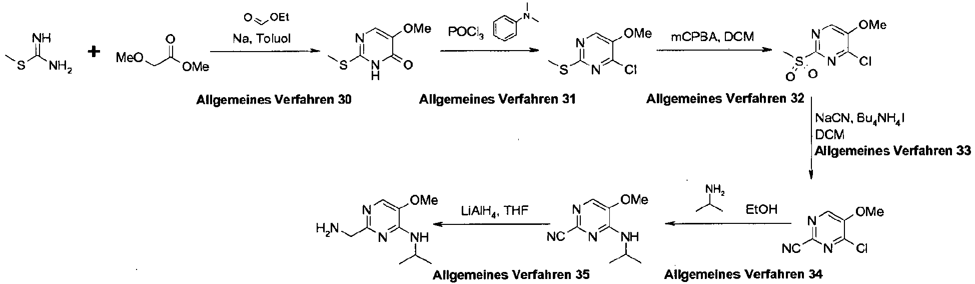

### Allgemeines Verfahren 30:

### 5-Methoxy-2-methylsulfonyl-3H-pyrimidin-4-on

Methyl-methoxyacetat (2.0 g, 19.2 mmol) und Ameisensäureethylester (1.42 g, 19.2 mmol) wurden gleichzeitig zu einer gerührten Suspension von Natrium (0.44 g, 19.2 mmol) in Toluol (20 ml) gegeben, und die Mischung bei Raumtemperatur für 12 h gerührt. Toluol wurde dekantiert, der Rohrückstand mit EtOH (20 ml) verdünnt, und S-Methylthioharnstoff (1.3 g, 15 mmol) wurde in einer Portion hinzugegeben, gefolgt von einer Lösung von Natriumethoxid (hergestellt aus Na 0.35 g, 15 mmol und 5 ml EtOH). Die Reaktionsmischung wurde unter Rückfluss für 15 h erhitzt. Die Mischung wurde filtriert und der Rückstand mit 1N HCl (10 ml) neutralisiert. Das Lösungsmittel wurde im Vakuum entfernt. Der Rohrückstand wurde mit MeOH (20 ml) verdünnt, für 0,25 h gerührt und über Zelithe filtriert. Das Filtrat wurde im Vakuum eingeengt, um die Titelverbindung zu ergeben (0.5 g, 21 %).
MW: 172.20
HPLCMS (Methode D): [m/z]: 173

### Allgemeines Verfahren 31:

### 4-Chloro-5-methoxy-2-methylsulfonyl-pyrimidin

Eine Lösung von 5-Methoxy-2-methylsulfonyl-3H-pyrimidin-4-on (0.77 g, 4.4 mmol) und POCl₃ (6.8 g, 44 mmol) in N,N-Dimethylanilin (0.4 ml) wurde unter Rückfluss für 1 h erhitzt. Die Reaktionsmischung wurde auf Eis (50 ml) gegossen und mit gesättigter wässriger NaHCO₃ auf pH 8 - 9 basisch gemacht, und die wässrige Phase wurde mit DCM (x 3) extrahiert. Die vereinigten organischen Phasen wurden getrocknet (Na₂SO₄) und im Vakuum eingeengt. Der Rohrückstand wurde durch Säulenchromatographie gereinigt mit EtOAc/Hexan (1:9-4:6) als Eluent, um die Titelverbindung zu ergeben (0.2 g, 33 %).
MW: 190.65
HPLCMS (Methode D): [m/z]: 191

### Allgemeines Verfahren 32:

### 4-Chlor-2-methansulfonyl-5-methoxy-pyrimidin

Eine Lösung von 3-Chlorperoxybenzoesäure (0.4 g, 2.3 mmol) in DCM (2 ml) wurde tropfenweise zu einer Lösung von 4-Chlor-5-methoxy-2-methylsulfonyl-pyrimidin (0.15 g, 0.78 mmol) in DCM (10 ml) gegeben, und die Mischung wurde bei Raumtemperatur für 12 h gerührt. Wasser (10 ml) wurde hinzugegeben, die wässrige Phase mit DCM extrahiert und im Vakuum eingeengt. Der Rohrückstand wurde durch Säulenchromatographie mit DCM/1% NH₃ in MeOH (98:2) als Eluent gereinigt, um die Titelverbindung zu ergeben (0.18 g, 100 %).
MW: 222.64
HPLCMS (Methode D): [m/z]: 223

### Allgemeines Verfahren 33:

### 4-Chlor-5-methoxy-pyrimidin-2-carbonitril

4-Chlor-2-methansulfonyl-5-methoxy-pyrimidin (0.18 g, 0.8 mmol) wurde zu einer Lösung von Natriumcyanid, Tetrabutylammoniumiodid (16 mg, 0.04 mmol) in DCM (3 ml) und Wasser (0.6 ml) gegeben, und die Mischung wurde bei Raumtemperatur für 16 h gerührt. Wasser (10 ml) wurde hinzugegeben, und die Mischung wurde mit DCM (x 2) extrahiert, die vereinigten organischen Phasen wurden getrocknet (Na₂SO₄) und im Vakuum eingeengt. Der Rohrückstand wurde durch Säulenchromatographie mit EtOAc/Hexan (1:9-4:6) als Eluent gereinigt, um die Titelverbindung zu ergeben (65 mg, 50 %).
MW: 169.56
HPLCMS (Methode D): [m/z]: 170

Allgemeines Verfahren 34:

### 4-Isopropylamino-5-methoxy-pyrimidin-2-carbonitril

4-Chlor-5-methoxy-pyrimidin-2-carbonitril (65 mg, 0.38 mmol), Isopropylamin (34 *µ*l, 0.42 mmol) und DIPEA (75 *µ*l, 0.46 mmol) wurden in EtOH (2 ml) gelöst. Die Mischung wurde bei Raumtemperatur für 15 h gerührt. Die Mischung wurde im Vakuum eingeengt. Der Rückstand wurde mit Wasser (15ml) verdünnt, und die Reaktionsmischung mit Ethylacetat (x 3) extrahiert. Die vereinigten organischen Phasen wurden getrocknet (Na₂SO₄) und im Vakuum eingeengt. Der Rohrückstand wurde aus Penthan verfestigt, um die Titelverbindung zu ergeben (30 mg, 40 %).
MW: 192.21
HPLCMS (Methode D): [m/z]: 193

### Beispiel 50

### Allgemeines Verfahren 35:

### (2-Aminomethyl-5-methoxy-pyrimidin-4-yl)-isopropyl-amin

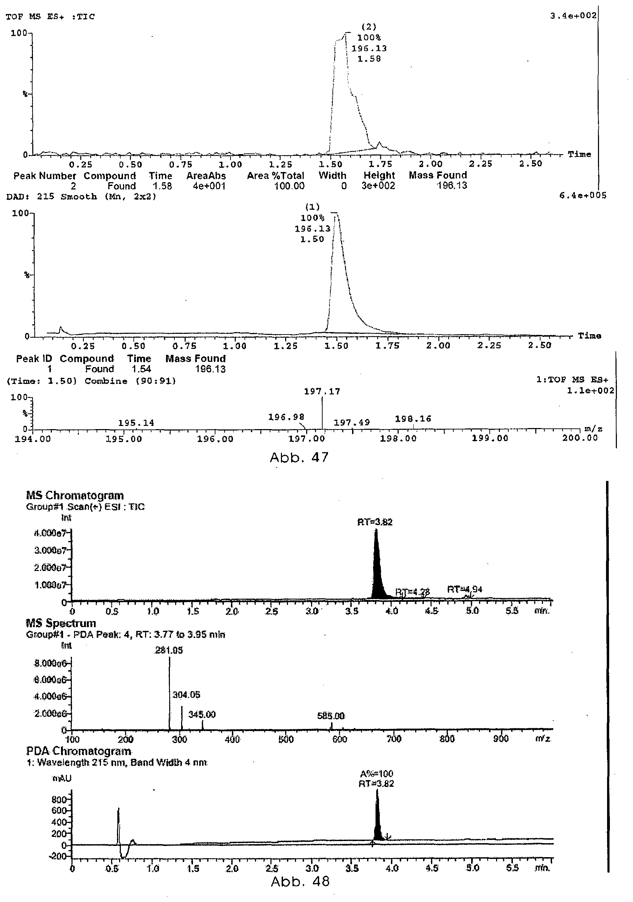

4-Isopropylamino-5-methoxy-pyrimidin-2-carbonitril (30 mg, 0.13 mmol) in THF (3 ml) wurde tropfenweise zu einer Lösung von Lithiumaluminiumhydrid (19 mg, 0.52 mmol) in THF (2 ml) bei 0°C gegeben, und die Mischung wurde bei Raumtemperatur für 0,75 h gerührt. Der Rückstand wurde mit 1N NaOH Lösung (5 ml) verdünnt, und die Mischung wurde im Vakuum eingeengt. Der Rohrückstand wurde durch Säulenchromatographie mit DCM/MeOH (98:2) als Eluent gereinigt, um die Titelverbindung zu ergeben (29 mg, 96 %).
MW: 196.27
HPLCMS (Methode D): [m/z]: 197
Abb. 47 zeigt die Spektren/Chromatogramme der Verbindung von Beispiel 50.
IC50 [*µ*M]: <50.

### ROUTE 13

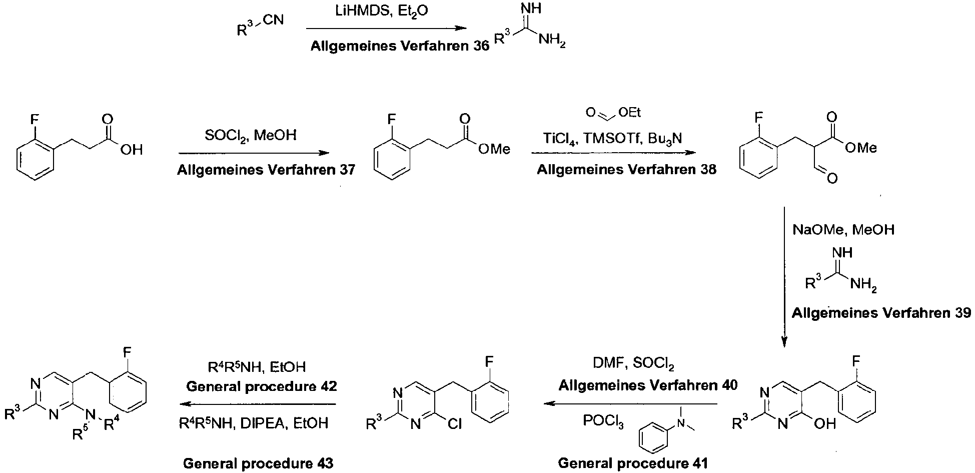

### Allgemeines Verfahren 36:

### Pyridin-2-carboxamidin

Lithiumhexamethyldisilazid (1 M Lösung in THF, 60.5 ml, 60.5 mmol) wurde zu einer Lösung von Pyridin-2-carbonitril (3.0 g, 28.8 mmol) in Et₂O (30 ml) bei 0°C gerührt. Der Ansatz wurde auf Raumtemperatur über Nacht erwärmen gelassen. Der Ansatz wurde auf 0°C abgekühlt, und 3 M HCl (54 ml) wurde hinzugegeben, und der Ansatz wurde für 30 min. gerührt. Wasser (135 ml) wurde hinzugegeben, und die organische Phase wurde abgetrennt und verworfen. Die wässrige Schicht wurde auf pH 14 mit gesättigter wässriger NaOH basisch gemacht und mit DCM (x 3) extrahiert. Die vereinigten organischen Phasen wurden getrocknet (Na₂SO₄) und im Vakuum eingeengt, um die Titelverbindung zu ergeben (1.70 g, 49 %).
MW: 121.14
HPLCMS (Methode B):[m/z]: 122

### Nicotinamidin

In ähnlicher Weise, unter Verwendung von Route 13, allgemeines Verfahren 36, ergaben Lithiumhexamethyldisilazid (1 M Lösung in THF, 40.4 ml, 40.4 mmol), Nicotinonitril (2.0 g, 19.2 mmol) in Et₂O (30 ml) die Titelverbindung (0.95 g, 41 %).
MW: 121.14
HPLCMS (Methode B):[m/z]: 122

### Allgemeines Verfahren 37:

### 3-(2-Fluorphenyl)-propionsäuremethylester

Thionylchlorid (0.65 ml, 9.82 mmol) wurde tropfenweise zu einer Lösung von 3-(2-Fluorphenyl)propionsäure (1.0 g, 5.95 mmol) in MeOH (10 ml) bei 0°C gerührt. Die Mischung wurde auf Raumtemperatur erwärmen gelassen und unter Rückfluss für 2 h erwärmt. Die Reaktionsmischung wurde im Vakuum eingeengt, mit gesättigter wässriger NaHCO₃-Lösung (10 ml) verdünnt und mit Et₂O (x 3) extrahiert. Die vereinigten organischen Phasen wurden mit Kochsalzlösung gewaschen, getrocknet (Na₂SO₄) und im Vakuum eingeengt, um die Titelverbindung zu ergeben (1.0 g, 93 %).
MW: 182.20
HPLCMS (Methode B):[m/z]: 183

### Allgemeines Verfahren 38:

### 2-(2-Fluorbenzyl)-3-oxo-propionsäuremethylester

Titan[IV)-chlorid (0.91 ml, 8.24 mmol), Trimethylsilyltrifluormethansulfonat (25 *µ*l, 0.14 mmol) gefolgt von Tri-n-butylamin (2.9 ml, 12.35 mmol) wurden tropfenweise zu einer Lösung von 3-(2-Fluorphenyl)-propionsäuremethylester (0.5 g, 2.74 mmol) und Ameisensäureethylester (0.33 ml, 4.11 mmol) in Toluol (20 ml) gegeben. Die Mischung wurde bei Raumtemperatur für 18 h gerührt. Wasser (20 ml) wurde hinzugegeben, und die wässrige Phase wurde mit EtOAc (x 2) extrahiert. Die vereinigten organischen Phasen wurden mit Kochsalzlösung gewaschen, getrocknet (Na₂SO₄) und im Vakuum eingeengt. Die partielle Reinigung der Säulenchromatographie mit EtOAc/Heptan (8:92) als Eluent ergab die Titelverbindung (200 mg, 35 %) in unreiner Form. Das Produkt wurde in die nächste Stufe ohne weitere Reinigung eingesetzt. Die Verbindung konnte nicht durch HPLCMS nachgewiesen werden, ihre Struktur wurde daher durch 1H-NMR bestätigt.

### Beispiel 51

### Allgemeines Verfahren 39:

### 5-(2-Fluorbenzyl)-2-pyridin-2-yl-pyrimidin-4-ol

NaOMe (133 mg, 2.48 mmol) wurde zu einer Lösung von 2-(2-Fluorbenzyl)-3-oxo-propionsäuremethylester (500 mg, 2.38 mmol) und Pyridin-2-carboxamidin 33 (200 mg, 1.65 mmol) in MeOH (10 ml) gegeben. Der Ansatz wurde bei Raumtemperatur für 65 h gerührt. Der Ansatz wurde im Vakuum eingeengt und durch Säulenchromatographie mit MeOH/DCM (5:95) als Eluent gereinigt. Der resultierende Feststoff wurde aus Et₂O verfestigt (trituriert), um die Titelverbindung zu ergeben (262 mg, 45 %).
MW: 281.28
HPLCMS (method A):[m/z]: 282
Abb. 48 zeigt das MS-Chromatogramm, das MS-Spektrum und das PDA-Chromatogramm der Verbindung von Beispiel 51.
IC50 [*µ*M]: <50.

### Beispiel 52

### 5-(2-Fluorbenzyl)-2-pyridin-3-yl-pyrimidin-4-ol

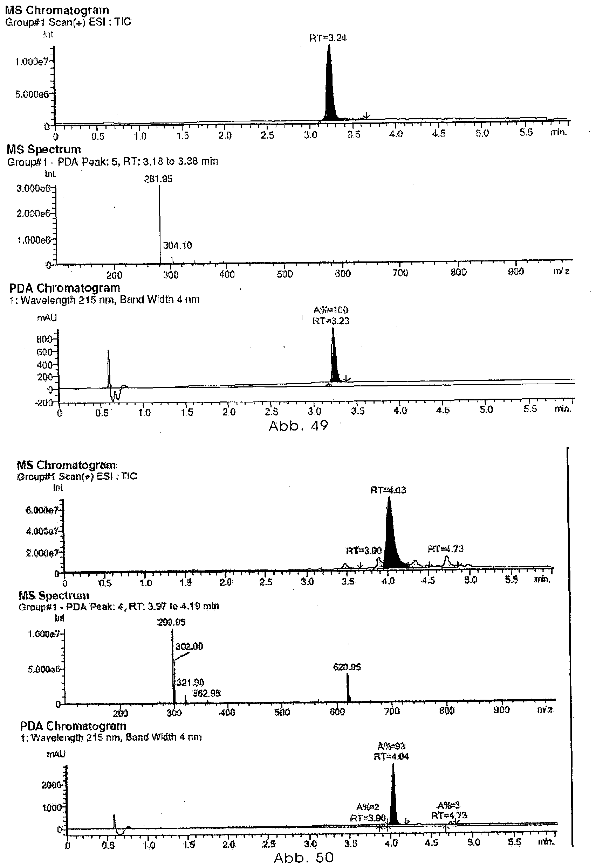

In ähnlicher Weise, unter Verwendung von Route 13, allgemeines Verfahren 39, ergaben NaOMe (167 mg, 3.10 mmol), 2-(2-Fluorbenzyl)-3-oxo-propionsäuremethylester (650 mg, 3.10 mmol) und Nicotinamidin 73 (250 mg, 2.06 mmol) die Titelverbindung (279 mg, 37 %) nach Reinigung durch Säulenchromatographie mit DCM/MeOH (97:3) als Eluent, gefolgt durch Verfestigung aus Et₂O.
MW: 281.28
HPLCMS (Methode A):[m/z]: 282
Abb. 49 zeigt das MS-Chromatogramm, das MS-Spektrum und das PDA-Chromatogramm der Verbindung von Beispiel 52.
IC50 [*µ*M]: >50.

### Beispiel 53

### Allgemeines Verfahren 40:

### 4-Chlor-5-(2-fluorbenzyl)-2-pyridin-2-yl-pyrimidin

DMF (Kat.) wurde zu einer Lösung von 5-(2-Fluorbenzyl)-2-pyridin-2-yl-pyrimidin-4-ol (100 mg, 0.35 mmol) in Thionylchlorid (1 ml) gegeben, und die Mischung wurde bei 80°C für 1 h erhitzt. Nach Abkühlen wurde die Reaktionsmischung im Vakuum eingeengt und der Rückstand wurde mit gesättigter wässriger NaHCO₃-Lösung (10 ml) basisch gemacht und mit DCM (x 3) extrahiert. Die vereinigten organischen Phasen wurden getrocknet (Na₂SO₄) und im Vakuum eingeengt, um die Titelverbindung zu ergeben (107 mg, 100 %).
MW: 299.73
HPLCMS (method A):[m/z]: 300
Abb. 50 zeigt das MS-Chromatogramm, das MS-Spektrum und das PDA-Chromatogramm der Verbindung von Beispiel 53.
IC50 [*µ*M]: <50.

### Beispiel 54

### 4-Chlor-5-(2-fluorbenzyl)-2-pyridin-3-yl-pyrimidin

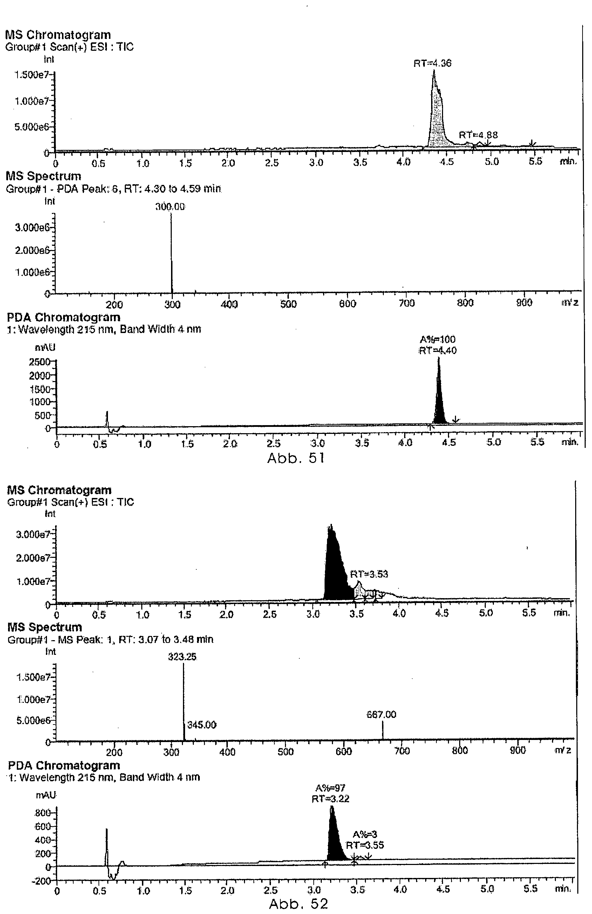

In ähnlicher Weise, unter Verwendung von Route 13, allgemeines Verfahren 40, ergaben DMF (Kat.), 5-(2-Fluorbenzyl)-2-pyridin-3-yl-pyrimidin-4-ol 77 (100 mg, 0.36 mmol) und Thionylchlorid (1 ml) die Titelverbindung (107 mg, 100 %) nach wässriger Aufarbeitung.
MW: 299.74
HPLCMS (Methode A):[m/z]: 300
Abb. 51 zeigt das MS-Chromatogramm, das MS-Spektrum und das PDA-Chromatogramm der Verbindung von Beispiel 54.
IC50 [*µ*M]: >50.

### Allgemeines Verfahren 41:

### 4-Chlor-5-(2-fluorbenzyl)-2-pyridin-2-yl-pyrimidin

5-(2-Fluorbenzyl)-2-(pyridin-2-yl)pyrimidin-4-ol (70 mg, 0.25 mmol) und POCl₃ (0.39 g, 2.5 mmol) in N,N-Dimethylanilin (0.07 ml) wurden unter Rückfluss für 1 h erhitzt. Die Reaktionsmischung wurde auf Eis (50 ml) gegossen und mit gesättiger wässriger NaHCO₃-Lösung auf pH 8 - 9 basisch gemacht. Die wässrige Phase wurde mit DCM (x 3) extrahiert. Die vereinigten organischen Phasen wurden getrocknet (Na₂SO₄) und im Vakuum eingeengt. Der Rohrückstand wurde durch Säulenchromatographie mit DCM als Eluent gereinigt, um die Titelverbindung zu ergeben (25 mg, 33 %).
MW: 299.74
HPLCMS (Methode D) [m/z]: 300

### Beispiel 55

### Allgemeines Verfahren 42:

### [5-(2-Fluorbenzyl)-2-pyridin-2-yl-pyrimidin-4-yl]-isopropyl-amin

Diisopropylamin (69 *µ*l, 0.80 mmol) wurde zu einer Lösung von 4-Chlor-5-(2-fluorbenzyl)-2-pyridin-2-yl-pyrimidin (107 mg, 0.36 mmol) in EtOH (1.1 ml) gegeben, und die Mischung wurde unter Rückfluss für 18 h erhitzt. Nach Abkühlen wurde die Reaktionsmischung im Vakuum eingeengt. Der Rückstand wurde mit gesättigter NaHCO₃-Lösung (1 ml) basisch gemacht und mit DCM (x 3) extrahiert. Die organische Phase wurde mit Wasser (x 2) gewaschen, getrocknet (Na₂SO₄) und im Vakuum eingeengt, um die Titelverbindung zu ergeben (92 mg, 78 %).
MW: 322.39
HPLCMS (Methode A):[m/z]: 323
Abb. 52 zeigt das MS-Chromatogramm, das MS-Spektrum und das PDA-Chromatogramm der Verbindung von Beispiel 55.
IC50 [*µ*M]: <50.

### Beispiel 56

### [5-(2-Fluorbenzyl)-2-pyridin-2-yl-pyrimidin-4-yl]-methyl-amin

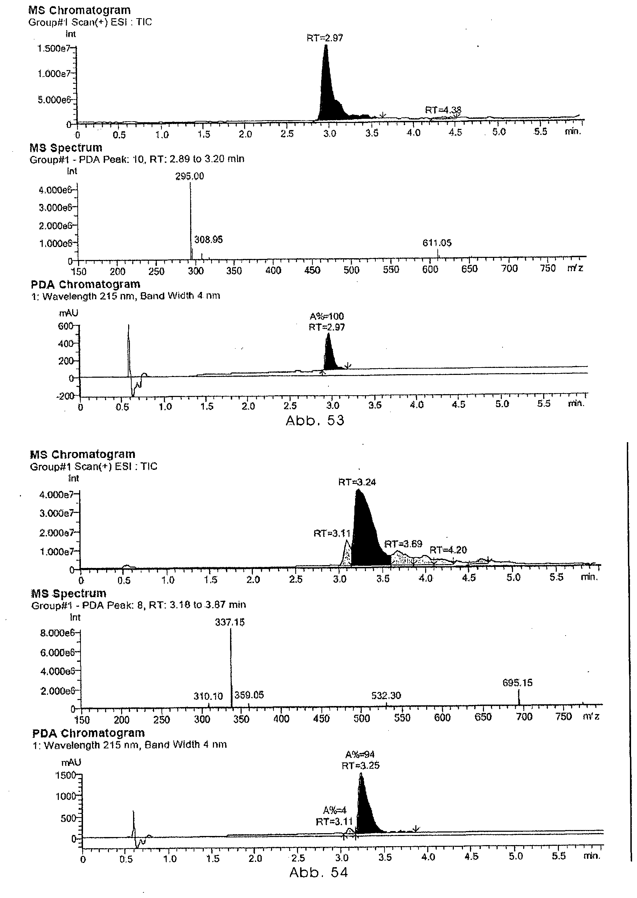

In ähnlicher Weise, unter Verwendung von Route 13, allgemeines Verfahren 42, ergaben 4-Chlor-5-(2-fluorbenzyl)-2-pyridin-2-yl-pyrimidin (103 mg, 0.34 mmol), Methylamin (2M in THF, 0.75 ml, 1.53 mmol) in EtOH (1 ml) die Titelverbindung (57 mg, 57 %) nach Reinigung durch präparative HPLC (saure Bedingungen).
MW: 294.32
HPLCMS (Methode A):[m/z]: 295
Abb. 53 zeigt das MS-Chromatogramm, das MS-Spektrum und das PDA-Chromatogramm der Verbindung von Beispiel 56.
IC50 [*µ*M]: >50.

### Beispiel 57

### Diethyl-[5-(2-fluorbenzyl)-2-pyridin-2-yl-pyrimidin-4-yl]-amin

In ähnlicher Weise, unter Verwendung von Route 13, allgemeines Verfahren 42, ergaben 4-Chlor-5-(2-fluorbenzyl)-2-pyridin-2-yl-pyrimidin (103 mg, 0.34 mmol), Diethylamin (0.16 ml, 1.53 mmol) in EtOH (1 ml) die Titelverbindung (88 mg, 77 %) nach basischer Aufarbeitung ohne weitere Reinigung.
MW: 336.41
HPLCMS (Methode A):[m/z]: 337
Abb. 54 zeigt das MS-Chromatogramm, das MS-Spektrum und das PDA-Chromatogramm der Verbindung von Beispiel 57.
IC50 [*µ*M]: <50.

### Beispiel 58

### Cyclohexylmethyl-[5-(2-fluorbenzyl)-2-pyridin-2-yl-pyrimidin-4-yl]-amin

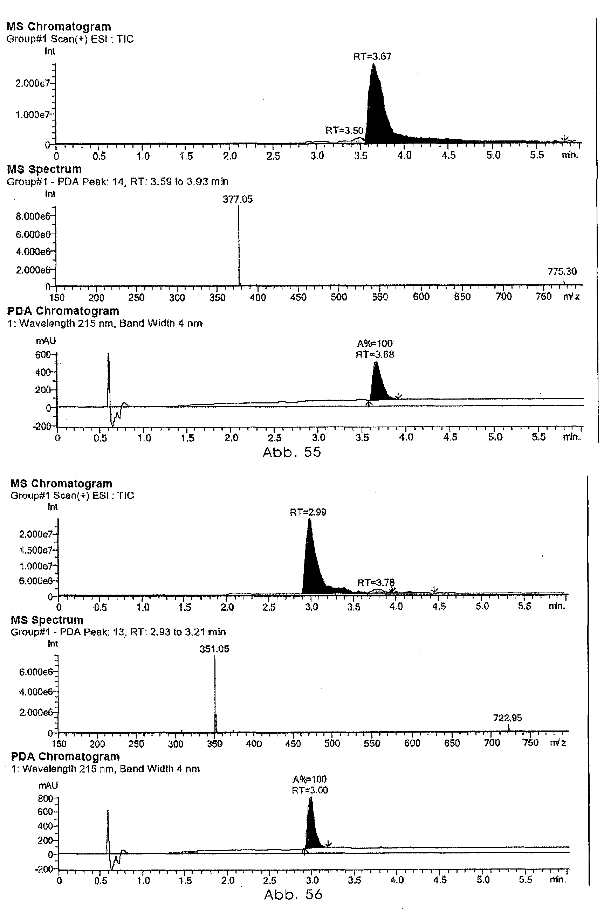

In ähnlicher Weise ergaben unter Verwendung von Route 13, allgemeines Verfahren 42, 4-Chlor-5-(2-fluorbenzyl)-2-pyridin-2-yl-pyrimidin (103 mg, 0.34 mmol), Cyclohexanmethylamin (0.20 ml, 1.53 mmol) in EtOH (1 ml) die Titelverbindung (80 mg, 63 %) nach Reinigung durch präparative HPLC (saure Bedingungen).
MW: 376.47
HPLCMS (Methode A):[m/z]: 377
Abb. 55 zeigt das MS-Chromatogramm, das MS-Spektrum und das PDA-Chromatogramm der Verbindung von Beispiel 58.
IC50 [*µ*M]: >50.

### Beispiel 59

### 4-[5-(2-Fluorbenzyl)-2-pyridin-2-yl-pyrimidin-4-yl]-morpholin

In ähnlicher Weise ergaben, unter Verwendung von Route 13, allgemeines Verfahren 42, 4-Chlor-5-(2-fluorbenzyl)-2-pyridin-2-yl-pyrimidin (103 mg, 0.34 mmol), Morpholin (0.13 ml, 1.53 mmol) in EtOH (1 ml) die Titelverbindung (82 mg, 69 %) nach basischer Aufarbeitung ohne weitere Reinigung.
MW: 350.39
HPLCMS (Methode A):[m/z]: 351
Abb. 56 zeigt das MS-Chromatogramm, das MS-Spektrum und das PDA-Chromatogramm der Verbindung von Beispiel 59.
IC50 [*µ*M] <50.

### Beispiel 60

### 5-(2-Fluorbenzyl)-4-piperidin-1-yl-2-pyridin-2-yl-pyrimidin

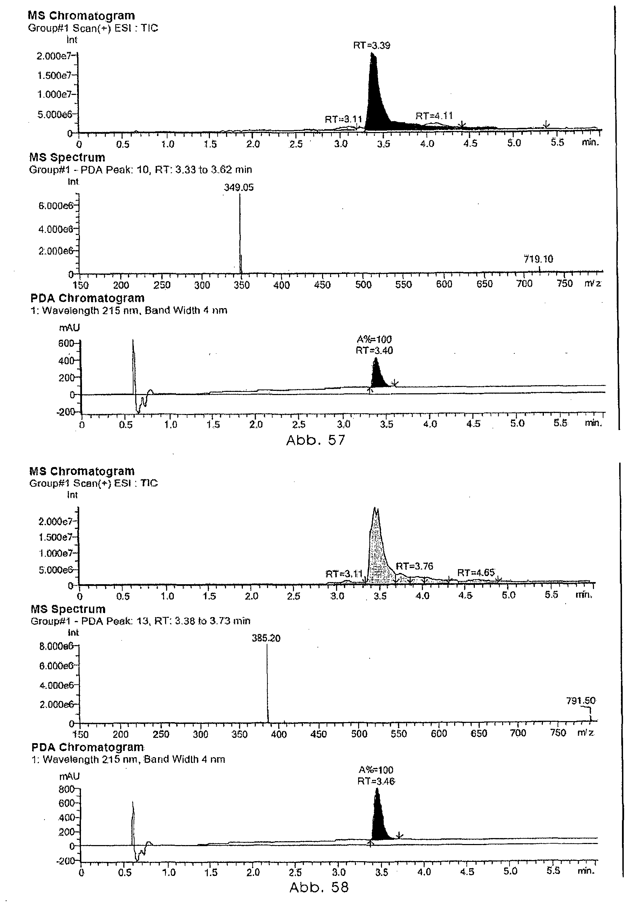

In ähnlicher Weise ergaben, unter Verwendung von Route 13, allgemeines Verfahren 42, 4-Chlor-5-(2-fluorbenzyl)-2-pyridin-2-yl-pyrimidin 78 (103 mg, 0.34 mmol), Piperidin (0.15 ml, 1.53 mmol) in EtOH (1 ml) die Titelverbindung (88 mg, 74 %) nach basischer Aufarbeitung ohne weitere Reinigung.
MW: 348.42
HPLCMS (Methode A):[m/z]: 349
Abb. 57 zeigt das MS-Chromatogramm, das MS-Spektrum und das PDA-Chromatogramm der Verbindung von Beispiel 60.
IC50 [*µ*M]: <50.

### Beispiel 61

### Benzyl-[5-(2-fluorbenzyl)-2-pyridin-2-yl-pyrimidin-4-yl]-methyl-amin

In ähnlicher Weise ergaben, unter Verwendung von Route 13, allgemeines Verfahren 42, 4-Chlor-5-(2-fluor-benzyl)-2-pyridin-2-yl-pyrimidin (103 mg, 0.34 mmol), N-Methylbenzylamin (0.20 ml, 1.53 mmol) in EtOH (1 ml) die Titelverbindung (97 mg, 74 %) nach Reinigung durch präparative HPLC (saure Bedingungen).
MW: 384.45
HPLCMS (Methode A):[m/z]: 385
Abb. 58 zeigt das MS-Chromatogramm, das MS-Spektrum und das PDA-Chromatogramm der Verbindung von Beispiel 61.
IC50 [*µ*M]: >50.

### Beispiel 62

### [5-(2-Fluorbenzyl)-2-pyridin-3-yl-pyrimidin-4-yl]-isopropyl-amin

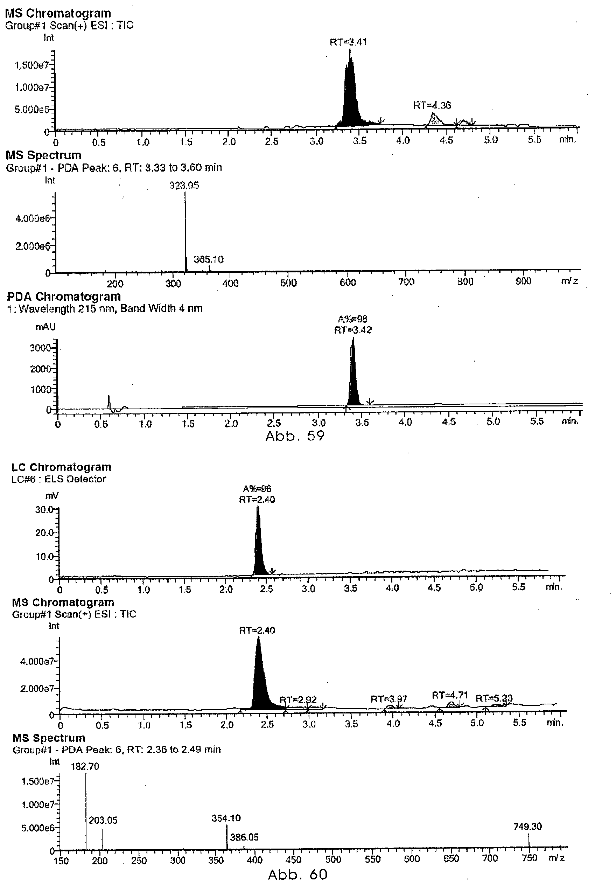

In ähnlicher Weise ergaben, unter Verwendung von Route 13, allgemeines Verfahren 42, Diisopropylamin (126 *µ*l, 1.49 mmol) und 4-Chlor-5-(2-fluorbenzyl)-2-pyridin-3-yl-pyrimidin (98 mg, 0.33 mmol) die Titelverbindung (83 mg, 79 %) nach wässriger Aufarbeitung.
MW: 322.39
HPLCMS (Methode A):[m/z]: 323
Abb. 59 zeigt das MS-Chromatogramm, das MS-Spektrum und das PDA-Chromatogramm der Verbindung von Beispiel 62.
IC50 [*µ*M]: >50.

### Beispiel 63

### Allgemeines Verfahren 43:

### 5-(2-Fluorbenzyl)-4-(4-methyl-piperazin-1-yl)-2-pyridin-2-yl-pyrimidin

4-Chlor-5-(2-fluorbenzyl)-2-pyridin-2-yl-pyrimidin (25 mg, 0.08 mmol), 1-Methylpiperazin (0.01 ml, 0.1 mmol) und DIPEA (17 *µ*l, 0.1 mmol) wurden in EtOH (2 ml) gelöst, und die Mischung wurde bei Raumtemperatur für 15 h gerührt. Die Mischung wurde im Vakuum eingeengt. Der Rückstand wurde mit Wasser (15 ml) verdünnt, und die Reaktionsmischung mit Ethylacetat (x 3) extrahiert. Die vereinigten organischen Phasen wurden getrocknet (Na₂SO₄) und im Vakuum eingeengt. Der Rohrückstand wurde durch Säulenchromatographie mit DCM/1% NH₃ in MeOH (96:4) als Eluent gereinigt, um die Titelverbindung zu ergeben (14 mg, 46 %).
MW: 364.44
HPLCMS (Methode A) [m/z]: 364
Abb. 60 zeigt das MS-Chromatogramm, das MS-Spektrum und das PDA-Chromatogramm der Verbindung von Beispiel 63.
IC50 [*µ*M] <50.

### ROUTE 14

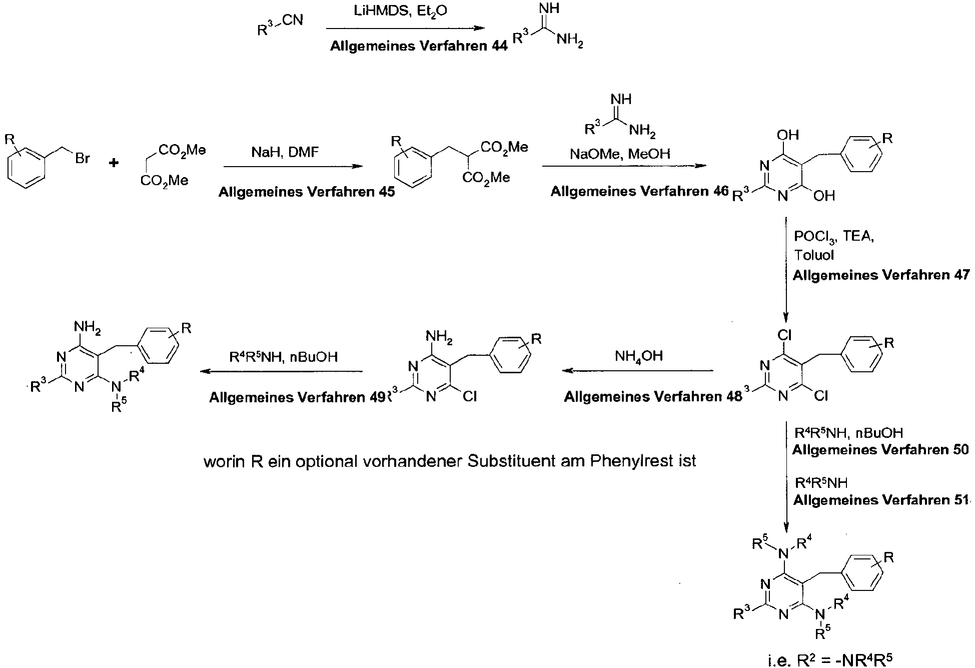

### Allgemeines Verfahren 44:

### 5-Fluorpyridin-2-carboxamidin

Trimethylaluminium (3.54 g, 49.14 mmol) wurde tropfenweise zu einer stark gerührten Lösung von NH₄Cl (2.63 g, 49.14 mmol) in trockenem Toluol (20 ml) bei 0°C gegeben. Die Mischung wurde auf Raumtemperatur erwärmt und für 15 min. gerührt. Eine Lösung von 5-Fluorpyridin-2-carbonitril (2.00 g, 16.38 mmol) in Toluol (20 ml) wurde tropfenweise hinzugegeben. Die Reaktionsmischung wurde auf 80°C für 18 h erhitzt. Nach Abkühlen wurde die Mischung in eine stark gerührte und abgekühlte (0°C) Aufschlämmung von Siliziumdioxid (20.0 g) in Chloroform (150 ml) gegeben und wurde für 10 min. gerührt. Die Mischung wurde filtriert und der Filterkuchen wurde mit MeOH (x 3) gewaschen. Das Filtrat wurde im Vakuum eingeengt. Der Rückstand wurde in 1 M HCl (150 ml) und Et₂O (70 ml) gelöst. Die oroganische Phase wurde abgetrennt und verworfen. Die wässrige Phase wurde mit gesättigter wässriger NaOH basisch gemacht und mit Chloroform (x 2) extrahiert. Die vereinigten organischen Extrakte wurden getrocknet (Na₂SO₄) und im Vakuum eingeengt, um die Titelverbindung zu ergeben (394 mg, 17 %). Die Verbindung konnte nicht durch HPLCMS nachgewiesen werden, daher wurde die Struktur durch 1H-NMR bestätigt.

### Allgemeines Verfahren 45:

### 2-Benzyl-malonsäuredimethylester

Malonsäuredimethylester (369 *µ*l, 3.22 mmol) wurde tropfenweise zu einer Suspension von NaH (60 %ige Dispersion in Mineralöl, 140 mg, 3.51 mmol) in DMF (5 ml) bei 0°C gegeben. Die Reaktionsmischung wurde bei Raumtemperatur für 30 min. gerührt. Die Reaktionsmischung wurde auf 0°C abgekühlt und Benzylbromid (350 *µ*l, 2.92 mmol) wurde tropfenweise zugegeben. Die Reaktionsmischung wurde auf Raumtemperatur über Nacht erwärmen gelassen. EtOAc (10 ml) wurde hinzugegeben, gefolgt von gesättigter wässriger NH₄Cl-Lösung (10 ml). Die Phasen wurden getrennt, und die organische Phase mit Wasser gewaschen, getrocknet (Na₂SO₄) und im Vakuum eingeengt. Der Rohrückstand wurde durch Säulenchromatographie mit EtOAc/Heptan (5:95) als Eluent gereinigt, um die Titelverbindung zu ergeben (325 mg, 25 %).
MW: 222.24
HPLCMS (Methode B):[m/z]: 223

### 2-(2-Fluorbenzyl)-malonsäuredimethylester

In ähnlicher Weise, unter Verwendung von Route 14, allgemeines Verfahren 45, ergaben Malonsäuredimethylester (2.0 ml, 17.46 mmol), NaH (60 %ige Dispersion in Mineralöl, 0.76 g, 19.05 mmol), 2-Fluorbenzylbromid (2.1 ml, 19.05 mmol) in THF (60 ml) die Titelverbindung (1.80 g, 47 %).
MW: 240.23
HPLCMS (Methode B):[m/z]: 241

### Beispiel 64

### Allgemeines Verfahren 46:

### 5-Benzyl-2-pyridin-2-yl-pyrimidin-4,6-diol

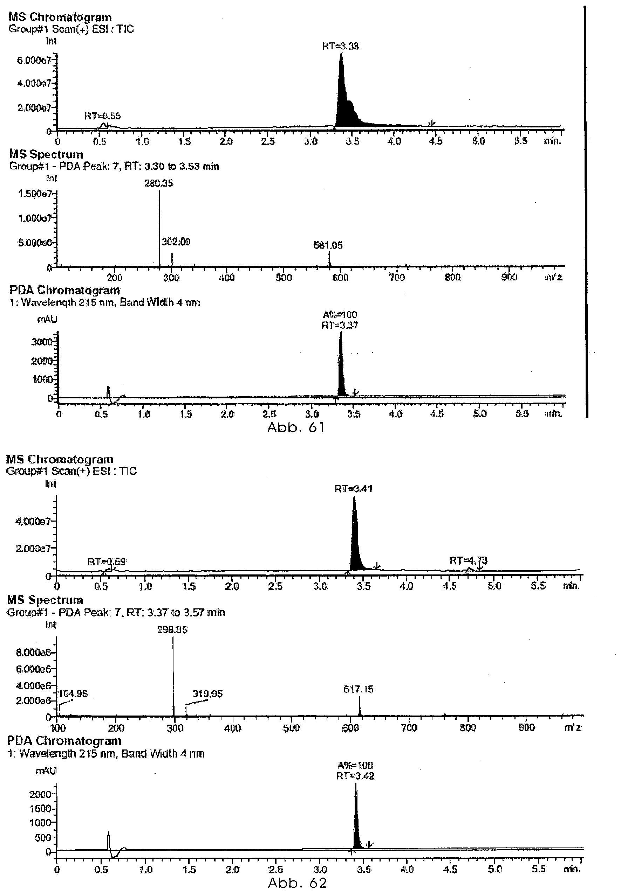

NaOMe (316 mg, 5.85 mmol) wurde zu einer Lösung von 2-Benzylmalosäuredimethylester (650 mg, 2.92 mmol) und Pyridin-2-carboxamidin (354 mg, 2.92 mmol) in MeOH (15 ml) gegeben. Die Reaktionsmischung wurde bei Raumtemperatur für 40 min. gelöst und dann bei 70°C für 1 h. Nach Abkühlen wurde die Reaktionsmischung im Vakuum eingeengt. Der Rohrückstand wurde durch Verfestigung aus EtOAc gereinigt, um die Titelverbindung zu ergeben (431 mg, 53 %).
MW: 279.29
HPLCMS (Methode A):[m/z]: 280
Abb. 61 zeigt das MS-Chromatogramm, das MS-Spektrum und das PDA-Chromatogramm der Verbindung von Beispiel 64.
IC50 [*µ*M]: >50.

### Beispiel 65

### 5-(2-Fluorbenzyl)-2-pyridin-2-yl-pyrimidin-4,6-diol

In ähnlicher Weise, unter Verwendung von Route 14, allgemeines Verfahren 46, ergaben NaOMe (111 mg, 2.06 mmol), 2-(2-Fluorbenzyl)-malonsäuredimethylester (496 mg, 2.06 mmol) und Pyridin-2-carboxamidin (250 mg, 2.06 mmol) die Titelverbindung (361 mg, 59 %).
MW: 297.28
HPLCMS (Methode A):[m/z]: 298
Abb. 62 zeigt das MS-Chromatogramm, das MS-Spektrum und das PDA-Chromatogramm der Verbindung von Beispiel 65.
IC50 [*µ*M]: >50.

### 5-(2-Fluorbenzyl)-2-(5-fluorpyridin-2-yl)-pyrimidin-4,6-diol

In ähnlicher Weise, unter Verwendung von Route 14, allgemeines Verfahren 46, ergaben NaOMe (153 mg, 2.83 mmol), 2-(2-Fluorbenzyl)-malonsäuredimethylester (680 mg, 2.83 mmol) und 5-Fluorpyridin-2-carboximidamid (394 mg, 2.83 mmol) die Titelverbindung (597 mg, 67 %).
MW: 315.27
HPLCMS (Methode B): [m/z]: 316

### Beispiel 66

### Allgemeines Verfahren 47:

### 5-Benzyl-4,6-dichlor-2-pyridin-2-yl-pyrimidin

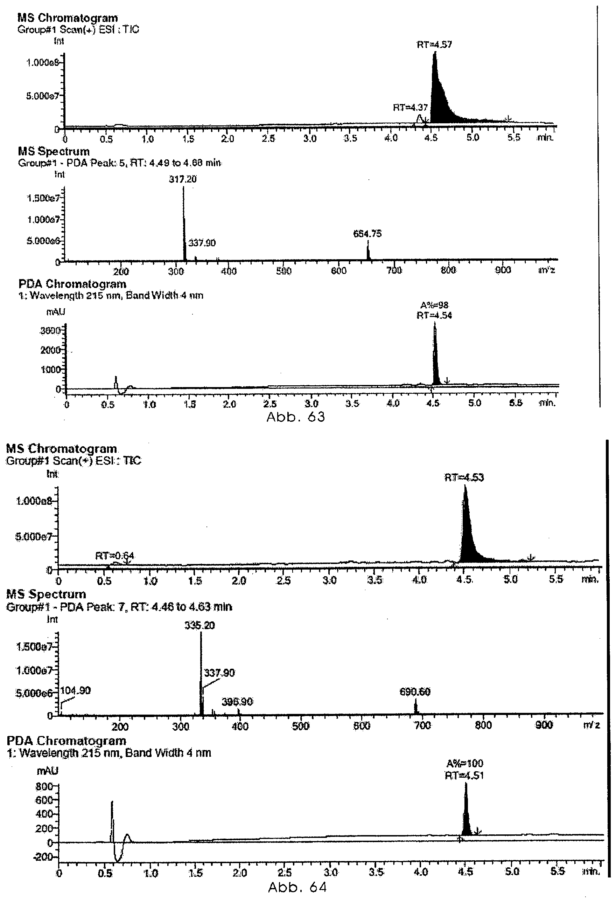

Eine Lösung von POCl₃ (316 *µ*l 3.4 mmol) in Toluol (3 ml) wurde tropfenweise zu einer Suspension von 5-Benzyl-2-(pyridin-2-yl)pyrimidin-4,6-diol (430 mg, 1.54 mmol) und TEA (215 µ*l*, 1.54 mmol) in Toluol (5 ml) bei 100°C gegeben. Die Reaktionsmischung wurde unter Rückfluss für 16 h erhitzt. Nach Abkühlen auf Raumtemperatur und dann auf 0°C, wurde Wasser (3 ml) tropfenweise hinzugegeben, und die Mischung wurde auf Raumtemperatur erwärmen gelassen. Der Versuch der Extraktion mit EtOAc scheiterte, daher wurde die Mischung im Vakuum eingeengt. Der Rückstand wurde basisch gemacht mit gesättigter wässriger NaHCO₃-Lösung und mit DCM (x 2) extrahiert, gefolgt von Chloroform (x 2). Die vereinigten organischen Phasen wurden getrocknet (MgSO₄) und im Vakuum eingeengt, um die Titelverbindung zu ergeben (327 mg, 67 %).
MW: 316.19
HPLCMS (Methode A):[m/z]: 317
Abb. 63 zeigt das MS-Chromatogramm, das MS-Spektrum und das PDA-Chromatogramm der Verbindung von Beispiel 66.
IC50 [*µ*M]: >50.

### Beispiel 67

### 4,6-Dichlor-5-(2-fluorbenzyl)-2-pyridin-2-yl-pyrimidin

In ähnlicher Weise, unter Verwendung von Route 14, allgemeines Verfahren 47, ergaben POCl₃ (69 *µ*l, 0.74 mmol), 5-(2-Fluorbenzyl)-2-pyridin-2-yl-pyrimidin-4,6-diol (100 mg, 0.34 mmol) und TEA (47 *µ*l, 0.34 mmol) die Titelverbindung (112 mg, 77 %).
MW: 334.18
HPLCMS (Methode A):[m/z]: 335
Abb. 64 zeigt das MS-Chromatogramm, das MS-Spektrum und das PDA-Chromatogramm der Verbindung von Beispiel 67.
IC50 [*µ*M] >50.

### Beispiel 68

### 4,6-Dichlor-5-(2-fluorbenzyl)-2-(5-fluorpyridin-2-yl)-pyrimidin

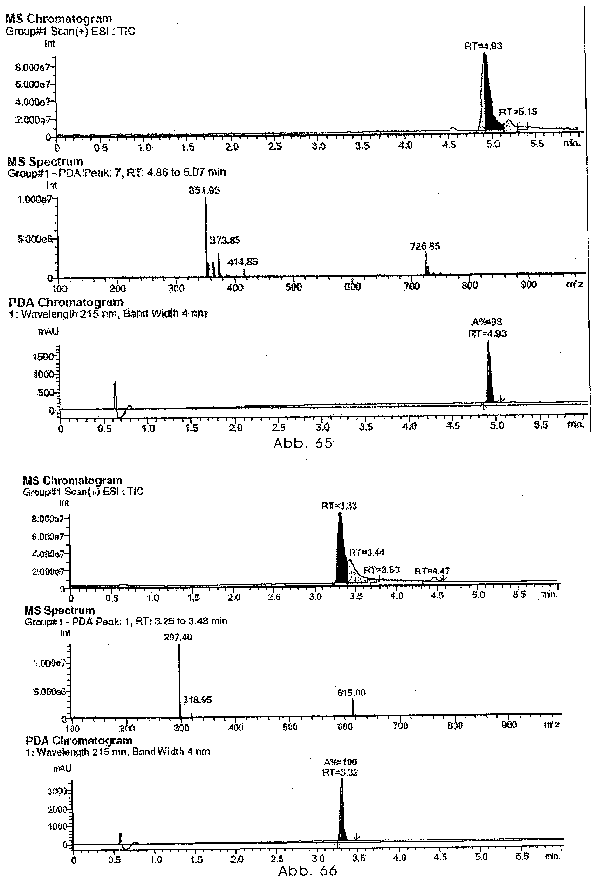

In ähnlicher Weise, unter Verwendung von Route 14, allgemeines Verfahren 47, ergaben POCl₃ (384 *µ*l, 4.12 mmol), 5-(2-Fluorbenzyl)-2-(5-fluorpyridin-2-yl)-pyrimidin-4,6-diol (590 mg, 1.87 mmol) und TEA (260 *µ*l, 1.87 mmol) die Titelverbindung (496 mg, 75 %).
MW: 352.17
HPLCMS (Methode A):[m/z]: 352
Abb. 65 zeigt das MS-Chromatogramm, das MS-Spektrum und das PDA-Chromatogramm der Verbindung von Beispiel 68.
IC50 [*µ*M]: >50.

### Beispiel 69

### Allgemeines Verfahren 48:

### 5-Benzyl-6-chlor-2-pyridin-2-yl-pyrimidin-4-ylamin

Eine Suspension von 5-Benzyl-4,6-dichlor-2-(pyridin-2-yl)pyrimidin (50 mg, 0.16 mmol) in NH₄OH (35 %ige Lösung in Wasser, 1 ml, 9.3 mmol) wurde in einer Mikrowellenröhre bei 100°C für 30 min in den Mikrowellen erwärmt. EtOH (1 ml) wurde hinzugegeben, und die Reaktionsmischung bei 100°C für weitere 30 min. in der Mikrowelle erhitzt. Der resultierende Feststoff wurde durch Filtration isoliert, gewaschen mit EtOH (1 ml) und unter Vakuum getrocknet, um die Titelverbindung zu ergeben (30 mg, 64 %).
MW: 296.75
HPLCMS (Methode A):[m/z]: 297
Abb. 66 zeigt das MS-Chromatogramm, das MS-Spektrum und das PDA-Chromatogramm der Verbindung von Beispiel 69.
IC50 [*µ*M]: >50.

### Beispiel 70

### 6-Chlor-5-(2-fluorbenzyl)-2-pyridin-2-yl-pyrimidin-4-ylamin

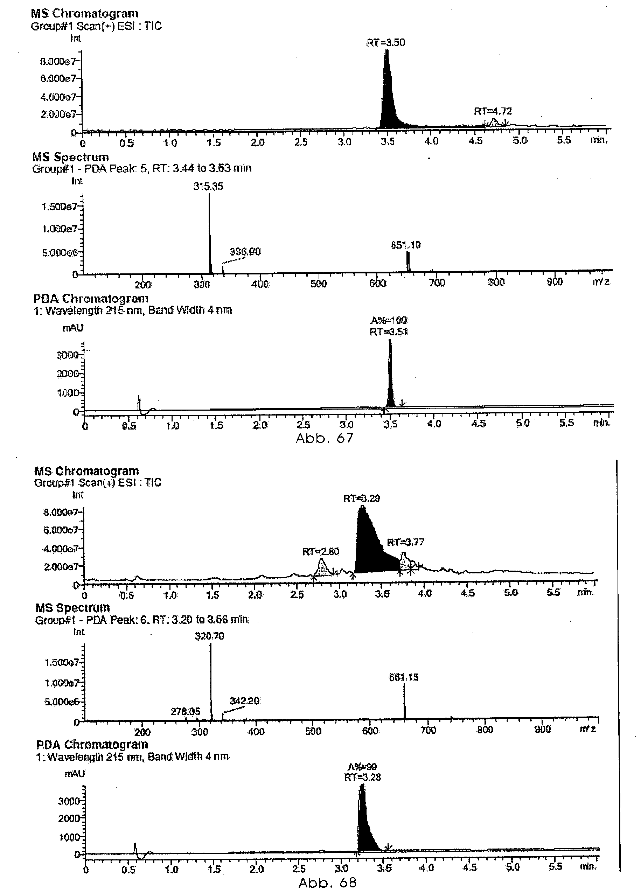

In ähnlicher Weise, unter Verwendung der Route 14, allgemeines Verfahren 48, ergaben 4,6-Dichlor-5-(2-fluorbenzyl)-2-pyridin-2-yl-pyrimidin (390 mg, 1.17 mmol) und NH₄OH (35 %ige Lösung in Wasser, 3.1 ml, 29.28 mmol) die Titelverbindung (334 mg, 91 %).
MW: 314.75
HPLCMS (Methode A):[m/z]: 315
Abb. 67 zeigt das MS-Chromatogramm, das MS-Spektrum und das PDA-Chromatogramm der Verbindung von Beispiel 70.
IC50 [*µ*M]: >50.

### 6-Chlor-5-(2-fluorbenzyl)-2-(5-fluorpyridin-2-yl)-pyrimidin-4-ylamin

In ähnlicher Weise, unter Verwendung der Route 14, allgemeines Verfahren 48, ergaben 4,6-Dichlor-5-(2-fluorbenzyl)-2-(5-fluorpyridin-2-yl)-pyrimidin (377 mg, 1.07 mmol) und NH₄OH (35 %ige Lösung in Wasser, 2.9 ml, 26.76 mmol) die Titelverbindung (307 mg, 86 %).
MW: 332.74
HPLCMS (Methode B):[m/z]: 333

### Beispiel 71

### Allgemeines Verfahren 49:

### 5-Benzyl-N-isopropyl-2-pyridin-2-yl-pyrimidin-4,6-diamin

Isopropylamin (87 *µ*l, 1.01 mmol) wurde zu einer Lösung von 5-Benzyl-6-chlor-2-(pyridin-2-yl)pyrimidin-4-amin (30 mg, 0.1 mmol) in n-BuOH (1 ml) in einer Mikrowellenröhre gegeben. Die Mischung wurde auf 193°C für 1 h in der Mikrowelle erwärmt. Isopropylamin (1.0 ml, 11.61 mmol) wurde hinzugegeben, und die Mischung wurde bei 193°C für weitere 150 min. in der Mikrowelle erwärmt. Nach Abkühlen wurde Wasser hinzugegeben, und der resultierende Niederschlag wurde durch Filtration isoliert, mit Et₂O gewaschen und im Vakuum getrocknet, um die Titelverbindung zu ergeben (29 mg, 90 %).
MW: 319.40
HPLCMS (Methode A):[m/z]: 320.70
Abb. 68 zeigt das MS-Chromatogramm, das MS-Spektrum und das PDA-Chromatogramm der Verbindung von Beispiel 71.
IC50 [*µ*M]: <50.

### Beispiel 72

### 5-(2-Fluorbenzyl)-N-isopropyl-2-pyridin-2-yl-pyrimidin-4,6-diamin

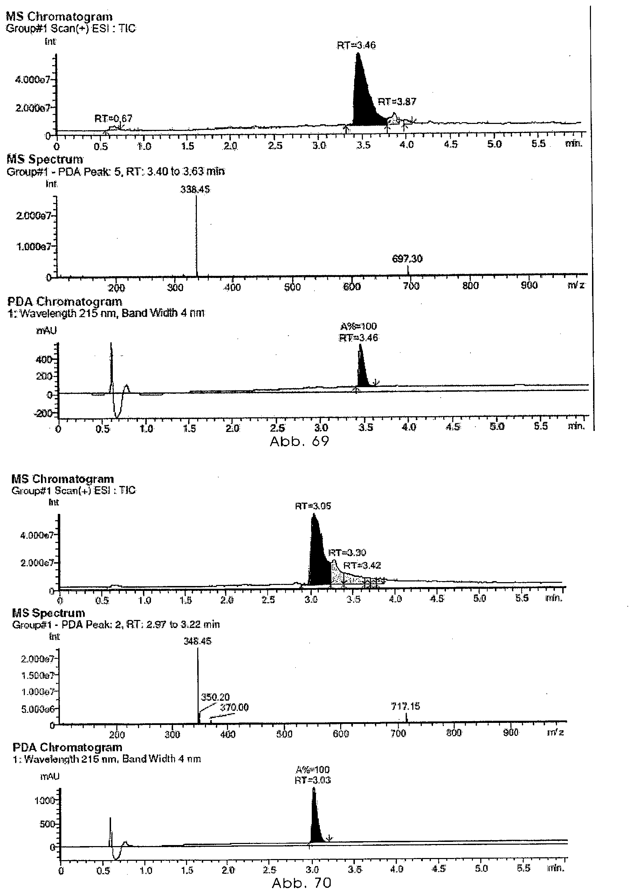

In ähnlicher, unter Verwendung von Route 14, allgemeines Verfahren 49, ergaben Isopropylamin (273 *µ*l, 3.18 mmol) und 6-Chlor-5-(2-fluorbenzyl)-2-pyridin-2-yl-pyrimidin-4-ylamin (100 mg, 0.32 mmol) die Titelverbindung (58 mg, 54 %).
MW: 337.40
HPLCMS (Methode A):[m/z]: 338
Abb. 69 zeigt das MS-Chromatogramm, das MS-Spektrum und das PDA-Chromatogramm der Verbindung von Beispiel 72.
IC50 [*µ*M]: <50.

### Beispiel 73

### 5-Benzyl-6-morpholin-4-yl-2-pyridin-2-yl-pyrimidin-4-ylamin

In ähnlicher Weise, unter Verwendung von Route 14, allgemeines Verfahren 49, ergaben 5-Benzyl-6-chlor-2-(pyridin-2-yl)pyrimidin-4-amin (30 mg, 0.1 mmol) und Morpholin (1 ml) die Titelverbindung (35 mg, 100 %).
MW: 347.41
HPLCMS (Methode A):[m/z]: 348
Abb. 70 zeigt das MS-Chromatogramm, das MS-Spektrum und das PDA-Chromatogramm der Verbindung von Beispiel 73.
IC50 [*µ*M]: <50.

### Beispiel 74

### 5-(2-Fluorbenzyl)-6-morpholin-4-yl-2-pyridin-2-yl-pyrimidin-4-ylamin

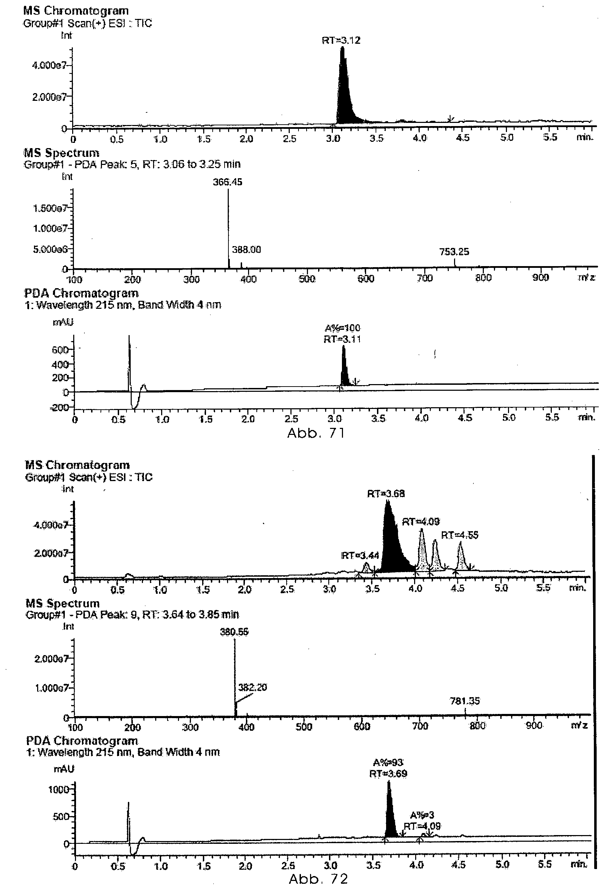

In ähnlicher Weise, unter Verwendung von Route 14, allgemeines Verfahren 49, ergaben 6-Chlor-5-(2-fluorbenzyl)-2-pyridin-2-yl-pyrimidin-4-ylamin (100 mg, 0.32 mmol) und Morpholin (1 ml) die Titelverbindung (111 mg, 96 %).
MW: 365.40
HPLCMS (Methode A):[m/z]: 366
Abb. 71 zeigt das MS-Chromatogramm, das MS-Spektrum und das PDA-Chromatogramm der Verbindung von Beispiel 74.
IC50 [*µ*M]: <50.

### Beispiel 75

### Allgemeines Verfahren 50:

### 5-(2-Fluorbenzyl)-N,N'-diisopropyl-2-pyridin-2-yl-pyrimidin-4,6-diamin

Isopropylamin (257 *µ*l, 2.99 mmol) wurde zu einer Lösung von 4,6-Dichlor-5-(2-fluorbenzyl)-2-pyridin-2-yl-pyrimidin (100 mg, 0.30 mmol) in n-BuOH (1 ml) in einer Mikrowellenröhre gegeben. Die Mischung wurde bei 200°C für 5 h in der Mikrowelle erwärmt. Die Reaktionsmischung wurde mit Wasser (1 ml) verdünnt und im Vakuum eingeengt. Der Rückstand wurde in EtOAc (2 ml) gelöst und mit gesättigter wässriger NaHCO₃-Lösung und Wasser gewaschen. Die organische Phase wurde getrocknet (Na₂SO₄) und im Vakuum eingeengt, um die Titelverbindung zu ergeben (82 mg, 72 %).
MW: 379.48
HPLCMS (Methode A):[m/z]: 380
Abb. 72 zeigt das MS-Chromatogramm, das MS-Spektrum und das PDA-Chromatogramm der Verbindung von Beispiel 75.
IC50 [*µ*M]: >50.

### Beispiel 76

### Allgemeines Verfahren 51 :

### 4-[5-Benzyl-6-(morpholin-4-yl)-2-(pyridin-2-yl)pyrimidin-4-yl]morpholin

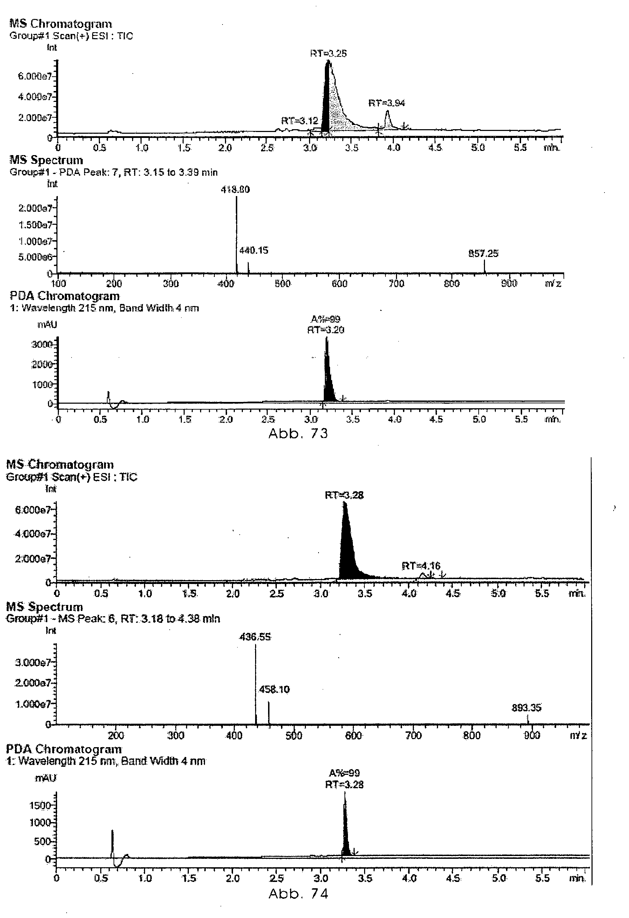

Eine Lösung von 5-Benzyl-4,6-dichlor-2-(pyridin-2-yl)pyrimidin (65 mg, 0.21 mmol) in Morpholin (1 ml) in einer Mikrowellenröhre wurde bei 200°C für 1 h in der Mikrowelle erwärmt. Die Lösung wurde mit Wasser (3 ml) verdünnt und extrahiert mit DCM (x 3). Die vereinigten organischen Phasen wurden getrocknet (MgSO₄) und im Vakuum eingeengt. Der Rückstand wurde in Et₂O (4 ml) gelöst und mit Wasser (x 2) und Kochsalzlösung gewaschen. Die organische Phase wurde getrocknet (MgSO₄) und im Vakuum eingeengt. Der Rohrückstand wurde gereinigt durch Trituration von Et₂O, um die Titelverbindung zu ergeben (25 mg, 29 %).
MW: 417.5
HPLCMS (Methode A):[m/z]: 418
Abb. 73 zeigt das MS-Chromatogramm, das MS-Spektrum und das PDA-Chromatogramm der Verbindung von Beispiel 76.
IC50 [*µ*M] >50.

### Beispiel 77

### 4-{5-[(2-Fluorphenyl)methyl]-6-(morpholin-4-yl)-2-(pyridin-2-yl)pyrimidin-4-yl}morpholin

In ähnlicher Weise, unter Verwendung von Route 14, allgemeines Verfahren 51, ergaben 4,6-Dichlor-5-(2-fluorbenzyl)-2-(5-fluorpyridin-2-yl)-pyrimidin (100 mg, 0.30 mmol) und Morpholin (1 ml) die Titelverbindung (60 mg, 46 %).
MW: 435.49
HPLCMS (Methode A):[m/z]: 436
Abb. 74 zeigt das MS-Chromatogramm, das MS-Spektrum und das PDA-Chromatogramm der Verbindung von Beispiel 77.
IC50 [*µ*M]: >50.

### ROUTE 15

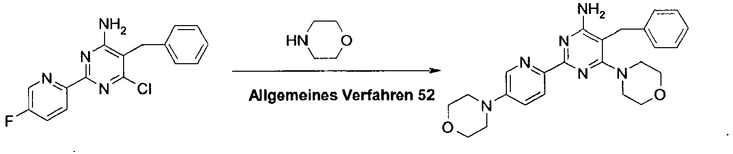

### Allgemeines Verfahren 52:

### Beispiel 78

### 5-(2-Fluorbenzyl)-6-morpholin-4-yl-2-(5-morpholin-4-yl-pyridin-2-yl)-pyrimidin-4-ylamin

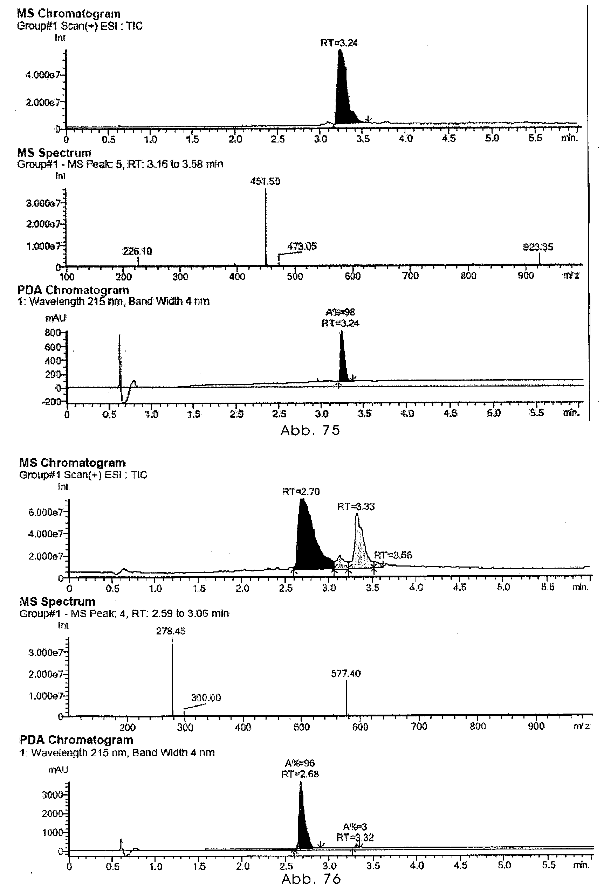

Eine Lösung von 6-Chlor-5-(2-fluorbenzyl)-2-(5-fluorpyridin-2-yl)-pyrimidin-4-ylamin (100 mg, 0.30 mmol) in Morpholin (1 ml) in einer Mikrowellenröhre wurde bei 200°C für 1 h in der Mikrowelle erwärmt. Et₂O (0.5 ml) wurde hinzugegeben und der resultierende Niederschlag wurde durch Filtration isoliert. Der Feststoff wurde gelöst in EtOAc (2 ml) und mit gesättigter wässriger NaHCO₃-Lösung und Wasser gewaschen. Die organische Phase wurde getrocknet (Na₂SO₄) und im Vakuum eingeengt. Der Rohrückstand wurde durch Trituration aus Et₂O gereinigt, um die Titelverbindung zu ergeben (100 mg, 74 %).
MW: 450.51.41
HPLCMS (Methode A):[m/z]: 451
Abb. 75 zeigt das MS-Chromatogramm, das MS-Spektrum und das PDA-Chromatogramm der Verbindung von Beispiel 78.
IC50 [*µ*M]: >50.

### Beispiel 79

### ROUTE 16

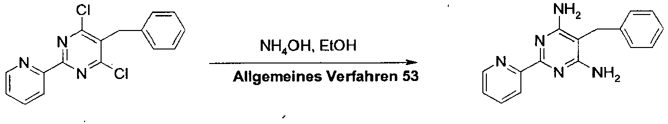

### Allgemeines Verfahren 53:

### 5-Benzyl-2-pyridin-2-yl-pyrimidin-4,6-diamin

Eine Suspension von 5-Benzyl-4,6-dichlor-2-(pyridin-2-yl)pyrimidin (50 mg, 0.16 mmol) in NH₄OH (1 ml, 9.3 mmol) und EtOH (1 ml) wurde in einer Mikrowellenröhre bei 130°C für 30 min. in der Mikrowelle erwärmt. Der Ansatz wurde erneut in Stufen bei 150°C für insgesamt 60.5 h erwärmt. Der Ansatz wurde mit Wasser verdünnt und der resultierende Feststoff durch Filtration isoliert, mit Et₂O gewaschen und im Vakuum getrocknet, um die Titelverbindung zu ergeben (32 mg, 73 %).
MW: 277.32
HPLCMS (Methode A):[m/z]: 278
Abb. 76 zeigt das MS-Chromatogramm, das MS-Spektrum und das PDA-Chromatogramm der Verbindung von Beispiel 79,
IC50 [*µ*M]: <50.

### ROUTE 17

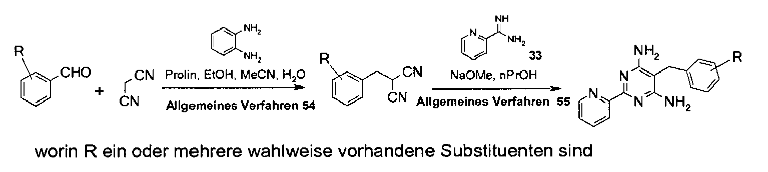

### Allgemeines Verfahren 54:

### 2-(2-Ethoxy-benzyl)-malononitril

Eine Lösung von 2-Ethoxybenzaldehyd (736 mg, 4.9 mmol) in EtOH (3 ml) wurde mit Malononitril (162 mg, 2.45 mmol) in EtOH (3 ml), Benzol-1,2-diamin (265 mg, 2.45 mol) in MeCN (3 ml) und schließlich Prolin (56 mg, 0.5 mmol) in Wasser (1 ml) behandelt, und die Lösung wurde bei Raumtemperatur für 1 h gelöst. Die Mischung wurde im Vakuum eingeengt und der Rückstand durch Säulenchromatographie mit DCM/Heptan (50:50 - 100) als Eluent gereinigt, um die Titelverbindung zu ergeben (407 mg, 42 %). Die Verbindung konnte nicht durch HPLCMS nachgewiesen werden, daher wurde die Struktur durch 1H-NMR bestätigt.

### 2-(2-Methoxy-5-methyl-benzyl)-malononitril

In ähnlicher Weise ergaben, unter Verwendung von Route 17, allgemeines Verfahren 54, 2-Methoxy-5-benzaldehyd (736 mg, 4.9 mmol), Malononitril (162 mg, 2.45 mmol), Benzol-1,2-diamin (265mg, 2.45 mol) und Prolin (56 mg, 0.5 mmol) die Titelverbindung (474 mg, 48 %) nach Reinigung durch Säulenchromatographie mit DCM/Heptan (25:75 - 100) als Eluent. Die Verbindung konnte nicht durch HPLCMS nachgewiesen werden, die Struktur wurde daher durch 1H-NMR bestätigt.

### 2-(2,4-Dimethoxy-benzyl)-malononitril

In ähnlicher Weise ergaben, unter Verwendung von Route 17, allgemeines Verfahren 54, 2,4-Dimethoxybenzaldehyd (814 mg, 4.9 mmol), Malononitril (162 mg, 2.45 mmol), Benzol-1,2-diamin (265 mg, 2.45 mol) und Prolin (56 mg, 0.5 mmol) die Titelverbindung (325 mg, 31 %) nach Reinigung durch Säulenchromatographie mit DCM/Heptan (25:75 - 100) als Eluent. Die Verbindung konnte nicht durch HPLCMS nachgewiesen werden. Die Struktur wurde daher durch 1H-NMR bestätigt.

### 2-(3-Methoxy-benzyl)-malononitril

In ähnlicher Weise ergaben, unter Verwendung von Route 17, allgemeines Verfahren 54, 3-Methoxybenzaldehyd (2.26 g, 16.6 mmol), Malononitril (0.55 g, 8.30 mmol), Benzol-1,2-diamin (0.90 g, 8.30 mol) und Prolin (0.19 g, 1.66 mmol) die Titelverbindung (481 mg, 31 %) nach Reinigung durch Säulenchromatographie mit DCM/Heptan (25:75 - 100). Die Verbindung konnte nicht durch HPLCMS nachgewiesen werden. Die Struktur wurde daher durch 1H-NMR bestätigt.

### 2-(2-Methyl-benzyl)-malononitril

In ähnlicher Weise ergaben, unter Verwendung von Route 17, allgemeines Verfahren 54, 2-Methylbenzaldehyd (1.99 g, 16.6 mmol), Malononitril (0.55 g, 8.30 mmol), Benzol-1,2-diamin (0.90 g, 8.30 mol) und Prolin (0.19 g, 1.66 mmol) die Titelverbindung (670 mg, 47 %) nach Reinigung durch Säulenchromatographie mit DCM/Heptan (25:75 - 100) als Eluent. Die Verbindung konnte nicht durch HPLCMS nachgewiesen werden. Die Struktur wurde daher durch 1H-NMR bestätigt.

### 2-(3-Methyl-benzyl)-malononitril

In ähnlicher Weise ergaben, unter Verwendung von Route 17, algemeines Verfahren 54, 3-Methylbenzaldehyd (1.99 g, 16.6 mmol), Malononitril (0.55 g, 8.30 mmol), Benzol-1,2-diamin (0.90 g, 8.30 mol) und Prolin (0.19 g, 1.66 mmol) die Titelverbindung (862 mg, 61 %) nach Reinigung durch Säulenchromatographie mit DCM/Heptan (25:75 - 100) als Eluent. Die Verbindung konnte nicht durch HPLCMS nachgewiesen werden. Die Struktur wurde daher durch 1H-NMR bestätigt.

### 2-(3-Fluorbenzyl)-malononitril

In ähnlicher Weise ergaben, unter Verwendung von Route 17, allgemeines Verfahren 54, 3-Fluorbenzaldehyd (2.06 g, 16.6 mmol), Malononitril (0.55 g, 8.30 mmol), Benzol-1,2-diamin (0.90 g, 8.30 mol) und Prolin (0.19 g, 1.66 mmol) die Titelverbindung (410 mg, 63 %) nach Reinigung durch Säulenchromatographie mit DCM/Heptan (25:75 - 100) als Eluent. Die Verbindung konnte nicht durch HPLCMS nachgewiesen werden. Die Struktur wurde daher durch 1H-NMR bestätigt.

### 2-(4-Fluorbenzyl)-malononitril

In ähnlicher Weise ergaben, unter Verwendung von Route 17, allgemeines Verfahren 54, 4-Fluorbenzaldehyd (2.06 g, 16.6 mmol), Malononitril (0.55 g, 8.30 mmol), Benzol-1,2-diamin (0.90 g, 8.30 mol) und Prolin (0.19 g, 1.66 mmol) die Titelverbindung (1.34 g, 92 %) nach Reinigung durch Säulenchromatographie mit DCM/Heptan (25:75 - 100) als Eluent. Die Verbindung konnte nicht durch HPLCMS nachgewiesen werden. Die Struktur wurde daher durch 1H-NMR bestätigt.

### 2-(3-Chlorbenzyl)-malononitril

In ähnlicher Weise ergaben, unter Verwendung von Route 17, allgemeines Verfahren 54, 3-Chlorbenzaldehyd (2.33 g, 16.6 mmol), Malononitril (0.55 g, 8.30 mmol), Benzol-1,2-diamin (0.90 g, 8.30 mol) und Prolin (0.19 g, 1.66 mmol) die Titelverbindung (784 mg, 50 %) nach Reinigung durch Säulenchromatographie mit DCM/Heptan (25:75 - 100) als Eluent. Die Verbindung konnte nicht durch HPLCMS nachgewiesen werden. Die Struktur wurde daher durch 1H-NMR bestätigt.

### 2-(2,5-Difluorbenzyl)-malononitril

In ähnlicher Weise ergaben, unter Verwendung von Route 17, allgemeines Verfahren 54, 2,5-Difluor-benzaldehyd (2.36 g, 16.6 mmol), Malononitril (0.55 g, 8.30 mmol), Benzol-1,2-diamin (0.90 g, 8.30 mol) und Prolin (0.19 g, 1.66 mmol) die Titelverbindung (650 mg, 41 %) nach Reinigung durch Säulenchromatographie mit DCM/Heptan (25:75 - 100) als Eluent. Die Verbindung konnte nicht durch HPLCMS nachgewiesen werden. Die Struktur wurde daher durch 1H-NMR bestätigt.

### 2-(2-Fluor-4-methoxy-benzyl)-malononitril

In ähnlicher Weise ergaben, unter Verwendung von Route 17, allgemeines Verfahren 54, 2-Fluor-4-methoxybenzaldehyd (2.36 g, 16.6 mmol), Malononitril (0.55 g, 8.30 mmol), Benzol-1,2-diamin (0.90 g, 8,30 mol) und Prolin (0.19 g, 1.66 mmol) die Titelverbindung (470 mg, 20 %) nach Reinigung durch Säulenchromatographie mit DCM/Heptan (25:75 - 100) als Eluent. Die Verbindung konnte nicht durch HPLCMS nachgewiesen werden. Die Struktur wurde daher durch 1H-NMR bestätigt.

### Beispiel 80

### Allgemeines Verfahren 55:

### 5-(2-Ethoxy-benzyl)-2-pyridin-2-yl-pyrimidin-4,6-diamin

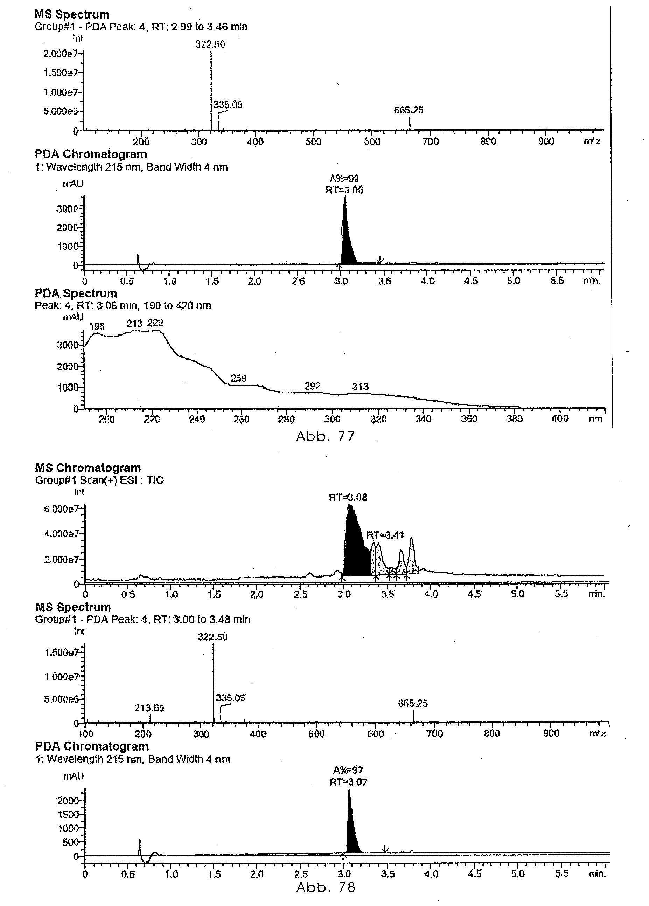

NaOMe (89 mg, 1.65 mmol) wurde zu einer Lösung von 2-(2-Ethoxybenzyl)-malononitril 110 (174 mg, 0.87 mmol) und Pyridin-2-carboximidamid (100 mg, 0.83 mmol) in n-PrOH (2 ml) in einer Mikrowellenröhre unter N₂ gegeben, und die Mischung wurde bei 150°C für 1 h in der Mikrowelle erwärmt. Die Roh-Reaktionsmischung wurde mit Wasser (8 ml) verdünnt. Die trübe Lösung wurde abdekantiert, und das zurückbleibende Gummi wurde mit Et₂O und MeCN (1:1, 2 ml) trituriert, um die Titelverbindung zu ergeben (26 mg, 10%).
MW: 321.38
HPLCMS (Methode A):[m/z]: 322
Abb. 77 zeigt das MS-Chromatogramm, das MS-Spektrum und das PDA-Chromatogramm der Verbindung von Beispiel 80.
IC50 [*µ*M]: >50.

### Beispiel 81

### 5-(2-Methoxy-5-methyl-benzyl)-2-pyridin-2-yl-pyrimidin-4,6-diamin

In ähnlicher Weise ergaben, unter Verwendung von Route 17, allgemeines Verfahren 55, Pyridin-2-carboximidamid (100 mg, 0.83 mmol), 2-(2-Methoxy-5-methyl-benzyl)-malononitril (174 mg, 0.87 mmol) und NaOMe (89 mg, 1.65 mmol) die Titelverbindung (58 mg, 22 %) nach Reinigung durch Trituration aus EtOH,
MW: 321.38
HPLCMS (Methode A):[m/z]: 322
Abb. 78 zeigt das MS-Chromatogramm, das MS-Spektrum und das PDA-Chromatogramm der Verbindung von Beispiel 81.
IC50 [*µ*M]: >50.

### Beispiel 82

### 5-(2,4-Dimethoxy-benzyl)-2-pyridin-2-yl-pyrimidin-4,6-diamin

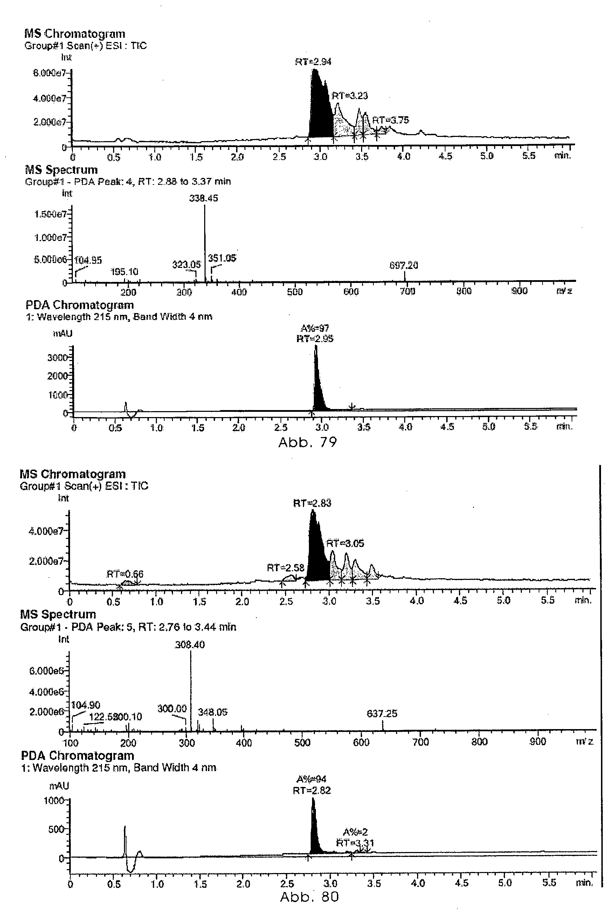

In ähnlicher Weise ergaben, unter Verwendung von Route 17, allgemeines Verfahren 55, Pyridin-2-carboximidamid (100 mg, 0.83 mmol), 2-(2,4-Dimethoxy-benzyl)-malononitril (188 mg, 0.87 mmol) und NaOMe (89 mg, 1.65 mmol) die Titelverbindung (11 mg, 4 %) nach Reinigung durch Trituration aus MeCN/Et₂O.
MW: 337.38
HPLCMS (Methode A):[m/z]: 338
Abb. 79 zeigt das MS-Chromatogramm, das MS-Spektrum und das PDA-Chromatogramm der Verbindung von Beispiel 82.
IC50 [*µ*M]: <50.

### Beispiel 83

### 5-(3-Methoxy-benzyl)-2-pyridin-2-yl-pyrimidin-4,6-diamin

In ähnlicher Weise ergaben, unter Verwendung von Route 17, allgemeines Verfahren 53, Pyridin-2-carboximidamid 33 (100 mg, 0.83 mmol), 2-(3-Methoxy-benzyl)-malononitril (162 mg, 0.87 mmol) und NaOMe (89 mg, 1.65 mmol) die Titelverbindung (15 mg, 6 %) nach Reinigung durch Trituration aus MeCN/Et₂O.
MW: 307.35
HPLCMS (Methode A):[m/z]: 308
Abb. 80 zeigt das MS-Chromatogramm, das MS-Spektrum und das PDA-Chromatogramm der Verbindung von Beispiel 83.
IC50 [*µ*M]: <50.

### Beispiel 84

### 5-(2-Methyl-benzyl)-2-pyridin-2-yl-pyrimidin-4,6-diamin

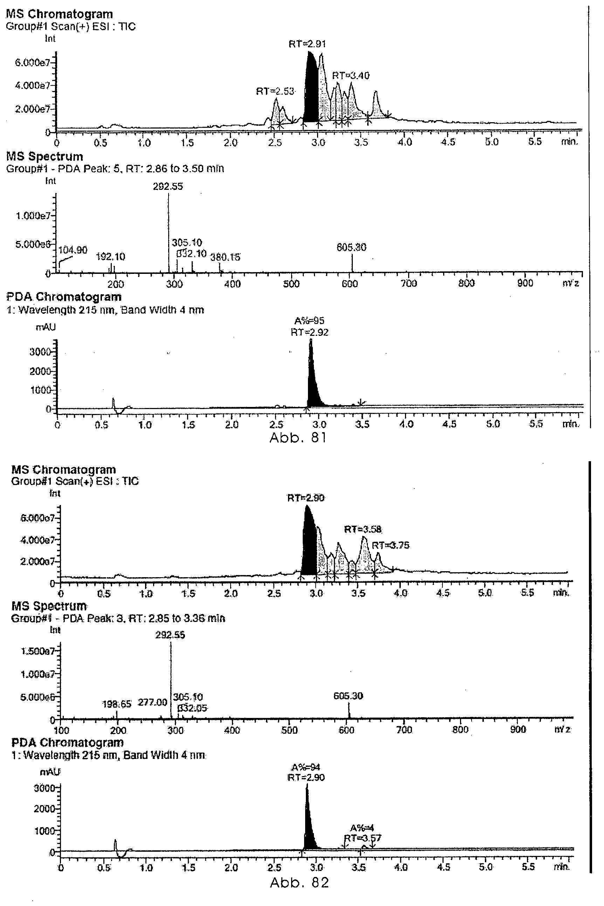

In ähnlicher Weise ergaben, unter Verwendung von Route 17, allgemeines Verfahren 55, Pyridin-2-carboximidamid (100 mg, 0.83 mmol), 2-(2-Methyl-benzyl)-malononitril (148 mg, 0.87 mmol) und NaOMe (89 mg, 1.65 mmol) die Titelverbindung (8 mg, 3 %) nach Reinigung durch Trituration aus MeCN/Et₂O.
MW: 291.35
HPLCMS (Methode A):[m/z]: 292
Abb. 81 zeigt das MS-Chromatogramm, das MS-Spektrum und das PDA-Chromatogramm der Verbindung von Beispiel 84.
IC50 [*µ*M]: <50.

### Beispiel 85

### 5-(3-Methyl-benzyl)-2-pyridin-2-yl-pyrimidin-4,6-diamin

In ähnlicher Weise ergaben, unter Verwendung von Route 17, allgemeines Verfahren 55, Pyridin-2-carboxamidin (100 mg, 0.83 mmol), 2-(3-Methyl-benzyl)-malononitril (155 mg, 0.91 mmol) und NaOMe (89 mg, 1.65 mmol) die Titelverbindung (40 mg, 15 %).
MW: 291.35
HPLCMS (Methode A): [m/z]: 292
Abb. 82 zeigt das MS-Chromatogramm, das MS-Spektrum und das PDA-Chromatogramm der Verbindung von Beispiel 85.
IC50 [*µ*M]: <50,

### Beispiel 86

### 5-(3-Fluorbenzyl)-2-pyridin-2-yl-pyrimidin-4,6-diamin

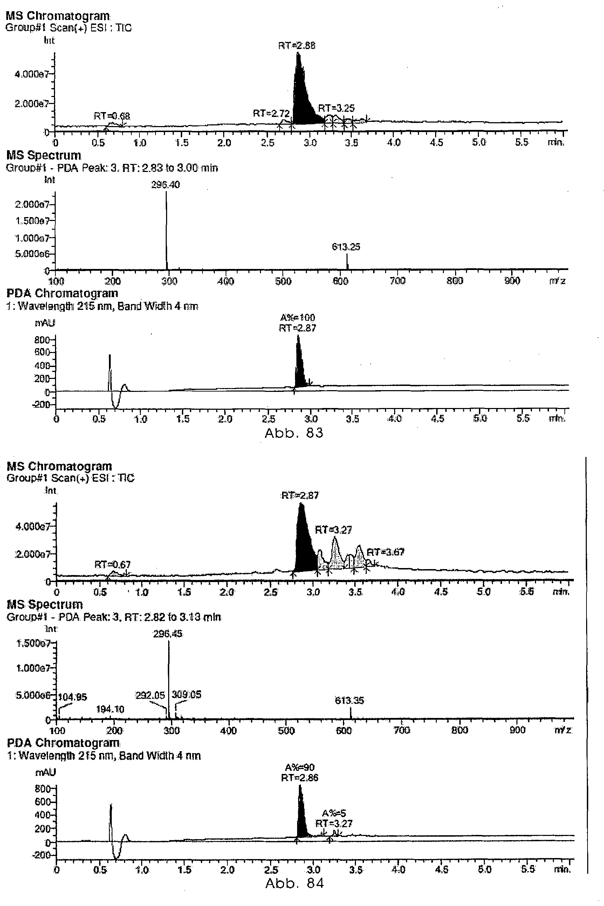

In ähnlicher Weise ergaben, unter Verwendung von Route 17, allgemeines Verfahren 55, Pyridin-2-carboxamidin (100 mg, 0.83 mmol), 2-(3-Fluorbenzyl)-malononitril (158 mg, 0.91 mmol) und NaOMe (89 mg, 1.65 mmol) die Titelverbindung (49 mg, 18 %) nach Reinigung durch Trituration aus MeCN/Et₂O.
MW: 295.31
HPLCMS (Methode A): [m/z]: 296
Abb. 83 zeigt das MS-Chromatogramm, das MS-Spektrum und das PDA-Chromatogramm der Verbindung von Beispiel 86.
IC50 [*µ*M]: <50.

### Beispiel 87

### 5-(4-Fluorbenzyl)-2-pyridin-2-yl-pyrimidin-4,6-diamin

In ähnlicher Weise ergaben, unter Verwendung von Route 17, allgemeines Verfahren 55, Pyridin-2-carboxamidin (100 mg, 0.83 mmol), 2-(4-Fluorbenzyl)-malononitril (158 mg, 0.91 mmol) und NaOMe (89 mg, 1.65 mmol) die Titelverbindung (23 mg, 9 %) nach Reinigung durch Trituration aus MeCN/Et₂O.
MW: 295.31
HPLCMS (Methode A): [m/z]: 296
Abb. 84 zeigt das MS-Chromatogramm, das MS-Spektrum und das PDA-Chromatogramm der Verbindung von Beispiel 87.
IC50 [*µ*M] <50.

### Beispiel 88

### 5-(3-Chlorbenzyl)-2-pyridin-2-yl-pyrimidin-4,6-diamin

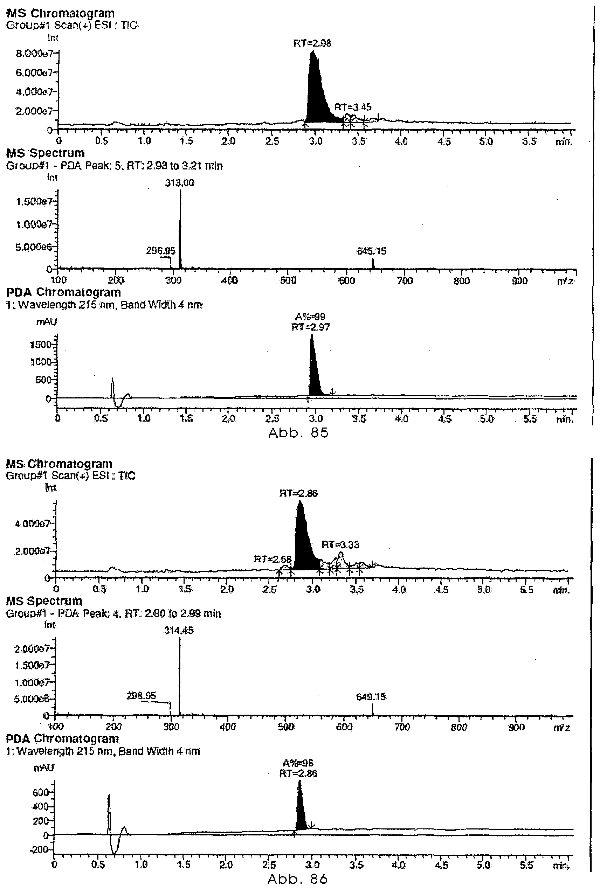

In ähnlicher Weise ergaben, unter Verwendung von Route 17, allgemeines Verfahren 55, Pyridin-2-carboxamidin (100 mg, 0.83 mmol), 2-(3-Chlorbenzyl)-malononitril (173 mg, 0.91 mmol) und NaOMe (89 mg, 1.65 mmol) die Titelverbindung (23 mg, 9 %) nach Reinigung durch Trituration aus MeCN/Et₂O.
MW: 311.77
HPLCMS (Methode A): [m/z]: 313
Abb. 85 zeigt das MS-Chromatogramm, das MS-Spektrum und das PDA-Chromatogramm der Verbindung von Beispiel 88.
IC50 [*µ*M]: <50.

### Beispiel 89

### 5-(2,5-Difluorbenzyl)-2-pyridin-2-yl-pyrimidin-4,6-diamin

In ähnlicher Weise ergaben, unter Verwendung von Route 17, allgemeines Verfahren 55, Pyridin-2-carboxamidin (100 mg, 0.83 mmol), 2-(2,5-Difluorbenzyl)-malononitril (175 mg, 0.91 mmol) und NaOMe (89 mg, 1.65 mmol) die Titelverbindung (21 mg, 7 %) nach Reinigung durch Trituration aus MeCN/Et₂O.
MW: 313.30
HPLCMS (Methode A): [m/z]: 314
Abb. 86 zeigt das MS-Chromatogramm, das MS-Spektrum und das PDA-Chromatogramm der Verbindung von Beispiel 89.
IC50 [*µ*M]: <50.

### Beispiel 90

### 5-(2-Fluor-4-methoxy-benzyl)-2-pyridin-2-yl-pyrimidin-4,6-diamin

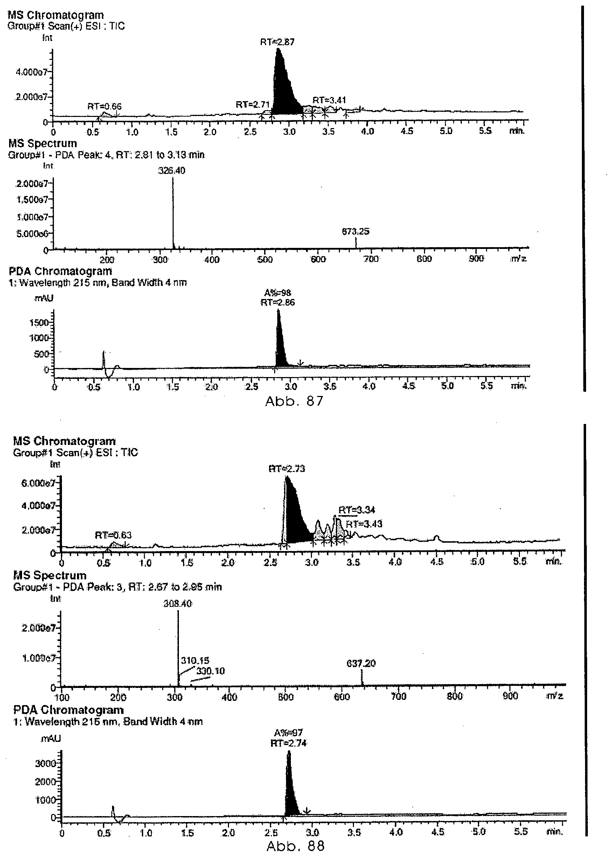

In ähnlicher Weise ergaben, unter Verwendung von Route 17, allgemeines Verfahren 55, Pyridin-2-carboxamidin (100 mg, 0.83 mmol), 2-(2-Fluor-4-methoxy-benzyl)-malononitril (186 mg, 0.91 mmol) und NaOMe (89 mg, 1.65 mmol) die Titelverbindung (14 mg, 5 %) nach Reinigung durch Trituration aus MeCN/Et₂O.
MW: 325.34
HPLCMS (Methode A): [m/z]: 326
Abb. 87 zeigt das MS-Chromatogramm, das MS-Spektrum und das PDA-Chromatogramm der Verbindung von Beispiel 90.
IC50 [*µ*M]: <50.

### Beispiel 91

### 5-(4-Methoxy-benzyl)-2-pyridin-2-yl-pyrimidin-4,6-diamin

In ähnlicher Weise ergaben, unter Verwendung von Route 17, allgemeines Verfahren 53, Pyridin-2-carboxamidin (100 mg, 0.83 mmol), 2-(4-Methoxy-benzyl)-malononitril (186 mg, 0.91 mmol) und NaOMe (89 mg, 1.65 mmol) in MeOH (2 ml) die Titelverbindung (72 mg, 28 %) nach Reinigung durch Trituration aus MeCN/Et₂O.
MW: 307.35
HPLCMS (Methode A):[m/z]: 308
Abb. 88 zeigt das MS-Chromatogramm, das MS-Spektrum und das PDA-Chromatogramm der Verbindung von Beispiel 91.
IC50 [*µ*M]: >50.

### Beispiel 92

### ROUTE 18

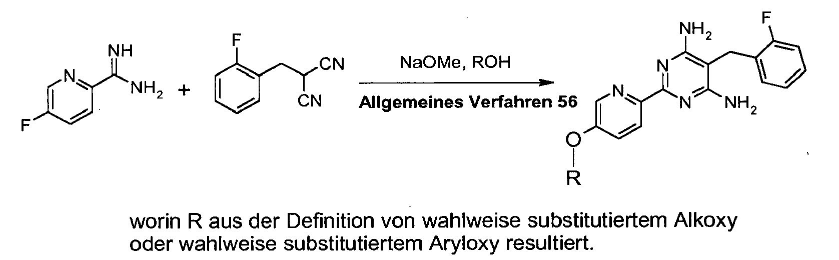

### Allgemeines Verfahren 56:

### 5-(2-Fluorbenzyl)-2-(5-methoxy-pyridin-2-yl)-pyrimidin-4,6-diamin

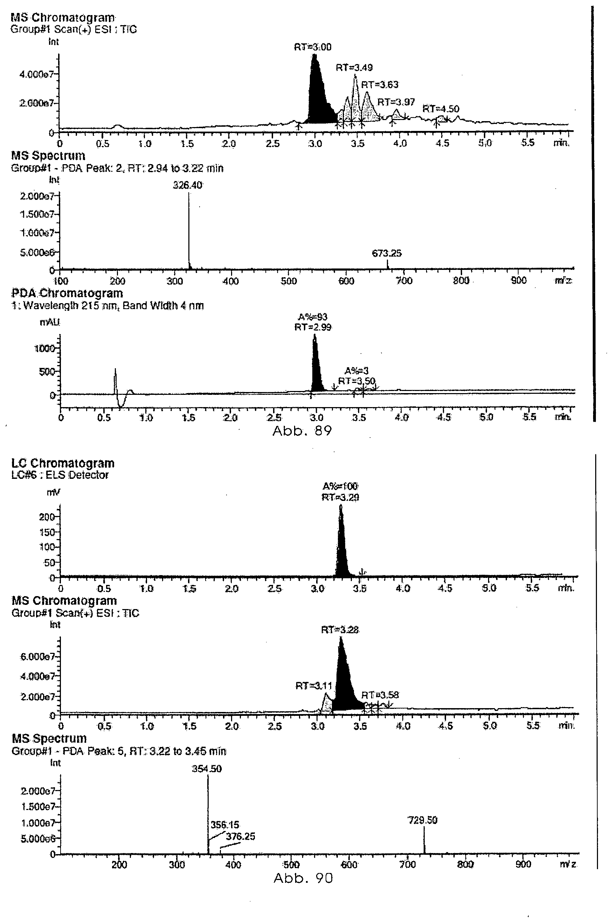

NaOMe (89 mg, 1.65 mmol) wurde zu einer Lösung von 2-(2-Fluor-benzyl)-malononitril (138 mg, 0.72 mmol) und 5-Fluor-pyridin-2-carboxamidin (100 mg, 0.72 mmol) in MeOH (2 ml) in einer Mikrowellenröhre gegeben unter N₂ und die Mischung wurde bei 150°C für 1 h in der Mikrowelle erwärmt. Die Rohreaktionsmischung wurde mit Wasser (8 ml) verdünnt. Die trübe Lösung wurde dekantiert, und das zurückbleibende Gummi wurde mit Et₂O und MeCN (1:1,2 ml) trituriert, um die Titelverbindung zu ergeben (33 mg, 14 %).
MW: 325.35
HPLCMS (Methode A): [m/z]: 326
Abb. 89 zeigt das MS-Chromatogramm, das MS-Spektrum und das PDA-Chromatogramm der Verbindung von Beispiel 92.
IC50 [*µ*M]: <50.

### Beispiel 93

### 5-(2-Fluorbenzyl)-2-(5-propoxy-pyridin-2-yl)-pyrimidin-4,6-diamin

In ähnlicher Weise ergaben, unter Verwendung von Route 17, allgemeines Verfahren 56, 5-Fluor-pyridin-2-carboxamidin (100 mg, 0.72 mmol), 2-(2-Fluor-benzyl)-malononitril (138 mg, 0.72 mmol) und NaOMe (89 mg, 1.65 mmol) in n-PrOH (2 ml) die Titelverbindung (9 mg, 4 %) nach Reinigung durch präparative HPLC (basische Bedingungen).
MW: 353.40
HPLCMS (Methode A): [m/z]: 355
Abb. 90 zeigt das LC-Chromatogramm, das MS-Spektrum und das MS-Chromatogramm der Verbindung von Beispiel 93.
IC50 [*µ*M]: <50.

### ROUTE 19

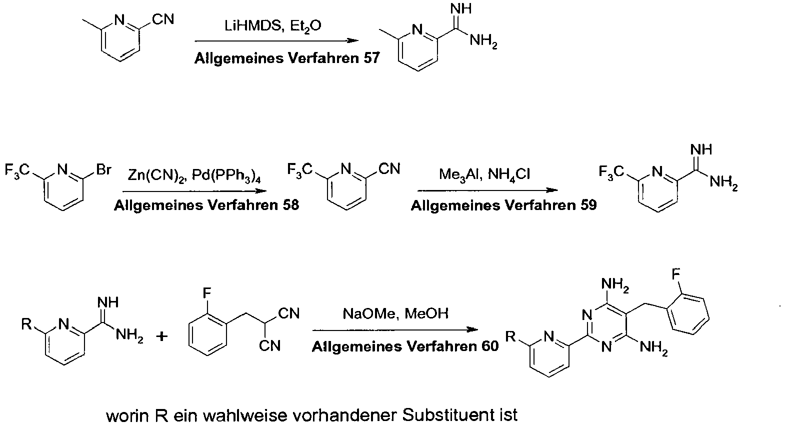

### Allgemeines Verfahren 57:

### 6-Methyl-pyridin-2-carboxamidin

Lithiumhexamethyldisilazid (1 M Lösung in THF, 36.0 ml, 36.0 mmol) wurde zu einer Lösung von 6-Methyl-2-pyridincarbonitril (2.0 g, 16.9 mmol) in Et₂O (30 ml) bei 0°C gegeben. Der Ansatz wurde auf Raumtemperatur über Nacht erwärmen gelassen. Der Ansatz wurde auf 0°C abgekühlt und 3 M HCl (54 ml) wurde hinzugegeben, und der Ansatz wurde für 30 min. gerührt. Wasser (135 ml) wurde hinzugegeben und die organische Phase abgetrennt und verworfen. Die wässrige Phase wurde mit gesättigter wässriger NaOH auf pH 14 basisch gemacht und mit DCM (x 3) extrahiert. Die vereinigten organischen Extrakte wurden getrocknet (Na₂SO₄) und im Vakuum eingeengt, um die Titelverbindung zu ergeben (1.55 g, 66 %). Die Verbindung konnte nicht durch HPLCMS nachgewiesen werden. Die Struktur wurde daher durch 1H-NMR bestätigt.

### Allgemeines Verfahren 58:

### 6-Trifluormethyl-pyridin-2-carbonitril

Tetrakis(triphenylphosphin)palladium(0) (3.20 g, 2.77 mmol) wurde zu einer Lösung von 2-Brom-6-trifluormethylpyridin (3.13 g, 13.85 mmol) und Zn(CN)₂ (1.63 g, 13.85 mmol) in DMF unter N₂ gegeben. Die Reaktionsmischung wurde bei 85°C über Nacht erhitzt. Nach Abkühlen wurde die Mischung verdünnt mit Wasser (200 ml) und mit EtOAc (x 2) extrahiert. Die vereinigten organischen Phasen wurden getrocknet (Na₂SO₄) und im Vakuum eingeengt. Der Rohrückstand wurde durch Säulenchromatographie mit EtOAc/Heptan (4:1 - 1:1) als Eluent gereinigt, um die Titelverbindung zu ergeben (1.34 g, 56 %). Die Verbindung konnte nicht durch HPLCMS nachgewiesen werden. Die Struktur wurde daher durch 1H-NMR bestätigt.

### Allgemeines Verfahren 59:

### 6-Trifluormethyl-pyridin-2-carboxamidin

Trimethylaluminium (2.10 g, 29.11 mmol) wurde tropfenweise zu einer stark gerührten Lösung von NH₄Cl (1.56 g, 29.11 mmol) in trockenem Toluol (15 ml) bei 0°C gegeben. Die Mischung wurde auf Raumtemperatur erwärmt und für 15 min. gerührt. Eine Lösung von 6-Trifluormethyl-pyridin-2-carbonitril (1.67 g, 9.703 mmol) in Toluol (15 ml) wurde tropfenweise hinzugegeben. Die Reaktionsmischung wurde bei 80°C für 18 h erwärmt. Nach Abkühlen wurde die Mischung in eine stark gerührte und gekühlte (0°C) Aufschlämmung von Siliziumdioxid (20.0 g) in Chloroform (150 ml) überführt und für 10 min. gerührt. Die Mischung wurde filtriert und der Filterkuchen mit MeOH (x 3) gewaschen. Das Filtrat wurde im Vakuum eingeengt. Der Rückstand wurde in 1 M HCl (150 ml) und Et₂O (70 ml) gelöst. Die organische Phase wurde abgetrennt und verworfen. Die wässrige Phase wurde mit gesättigter wässriger NaOH basisch gemacht und extrahiert mit Chloroform (x 2). Die vereinigten organischen Phasen wurden getrocknet (Na₂SO₄) und im Vakuum eingeengt, um die Titelverbindung zu ergeben (980 mg, 53 %). Die Verbindung konnte nicht durch HPLCMS nachgewiesen werden. Die Struktur wurde daher durch NMR bestätigt.

### Beispiel 94

### Allgemeines Verfahren 60:

### 5-(2-Fluorbenzyl)-2-(6-methyl-pyridin-2-yl)-pyrimidin-4,6-diamin

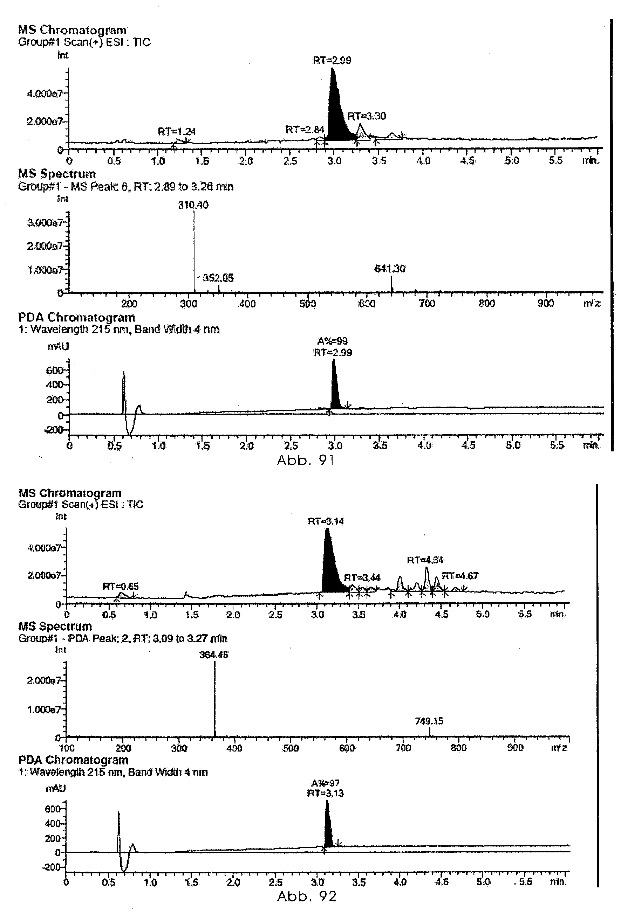

NaOMe (200 mg, 3.70 mmol) wurde zu einer Lösung von 2-(2-Fluorbenzyl)-malononitril (387 mg, 2.22 mmol) und 6-Methyl-pyridin-2-carboximidamid (200 mg, 1.48 mmol) in MeOH (4 ml) in einer Mikrowellenröhre unter N₂ gegeben, und die Mischung wurde bei 150°C für 1 h in der Mikrowelle erwärmt. Nach Abkühlen wurde die Mischung mit Wasser (8 ml) verdünnt und mit Ultraschall beschallt, der resultierende Niederschlag wurde durch Filtration entfernt. Das Filtrat wurde im Vakuum eingeengt, der Rückstand aus EtOAc trituriert und getrocknet unter Vakuum, um die Titelverbindung zu ergeben (24 mg, 5 %).
MW: 309.34
HPLCMS (Methode A): [m/z]: 310
Abb. 91 zeigt das MS-Chromatogramm, das MS-Spektrum und das PDA-Chromatogramm der Verbindung von Beispiel 94.
IC50 [*µ*M]: >50.

### Beispiel 95

### 5-(2-Fluorbenzyl)-2-(6-trifluormethyl-pyridin-2-yl)-pyrimidin-4,6-diamin

In ähnlicher Weise ergaben unter Verwendung von Route 19, allgemeines Verfahren 60, 2-(2-Fluorbenzyl)-malononitril (101 mg, 0.58 mmol), 6-Trifluormethyl-pyridin-2-carboximidamid (100 mg, 0.53 mmol) und NaOMe (57 mg, 1.06 mmol) in MeOH (2 ml) die Titelverbindung (31 mg, 16 %) nach Reinigung durch Trituration aus Et₂O/MeCN.
MW: 363.31
HPLCMS (Methode A): [m/z]: 364
Abb. 92 zeigt das MS-Chromatogramm, das MS-Spektrum und das PDA-Chromatogramm der Verbindung von Beispiel 95.
IC50 [*µ*M]: >50.

### ROUTE 20

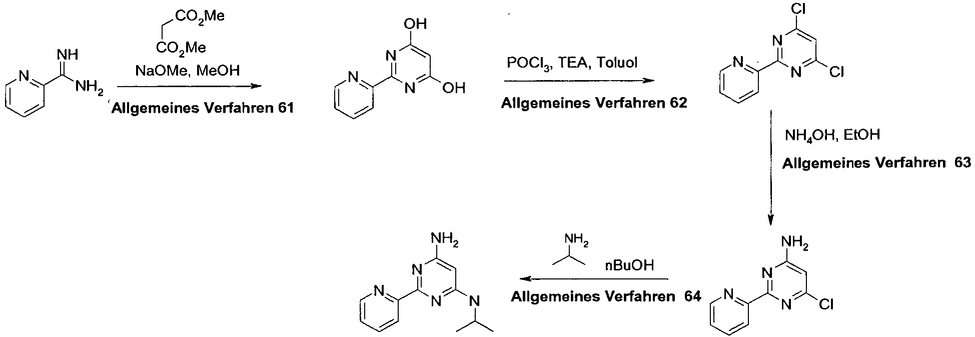

### Beispiel 96

### Allgemeines Verfahren 61:

### 2-Pyridin-2-yl-pyrimidin-4,6-diol

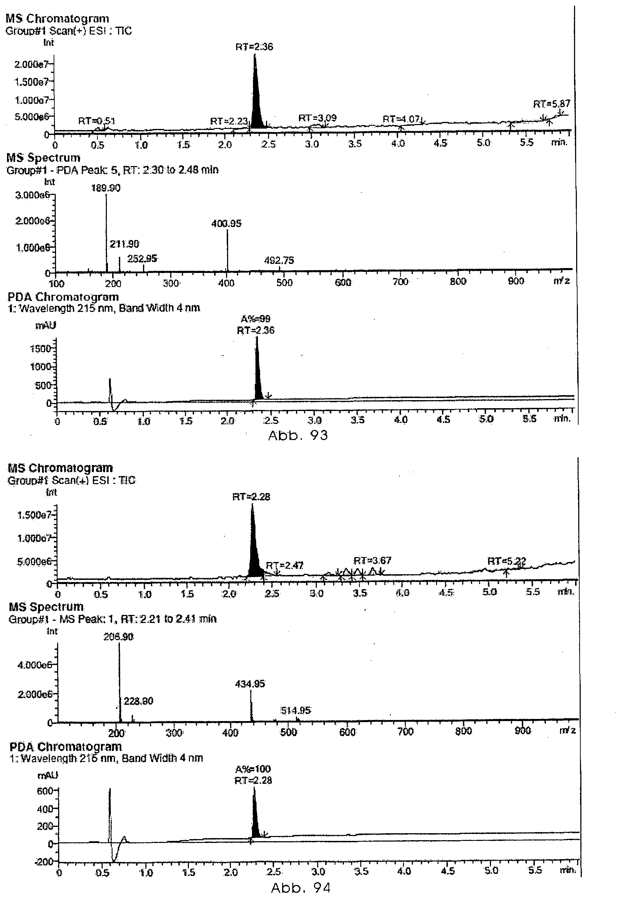

NaOMe (0.22 g, 4.13 mmol) wurde zu einer Lösung von Malonsäuredimethylester (0.55 g, 4.13 mmol) und Pyridin-2-carboxamidin (0.5 g, 84.13 mmol) in MeOH (5 ml) gegeben. Die Reaktionsmischung wurde unter Rückfluss für 40 min. erwärmt, was zur Bildung eines Niederschlags führte. Die Reaktionsmischung wurde mit MeOH (2 ml) und EtOAc (2 ml) verdünnt und der Niederschlag wurde trituriert und isoliert durch Filtration, um die Titelverbindung zu ergeben (0.54 g, 69 %).
MW: 189.17
HPLCMS (Methode A):[m/z]: 190
Abb. 93 zeigt das MS-Chromatogramm, das MS-Spektrum und das PDA-Chromatogramm der Verbindung von Beispiel 96.
IC50 [*µ*M]: >50.

### Allgemeines Verfahren 62:

### 4,6-Dichlor-2-pyridin-2-yl-pyrimidin

POCl₃ (2.7 ml, 28.97 mmol) wurde tropfenweise zu einer Lösung von 2-Pyridin-2-yl-pyrimidin-4,6-diol 140 (532 mg, 2.81 mmol) in Toluol (3.7 ml) bei 0°C gegeben. TEA (1.57 ml, 11.25 mmol) wurde tropfenweise hinzugegeben, und die Mischung wurde auf Raumtemperatur erwärmen gelassen, bevor sie für 1 h auf 110°C erwärmt wurde. Die Reaktionsmischung wurde im Vakuum eingeengt, und der Rückstand wurde durch Zugabe von Eis/Wasser (10 ml) abgeschreckt. Die wässrige Phase wurde mit EtOAc (x 3) extrahiert. Die vereinigten organischen Phasen wurden mit NaHCO₃ und Wasser gewaschen, getrocknet (Na₂SO₄) und im Vakuum eingeengt, um die Titelverbindung zu ergeben (310 mg, 49 %).
MW: 226.06
HPLCMS (Methode B):[m/z]: 226

### Beispiel 97

### Allgemeines Verfahren 63:

### 6-Chlor-2-pyridin-2-yl-pyrimidin-4-ylamin

NH₄OH (35 % Lösung in Wasser, 2.0 ml, 18.58 mmol) wurde zu einer Lösung von 4,6-Dichlor-2-pyridin-2-yl-pyrimidin (210 mg, 0.93 mmol) in EtOH (2 ml) in eine Mikrowellenröhre gegeben, und die Mischung wurde bei 100°C für 30 min. in der Mikrowelle erwärmt. Die Reaktionsmischung wurde im Vakuum eingeengt und der resultierende Rückstand wurde durch Trituration aus Isopropylalkohol gereinigt, um die Titelverbindung zu ergeben (135 mg, 70 %).
MW: 206.63
HPLCMS (Methode A):[m/z]: 207
Abb. 94 zeigt das MS-Chromatogramm, das MS-Spektrum und das PDA-Chromatogramm der Verbindung von Beispiel 97.
IC50 [*µ*M]: >50.

### Beispiel 98

### Allgemeines Verfahren 64:

### N-Isopropyl-2-pyridin-2-yl-pyrimidin-4,6-diamin

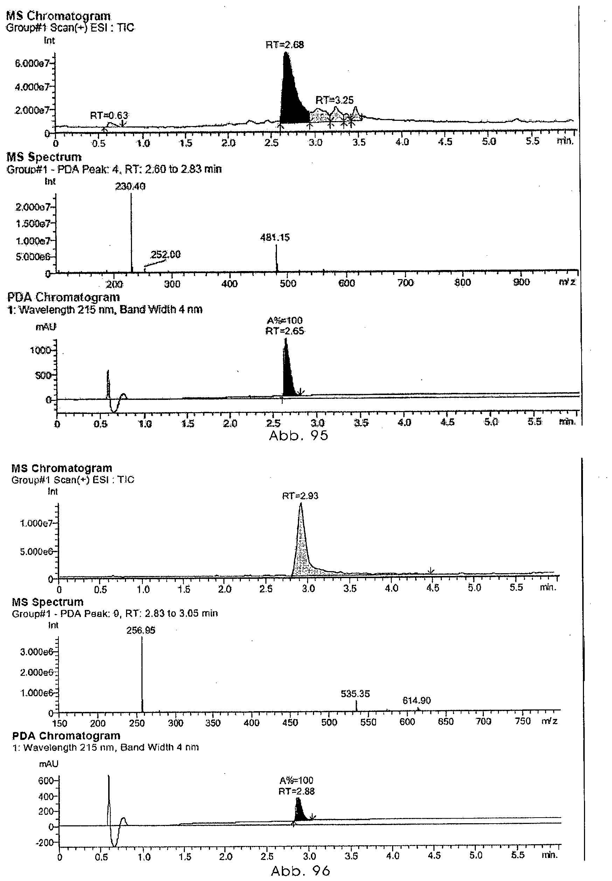

Isopropylamin (181 *µ*l, 2.42 mmol) wurde zu einer Lösung von 6-Chlor-2-pyridin-2-yl-pyrimidin-4-ylamin (100 mg, 0.48 mmol) in n-BuOH (1 ml) in eine Mikrowellenröhre gegeben, und die Mischung wurde bei 180°C für 1 h in der Mikrowelle erwärmt. Isopropylamin (181 *µ*l, 2.42 mmol) wurde hinzugegeben, und die Mischung wurde bei 180°C für weitere 7 h in der Mikrowelle erwärmt. Die Reaktionsmischung wurde mit Wasser (1 ml) verdünnt und im Vakuum eingeengt. Der Rückstand wurde in EtOAc (2 ml) gelöst und mit gesättigter wässriger NaHCO₃-Lösung (2 ml) und Wasser (2 ml) gewaschen. Die organische Phase wurde getrocknet (Na₂SO₄) und im Vakuum eingeengt. Der Rohrückstand wurde durch Trituration aus Et₂O gereinigt, um die Titelverbindung zu ergeben (32 mg, 29 %).
MW: 229.28
HPLCMS (Methode A):[m/z]: 230
Abb. 95 zeigt das MS-Chromatogramm, das MS-Spektrum und das PDA-Chromatogramm der Verbindung von Beispiel 98.
IC50 [*µ*M]: <50.

### Beispiel 99

### 5-Methoxy-2-pyridin-2-yl-4-pyrrolidin-1-yl-pyrimidin

MW: 256.30
Hersteller: Key Organics
HPLCMS (Methode A):[m/z]: 256.95
Abb. 96 zeigt das MS-Chromatogramm, das MS-Spektrum und das PDA-Chromatogramm der Verbindung von Beispiel 99.
IC50 [*µ*M] <50.

### Beispiel 100

### 5-Methoxy-4-(4-methyl-piperazin-1-yl)-2-pyridin-2-yl-pyrimidin

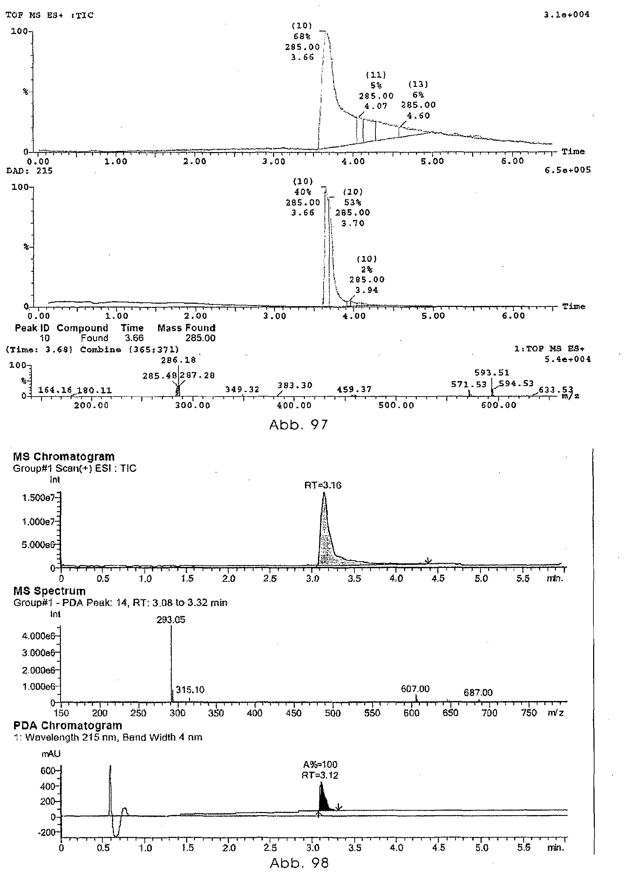

MW: 285.34
Hersteller: Key Organics
HPLCMS (Methode E):[m/z]: 286
Abb. 97 zeigt die Spektren/Chromatogramme der Verbindung von Beispiel 100.
IC50 [*µ*M]: >50.

### Beispiel 101

### (5-Methoxy-2-pyridin-2-yl-pyrimidin-4-yl)-methyl-phenyl-amin

MW: 292.36
Hersteller: Key Organics
HPLCMS (Methode A):[m/z]: 293
Abb. 98 zeigt das MS-Chromatogramm, das MS-Spektrum und das PDA-Chromatogramm der Verbindung von Beispiel 101.
IC50 [*µ*M]: <50.

### Beispiel 102

### 5-Methoxy-4-phenoxy-2-pyridin-2-yl-pyrimidin

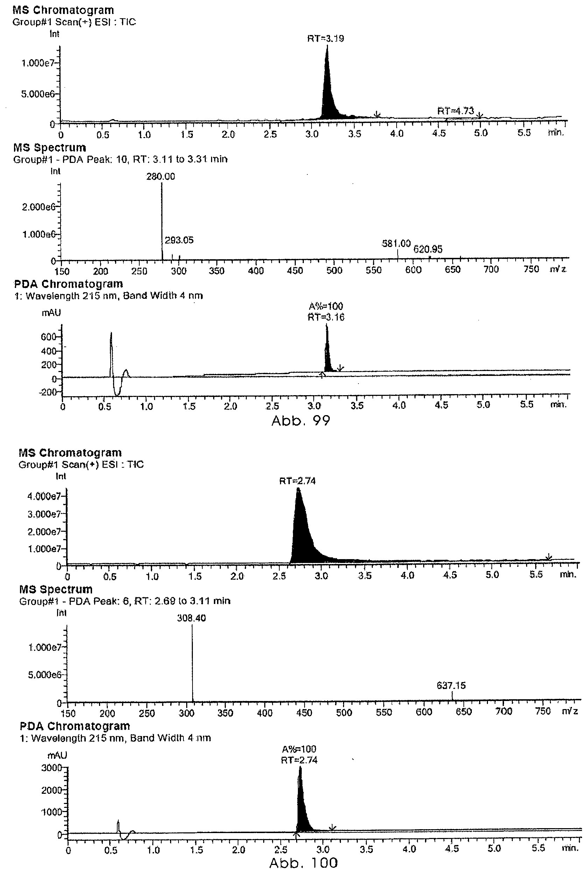

MW: 279.29
Hersteller: Key Organics
HPLCMS (Methode A):[m/z]: 280
Abb. 99 zeigt das MS-Chromatogramm, das MS-Spektrum und das PDA-Chromatogramm der Verbindung von Beispiel 102.
IC50 [*µ*M]: >50.

### Beispiel 103

### 5-(2-Methoxy-benzyl)-2-pyridin-2-yl-pyrimidin-4,6-diamin

MW: 307.35
Hersteller: Key Organics
HPLCMS (Methode A):[m/z]: 308
Abb. 100 zeigt das MS-Chromatogramm, das MS-Spektrum und das PDA-Chromatogramm der Verbindung von Beispiel 103.
IC50 [*µ*M]: <50.

### Beispiel 104

### 5-(2,4-Dichlorbenzyl)-2-pyridin-2-yl-pyrimidin-4,6-diamin

MW: 346.21
Hersteller: Key Organics
HPLCMS (Methode A):[m/z]: 347
Abb. 101 zeigt das MS-Chromatogramm, das MS-Spektrum und das PDA-Chromatogramm der Verbindung von Beispiel 104.
IC50 [*µ*M] : <50.

### Beispiele 105-112

In den folgenden Beispielen wurden die nachfolgend beschriebenen analytischen Methoden verwendet:
Analytische HPLC-MS

### Methode A

Säule: Waters Atlantis dC18 (2.1 x100 mm, 3 mm Säule)
Fließgeschwindigkeit: 0.6 ml/min
Lösungsmittel A: 0.1 % Ameisensäure/Wasser
Lösungsmittel B: 0.1 % Ameisensäure/Acetonitril
Injektionsvolumen: 3 *µ*l
Säulentemperatur: 40°C
UV Detektionswellenlänge: 215 nm
Eluent: 0 min. bis 5 min., konstanter Gradient von 95 % Lösungsmittel A + 5 % Lösungsmittel B bis 100 % Lösungsmittel B; 5 min. bis 5.4 min., 100 % Lösungsmittel B; 5.4 min. bis 5.42 min., konstanter Gradient von 100 % Lösungsmittel B bis 95 % Lösungsmittel A + 5 % Lösungsmittel B; 5.42 min.
bis 7.00 min., 95 % Lösungsmittel A + 5 % Lösungsmittel B

### Methode B

Säule: Waters Atlantis dC18 (2.1 x 50 mm, 3 mm)
Lösungsmittel A: 0.1 % Ameisensäure/Wasser
Lösungsmittel B: 0.1 % Ameisensäure/Acetonitril
Fließgeschwindigkeit 1 ml/min
Injektionsvolumen 3 ml
UV Detektionswellenlänge: 215 nm
Eluent: 0 bis 2.5 min., konstanter Gradient von 95 % Lösungsmittel A + 5 % Lösungsmittel B bis 100 % Lösungsmittel B; 2.5 min. bis 2.7 min., 100 % Lösungsmittel B; 2.71 bis 3.0 min., 95 % Lösungsmittel A + 5 % Lösungsmittel B.

### Methode C

Säule: Waters Atlantis dC18 (2.1 x 30 mm, 3 mm Säule)
Fließgeschwindigkeit: 1 ml/min
Lösungsmittel A: 0.1 % Ameisensäure/Wasser
Lösungsmittel B: 0.1 % Ameisensäure/Acetonitril
Injektionsvolumen: 3 ml
UV Detektionswellenlänge: 215 nm
Eluent: 0 min. bis 1.5 min., konstanter Gradient von 95 % Lösungsmittel A + 5 % Lösungsmittel B bis 100 % Lösungsmittel B; 1.5 min. bis 1.6 min., 100 % Lösungsmittel B; 1.60 min. bis 1.61 min., konstanter Gradient von 100 % Lösungsmittel B bis 95 % Lösungsmittel A + 5 % Lösungsmittel B; 1.61 min. bis 2.00 min., 95 % Lösungsmittel A + 5 % Lösungsmittel B.
MS-Detektion unter Verwendung von Waters LCT oder LCT Premier oder ZQ oder ZMD
UV-Detektion unter Verwendung von Waters 2996 Photodioden Array oder Waters 2787 UV oder Waters 2788 UV
Präparative HPLC - neutrale Bedingungen
Säule: Waters SunFire Prep C18 OBD (5 mm 19 x 100 mm)
Fließgeschwindigkeit: 20 ml/min
Lösungsmittel A: Wasser
Lösungsmittel B: Acetonitril
Injektionsvolumen: 1000 *µ*l
Säulentemperatur: Raumtemperatur
Detektion: UV-gerichtet
Eluent: 0 min. bis 2 min., 5 % Lösungsmittel B + 95 % Lösungsmittel A; 2 min. bis 2.5 min konstanter Gradient bis 10 % Lösungsmittel B + 90 % Lösungsmittel A, 2.5 min. bis 14.5 min. konstanter Gradient bis 100 % Lösungsmittel B; 14.5 min. bis 16.5 min. 100 % Lösungsmittel B; 16.5 bis 16.7 min. konstanter Gradient bis 5 % B + 95 % A; 16.7 min. bis 17.2 min. 5 % Lösungsmittel B + 95 % Lösungsmittel A.
Gilson semi-präparative HPLC-Module mit 119 UV-Detektor und 5.11 Unipoint Steuerungssoftware
Waters 515 Hilfspumpen
Waters 2487 UV-Detektor
Gilson 215 Autosampler und Fraktionssammler

Flash-Silikagel-Chromatographie wurde durchgeführt an Silikagel 230-400 mesh oder an vorgepackten Siliziumdioxid-Kartuschen.

Mikrowellenreaktionen wurden durchgeführt unter Verwendung von CEM Discover oder Explorer - fokussierende Mikrowellenapparate.

### Verbindungsbezeichnungen

Einige Verbindungen wurden als TFA- oder HCl-Salze isoliert, was sich nicht in deren chemischen Namen widerspiegelt. Im Rahmen der vorliegenden Erfindung bedeuten die chemischen Namen jeweils die Verbindung in neutraler Form, ebenso wie ihre TFA-Salze oder irgendein anderes Salz, insbesondere pharmazeutisch verträgliches Salz, soweit anwendbar.

### Abkürzungen

- AcOH: Essigsäure
- n-BuOH: n-Butanol
- Kat.: Katalytisch
- d: Tag(e)
- DCE: 1,2-Dichlorethan
- DCM: Dichlormethan
- DIPEA: N,N-Diisoproylethylamin
- DMAP: 4-Dimethylaminopyridin
- EDC.HCl: N-[3-(Dimethylamino)propyl]-N'-ethylcarbodiimid-hydrochlorid
- Et₂O: Diethylether
- EtOAc: Ethylacetat
- EtOH: Ethanol
- h: Stunde(n)
- HPLC: Hochleistungsflüssigkeits-Chromatographie
- MeOH: Methanol
- min: Minute(n)
- MW: Molekulargewicht
- i-PrOH: Isopropanol
- STAB: Natriumtriacetoxyborhydrid
- TEA: Triethylamin
- TFA: Trifluoressigsäure
- THF: Tetrahydrofuran
- p-TSA: para-Toluolsulfonsäure

### ROUTE 1

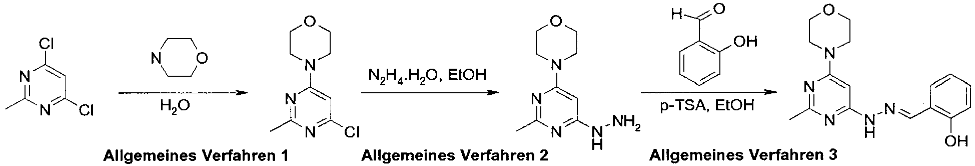

### Allgemeines Verfahren 1:

### 4-(6-Chlor-2-methyl-pyrimidin-4-yl)-morpholin

Eine Mischung von Morpholin (2.36 ml, 27.0 mmol) und 4,6-Dichlor-2-methyl-pyrimidin (2.0 g, 12.3 mmol) in Wasser (20 ml) wurde bei 100°C für 2 h erhitzt. Der Ansatz wurde auf Raumtemperatur abkühlen gelassen und mit Wasser (20 ml) verdünnt. Der resultierende Niederschlag wurde durch Filtration isoliert, um die Titelverbindung zu ergeben (1.90 g, 72 % Ausbeute).
MW: 213.67
HPLCMS (Methode B):[m/z]: 214

### Allgemeines Verfahren 2:

### (2-Methyl-6-morpholin-4-yl-pyrimidin-4-yl)-hydrazin

Eine Mischung von Hydrazinmonohydrat (150 ml, 3.09 mmol) und 4-(6-Chlor-2-methyl-pyrimidin-4-yl)-morpholin (300 mg, 1.40 mmol) in EtOH (3 ml) wurde unter Rückfluss über Nacht erhitzt. Zusätzliches Hydrazinmonohydrat (200 ml, 4.20 mmol) wurde hinzugegeben, und der Ansatz wurde unter Rückfluss für weitere 24 h erhitzt. Der Ansatz wurde auf Raumtemperatur abkühlen gelassen. Der resultierende Niederschlag wurde durch Filtration isoliert, um die Titelverbindung zu ergeben (246 mg, 84 % Ausbeute).
MW: 209.25
HPLCMS (Methode B):[m/z]: 210

### Beispiel 105

### Allgemeines Verfahren 3:

2-[(2-Methyl-6-morpholin-4-yl-pyrimidin-4-yl)-hydrazonmethyl]-phenol 2-Hydroxy-benzaldehyd (15 ml, 0.14 mmol) und p-Toluolsulfonsäuremonohydrat (Kat.) wurden zu einer Lösung von (2-Methyl-6-morpholin-4-yl-pyrimidin-4-yl)-hydrazin (30 mg, 0.14 mmol) in EtOH (0.6 ml) gegeben. Der Ansatz wurde bei Raumtemperatur für 20 min. gerührt. Der resultierende Niederschlag wurde durch Filtration isoliert. Der Rohrückstand wurde durch Säulenchromatographie mit EtOAc/Heptan (55 %) als Eluent gereinigt, um die Titelverbindung zu ergeben (24 mg, 55 % Ausbeute).
MW: 313.36

Die Titelverbindung war nicht stabil unter HPLCMS-Bedingungen - Struktur durch NMR bestätigt.

### ROUTE 2

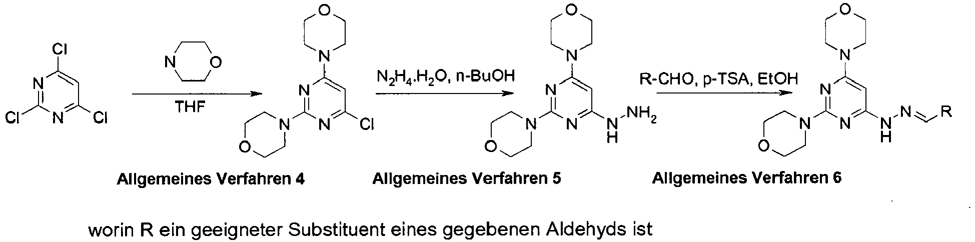

### Allgemeines Verfahren 4:

### 2,6-Di-morpholinyl-4-chlorpyrimidin

Morpholin (4.74 ml, 54.52 mmol) wurde tropfenweise zu einer Lösung von 2,4,6-Trichlor-pyrimidin (2.0 g 10.90 mmol) in THF (30 ml) bei 0°C gegeben. Der Ansatz wurde auf Raumtemperatur erwärmen gelassen und für 16 h bei 50°C erhitzt. Der Ansatz wurde auf Raumtemperatur abgekühlt, mit Wasser (60 ml) verdünnt und mit Et₂O (x 3) extrahiert. Die vereinigten organischen Phasen wurden getrocknet (Na₂SO₄) und im Vakuum eingeengt. Der Rohrückstand wurde durch Säulenchromatographie mit EtOAc/Heptan (20-30 % Gradient) als Eluent gereinigt, um die Titelverbindung zu ergeben (2.52 g, 82 % Ausbeute).
MW: 284.75
HPLCMS (Methode B):[m/z]: 285

### Allgemeines Verfahren 5:

### (2,6-Di-Morpholin-4-yl-pyrimidin-4-yl)-hydrazin

Hydrazinmonohydrat (256 ml, 5.27 mmol) wurde tropfenweise zu einer Lösung von 2,6-Di-morpholinyl-4-chlorpyrimidin (300 mg, 1.05 mmol) in n-BuOH (1.2 ml) gegeben. Der Ansatz wurde unter Rückfluss für 16 h erhitzt. Der Ansatz wurde im Vakuum eingeengt. Der Rohrückstand wurde mit EtOH trituriert, um die Titelverbindung zu ergeben (276 mg, 94 % Ausbeute).
MW: 280.33
HPLCMS (Methode B):[m/z]: 281

### Beispiel 106

### Allgemeines Verfahren 6:

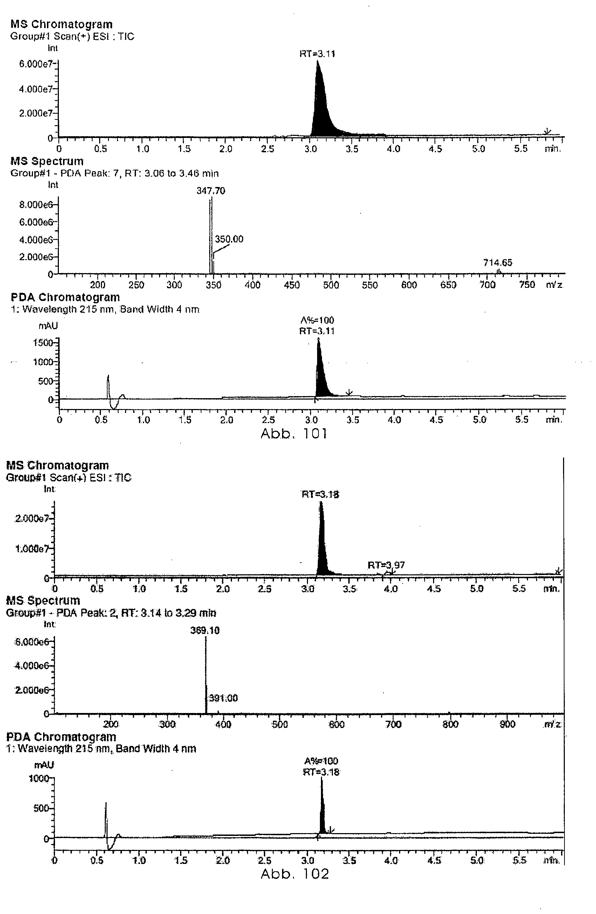

N-Benzyliden-N'-(2,6-di-morpholin-4-yl-pyrimidin-4-yl)-hydrazin p-Toluolsulfonsäuremonohydrat (Kat.) wurde zu einer Lösung von (2,6-Di-morpholin-4-yl-pyrimidin-4-yl)-hydrazin (50 mg, 0.18 mmol) und Benzaldehyd (18.2 ml, 0.18 mmol) in EtOH (2 ml) gegeben. Der resultierende Niederschlag wurde durch Filtration isoliert und mit einer Lösung von Et₂O, MeOH und DCM (1:1:1) trituriert, um die Titelverbindung zu ergeben (13 mg, 18 % Ausbeute).
MW: 368.44
HPLCMS (Methode A):[m/z]: 369
Abb. 102 zeigt das MS-Chromatogramm, das MS-Spektrum und das PDA-Chromatogramm der Verbindung von Beispiel 106.
IC50 [*µ*M]: >50.

### Beispiel 107

### 4-[(2,6-Di-morpholin-4-yl-pyrimidin-4-yl)-hydrazonomethyl]-benzol-1,3-diol

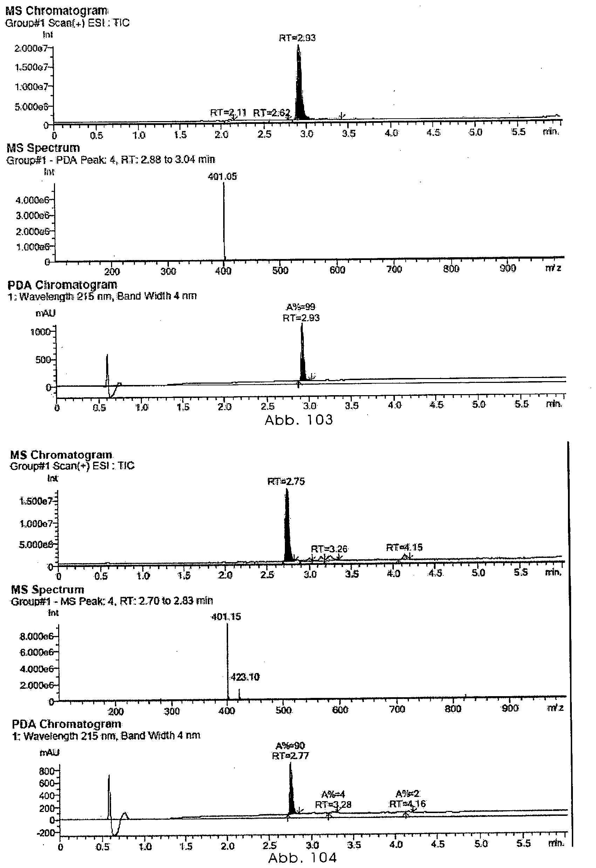

In ähnlicher Weise, unter Verwendung von Route 2, allgemeines Verfahren 6, ergaben p-Toluolsulfonsäuremonohydrat (Kat.), (2,6-Di-morpholin-4-yl-pyrimidin-4-yl)-hydrazin (50 mg, 0.18 mmol) und 2,4-Dihydroxy-benzaldehyd (24.6 mg, 0.18 mmol) in EtOH (2 ml) die Titelverbindung (34 mg, 51 % Ausbeute) nach Reinigung durch Trituration aus EtOH.
MW: 400.44
HPLCMS (Methode A):[m/z]: 401
Abb. 103 zeigt das MS-Chromatogramm, das MS-Spektrum und das PDA-Chromatogramm der Verbindung von Beispiel 107.
IC50 [*µ*M]: >50.

### Beispiel 108

### ROUTE 3

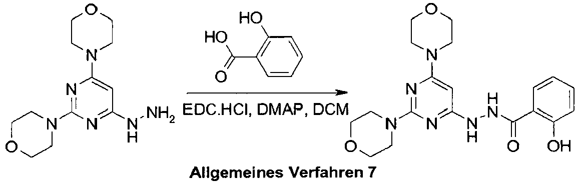

### Allgemeines Verfahren 7:

2-Hydroxy-benzoesäure-N'-(2,6-di-morpholin-4-yl-pyrimidin-4-yl)-hydrazid EDC.HCl (97 mg, 0.49 mmol) wurde zu einer Lösung von (2,6-Di-morpholin-4-yl-pyrimidin-4-yl)-hydrazin (127 mg, 0.48 mmol), 2-Hydroxy-benzoesäure (63 mg, 0.48 mmol) und DMAP (Kat.) in DCM gegeben, und die Mischung wurde für 16 h bei Raumtemperatur gerührt. Die Reaktionsmischung wurde im Vakuum eingeengt. Der Rohrückstand wurde durch präparative HPLC (neutrale Bedingungen), gefolgt von Trituration aus Et₂O/EtOAc, gereinigt, um die Titelverbindung zu ergeben (8 mg, 4 % Ausbeute).
MW: 400.44
HPLCMS (Methode A):[m/z]: 401
Abb. 104 zeigt das MS-Chromatogramm, das MS-Spektrum und das PDA-Chromatogramm der Verbindung von Beispiel 108.
IC50 [*µ*M] : >50.

### Beispiel 109

### ROUTE 4

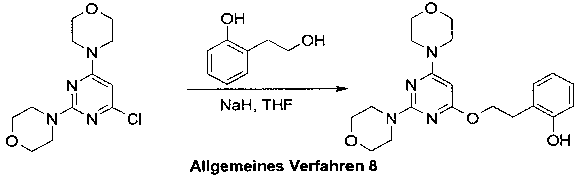

### Allgemeines Verfahren 8:

### 2-[2-(2,6-Di-morpholin-4-yl-pyrimidin-4-yloxy)-ethyl]-phenol

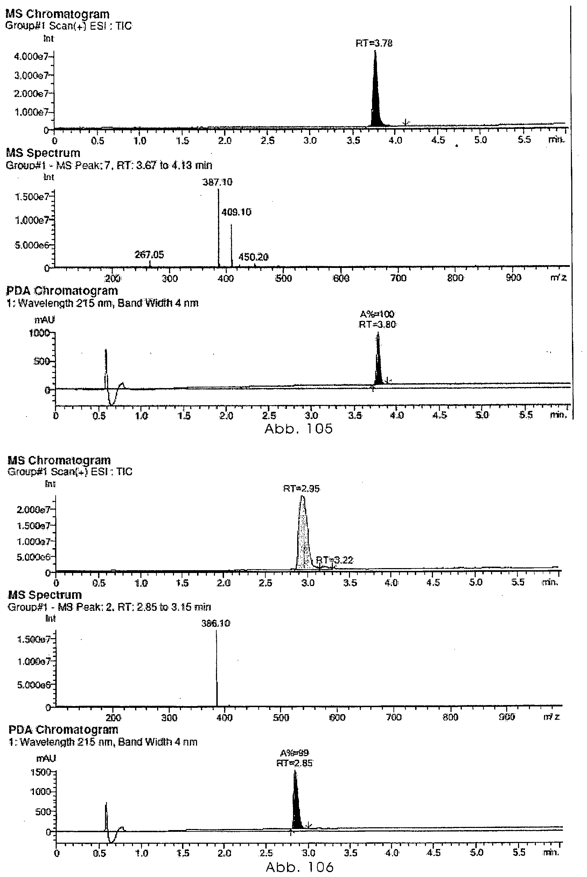

Natriumhydrid (60 %ige Dispersion in Mineralöl, 14 mg, 0.35 mmol) wurde zu einer Lösung von 2,6-Di-morpholinyl-4-chlor-pyrimidin (50 mg, 0.18 mmol) und 2-(2-Hydroxy-ethyl)-phenol (24 mg, 0.18 mmol) in THF (1 ml) bei 0°C unter N₂ in einer Mikrowellenröhre gegeben. Der Ansatz wurde auf Raumtemperatur erwärmen gelassen und dann bei Raumtemperatur für 1 h gerührt. Die Mikrowellenröhre wurde dann mit N₂ gespült, versiegelt und auf 120°C in der Mikrowelle für 11 h erhitzt. Der Ansatz wurde mit Wasser (1 ml) verdünnt und durch tropfenweise Zugabe von 0,1 M wässriger HCl neutralisiert. Die resultierende Lösung wurde extrahiert mit EtOAc (x 3). Die vereinigten organischen Phasen wurden getrocknet (Na₂SO₄) und im Vakuum eingeengt. Der Rohrückstand wurde durch Säulenchromatographie mit EtOAc/Heptan (30-45 % Gradient) als Eluent gereinigt, um die Titelverbindung zu ergeben (17 mg, 25 % Ausbeute).
MW: 386.45
HPLCMS (Methode A):[m/z]: 387
Abb. 105 zeigt das MS-Chromatogramm, das MS-Spektrum und das PDA-Chromatogramm der Verbindung von Beispiel 109.
IC50 [*µ*M]: >50.

### ROUTE 5

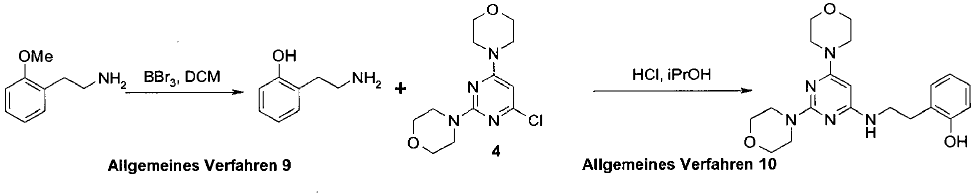

### Allgemeines Verfahren 9:

### 2-(2-Amino-ethyl)-phenol

Bortribromid (1.0 M Lösung in DCM, 33.1 ml, 33.1 mmol) wurde tropfenweise zu einer Lösung von 2-(2-Methoxy-phenyl)-ethylamin (2.0 g, 13.2 mmol) in DCM (20 ml) bei -78°C gegeben. Der Ansatz wurde auf Raumtemperatur über Nacht erwärmen gelassen. Der Ansatz wurde abgeschreckt durch Zugabe von MeOH (20 ml) bei -78°C. Der Ansatz wurde auf Raumtemperatur erwärmen gelassen und dann für 1 h gerührt. Die resultierende Lösung wurde im Vakuum eingeengt, mit gesättigter wässriger NaHCO₃-Lösung (100 ml) verdünnt und mit i-PrOH/CHCl₃ (1:1, x 3) extrahiert. Die vereinigten organischen Phasen wurden getrocknet (Na₂SO₄) und im Vakuum eingeengt, um die Titelverbindung zu ergeben (0.34 g, 19 % Ausbeute).
MW: 137.18
HPLCMS (Methode B):[m/z]: 138

### Beispiel 110

### Allgemeines Verfahren 10:

### 2-[2-(2,6-Di-morpholin-4-yl-pyrimidin-4-ylamino)-ethyl]-phenol

Konzentrierte HCl (2 Tropfen) wurde zu einer Lösung von 2,6-Di-morpholinyl-4-chlor-pyrimidin (180 mg, 0.63 mmol) und 2-(2-Amino-ethyl)-phenol 10 (130 mg, 0.95 mmol) in i-PrOH (3.5 ml) gegeben. Der Ansatz wurde bei 170°C in einer Mikrowelle für 1 h erwärmt. Der Ansatz wurde mit gesättigter wässriger NaHCO₃-Lösung basisch gemacht und mit DCM (x 3) extrahiert. Die vereinigten organischen Phasen wurden getrocknet (Na₂SO₄) und im Vakuum eingeengt. Der Rohrückstand wurde durch Säulenchromatographie mit EtOAc/Heptan (75 %) als Eluent gereinigt, um die Titelverbindung zu ergeben (30 mg, 12 % Ausbeute).
MW: 385.47
HPLCMS (Methode A):[m/z]: 386
Abb. 106 zeigt das MS-Chromatogramm, das MS-Spektrum und das PDA-Chromatogramm der Verbindung von Beispiel 110.
IC50 [*µ*M]: >50.

### ROUTE 6

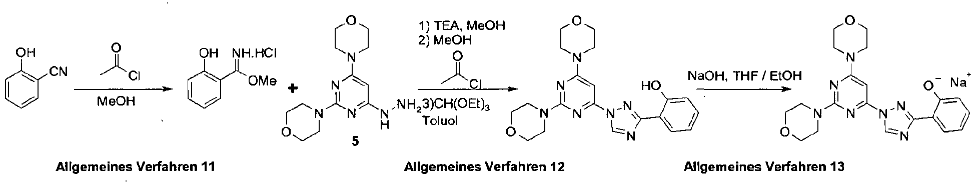

### Allgemeines Verfahren 11:

### 2-Hydroxy-benziminsäuremethylester

Acetylchlorid (5.9 ml, 83.95 mmol) wurde tropfenweise zu MeOH (11 ml) bei Raumtemperatur unter N₂ gegeben. Der Ansatz wurde für 2 h gerührt und 2-Hydroxy-benzonitril (2.0 g, 16.79 mmol) wurde hinzugegeben. Nach 48 h wurde der Ansatz im Vakuum eingeengt. Der Rückstand wurde in DCM (5 ml) gelöst und Et₂O wurde tropfenweise hinzugegeben, um einen Niederschlag zu ergeben. Der Niederschlag wurde durch Filtration isoliert, um die Titelverbindung als HCl-Salz zu ergeben (0.59 g, 19 % Ausbeute).
MW: 151.17
HPLCMS (Methode B):[m/z]: 152

### Allgemeines Verfahren 12:

### 2-[1-(2,6-Di-morpholin-4-yl-pyrimidin-4-yl)-1H-[1,2,4]triazol-3-yl]-phenol

TEA (148 ml, 1.07 mmol) wurde zu einer Lösung von 2-Hydroxy-benziminsäuremethylester HCl (167 mg, 0.89 mmol) in MeOH (3.5 ml) gegeben. Nach 30 min. wurde (2,6-Di-morpholin-4-yl-pyrimidin-4-yl)-hydrazin (275 mg, 0.98 mmol) hinzugegeben, und der Ansatz wurde unter Rückfluss für 6 h erhitzt. In einem separaten Kolben wurde Acetylchlorid (69 ml, 0.98 mmol) tropfenweise zu MeOH (3.5 ml) gegeben und bei Raumtemperatur für 30 min. gerührt. Dies wurde zur Hauptreaktionsmischung bei 0°C gegeben. Der Ansatz wurde bei Raumtemperatur für 10 min. gerührt, bevor im Vakuum eingeengt wurde. Der Rückstand wurde in Toluol (5 ml) gelöst und Orthoameisensäuretriethylester (5 ml) wurde hinzugegeben. Der Ansatz wurde bei 100°C für 30 min. erhitzt. Nach Abkühlen auf 85°C wurde EtOH (3 ml) weiter hinzugegeben und der Ansatz wurde bei 85°C für 30 min. gehalten. Nach Abkühlen auf Raumtemperatur wurde die Mischung mit gesättigter wässriger NaHCO₃-Lösung basisch gemacht. Die Phasen wurden abgetrennt und die wässrige Phase mit DCM (x 3) extrahiert. Die vereinigten organischen Phasen wurden getrocknet (Na₂SO₄) und im Vakuum eingeengt. Der Rohrückstand wurde durch Säulenchromatographie mit EtOAc/Heptan (25 %) als Eluent gereinigt. Der resultierende Feststoff wurde mit MeOH trituriert, um die Titelverbindung zu ergeben (40 mg, 11 % Ausbeute).
MW: 409.45
HPLCMS (Methode A):[m/z]: 410

### Beispiel 111

### Allgemeines Verfahren 13:

### Natrium-2-[1-(2,6-di-morpholin-4-yl-pyrimidin-4-yl)-1H-[1,2,4]triazol-3-yl]-phenoxid

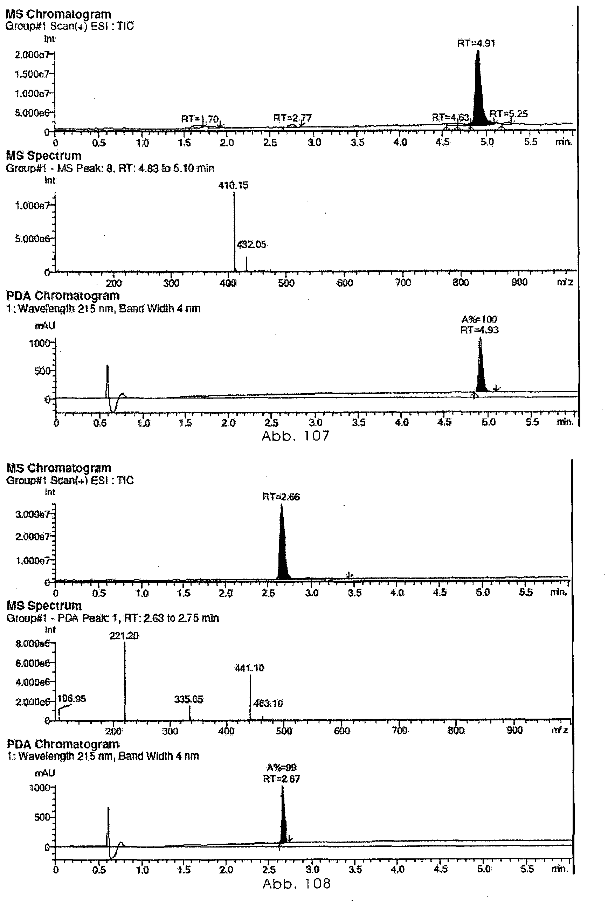

NaOH (0.1 M Lösung in Wasser, 0.5 ml, 48.8 mmol) wurde zu einer Suspension von 2-[1-(2,6-Di-morpholin-4-yl-pyrimidin-4-yl)-1 H-[1,2,4]triazol-3-yl]-phenol (20 mg, 48.8 mmol) in EtOH/THF (1:20, 5.25 ml) gegeben. Die Reaktionsmischung wurde im Vakuum eingeengt, um die Titelverbindung zu ergeben (21 mg, 100 % Ausbeute).
MW: 408.44 (anion)
HPLCMS (Methode A):[m/z]: 410
Abb. 107 zeigt das MS-Chromatogramm, das MS-Spektrum und das PDA-Chromatogramm der Verbindung von Beispiel 111.
IC50 [*µ*M]: >50.

### ROUTE 7

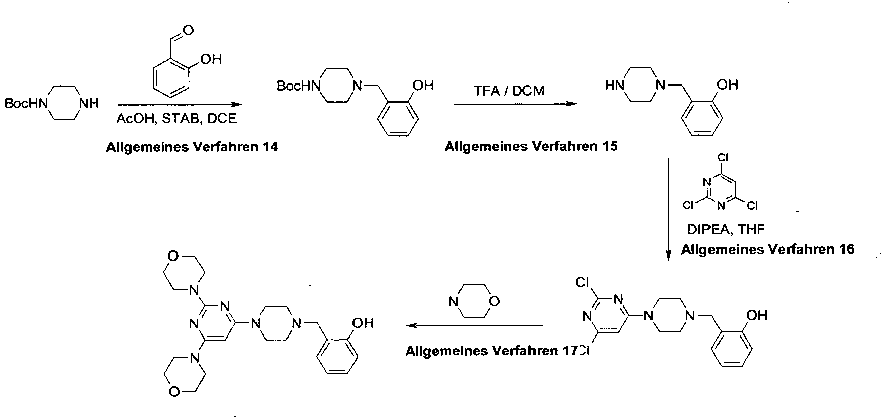

### Allgemeines Verfahren 14:

### 4-(2-Hydroxy-benzyl)-piperazin-1-carbonsäure-tert-butylester

Essigsäure (308 ml, 5.37 mmol) wurde zu einer Lösung von Piperazine-1-carbonsäure-tert-butylester (1.0 g, 5.37 mmol) und 2-Hydroxy-benzaldehyd (570 ml, 5.37 mmol) in DCE über 4 *µ* Molekularsieb gegeben. Der Ansatz wurde für 1 h bei Raumtemperatur gerührt, und Natriumtriacetoxyborhydrid (2.28 g, 10.74 mmol) wurde hinzugegeben. Nach Rühren für weitere 16 h wurde der Ansatz mit MeOH (10 ml) gequentscht. Nach Rühren für 30 min. wurde die Mischung filtriert und das Filtrat im Vakuum eingeengt. Der Rohrückstand wurde durch Säulenchromatographie mit EtOAc/Heptan (25 %) als Eluent gereinigt, um die Titelverbindung zu ergeben (0.69 g, 44 % Ausbeute).
MW: 292.38
HPLCMS (Methode B):[m/z]: 293

### Allgemeines Verfahren 15:

### 2-Piperazin-1-ylmethyl-phenol

4-(2-Hydroxy-benzyl)-piperazin-1-carbonsäure-tert-butytester (0.69 g, 2.36 mmol) wurde in TFA/DCM (1:3, 7 ml) gelöst, und die Mischung wurde bei Raumtemperatur für 18 h gerührt. Die Reaktionsmischung wurde im Vakuum eingeengt, um die Titelverbindung als TFA-Salz zu ergeben (0.99 g, 100 % Ausbeute).
MW: 192.26
HPLCMS (Methode B):[m/z]: 193

### Allgemeines Verfahren 16:

### 2-[4-(2,6-Dichlorpyrimidin-4-yl)-piperozin-1-ylmethyl]-phenol

DIPEA (0.5 ml, 2.85 mmol) wurde zu einer Lösung von 2-Piperazin-1-ylmethylphenol-trifluoressigsäuresalz (400 mg, 0.95 mmol) in THF (5 ml) gegeben, und für 30 min. bei Raumtemperatur gerührt. Die resultierende Lösung wurde tropfenweise zu einer gerührten Lösung von 2,4,6-Trichlorpyrimidin (109 ml, 0.95 mmol) in THF (1 ml) bei 0°C gegeben und der Ansatz wurde für 18 h bei Raumtemperatur gerührt. Die Reaktionsmischung wurde mit Wasser (6 ml) verdünnt und mit EtOAc (x 3) extrahiert. Die vereinigten organischen Phasen wurden getrocknet (Na₂SO₄) und im Vakuum eingeengt. Der Rohrückstand wurde durch Säulenchromatographie mit EtOAc/Heptan (25 %) als Eluent gereinigt, um die Titelverbindung zu ergeben (145 mg, 35 % Ausbeute).
MW: 339.23
HPLCMS (Methode B):[m/z]: 339

### Beispiel 112

### Allgemeines Verfahren 17:

### 2-[4-(2,6-Di-morpholin-4-yl-pyrimidin-4-yl)-piperazin-1-ylmethyl]-phenol

Eine Lösung von 2-[4-(2,6-Dichlor-pyrimidin-4-yl)-piperazin-1-ylmethyl]-phenol (128 mg, 0.38 mmol) in Morpholin (4 ml) wurde unter Rückfluss für 18 h erhitzt. Die Mischung wurde im Vakuum eingeengt, und der Rückstand in EtOAc (10 ml) gelöst. Die organische Phase wurde mit gesättigter wässriger NaHCO₃-Lösung (10 ml) gewaschen. Die organische Phase wurde getrocknet (Na₂SO₄) und im Vakkum eingeengt. Der Rohrückstand wurde in EtOAc trituriert, um die Titelverbindung zu ergeben (84 mg, 50 % Ausbeute).
MW: 440.55
HPLCMS (Methode A):[m/z]: 441
Abb. 108 zeigt das MS-Chromatogramm, das MS-Spektrum und das PDA-Chromatogramm der Verbindung von Beispiel 112.
IC50 [*µ*M]: >50.

### Beispiele 113-117

In den folgenden Beispielen wurden die nachfolgend beschriebenen analytischen Methoden verwendet:

### Analytische HPLC-MS

### Methode A

Säule: Waters Atlantis dC18 (2.1 x 100 mm, 3 *µ*m Säule)
Fließgeschwindigkeit: 0.6 ml/min
Lösungsmittel A: 0.1 % Ameisensäure/Wasser
Lösungsmittel B: 0.1 % Ameisensäure/Acetonitril
Injektionsvolumen: 3 *µ*l
Säulentemperatur: 40°C
UV-Detektionswellenlänge: 215 nm
Eluent: 0 min. bis 5 min., konstanter Gradient von 95 % Lösungsmittel A + 5 % Lösungsmittel B bis 100 % Lösungsmittel B; 5 min. bis 5.4 min., 100 % Lösungsmittel B; 5.4 min. bis 5.42 min., konstanter Gradient von 100 % Lösungsmittel B bis 95 % Lösungsmittel A + 5 % Lösungsmittel B; 5.42 min. bis 7.00 min., 95 % Lösungsmittel A + 5 % Lösungsmittel B

### Methode B

Säule: Waters Atlantis dC18 (2.1 x 50 mm, 3 *µ*m)
Lösungsmittel A: 0.1 % Ameisensäure/Wasser
Lösungsmittel B: 0.1 % Ameisensäure/Acetonitril
Fließgeschwindigkeit 1 ml/min
Injektionsvolumen 3 *µ*l
UV-Detektionswellenlänge: 215nm
Eluent: 0 bis 2.5 min., konstanter Gradient von 95 % Lösungsmittel A + 5 % Lösungsmittel B bis 100 % Lösungsmittel B; 2.5 min. bis 2.7 min., 100 % Lösungsmittel B; 2.71 bis 3.0 min., 95 % Lösungsmittel A + 5 % Lösungsmittel B.
MS-Detektion unter Verwendung von Waters LCT oder LCT Premier oder ZQ oder ZMD
UV-Detektion unter Verwendung von Waters 2996 Photodioden Array oder Waters 2787 UV oder Waters 2788 UV

Flash-Silikagel-Chromatographie wurde durchgeführt an Silikagel 230-400 mesh oder an vorgepackten Silika-Kartuschen.

### Abkürzungen

- d: Tag(e)
- DCM: Dichlormethan
- DIPEA: N,N-Disopropylethylamin
- EtOAc: Ethylacetat
- EtOH: Ethanol
- h: Stunde(n)
- HPLC: Hochleistungsflüssigkeits-Chromatographie
- min: Minuten
- MW: Molekulargewicht
- p-TSA: para-Toluolsulfonsäure
- TFA: Trifluoressigsäure
- THF: Tetrahydrofuran

### ROUTE 1

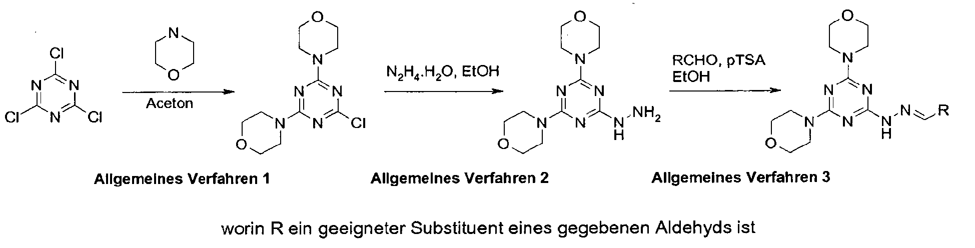

### Allgemeines Verfahren 1:

### 2-Chlor-4,6-di-morpholin-4-yl-[1,3,5]triazin

Eine Lösung von Morpholin (4.0 ml, 45.8 mmol) in Wasser (2 ml) wurde zu einer Lösung von Cyanurchlorid (2.0 g, 10.9 mmol) in Aceton (30 ml) bei 0°C gegeben, und die Mischung wurde bei 0°C für 1.75 h gerührt. Wasser (50 ml) wurde hinzugegeben und der resultierende Niederschlag durch Filtration isoliert, mit Wasser gewaschen und bei 40°C unter Vakuum getrocknet, um die Titelverbindung zu ergeben (2.84 g, 91 %).
MW: 285.74
HPLCMS (Methode B):[m/z]: 286

### Allgemeines Verfahren 2:

### (4,6-Di-morpholin-4-yl-[1,3,5]triazin-2-yl)-hydrazin

Hydrazinhydrat (0.88 ml, 1.75 mmol) wurde zu einer Lösung von 2-Chlor-4,6-di-morpholin-4-yl-[1,3,5]triazin 1 (100 mg, 0.35 mmol) in EtOH (1 ml) gegeben, und die Mischung wurde unter Rückfluss für 1,5 h erhitzt. Nach Abkühlen wurde der resultierende Feststoff durch Filtration isoliert und mit EtOH gewaschen, um die Titelverbindung zu ergeben (85 mg, 86 %).
MW: 281.32
HPLCMS (Methode B):[m/z]: 282

### Beispiel 113

### Allgemeines Verfahren 3:

### 2-[(4,6-Di-morpholin-4-yl-[1,3,5]triazin-2-yl)-hydrazonomethyl]-phenol

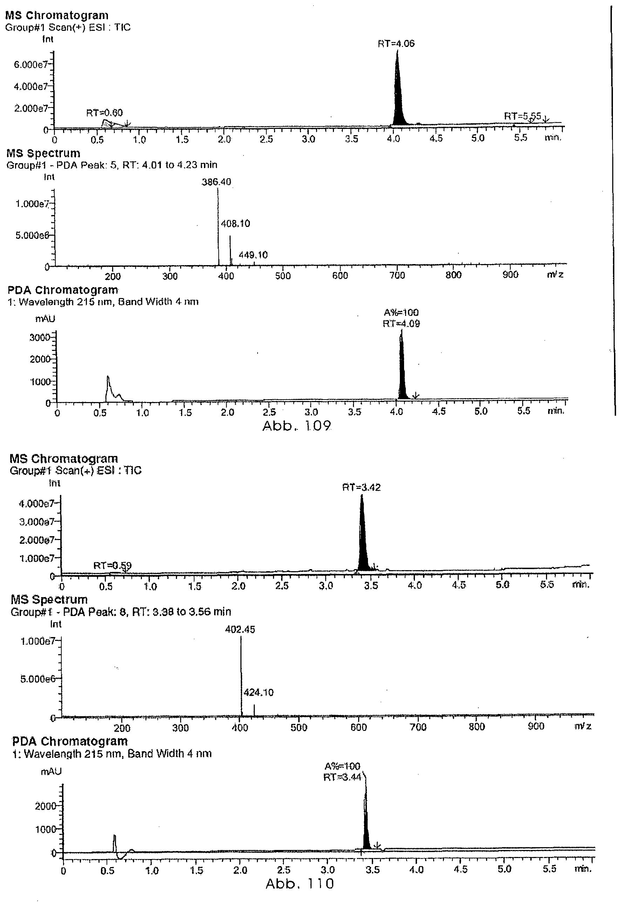

2-Hydroxybenzaldehyd (15 *µ*l, 0.14 mmol) und p-Toluolsulfonsäure (2 mg, 0.01 mmol) wurde zu einer Lösung von (4,6-Di-morpholin-4-yl-[1,3,5]triazin-2-yl)-hydrazin (40 mg, 0.14 mmol) in EtOH (0.5 ml) bei 0°C gegeben, und die Mischung wurde für 1,25 h gerührt. Zusätzliches 2-Hydroxybenzaldehyd (3 *µ*l) wurde hinzugegeben und das Rühren für 30 min. bei 0°C und bei Raumtemperatur für 18 h fortgesetzt. Schließlich wurde die Mischung auf 50°C für 3 h erwärmt. Nach Abkühlen wurde der resultierende Niederschlag durch Filtration isoliert und mit EtOH gewaschen. Der Rohfeststoff wurde durch Säulenchromatographie mit MeOH/DCM (2 %) als Eluent gereinigt, um die Titelverbindung zu ergeben (25 mg, 46 %).
MW: 385.43
HPLCMS (Methode A):[m/z]: 386
Abb. 109 zeigt das MS-Chromatogramm, das MS-Spektrum und das PDA-Chromatogramm der Verbindung von Beispiel 113.
IC50 [*µ*M]: >50.

### Beispiel 114

### 4-[(4,6-Di-morpholin-4-yl-[1,3,5]triazin-2-yl)-hydrozonomethyl]-benzol-1,3-diol

In ähnlicher Weise ergaben, unter Verwendung von Route 1, allgemeines Verfahren 3, (4,6-Di-morpholin-4-yl-[1,3,5]triazin-2-yl)-hydrazin (40 mg, 0.14 mmol), 2,4-Dihydroxybenzaldehyd (19 mg, 0.14 mmol) und p-Toluolsulfonsäure (2 mg, 0.08 mmol) die Titelverbindung (20 mg, 36 %) nach Reinigung durch Säulenchromatographie mit MeOH/DCM (0 - 3 % Gradient) als Eluent.
MW: 401.43
HPLCMS (Methode A):[m/z]: 402
Abb. 110 zeigt das MS-Chromatogramm, das MS-Spektrum und das PDA-Chromatogramm der Verbindung von Beispiel 114.
IC50 [*µ*M] : >50.

### ROUTE 2

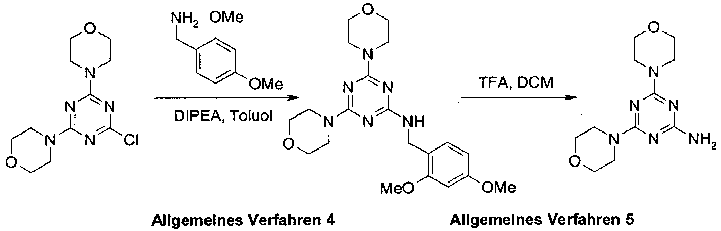

### Allgemeines Verfahren 4:

### (2,4-Dimethoxy-benzyl)-(4,6-di-morpholin-4-yl-[1,3,5]triazin-2-yl)-amin (von der Erfindung umfasst)

2,4-Dimethoxybenzylamin (0.79 ml, 5.25 mmol) wurde zu einer Lösung von 2-Chlor-4,6-di-morpholin-4-yl-[1,3,5]triazin (0.5 g, 1,75 mmol) in Toluol (10 ml), gefolgt von DIPEA (0.61 ml, 3.50 mmol) gegeben, und die Mischung wurde bei 90°C für 18 h erwärmt. Nach Abkühlen wurde die resultierende Suspension über Zelithe filtriert und mit Toluol gewaschen. Das Filtrat wurde im Vakuum eingeengt, und der Rückstand wurde in DCM gelöst. Die organische Phase wurde mit Wasser (x 2) und Kochsalzlösung gewaschen, getrocknet (MgSO₄) und im Vakuum eingeengt. Der Rohrückstand wurde durch Säulenchromatographie mit MeOH/DCM (0-5 % Gradient) als Eluent gereinigt, um die Titelverbindung zu ergeben (0.64 g, 88 %).
MW: 416.48
HPLCMS (Methode B):[m/z]: 417

### Beispiel 115

### Allgemeines Verfahren 5:

### 4,6-Di-morpholin-4-yl-[1,3,5]triazin-2-ylamin

TFA (2.5 ml) wurde zu einer Lösung von (2,4-Dimethoxy-benzyl)-(4,6-di-morpholin-4-yl-[1,3,5]triazin-2-yl)-amin (0.50 g, 1.20 mmol) in DCM (5 ml) gegeben, und die Mischung wurde bei Raumtemperatur für 18 h gerührt. Wasser wurde hinzugegeben und die Mischung wurde für 1 h gerührt. Die Phasen wurden getrennt und die organische Phase getrocknet (MgSO₄) und im Vakuum eingeengt. Der Rückstand wurde in EtOAc und Na₂CO₃ (aq) gelöst und die resultierende Mischung für 30 min. gerührt. Die Phasen wurden getrennt und die wässrige Phase mit EtOAc extrahiert. Die vereinigten organischen Phasen wurden getrocknet (MgSO₄) und im Vakuum eingeengt. Ein Viertel des Rohrückstands (60 mg) wurde durch Säulenchromatographie mit MeOH/DCM als Eluent gereinigt, um die Titelverbindung zu ergeben (54 mg, ∼68 % gesamt).
MW: 266.31
HPLCMS (Methode A):[m/z]: 267
Abb. 111 zeigt das MS-Chromatogramm, das MS-Spektrum und das PDA-Chromatogramm der Verbindung von Beispiel 115.
IC50 [*µ*M]: >50.

### Beispiel 116

### ROUTE 4 (Route 3 siehe Beispiel 12).

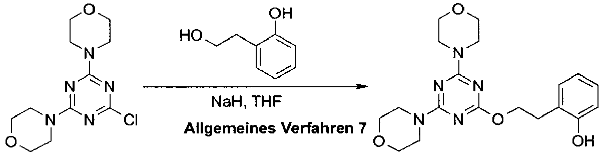

### 2-[2-(4,6-Di-morpholin-4-yl-[1,3,5]triazin-2-yloxy)-ethyl]-phenol

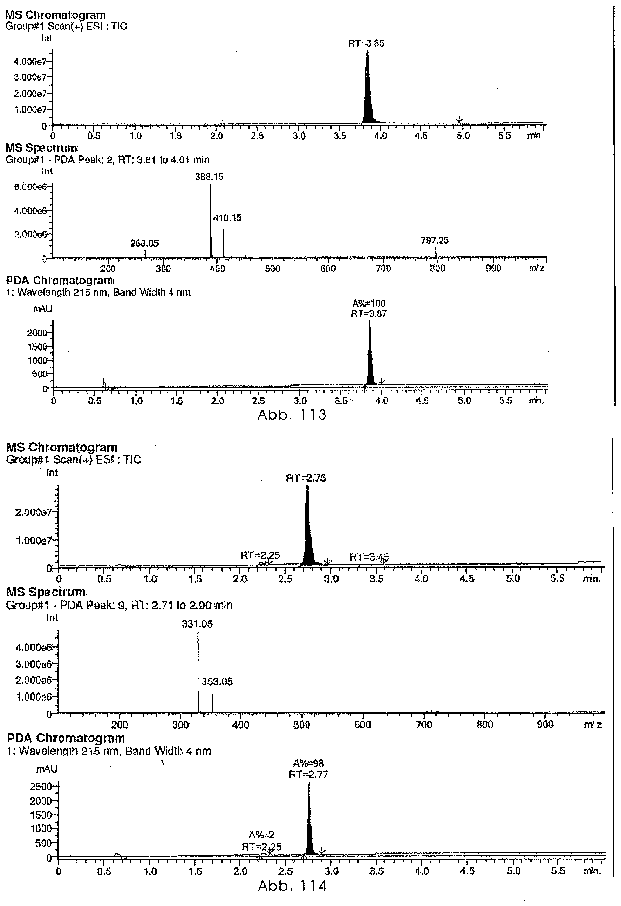

2-(2-Hydroxy-ethyl)-phenol (73 mg, 0.53 mmol) wurde zu einer Lösung von 2-Chlor-4,6-di-morpholin-4-yl-[1,3,5]triazin (151 mg, 0.53 mmol) in THF (3 ml), gefolgt von Natriumhydrid (60 %ige Suspension in Mineralöl; 14 mg, 1.06 mmol) gegeben, und die Mischung wurde bei Raumtemperatur für 1 h gerührt und auf 70°C für 18 h erhitzt. Nach Abkühlen wurde die Mischung zwischen Wasser und EtOAc verteilt und die wässrige Phase mit EtOAc extrahiert. Die vereinigten organischen Phasen wurden getrocknet (MgSO₄) und im Vakuum eingeengt. Der Rohrückstand wurde durch Säulenchromatographie mit EtOAc/Heptan (0-40 % Gradient) als Eluent, gefolgt durch Trituration aus DCM/Heptan gereinigt, um die Titelverbindung zu ergeben (30 mg, 15 %).
MW: 387.44
HPLCMS (Methode A):[m/z]: 388
Abb. 113 zeigt das MS-Chromatogramm, das MS-Spektrum und das PDA-Chromatogramm der Verbindung von Beispiel 116.
IC50 [*µ*M]: >50.

### ROUTE 5

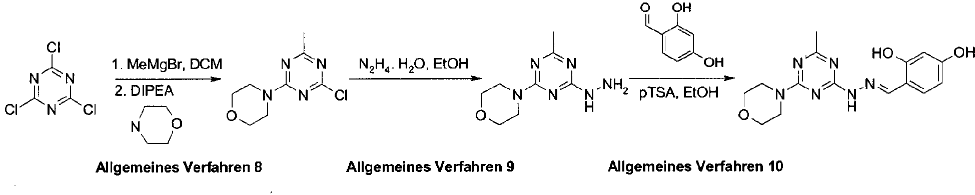

### Allgemeines Verfahren 8:

### 2-Chlor-4-methyl-6-morpholin-4-yl-[1,3,5]triazin

Methylmagnesiumbromid (3M in Ether; 3.4 ml, 10.4 mmol) wurde zu einer Lösung von Cyanurchlorid (2.0 g, 10.9 mmol) in wasserfreiem DCM (40 ml) bei 0°C gegeben. Nach vollständiger Zugabe wurde die Mischung auf Raumtemperatur während 2 h erwärmen gelassen. Die Mischung wurde auf 0°C abgekühlt und Morpholin (0.96 ml, 10.9 mmol) wurde tropfenweise, gefolgt von DIPEA (1.9 ml, 10.9 mmol) zugegeben, und der Ansatz wurde auf Raumtemperatur über 1 h erwärmen gelassen. Wasser wurde hinzugegeben und die resultierende Mischung über Zelithe filtriert. Die organische Phase wurde mit Wasser und Kochsalzlösung gewaschen, getrocknet (MgSO₄) und im Vakuum eingeengt. Der Rohrückstand wurde durch Säulenchromatographie mit MeOH/DCM (0-10 % Gradient) als Eluent gereinigt, um die Titelverbindung zu ergeben (0.54 g, 23 %).
MW: 214.66
HPLCMS (Methode B):[m/z]: 215

### Allgemeines Verfahren 9:

### (4-Methyl-6-morpholin-4-yl-[1,3,5]triazin-2-yl)-hydrazin

Hydrazinhydrat (0.18 ml, 2.35 mmol) wurde zu einer Lösung von 2-Chlor-4-methyl-6-morpholin-4-yl-[1,3,5]triazin (100 mg, 0.47 mmol) in EtOH (1 ml) gegeben, und die Mischung wurde unter Rückfluss für 1.5 h erwärmt. Nach Abkühlen auf 0°C wurde der resultierende Feststoff durch Filtration isoliert und mit EtOH gewaschen, um die Titelverbindung zu ergeben (64 mg, 65 %).
MW: 210.24
HPLCMS (Methode B):[m/z]: 211

### Beispiel 117

### Allgemeines Verfahren 10:

### 4-[(4-Methyl-6-morpholin-4-yl-[1,3,5]triazin-2-yl)-hydrazonomethyl]-benzol-1,3-diol

2,4-Dihydroxybenzaldehyd (40 mg, 0.29 mmol) und p-Toluolsulfonsäure (3.5 mg, 0.02 mmol) wurden zu einer Lösung von (4-Methyl-6-morpholin-4-yl-[1,3,5]triazin-2-yl)-hydrazin (60 mg, 0.29 mmol) in EtOH (1 ml) bei 0°C gegeben, und die Mischung wurde für 1.5 h gerührt. Der resultierende Niederschlag wurde durch Filtration isoliert und mit EtOH gewaschen. Die vereinigte Feststoff und das Filtrat wurden durch Säulenchromatographie mit 2M Ammoniak in MeOH/DCM (0-7 % Gradient) als Eluent, gefolgt durch Trituration aus Isopropylalkohol gereinigt, um die Titelverbindung zu ergeben (13.5 mg, 14 %).
MW: 330.35
HPLCMS (Methode A):[m/z]: 331
Abb. 114 zeigt das MS-Chromatogramm, das MS-Spektrum und das PDA-Chromatogramm der Verbindung von Beispiel 117.
IC50 [*µ*M]: >50.

## Patentansprüche

1. Hepcidin Antagonisten der allgemeinen Formel (I') 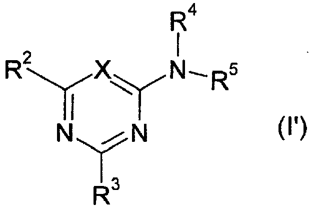 worin
X Die Bedeutung von C-R¹ aufweist, worin
R¹ ausgewählt wird aus der Gruppe, die besteht aus:
- Wasserstoff,
- Halogen,
- gegebenenfalls substituiertem Alkyl,
- gegebenenfalls substituiertem Alkoxy
R² und R³ gleich oder verschieden sind, und jeweils ausgewählt werden aus der Gruppe, die besteht aus:
- Wasserstoff,
- Hydroxyl,
- Halogen,
- Carboxyl,
- Sulfonsäurerest (-SO₃H),
- gegebenenfalls substituiertem Aminocarbonyl,
- gegebenenfalls substituiertem Aminosulfonyl,
- gegebenenfalls substituiertem Amino,
- gegebenenfalls substituiertem Alkyl,
- gegebenenfalls substituiertem Acyl,
- gegebenenfalls substituiertem Alkoxycarbonyl,
- gegebenenfalls substituiertem Acyloxy,
- gegebenenfalls substituiertem Alkoxy,
- gegebenenfalls substituiertem Alkenyl,
- gegebenenfalls substituiertem Alkinyl,
- gegebenenfalls substituiertem Aryl,
- gegebenenfalls substituiertem Heterocyclyl;
R⁴ und R⁵ gleich oder verschieden sind, und jeweils ausgewählt werden aus der Gruppe, die besteht aus:
- Wasserstoff,
- gegebenenfalls substituiertem Amino,
- gegebenenfalls substituiertem Alkyl-, Aryl- oder Heterocyclylsulfonyl,
- gegebenenfalls substituiertem Alkyl,
- gegebenenfalls substituiertem Alkenyl,
- gegebenenfalls substituiertem Alkinyl,
- gegebenenfalls substituiertem Acyl,
- gegebenenfalls substituiertem Aryl,
- gegebenenfalls substituiertem Heterocyclyl oder
- worin R⁴ und R⁵ zusammen mit dem Stickstoffatom, an das sie gebunden sind, einen gesättigten oder ungesättigten, gegebenenfalls substituierten 3- bis 8-gliedrigen Ring ausbilden, der gegebenenfalls weitere Heteroatome enthalten kann;
oder pharmazeutisch verträgliche Salze hiervon,
zur Verwendung in der Behandlung von Eisenmangelerkrankungen und/oder Eisenmangelanämien.

2. Hepcidin Antagonisten zur Verwendung nach Anspruch 1, worin
R² und R³ gleich oder verschieden sind, und jeweils ausgewählt werden aus der Gruppe, die besteht aus:
- Wasserstoff,
- Halogen,
- gegebenenfalls substituiertem Amino,
- gegebenenfalls substituiertem Alkyl,
- gegebenenfalls substituiertem Alkoxy,
- gegebenenfalls substituiertem Aryl,
- gegebenenfalls substituiertem Heterocyclyl;
R⁴ und R⁵ gleich oder verschieden sind, und jeweils ausgewählt werden aus der Gruppe, die besteht aus:
- Wasserstoff,
- gegebenenfalls substituiertem Amino,
- gegebenenfalls substituiertem Alkyl,
- gegebenenfalls substituiertem Aryl,
- gegebenenfalls substituiertem Heterocyclyl oder
- worin R⁴ und R⁵ zusammen mit dem Stickstoffatom, an das sie gebunden, sind einen gesättigten oder ungesättigten, gegebenenfalls substituierten 5- bis 6-gliedrigen Ring ausbilden, der gegebenenfalls weitere Heteroatome enthalten kann;
oder pharmazeutisch verträgliche Salze hiervon.

3. Hepcidin Antagonisten zur Verwendung nach einem oder mehreren der Ansprüche 1 bis 2, worin
R² und R³ gleich oder verschieden sind und ausgewählt werden aus der Gruppe, die besteht aus:
- Wasserstoff,
- gegebenenfalls substituiertem Amino,
- gegebenenfalls substituiertem Alkyl,
- gegebenenfalls substituiertem Heterocyclyl,
R⁴ und R⁵ gleich oder verschieden sind, und jeweils ausgewählt werden aus der Gruppe, die besteht aus:
- Wasserstoff,
- gegebenenfalls substituiertem Amino,
- gegebenenfalls substituiertem Alkyl;
- gegebenenfalls substituiertem Heterocyclyl; oder
R⁴ und R⁵ bilden zusammen mit dem Stickstoffatom, an das sie gebunden sind, einen gesättigten oder ungesättigten, gegebenenfalls substituierten 5- bis 6-gliedrigen Ring aus, der gegebenenfalls ein bis zwei weitere Heteroatome enthalten kann;
oder pharmazeutisch verträgliche Salze hiervon,

4. Hepcidin Antagonisten zur Verwendung nach einem oder mehreren der Ansprüche 1 bis 3 ausgewählt aus : 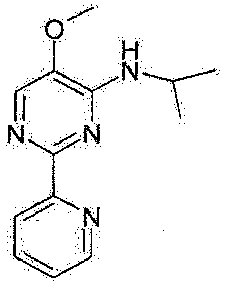 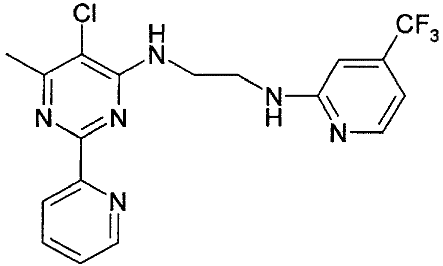 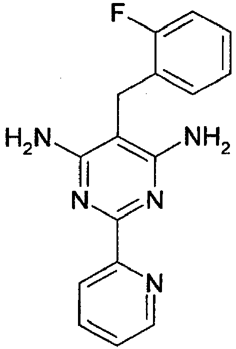 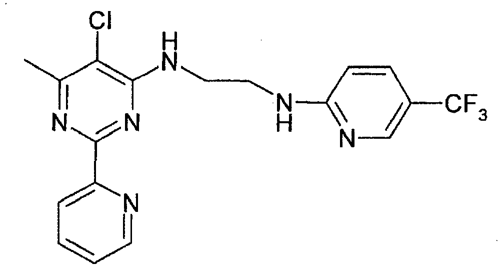 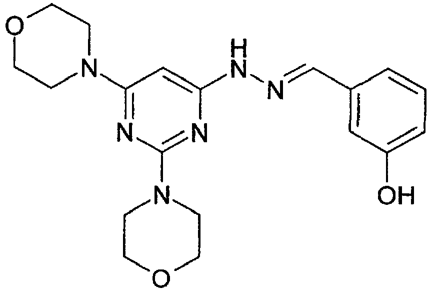 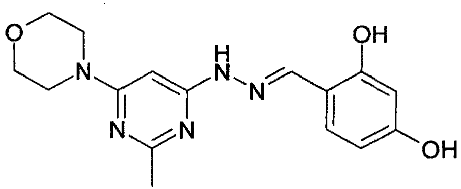 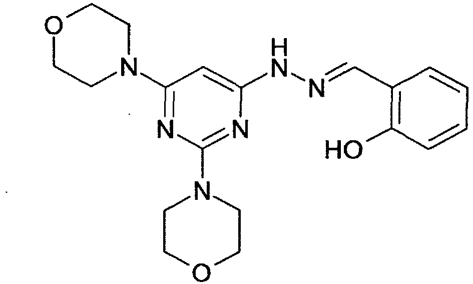 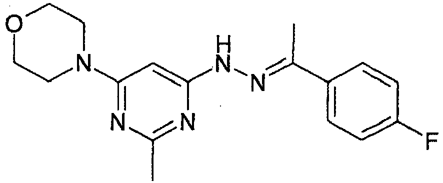 oder pharmazeutisch verträgliche Salze hiervon.

5. Hepcidin Antagonisten zur Verwendung nach einem oder mehreren der Ansprüche 1 bis 4, worin die Eisenmangelerkrankungen und/oder Eisenmangelanämien ausgewählt sind aus: Eisenmangelanämie bei Krebs, Eisenmangelanämie ausgelöst durch Chemotherapie, Eisenmangelanämie ausgelöst durch Inflammation (AI), Eisenmangelanämie bei kongestiver Herzinsuffizienz (CHF;congestive heart failure), Eisenmangelanämie bei chronischer Niereninsuffizienz Stadium 3 -5 (CKD 3-5; chronic kidney diseases stage 3-5), Eisenmangelanämie ausgelöst durch chronische Inflammation (ACD), Eisenmangelanämie bei rheumatischer Arthritis (RA; rheumatoid arthritis), Eisenmangelanämie bei systemischem Lupus erythemotodes (SLE; systemic lupus erythematosus) und Eisenmangelanämie bei inflammatorischen Darmerkrankungen (IBD; inflammatory bowel diseases),

6. Zusammensetzung, enthaltend einen oder mehreren der Hepcidin Antagonisten, wie in einem oder mehreren der Ansprüche 1 bis 5 definiert, sowie einen oder mehrere pharmazeutische Träger und/oder Hilfsstoffe und/oder Lösungsmittel, zur Verwendung in der Behandlung von Eisenmangelerkrankungen und/oder Eisenmangelanämlen.

7. Kombinationspräparat, enthaltend einen oder mehreren der Hepcldin Antagonisten, wie in einem oder mehreren der Ansprüche 1 bis 5 definiert, sowie mindestens eine weitere Verbindung zur Behandlung von Eisenmetabolismus-Störungen sowie der damit einhergehenden Symptome, bevorzugt eine Eisen-haltige Verbindung, zur Verwendung in der Behandlung von Eisenmetabolismus-Störungen,

## Claims

1. Hepcidin antagonists of general formula (I') 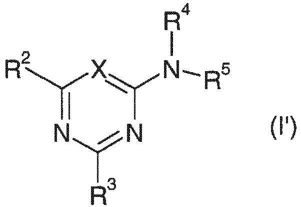 wherein
X has the meaning of C-R¹, wherein
R¹ is selected from the group consisting of:
- hydrogen,
- halogen,
- optionally substituted alkyl,
- optionally substituted alkoxy
R² and R³ are the same or different and are each selected from the group consisting of:
- hydrogen,
- hydroxyl,
- halogen,
- carboxyl,
- sulfonic acid residue (-SO₃H),
- optionally substituted aminocarbonyl,
- optionally substituted aminosulfonyl,
- optionally substituted amino,
- optionally substituted alkyl,
- optionally substituted acyl,
- optionally substituted alkoxycarbonyl,
- optionally substituted acyloxy,
- optionally substituted alkoxy
- optionally substituted alkenyl,
- optionally substituted alkynyl,
- optionally substituted aryl,
- optionally substituted heterocyclyl;
R⁴ and R⁵ are the same or different and are each selected from the group consisting of:
- hydrogen,
- optionally substituted amino,
- optionally substituted alkyl-, aryl- or heterocyclylsulfonyl,
- optionally substituted alkyl,
- optionally substituted alkenyl,
- optionally substituted alkynyl,
- optionally substituted acyl,
- optionally substituted aryl,
- optionally substituted heterocyclyl or
- wherein R⁴ and R⁵, together with the nitrogen atom to which they are bound, form a saturated or unsaturated, optionally substituted 3- to 8-membered ring, which can optionally contain further heteroatoms;
or pharmaceutically acceptable salts thereof,
for the use in the treatment of iron deficiency diseases and/or iron deficiency anemia.

2. Hepcidin antagonists for the use according to claim 1, wherein
R² and R³ are the same or different and are each selected from the group consisting of:
- hydrogen,
- halogen,
- optionally substituted amino,
- optionally substituted alkyl,
- optionally substituted alkoxy,
- optionally substituted aryl,
- optionally substituted heterocyclyl;
R⁴ and R⁵ are the same or different and are each selected from the group consisting of:
- hydrogen,
- optionally substituted amino,
- optionally substituted alkyl,
- optionally substituted aryl,
- optionally substituted heterocyclyl or
- wherein R⁴ and R⁵ together with the nitrogen atom, to which they are bound, form a saturated or unsaturated, optionally substituted 5- to 6-membered ring, which can optionally contain further heteroatoms;
or pharmaceutically acceptable salts thereof.

3. Hepcidin antagonists for the use according to one or more of claims 1 to 2, wherein
R² and R³ are the same or different and are selected from the group consisting of:
- hydrogen,
- optionally substituted amino,
- optionally substituted alkyl,
- optionally substituted heterocyclyl,
R⁴ and R⁵ are the same or different and are each selected from the group consisting of:
- hydrogen,
- optionally substituted amino,
- optionally substituted alkyl;
- optionally substituted heterocyclyl; or
R⁴ and R⁵ together with the nitrogen atom, to which they are bound, form a saturated or unsaturated, optionally substituted 5- to 6-membered ring, which can optionally contain one to two further heteroatoms;
or pharmaceutically acceptable salts thereof.

4. Hepcidin antagonists for the use according to one or more of claims 1 to 3, selected from: 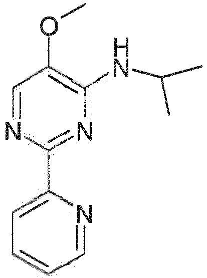 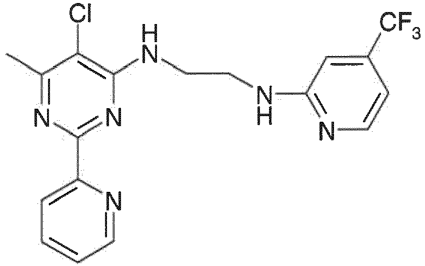 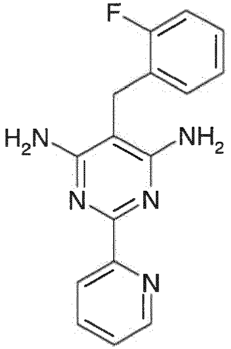 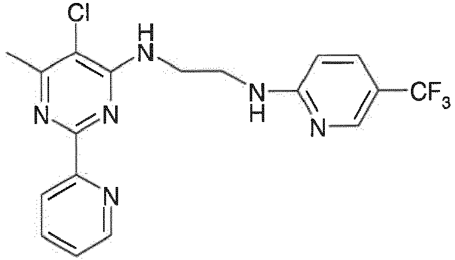 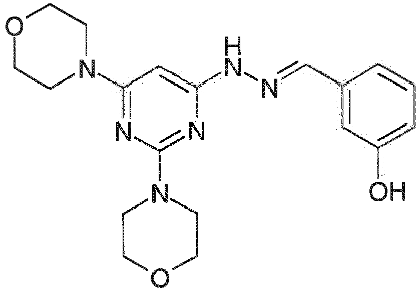 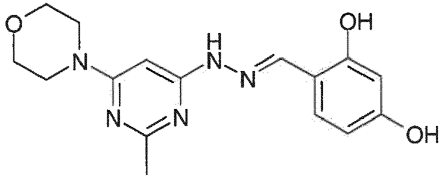 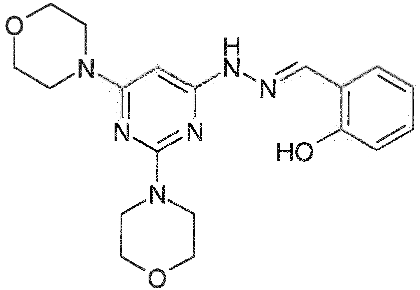 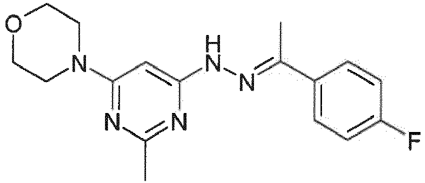 or pharmaceutically acceptable salts thereof.

5. Hepcidin antagonists for the use according to one or more of claims 1 to 4, wherein the iron deficiency diseases and/or iron deficiency anaemia are selected from: iron deficiency anaemia in cancer, iron deficiency anaemia triggered by chemotherapy, iron deficiency anaemia triggered by inflammation (AI), iron deficiency anaemia in congestive heart failure (CHF), iron deficiency anaemia in chronic kidney disease stage 3-5 (CKD 3-5), iron deficiency anaemia trigged by chronic inflammation (ACD), iron deficiency anaemia in rheumatoid arthritis (RA), iron deficiency anaemia in systemic lupus erythematosus (SLE) and iron deficiency anaemia in inflammatory bowel disease (IBD).

6. Composition containing one or more of the hepcidin antagonists as defined in one or more of claims 1 to 5 and one or more pharmaceutical carriers and/or auxiliaries and/or solvents, for the use in the treatment of iron deficiency diseases and/or iron deficiency anaemia.

7. Combined preparation containing one or more of the hepcidin antagonists as defined in one or more of claims 1 to 5 and at least one further compound for the treatment of iron metabolism disorders and the associated symptoms, preferably an ironcontaining compound, for the use in the treatment of iron metabolism disorders.

## Revendications

1. Antagonistes de l'hepcidine de formule générale (I') 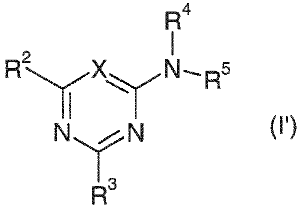 dans laquelle
X a la signification C-R¹, où
R¹ est choisi parmi le groupe consistant en:
- hydrogène,
- halogène,
- alkyle éventuellement substitué,
- alkoxy éventuellement substitué,
R² et R³ sont identiques ou différents et sont chacun choi- sis parmi le groupe consistant en:
- hydrogène,
- hydroxyle,
- halogène,
- carboxyle,
- résidu d'acide sulfonique (-SO₃H),
- aminocarbonyle éventuellement substitué,
- aminosulfonyle éventuellement substitué,
- amino éventuellement substitué,
- alkyle éventuellement substitué,
- acyle éventuellement substitué,
- alkoxycarbonyle éventuellement substitué,
- acyloxy éventuellement substitué,
- alkoxy éventuellement substitué,
- alkényle éventuellement substitué,
- alkynyle éventuellement substitué,
- aryle éventuellement substitué,
- hétérocyclyle éventuellement substitué;
R⁴ et R⁵ sont identiques ou différents et sont chacun choisis parmi le groupe consistant en:
- hydrogène,
- amino éventuellement substitué,
- alkyl- aryl- ou hétérocyclylsulfonyle, éventuellement substitué,
- alkyle éventuellement substitué,
- alkényle éventuellement substitué,
- alkynyle éventuellement substitué,
- acyle éventuellement substitué,
- aryle éventuellement substitué,
- hétérocyclyle éventuellement substitué ou bien
- où R⁴ et R⁵, conjointement avec l'atome d'azote auquel ils sont liés, forment un cycle de 3 à 8 chaînons éventuellement substitué, saturé ou non saturé, qui peut éventuellement contenir d'autres hétéroatomes;
ou leurs sels pharmaceutiquement acceptables,
pour l'utilisation dans le traitement des maladies dues à la carence en fer et/ou l'anémie ferriprive.

2. Antagonistes de l'hepcidine pour l'utilisation selon la revendication 1, dans laquelle
R² et R³ sont identiques ou différents et sont chacun choisis parmi le groupe consistant en:
- hydrogène,
- halogène,
- amino éventuellement substitué,
- alkyle éventuellement substitué,
- alkoxy éventuellement substitué,
- aryle éventuellement substitué,
- hétérocyclyle éventuellement substitué;
R⁴ et R⁵ sont identiques ou différents et sont chacun choisis parmi le groupe consistant en:
- hydrogène,
- amino éventuellement substitué,
- alkyle éventuellement substitué,
- aryle éventuellement substitué,
- hétérocyclyle éventuellement substitué ou bien
- où R⁴ et R⁵ conjointement avec l'atome d'azote auquel ils sont liés, forment un cycle de 5 à 6 chaînons éventuellement substitué, saturé ou non saturé, qui peut éventuellement contenir d'autres hétéroatomes;
ou leurs sels pharmaceutiquement acceptables.

3. Antagonistes de l'hepcidine pour l'utilisation selon l'une ou plu sieurs des revendications 1 ou 2 dans lesquels
R² et R³ sont identiques ou différents et sont choisis parmi le groupe consistant en:
- hydrogène,
- amino éventuellement substitué,
- alkyle éventuellement substitué,
- hétérocyclyle éventuellement substitué;
R⁴ et R⁵ sont identiques ou différents et sont chacun choisis parmi le groupe consistant en:
- hydrogène,
- amino éventuellement substitué,
- alkyle éventuellement substitué,
- hétérocyclyle éventuellement substitué ou bien
- où R⁴ et R⁵ conjointement avec l'atome d'azote auquel ils sont liés, forment un cycle de 5 à 6 chaînons éventuellement substitué, saturé ou non saturé, qui peut éventuellement contenir un à deux autres hétéro- atomes;
ou leurs sels pharmaceutiquement acceptables.

4. Antagonistes de l'hepcidine pour l'utilisation selon l'une ou plu sieurs des revendications 1 à 3, choisis parmi: 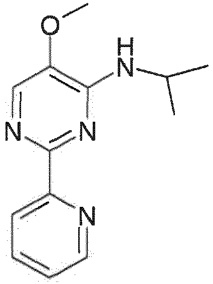 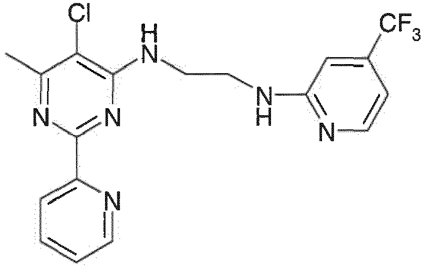 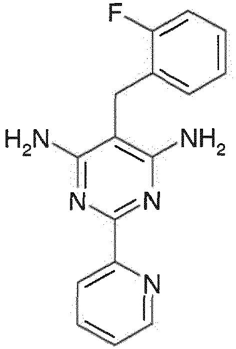 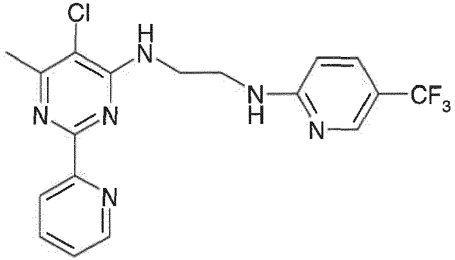 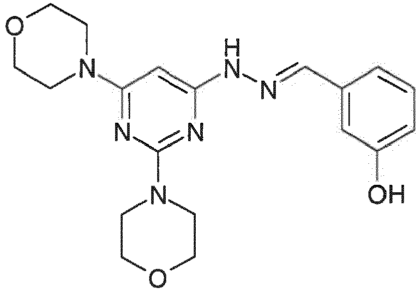 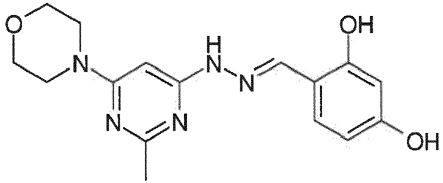 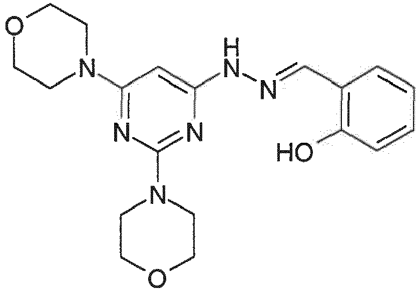 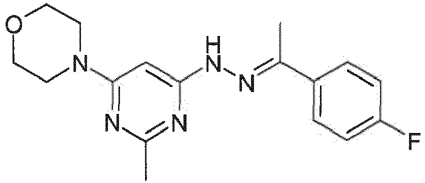 ou leurs sels pharmaceutiquement acceptables.

5. Antagonistes de l'hepcidine pour l'utilisation selon l'une ou plusieurs des revendications 1 à 4, où les maladies dues à la carence en fer et/ou l'anémie ferriprive sont choisis parmi: l'anémie ferriprive dans le cancer, l'anémie ferriprive provoquée par la chimiothérapie, l'anémie ferriprive provoquée par une inflammation (AI), l'anémie ferriprive dans l'insuffisance cardiaque congestive (ICC), l'anémie ferriprive dans la maladie rénale chronique au stade 3-5 (CKD 3-5), l'anémie ferriprive provoquée par une inflammation chronique (AMC), l'anémie ferriprive dans l'arthrite rhumatoïde (AR), l'anémie ferriprive dans le lupus érythémateux disséminé (LED) et l'anémie ferriprive dans la maladie inflammatoire de l'intestin (MII).

6. Composition contenant un ou plusieurs des antagonistes de l'hepcidine comme définis dans l'une ou plusieurs des revendications 1 à 5 et un ou plusieurs supports et/ou auxiliaires et/ou solvants pharmaceutiques pour l'utilisation dans le traitement des maladies dues à la carence en fer et/ou l'anémie ferriprive.

7. Préparation combinée contenant un ou plusieurs des antagonistes de l'hepcidine comme définis dans l'une ou plusieurs des revendications 1 à 5 et au moins un autre composé pour le traitement des troubles du métabolisme du fer et les symptômes associés, de préférence un composé contenant du fer, pour l'utilisation dans le traitement des troubles du métabolisme du fer.
